# EUROPÄISCHE PATENTSCHRIFT

(11) **EP 3 484 868 B1**
(45) Veröffentlichungstag und Bekanntmachung des Hinweises auf die Patenterteilung: **25.11.2020**
(21) Anmeldenummer: 17737282.8
(22) Anmeldetag: 11.07.2017
(51) Int. Cl.: C07F 15/00, H01L 51/00, C07D 401/14, C07D 215/30, H01L 51/50, C07D 213/22

(54) **METALLKOMPLEXE**
METAL COMPLEXES
COMPLEXES MÉTALLIQUES

(30) Priorität: 14.07.2016 EP 16179378
(43) Veröffentlichungstag der Anmeldung: 22.05.2019
(73) Patentinhaber: Merck Patent GmbH, 64293 Darmstadt (DE)
(72) Erfinder: STOESSEL, Philipp, 60389 FRANKFURT AM MAIN (DE); EHRENREICH, Christian, 64285 DARMSTADT (DE)
(86) Internationale Anmeldenummer: PCT/EP2017/067359
(87) Internationale Veröffentlichungsnummer: WO 2018/011186

(56) Entgegenhaltungen:
- WO-A1-2016/124304
- WO-A1-2017/032439
- US-A1- 2005 170 207

## Beschreibung

Die vorliegende Erfindung betrifft Metallkomplexe, welche sich für den Einsatz als Emitter in organischen Elektrolumineszenzvorrichtungen eignen.

Gemäß dem Stand der Technik werden in phosphoreszierenden organischen Elektrolumineszenzvorrichtungen (OLEDs) als Triplettemitter vor allem Iridiumkomplexe eingesetzt, insbesondere bis- und tris-orthometallierte Komplexe mit aromatischen Liganden, wobei die Liganden über ein negativ geladenes Kohlenstoffatom und ein neutrales Stickstoffatom oder über ein negativ geladenes Kohlenstoffatom und ein neutrales Carben-Kohlenstoffatom an das Metall binden. Beispiele für solche Komplexe sind Tris(phenylpyridyl)iridium(III) und Derivate davon, wie beispielsweise Komplexe mit 1- oder 3-Phenylisochinolinliganden, mit 2-Phenylchinolinen oder mit Phenylcarbenen.

Eine Verbesserung der Stabilität der Komplexe konnte durch die Verwendung polypodaler Liganden erreicht werden, wie beispielsweise in WO 2004/081017 oder US 7,332,232 beschrieben. Auch wenn diese Komplexe mit polypodalen Liganden Vorteile gegenüber den Komplexen zeigen, die ansonsten die gleiche Ligandenstruktur aufweisen, deren einzelne Liganden jedoch nicht polypodal verbrückt sind, gibt es jedoch noch Verbesserungsbedarf. Dieser liegt insbesondere in der aufwändigeren Synthese der Verbindungen, so dass beispielsweise die Komplexierungsreaktion sehr lange Reaktionszeiten und hohe Reaktionstemperaturen erfordert. Weiterhin sind auch bei den Komplexen mit polypodalen Liganden noch Verbesserungen in Bezug auf die Eigenschaften bei Verwendung in einer organischen Elektrolumineszenzvorrichtung, insbesondere in Bezug auf Effizienz, Spannung und/oder Lebensdauer, wünschenswert.

Aus US 2005/0170207 sind polypodale Metallkomplexe für die Verwendung in OLEDs bekannt, wobei als Brückenkopf unter anderem eine Struktur der Formel A-B²(A)-A offenbart wird. Dabei steht B² für N, C, Alkyl, Aryl, Heteroaryl, Cycloalkyl oder eine heterocyclische Gruppe, und A steht für -(CR₂)_{f}- oder -Z-(CR₂)_{g}, wobei Z für O, NR oder SiR₂ steht. Aufgabe der vorliegenden Erfindung ist daher die Bereitstellung neuer Metallkomplexe, welche sich als Emitter für die Verwendung in OLEDs eignen. Insbesondere ist die Aufgabe, Emitter bereitzustellen, welche verbesserte Eigenschaften in Bezug auf Effizienz, Betriebsspannung und/oder Lebensdauer zeigen. Weiterhin ist es die Aufgabe der vorliegenden Erfindung, Metallkomplexe bereitzustellen, deren Synthese bei milderen Synthesebedingungen, insbesondere in Bezug auf Reaktionsdauer und Reaktionstemperatur, durchgeführt werden kann, jeweils verglichen mit Komplexen, die strukturell vergleichbare Liganden aufweisen. Weiterhin ist es die Aufgabe der vorliegenden Erfindung, Metallkomplexe bereitzustellen, die keine facial-meridional-Isomerisierung zeigen, was bei Komplexen gemäß dem Stand der Technik ein Problem darstellen kann.

Überraschend wurde gefunden, dass Metallkomplexe mit einem hexadentaten tripodalen Liganden, wobei die Brücke des Liganden, die die einzelnen Teilliganden verknüpft, die nachfolgend beschriebene Struktur aufweist, diese Aufgabe lösen und sich sehr gut für die Verwendung in einer organische Elektrolumineszenzvorrichtung eignen. Diese Metallkomplexe und organische Elektrolumineszenzvorrichtungen, welche diese Komplexe enthalten, sind daher der Gegenstand der vorliegenden Erfindung.

Gegenstand der Erfindung ist somit ein monometallischer Metallkomplex enthaltend einen hexadentaten tripodalen Liganden, in dem drei bidentate Teilliganden, die gleich oder verschieden sein können, an ein Metall koordinieren und die drei bidentaten Teilliganden über eine Brücke der folgenden Formel (1) verknüpft sind: 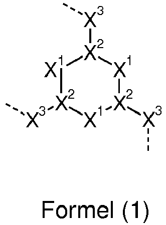 wobei die gestrichelte Bindung die Bindung der bidentaten Teilliganden an diese Struktur darstellt und für die verwendeten Symbole gilt:
- X¹: ist bei jedem Auftreten gleich oder verschieden CR₂ oder O;
- X²: ist bei jedem Auftreten gleich oder verschieden CR, P=O, B oder Si, welches optional substituiert ist, mit der Maßgabe, dass für X² gleich P=O, B oder Si, welches optional substituiert ist, X¹ für O steht; dabei können die optional vorhandenen Substituenten an X¹ und X² jeweils und auch miteinander ein aliphatisches oder heteroaliphatisches Ringsystem bilden;
- X³: ist bei jedem Auftreten gleich oder verschieden -CR=CR-, -CR=N-, -C(=O)-O-, -C(=O)-NR"-,-C(=O)-S-, -C(=S)-O-, -C(=S)-NR"-, -C(=S)-S-;
- R: ist bei jedem Auftreten gleich oder verschieden H, D, F, Cl, Br, I, N(R¹)₂, CN, NO₂, OR¹, SR¹, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei oder mehr Reste R, die an X¹ und/oder X² binden, auch miteinander ein aliphatisches oder heteroaliphatisches Ringsystem bilden; weiterhin können zwei Reste R für X³ = -CR=CR- auch miteinander ein aliphatisches, heteroaliphatisches, aromatisches oder heteroaromatisches Ringsystem bilden;
- R": ist bei jedem Auftreten gleich oder verschieden H, D, eine geradkettige Alkylgruppe mit 1 bis 20 C-Atomen oder eine verzweigte oder cyclische Alkylgruppe mit 3 bis 20 C-Atomen oder eine Alkenylgruppe mit 2 bis 20 C-Atomen, wobei die Alkylgruppe bzw. Alkenylgruppe jeweils mit einem oder mehreren Resten R¹ substituiert sein kann und wobei eine oder mehrere nicht benachbarte CH₂-Gruppen durch Si(R¹)₂ ersetzt sein können, oder ein aromatisches oder heteroaromatisches Ringsystem mit 5 bis 40 aromatischen Ringatomen, das jeweils durch einen oder mehrere Reste R¹ substituiert sein kann;
- R¹: ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch R²C=CR², C=C, Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können mehrere Substituenten R¹ auch miteinander ein aliphatisches, heteroaliphatisches, aromatisches oder heteroaromatisches Ringsystem bilden;
- R²: ist bei jedem Auftreten gleich oder verschieden H, D, F oder ein aliphatischer, aromatischer und/oder heteroaromatischer organischer Rest, insbesondere ein Kohlenwasserstoffrest, mit 1 bis 20 C-Atomen, in dem auch ein oder mehrere H-Atome durch F ersetzt sein können;
dabei können die drei bidentaten Liganden außer durch die Brücke der Formel (1) auch noch durch eine weitere Brücke zu einem Kryptat geschlossen sein.

Wenn X¹ für C steht, dann trägt dieses C-Atom entweder ein Wasserstoffatom, und es ist substituiert mit einem Substituenten ungleich Wasserstoff, oder es trägt zwei Wasserstoffatome oder zwei Substituenten ungleich Wasserstoff.

Bei dem Liganden handelt es sich erfindungsgemäß somit um einen hexadentaten, tripodalen Liganden mit drei bidentaten Teilliganden. Die Struktur des hexadentaten, tripodalen Liganden wird schematisch durch die folgende Formel (Lig) dargestellt: 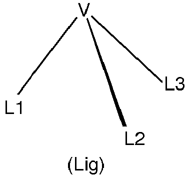 wobei V die Brücke gemäß Formel (1) darstellt und L1, L2 und L3 gleich oder verschieden bei jedem Auftreten jeweils bidentate Teilliganden darstellen. Dabei bedeutet bidentat, dass der jeweilige Teilligand im Komplex über zwei Koordinationsstellen an das Metall koordiniert bzw. bindet. Tripodal bedeutet, dass der Ligand drei Teilliganden aufweist, die an die Brücke V bzw. die Brücke der Formel (1) gebunden sind. Da der Ligand drei bidentate Teilliganden aufweist, ergibt sich insgesamt ein hexadentater Ligand, also ein Ligand, der über sechs Koordinationsstellen an das Metall koordiniert bzw. bindet. Der Begriff "bidentater Teilligand" bedeutet im Sinne dieser Anmeldung, dass es sich bei dieser Einheit um einen bidentaten Liganden handeln würde, wenn die Brücke der Formel (1) nicht vorhanden wäre. Durch die formale Abstraktion eines Wasserstoffatoms an diesem bidentaten Liganden und die Anknüpfung an die Brücke der Formel (1) ist dieser jedoch kein separater Ligand mehr, sondern ein Teil des so entstehenden hexadentaten Liganden, so dass hierfür der Begriff "Teilligand" verwendet wird.

Der mit diesem Liganden der Formel (Lig) gebildete Metallkomplex M(Lig) kann somit schematisch durch die folgende Formel dargestellt werden: 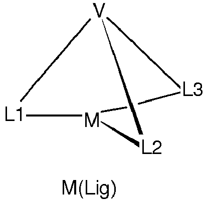 wobei V die Brücke gemäß Formel (1) darstellt, L1, L2 und L3 gleich oder verschieden bei jedem Auftreten jeweils bidentate Teilliganden darstellen und M für ein Metall steht. Wie man der Schemazeichnung entnehmen kann, koordinieren alle drei bidentaten Teilliganden über jeweils beide Koordinationsstellen in den erfindungsgemäßen Verbindungen an das Metall.

Monometallisch im Sinne der vorliegenden Erfindung bedeutet, dass der Metallkomplex nur ein einziges Metallatom enthält, wie auch durch M(Lig) schematisch dargestellt. Metallkomplexe, in denen beispielsweise jeder der drei bidentaten Teilliganden an ein anderes Metallatom koordiniert ist, sind somit nicht von der Erfindung umfasst.

Die Bindung des Liganden an das Metall kann sowohl eine Koordinationsbindung als auch eine kovalente Bindung sein bzw. der kovalente Anteil an der Bindung kann je nach Ligand und Metall variieren. Wenn in der vorliegenden Anmeldung die Rede davon ist, dass der Ligand bzw. der Teilligand an das Metall koordiniert oder bindet, so bezeichnet dies im Sinne der vorliegenden Anmeldung jede Art der Bindung des Liganden bzw. Teilliganden an das Metall, unabhängig vom kovalenten Anteil der Bindung.

Bevorzugt sind die erfindungsgemäßen Verbindungen dadurch gekennzeichnet, dass diese nicht geladen, d. h. elektrisch neutral, sind. Dies wird auf einfache Weise dadurch erreicht, dass die Ladungen der drei bidentaten Teilliganden und der Brücke der Formel (1) so gewählt werden, dass sie die Ladung des komplexierten Metallatoms kompensieren. Wenn also beispielsweise ein Metallatom in der Oxidationsstufe +3 verwendet wird, kann Ladungsneutralität erreicht werden, indem jeder der drei bidentaten Teilliganden monoanionisch ist.

Im Folgenden werden bevorzugte Ausführungsformen der Brücke der Formel (1) ausgeführt. Die Gruppe X³ kann eine Alkenylgruppe, eine Imingruppe, eine Amidgruppe, eine Estergruppe oder die entsprechenden Schwefelanaloga von Amid- oder Estergruppen darstellen. Die Gruppe X³ kann, wenn X³ für-CR=CR- steht und die Reste R miteinander ein aromatisches oder heteroaromatisches Ringsystem bilden, auch für eine ortho-verknüpfte Arylen- oder Heteroarylengruppe stehen. Bei unsymmetrischen Gruppen X³ ist jede Orientierung der Gruppen möglich. Dies ist nachfolgend schematisch am Beispiel von X³ = -C(=O)-O-erläutert. Hieraus ergeben sich die folgenden möglichen Orientierungen von X³, die alle von der vorliegenden Erfindung umfasst sind:

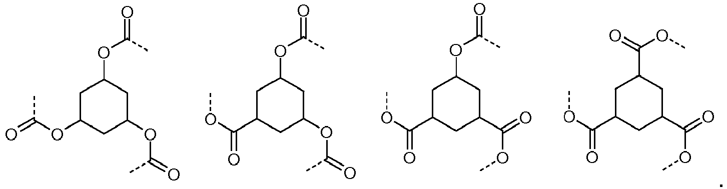

Wenn X² für CR steht, insbesondere wenn alle X² für CR stehen, ganz besonders wenn zusätzlich 0, 1, 2 oder 3, insbesondere 3, der X¹ für CR₂ stehen, können die Reste R an X² abhängig von der Konfiguration unterschiedliche Positionen einnehmen. Bevorzugt sind dabei kleine Reste R wie H oder D. Bevorzugt ist, dass sie entweder alle weg vom Metall gerichtet sind (apical) oder alle nach innen zum Metall hin gerichtet sind (endohedral). Dies ist nachfolgend am Beispiel eines Komplexes mit Esterbrücken veranschaulicht. Es gilt in gleicher Weise für ortho-Arylen-, ortho-Heteroarylen-, 1,2-Olefin-, Imin- und Amidbrücken und zwar unabhängig davon, wie die Brücke orientiert ist, d.h. ob die Carbonyl-Gruppe der Ester-/Amid-Brücke bzw. das N-Atom der Iminbrücke an den Cyclohexanring bindet oder an den Aromaten des zweizähnigen Teilliganden.

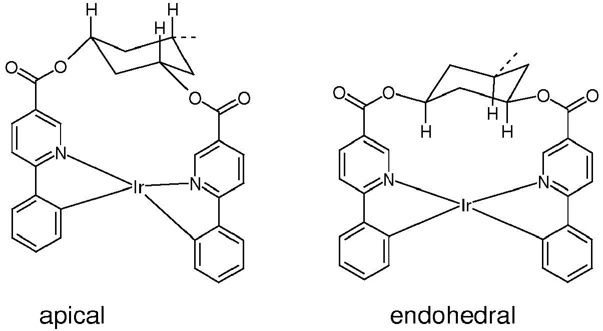

Der dritte Teilligand ist der Übersichtlichkeit halber nicht dargestellt, sondern nur durch die gestrichelte Bindung angedeutet. Bevorzugt sind daher Komplexe, welche mindestens eine der beiden Konfigurationen einnehmen können. Dies sind Komplexe, bei welchen alle drei Gruppen X³ äquatorial am zentralen Ring angeordnet sind.

Wenn X³ für eine Alkenylgruppe bzw. eine Imingruppe stehen, dann handelt es sich um cis-verknüpfte Alkenyl- bzw. Imingruppen.

Wenn X³ für -CR=CR- steht, dann stellt die Gruppe X³ eine Alkengruppe bzw. bei Ringschluss der optional vorhandenen Substituenten auch eine Arylen- bzw. Heteroarylengruppe dar.

Wenn X³ für -C(=O)-NR"- steht, dann steht R" bevorzugt gleich oder verschieden bei jedem Auftreten für eine geradkettige Alkylgruppe mit 1 bis 10 C-Atomen oder eine verzweigte oder cyclische Alkylgruppe mit 3 bis 10 C-Atomen oder ein aromatisches oder heteroaromatisches Ringsystem mit 6 bis 24 aromatischen Ringatomen, das jeweils durch einen oder mehrere Reste R¹ substituiert sein kann. Besonders bevorzugt steht R" gleich oder verschieden bei jedem Auftreten für eine geradkettige Alkylgruppe mit 1 bis 5 C-Atomen oder eine verzweigte oder cyclische Alkylgruppe mit 3 bis 6 C-Atomen oder ein aromatisches oder heteroaromatisches Ringsystem mit 6 bis 12 aromatischen Ringatomen, das jeweils durch einen oder mehrere Reste R¹ substituiert sein kann, bevorzugt aber unsubstituiert ist.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

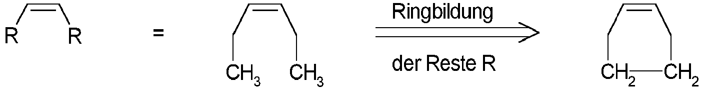

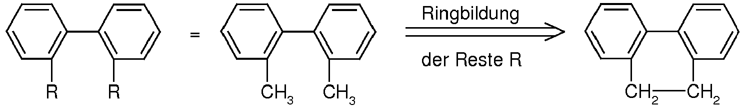

Entsprechend ist auch die Bildung von bicyclischen, tricyclischen und oligocyclischen Ringsystemen möglich. Weiterhin soll unter der oben genannten Formulierung auch verstanden werden, dass für den Fall, dass einer der beiden Reste Wasserstoff darstellt, der zweite Rest unter Bildung eines Rings an die Position, an die das Wasserstoffatom gebunden war, bindet. Dies wird durch das folgende Schema verdeutlicht:

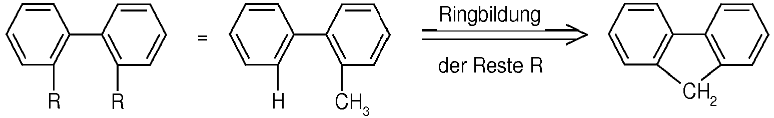

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1 -yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Geeignete Ausführungsformen der Gruppe der Formel (1) sind die Strukturen der folgenden Formeln (2) bis (6), 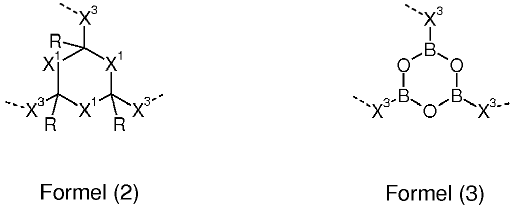 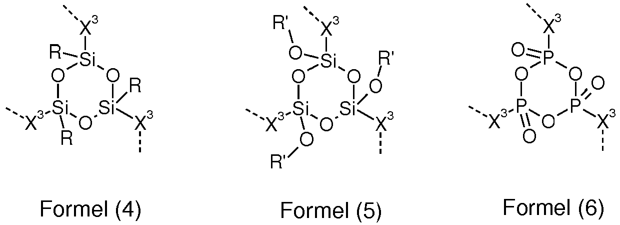 wobei die verwendeten Symbole die oben genannten Bedeutungen aufweisen, wobei zusätzlich gilt:
- R': ist bei jedem Auftreten gleich oder verschieden H, D, F, Cl, Br, I, N(R¹)₂, CN, NO₂, OR¹, SR¹, COOH, C(=O)N(R¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann.

Bevorzugte Ausführungsformen der Gruppen der Formel (2) sind die Formeln (2a) oder (2b), 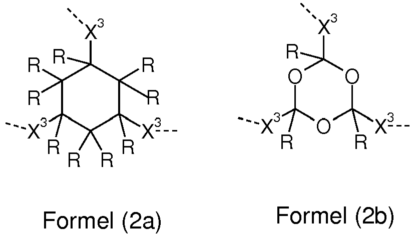 wobei die verwendeten Symbole die zuvor genannten Bedeutungen aufweisen.

In einer bevorzugten Ausführungsform der Erfindung stehen alle Gruppen X¹ und X² in der Gruppe der Formel (1) für ein optional substituiertes Kohlenstoffatom, wobei der Substituent bevorzugt ausgewählt ist aus den oben genannten Gruppen R, so dass der zentrale trivalente Cyclus der Formel (1) ein Cyclohexan darstellt. Bevorzugte Ausführungsform der Formel (1) ist somit die Struktur der Formel (2a).

Besonders bevorzugt stehen alle R der Gruppen X¹ und X² für bei jedem Auftreten gleich oder verschieden für H oder D, insbesondere für H.

Weitere bevorzugte Ausführungsformen der Formel (2a) sind die folgenden Formeln (2a-1) bis (2a-4): 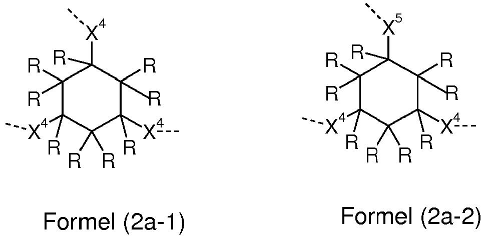 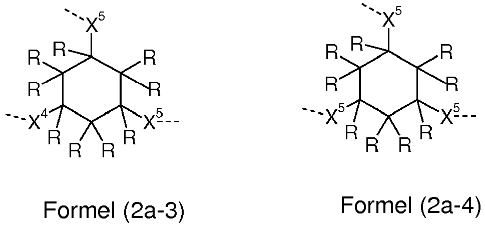 wobei zusätzlich gilt:
- X⁴: ist bei jedem Auftreten gleich oder verschieden -CR=CR-, -CR=N-, -C(=O)-O-, -C(=O)-NR"-, -C(=O)-S-, -C(=S)-O-, -C(=S)-NR"-,-C(=S)-S-;
- X⁵: ist bei jedem Auftreten gleich oder verschieden -CR=CR-, -CR=N-, -C(=O)-O-, -C(=O)-NR"-, -C(=O)-S-, -C(=S)-O-, -C(=S)-NR"-, -C(=S)-S-;
- R: ist bei jedem Auftreten gleich oder verschieden H, D, F, Cl, Br, I, N(R¹)₂, CN, NO₂, OR¹, SR¹, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei oder mehr Reste R, die an den zentralen Cyclohexanring gebunden sind, auch miteinander ein aliphatisches oder heteroaliphatisches Ringsystem bilden; weiterhin können zwei Reste R für X⁴ = -CR=CR- auch miteinander ein aliphatisches, heteroaliphatisches, aromatisches oder heteroaromatisches Ringsystem, bevorzugt ein aromatisches oder heteroaromatisches Ringsystem, bilden; weiterhin bilden zwei Reste R für X⁵ = -CR=CR-auch miteinander ein aliphatisches, heteroaliphatisches, aromatisches oder heteroaromatisches Ringsystem.

Für Reste R insbesondere am zentralen Cyclohexanring der Formel (2) oder den bevorzugten Ausführungsformen gilt bevorzugt:
- R: ist bei jedem Auftreten gleich oder verschieden H, D, F, CN, OR¹, eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, die jeweils mit einem oder mehreren Resten R¹ substituiert sein kann, oder ein aromatisches oder heteroaromatisches Ringsystem mit 5 bis 24 aromatischen Ringatomen, das jeweils durch einen oder mehrere Reste R¹ substituiert sein kann;
- R¹: ist bei jedem Auftreten gleich oder verschieden H, D, F, CN, OR², eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, die jeweils mit einem oder mehreren Resten R² substituiert sein kann, oder ein aromatisches oder heteroaromatisches Ringsystem mit 5 bis 24 aromatischen Ringatomen, das jeweils durch einen oder mehrere Reste R² substituiert sein kann;
- R²: ist bei jedem Auftreten gleich oder verschieden H, D, F oder ein aliphatischer, aromatischer und/oder heteroaromatischer organischer Rest mit 1 bis 20 C-Atomen, in dem auch ein oder mehrere H-Atome durch F ersetzt sein können.

Für Reste R insbesondere am trivalenten zentralen Cyclohexanring der Formel (2) oder den bevorzugten Ausführungsformen gilt besonders bevorzugt:
- R: ist bei jedem Auftreten gleich oder verschieden H, D, F, CN, eine geradkettige Alkylgruppe mit 1 bis 4 C-Atomen oder eine verzweigte oder cyclische Alkylgruppe mit 3 bis 6 C-Atomen, die jeweils mit einem oder mehreren Resten R¹ substituiert sein kann, oder ein aromatisches oder heteroaromatisches Ringsystem mit 6 bis 12 aromatischen Ringatomen, das jeweils durch einen oder mehrere Reste R¹ substituiert sein kann;
- R¹: ist bei jedem Auftreten gleich oder verschieden H, D, F, CN, eine geradkettige Alkylgruppe mit 1 bis 4 C-Atomen oder eine verzweigte oder cyclische Alkylgruppe mit 3 bis 6 C-Atomen, die jeweils mit einem oder mehreren Resten R² substituiert sein kann, oder ein aromatisches oder heteroaromatisches Ringsystem mit 6 bis 12 aromatischen Ringatomen, das jeweils durch einen oder mehrere Reste R² substituiert sein kann;
- R²: ist bei jedem Auftreten gleich oder verschieden H, D, F oder ein aliphatischer oder aromatischer Kohlenwasserstoffrest mit 1 bis 12 C-Atomen.

Im Folgenden werden bevorzugte bivalente Gruppen X³ bzw. die bevorzugten Ausführungsformen X⁴ oder X⁵, beschrieben, wie sie in den Strukturen der Formeln (1) bis (6) oder ihren bevorzugten Ausführungsformen vorkommen.

In einer bevorzugten Ausführungsform der Erfindung steht das Symbol X³ gleich oder verschieden bei jedem Auftreten für -CR=CR-, -CR=N- -C(=O)-O- oder -C(=O)-NR"-. Bevorzugte Kombinationen für X³ sind:

| X³ | X³ | X³ |
|---|---|---|
| -CR=CR- | -CR=CR- | -CR=CR- |
| -C(=O)-O- | -C(=O)-O- | -C(=O)-O- |
| -C(=O)-O- | -C(=O)-O- | -CR=CR- |
| -C(=O)-O- | -CR=CR- | -CR=CR- |
| -C(=O)-NR"- | -C(=O)-NR"- | -C(=O)-NR"- |
| -C(=O)-NR"- | -C(=O)-NR"- | -CR=CR- |
| -C(=O)-NR"- | -CR=CR- | -CR=CR- |
| -CR=N- | -CR=N- | -CR=N- |
| -CR=CR- | -CR=CR- | -CR=N- |
| -CR=CR- | -CR=N- | -CR=N- |

Die Gruppe der Formel (1) kann bevorzugt durch die folgenden Formeln (1a) bis (1q) dargestellt werden: 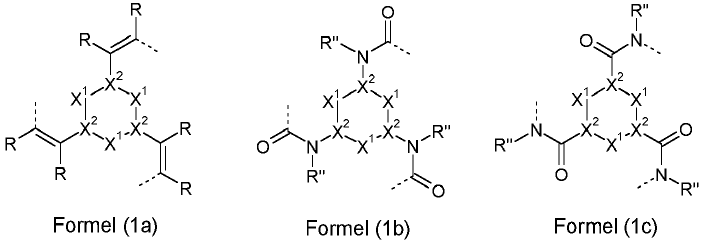 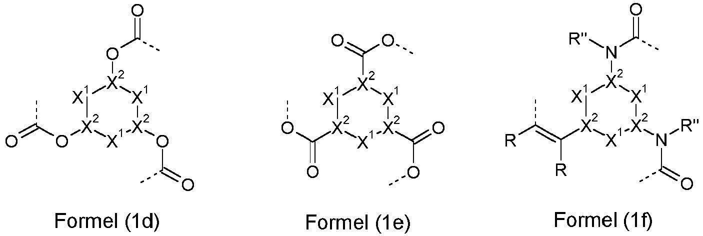 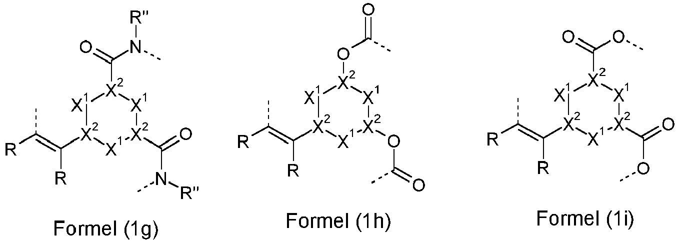 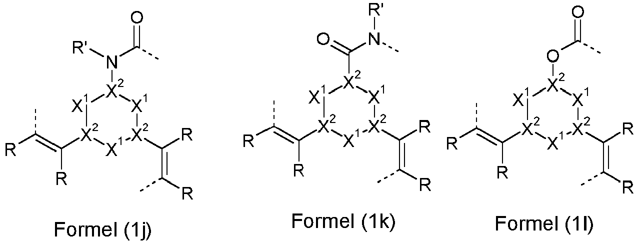 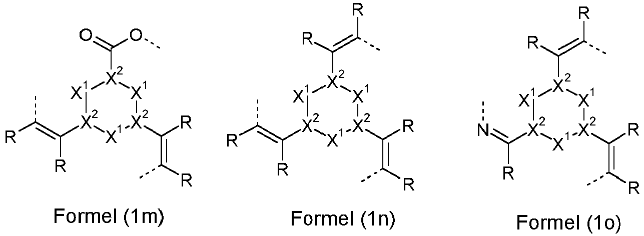 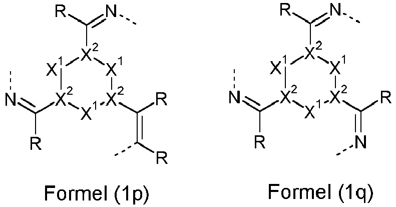 wobei die Symbole die oben genannten Bedeutungen aufweisen. Dabei bilden die Reste R in den Formeln (1a) und (1f) bis (1p) bevorzugt ein aromatisches oder heteroaromatisches Ringsystem miteinander.

In einer bevorzugten Ausführungsform der Erfindung steht das Symbol X⁴ gleich oder verschieden bei jedem Auftreten für -CR=CR-, -CR=N- -C(=O)-O- oder -C(=O)-NR"- und das Symbol X⁵ gleich oder verschieden bei jedem Auftreten für -CR=CR-, -CR=N-, -C(=O)-O- oder -C(=O)-NR"-.

Wie oben beschrieben, handelt es sich bei der Gruppe X³, bzw. X⁴ oder X⁵, in einer Ausführungsform der Erfindung um eine cis-verknüpfte Alkenylgruppe. Insbesondere kann es bevorzugt sein, wenn die Reste R in X³, X⁴ bzw. X⁵ miteinander ein aliphatisches oder heteroaliphatisches Ringsystem bilden. Wie eine solche Ringbildung der Substituenten aussieht, wird weiter unten ausführlich beschrieben.

Wenn die Substituenten der Gruppe X³, X⁴ bzw. X⁵, für eine cis-verknüpfte Alkenylgruppe stehen und miteinander ein aromatisches oder heteroaromatisches Ringsystem bilden, handelt es sich dabei bevorzugt um eine Arylen- oder Heteroarylengruppe mit 5 bis 13 aromatischen Ringatomen, die bevorzugt maximal zwei Heteroatome enthält, besonders bevorzugt maximal ein Heteroatom, wobei die Heteroatome ausgewählt sind aus N, O oder S, bevorzugt N oder O, besonders bevorzugt N. Dies schließt nicht aus, dass Substituenten, die gegebenenfalls an dieser Gruppe gebunden sind, auch Heteroatome enthalten können.

Bevorzugte Ausführungsformen der Gruppe X³, X⁴ bzw. X⁵, umfassend solch ein aromatisches oder heteroaromatisches Ringsystem, sind die Strukturen der folgenden Formeln (7) bis (23), 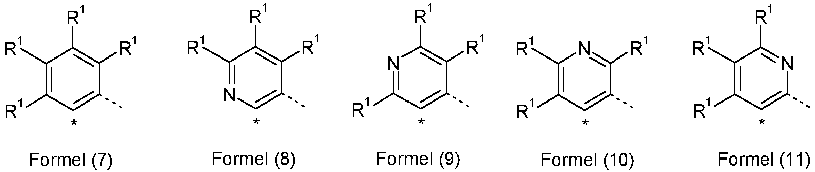 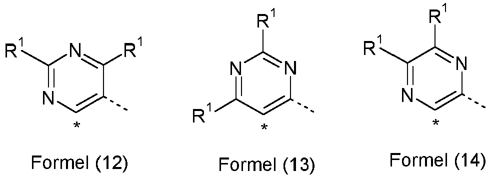 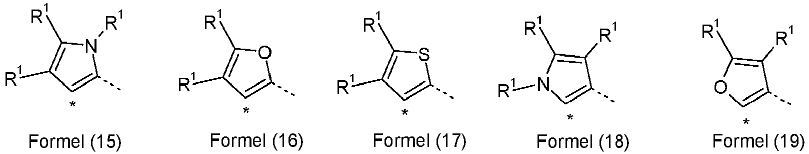 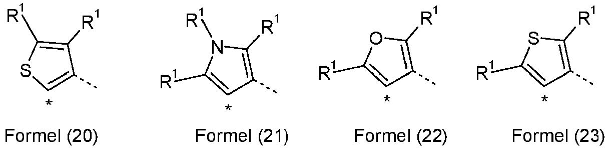 wobei die gestrichelte Bindung jeweils die Position der Bindung der bidentaten Teilliganden an diese Struktur darstellt, * die Position der Verknüpfung der Einheit der Formel (7) bis (23) mit der zentralen trivalenten cyclischen Gruppe darstellt und die weiteren verwendeten Symbole die oben genannten Bedeutungen aufweisen.

Besonders bevorzugt sind die gegebenenfalls substituierten Sechsring-Aromaten und Sechsring-Heteroaromaten der oben abgebildeten Formeln (7) bis (11). Ganz besonders bevorzugt ist ortho-Phenylen, also eine Gruppe der oben genannten Formel (7).

Dabei können, wie oben beschrieben, auch benachbarte Substituenten miteinander ein Ringsystem bilden, so dass kondensierte Strukturen, auch kondensierte Arylen- und Heteroarylengruppen, wie beispielsweise Naphthalin, Chinolin, Benzimidazol, Carbazol, Dibenzofuran oder Dibenzothiophen, entstehen können. Eine solche Ringbildung ist im Folgenden schematisch an Gruppen der oben genannten Formel (7) aufgeführt, was zu Gruppen der folgenden Formeln (7a) bis (7j) führt: 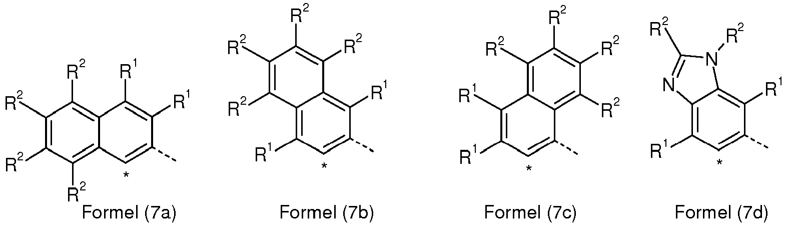 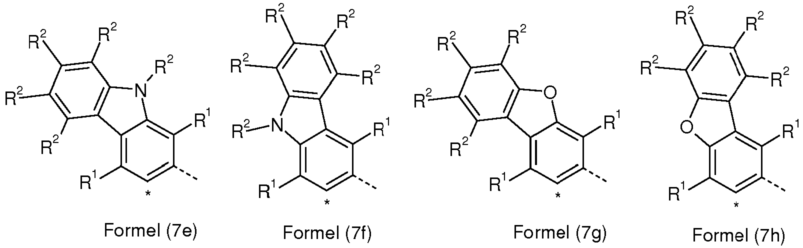 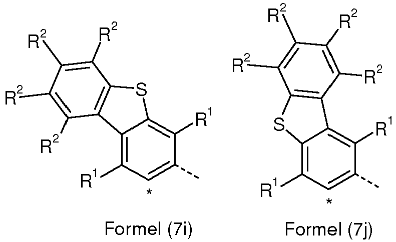 wobei die verwendeten Symbole die oben genannten Bedeutungen aufweisen.

Generell können die drei Gruppen X³, X⁴ bzw. X⁵, die in der Einheit der Formeln (1) bis (6) oder ihren bevorzugten Ausführungsformen vorhanden sind, gleich oder verschieden sein. In einer bevorzugten Ausführungsform der Erfindung sind alle drei Gruppen X³ gleich und sind auch gleich substituiert. Diese Bevorzugung wird durch die bessere synthetische Zugänglichkeit begründet. In einer weiteren bevorzugten Ausführungsform der Erfindung ist eine Gruppe X³ unterschiedlich, wobei die beiden anderen Gruppen X³ ebenfalls gleich oder unterschiedlich sein können. Diese Bevorzugung wird durch eine bessere Löslichkeit sowie im Allgemeinen geringere Sublimationstemperatur der Verbindungen begründet.

Im Folgenden werden die bevorzugten Metalle des erfindungsgemäßen Metallkomplexes beschrieben. In einer bevorzugten Ausführungsform der Erfindung ist das Metall ein Übergangsmetall, wobei Übergangsmetalle im Sinne der vorliegenden Erfindung nicht die Lanthanide und Actinide umfassen, oder für ein Hauptgruppenmetall. Wenn das Metall für ein Hauptgruppenmetall steht, dann ist es bevorzugt ausgewählt aus Metallen der dritten oder vierten Hauptgruppe, bevorzugt Al(III), In(III), Ga(III) oder Sn(IV), insbesondere Al(III). Wenn das Metall für ein Übergangsmetall steht, dann ist es bevorzugt ausgewählt aus der Gruppe bestehend aus Chrom, Molybdän, Wolfram, Rhenium, Ruthenium, Osmium, Rhodium, Iridium, Eisen, Kobalt, Nickel, Palladium, Platin, Kupfer, Silber und Gold, insbesondere Molybdän, Wolfram, Rhenium, Ruthenium, Osmium, Iridium, Kupfer, Platin und Gold. Ganz besonders bevorzugt ist Iridium. Die Metalle können dabei in verschiedenen Oxidationsstufen vorliegen. Bevorzugt sind dabei die oben genannten Metalle in den Oxidationsstufen Cr(0), Cr(III), Cr(VI), Mo(0), Mo(III), Mo(VI), W(0), W(III), W(VI), Re(I), Re(III), Re(IV), Ru(II), Ru(III), Os(II), Os(III), Os(IV), Rh(III), Ir(III), Ir(IV), Fe(II), Fe(III), Co(II), Co(III), Ni(II), Ni(IV), Pt(IV),Cu(II), Cu(III), Au(III) und Au(V). Besonders bevorzugt sind Mo(0), W(0), Re(I), Ru(II), Os(II), Rh(III) und Ir(III). Ganz besonders bevorzugt ist Ir(III).

Es ist besonders bevorzugt, wenn die bevorzugten Ausführungsformen der Teilliganden, wie sie unten genauer ausgeführt werden, und der Brücke der Formel (1) mit den bevorzugten Ausführungsformen des Metalls kombiniert werden. Besonders bevorzugt sind also Metallkomplexe, bei denen das Metall Ir(III) ist und die eine Brücke der Formel (1a) bis (1d) bzw. der Formeln (2) bis (6) bzw. (2a) oder (2b) aufweisen und die als bivalente Alkenyl- bzw. Arylen- bzw. Heteroarylengruppe X³ in der Gruppe der Formel (1) bis (6) bzw. den bevorzugten Ausführungsformen die oben aufgeführten bevorzugten Ausführungsformen aufweisen.

Im Folgenden werden die bidentaten Teilliganden beschrieben, die mit der Brücke der Formel (1) bzw. den oben genannten bevorzugten Ausführungsformen verknüpft sind. Die bevorzugten Ausführungsformen der bidentaten Teilliganden hängen insbesondere von dem jeweiligen verwendeten Metall ab. Die drei bidentaten Teilliganden können gleich oder verschieden sein. Wenn alle drei bidentaten Teilliganden gleich gewählt sind, entstehen dadurch C₃-symmetrische Metallkomplexe, wenn auch die Einheit der Formel (1) C₃-symmetrisch ist, was vorteilhaft bezüglich der Synthese der Liganden ist. Es kann aber auch vorteilhaft sein, die drei bidentaten Teilliganden unterschiedlich zu wählen bzw. zwei Teilliganden gleich und den dritten Teilliganden davon verschieden zu wählen, so dass C₁-symmetrische Metallkomplexe entstehen, weil dies größere Variationsmöglichkeiten der Liganden zulässt, so dass sich die gewünschten Eigenschaften des Komplexes, wie beispielsweise die Lage von HOMO und LUMO bzw. die Emissionsfarbe leichter variieren lassen. Außerdem lässt sich so auch die Löslichkeit der Komplexe verbessern, ohne lange aliphatische oder aromatische, löslichkeitsvermittelnde Gruppen verwenden zu müssen. Weiterhin weisen unsymmetrische Komplexe häufig eine geringere Sublimationstemperatur auf als ähnliche symmetrische Komplexe.

In einer bevorzugten Ausführungsform der Erfindung sind die drei bidentaten Teilliganden entweder gleich gewählt oder zwei der bidentaten Teilliganden sind gleich gewählt und der dritte bidentate Teilligand ist unterschiedlich von den ersten beiden bidentaten Teilliganden. Dabei bedeutet "gleiche Teilliganden", dass zum einen die Ligandenstruktur selber gleich gewählt ist und zum anderen, dass diese Strukturen auch gleich substituiert sind.

In einer bevorzugten Ausführungsform der Erfindung ist jeder der bidentaten Teilliganden gleich oder verschieden entweder monoanionisch oder neutral. Besonders bevorzugt ist jeder der bidentaten Teilliganden monoanionisch.

In einer weiteren bevorzugten Ausführungsform der Erfindung sind die koordinierenden Atome der bidentaten Teilliganden gleich oder verschieden bei jedem Auftreten ausgewählt aus C, N, P, O, S und/oder B, besonders bevorzugt C, N und/oder O.

Wenn das Metall ausgewählt ist aus den Hauptgruppenmetallen, dann sind die koordinierenden Atome der bidentaten Teilliganden bevorzugt gleich oder verschieden bei jedem Auftreten ausgewählt aus N, O und/oder S. Besonders bevorzugt weisen die bidentaten Teilliganden zwei Stickstoffatome oder zwei Sauerstoffatome oder ein Stickstoffatom und ein Sauerstoffatom pro Teilligand als koordinierende Atome auf. Dabei können die koordinierenden Atome von jedem der drei Teilliganden gleich sein, oder sie können unterschiedlich sein.

Wenn das Metall ausgewählt ist aus den Übergangsmetallen, dann sind die koordinierenden Atome der bidentaten Teilliganden bevorzugt gleich oder verschieden bei jedem Auftreten ausgewählt aus C, N, O und/oder S, besonders bevorzugt C, N und/oder O und ganz besonders bevorzugt C und/oder N. Dabei weisen die bidentaten Teilliganden bevorzugt ein Kohlenstoffatom und ein Stickstoffatom oder zwei Kohlenstoffatome oder zwei Stickstoffatome oder zwei Sauerstoffatome oder ein Sauerstoffatom und ein Stickstoffatom pro Teilligand als koordinierende Atome auf. Dabei können die koordinierenden Atome von jedem der drei Teilliganden gleich sein, oder sie können unterschiedlich sein. Besonders bevorzugt weist mindestens einer der bidentaten Teilliganden ein Kohlenstoffatom und ein Stickstoffatom oder zwei Kohlenstoffatome als koordinierende Atome auf, insbesondere ein Kohlenstoffatom und ein Stickstoffatom. Ganz besonders bevorzugt weisen mindestens zwei der bidentaten Teilliganden und insbesondere alle drei bidentaten Teilliganden ein Kohlenstoffatom und ein Stickstoffatom oder zwei Kohlenstoffatome als koordinierende Atome auf, insbesondere ein Kohlenstoffatom und ein Stickstoffatom. Dies gilt insbesondere, wenn das Metall Ir(III) ist. Wenn das Metall Ru, Co, Fe, Os, Cu oder Ag ist, sind als koordinierende Atome der bidentaten Teilliganden auch zwei Stickstoffatome besonders bevorzugt.

In einer besonders bevorzugten Ausführungsform der Erfindung ist das Metall Ir(III) und zwei der bidentaten Teilliganden koordinieren an das Iridium über jeweils ein Kohlenstoffatom und ein Stickstoffatom und der dritte der bidentaten Teilliganden koordiniert an das Iridium über ein Kohlenstoffatom und ein Stickstoffatom oder über zwei Stickstoffatome oder über ein Stickstoffatom und ein Sauerstoffatom oder über zwei Sauerstoffatome, insbesondere über ein Kohlenstoffatom und ein Stickstoffatom. Besonders bevorzugt handelt es sich somit um einen Iridiumkomplex, in dem alle drei bidentaten Teilliganden ortho-metalliert sind, d. h. mit dem Iridium einen Metallacyclus bilden, in dem eine Metall-Kohlenstoff-Bindung vorliegt.

Es ist weiterhin bevorzugt, wenn es sich bei dem Metallacyclus, der aus dem Metall und dem bidentaten Teilliganden aufgespannt wird, um einen Fünfring handelt, der vor allem dann bevorzugt ist, wenn die koordinierenden Atome C und N, N und N oder N und O sind. Wenn es sich bei den koordinierenden Atomen um O handelt, kann auch ein Metallasechsring bevorzugt sein. Dies wird im Folgenden schematisch dargestellt: 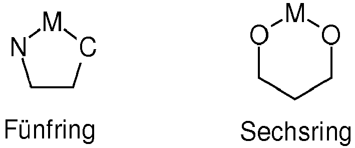 wobei M das Metall, N ein koordinierendes Stickstoffatom, C ein koordinierendes Kohlenstoffatom und O koordinierende Sauerstoffatome darstellen und die eingezeichneten Kohlenstoffatome Atome des bidentaten Liganden darstellen.

Im Folgenden werden die Strukturen der bidentaten Teilliganden beschrieben, die bevorzugt sind, wenn das Metall ein Übergangsmetall ist.

In einer bevorzugten Ausführungsform der Erfindung steht mindestens einer der bidentaten Teilliganden, besonders bevorzugt mindestens zwei der bidentaten Teilliganden, ganz besonders bevorzugt alle drei der bidentaten Teilliganden gleich oder verschieden bei jedem Auftreten für eine Struktur gemäß den folgenden Formeln (L-1), (L-2), (L-3) oder (L-4), 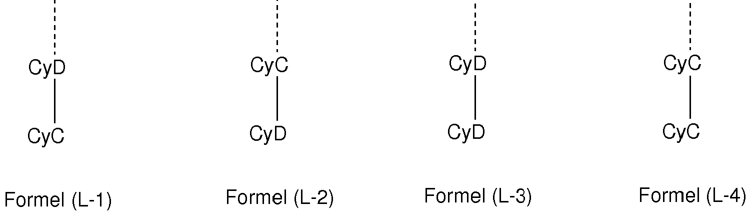 wobei die gestrichelte Bindung die Bindung des Teilliganden an die Brücke der Formeln (1) bis (6) bzw. die bevorzugten Ausführungsformen darstellt und für die weiteren verwendeten Symbole gilt:
- CyC: ist gleich oder verschieden bei jedem Auftreten eine optional substituierte Aryl- oder Heteroarylgruppe mit 5 bis 14 aromatischen Ringatomen, welche jeweils über ein Kohlenstoffatom an das Metall koordiniert und welche jeweils über eine kovalente Bindung mit CyD verbunden ist;
- CyD: ist gleich oder verschieden bei jedem Auftreten eine optional substituierte Heteroarylgruppe mit 5 bis 14 aromatischen Ringatomen, welche über ein Stickstoffatom oder über ein Carben-Kohlenstoffatom an das Metall koordiniert und welche über eine kovalente Bindung mit CyC verbunden ist;
dabei können mehrere der optionalen Substituenten miteinander ein Ringsystem bilden; weiterhin sind die optionalen Reste bevorzugt ausgewählt aus den oben genannten Resten R.

Dabei koordiniert CyD in den Teilliganden der Formeln (L-1) und (L-2) bevorzugt über ein neutrales Stickstoffatom oder über ein Carben-Kohlenstoffatom. Weiterhin bevorzugt koordiniert eine der beiden Gruppen CyD in dem Liganden der Formel (L-3) über ein neutrales Stickstoffatom und die andere der beiden Gruppen CyD über ein anionisches Stickstoffatom. Weiterhin bevorzugt koordiniert CyC in den Teilliganden der Formeln (L-1), (L-2) und (L-4) über anionische Kohlenstoffatome.

Besonders bevorzugt sind die bidentaten Teilliganden (L-1) und (L-2).

Wenn mehrere der Substituenten, insbesondere mehrere Reste R, miteinander ein Ringsystem bilden, so ist die Bildung eines Ringsystems aus Substituenten, die an direkt benachbarten Kohlenstoffatomen gebunden sind, möglich. Weiterhin ist es auch möglich, dass die Substituenten an CyC und CyD in den Formeln (L-1) und (L-2) bzw. die Substituenten an den beiden Gruppen CyD in Formel (L-3) bzw. die Substituenten an den beiden Gruppen CyC in Formel (L-4) miteinander einen Ring bilden, wodurch CyC und CyD bzw. die beiden Gruppen CyD bzw. die beiden Gruppen CyC auch zusammen eine einzige kondensierte Aryl- bzw. Heteroarylgruppe als bidentaten Liganden bilden können.

In einer bevorzugten Ausführungsform der vorliegenden Erfindung ist CyC eine Aryl- oder Heteroarylgruppe mit 6 bis 13 aromatischen Ringatomen, besonders bevorzugt mit 6 bis 10 aromatischen Ringatomen, ganz besonders bevorzugt mit 6 aromatischen Ringatomen, welche über ein Kohlenstoffatom an das Metall koordiniert, welche mit einem oder mehreren Resten R substituiert sein kann und welche über eine kovalente Bindung mit CyD verbunden ist.

Bevorzugte Ausführungsformen der Gruppe CyC sind die Strukturen der folgenden Formeln (CyC-1) bis (CyC-20), 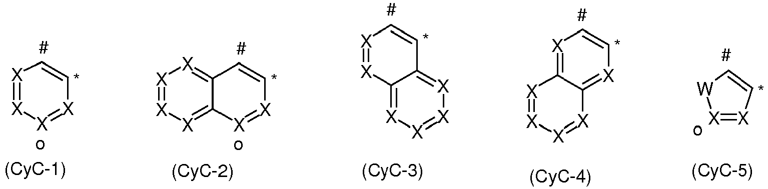 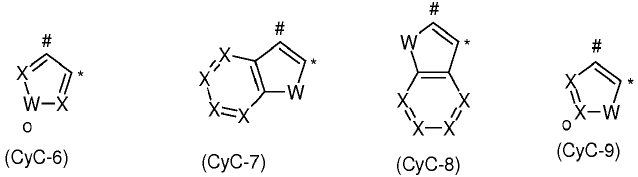 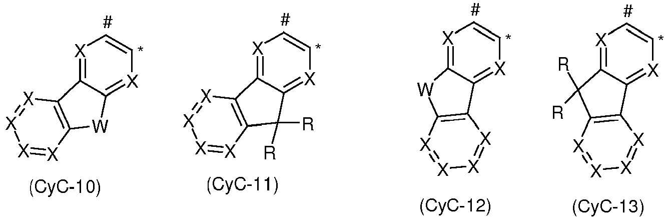 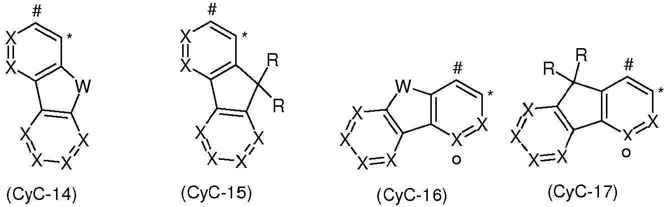 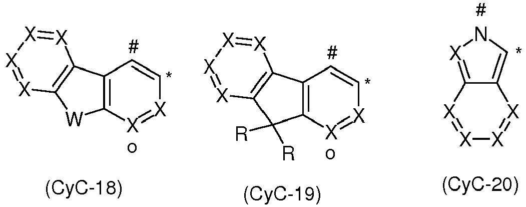 wobei die Gruppe jeweils an der durch # gekennzeichneten Position an CyD in (L-1) bzw. (L-2) bzw. an CyC in (L-4) bindet und an der durch * gekennzeichneten Position an das Metall koordiniert, R die oben genannten Bedeutungen aufweist und für die weiteren verwendeten Symbole gilt:
- X: ist bei jedem Auftreten gleich oder verschieden CR oder N mit der Maßgabe, dass maximal zwei Symbole X pro Cyclus für N stehen;
- W: ist bei jedem Auftreten gleich oder verschieden NR, O oder S;
mit der Maßgabe, dass, wenn die Brücke der Formeln (1) bis (6) bzw. die bevorzugten Ausführungsformen an CyC gebunden ist, ein Symbol X für C steht und die Brücke der Formeln (1) bis (6) bzw. die bevorzugten Ausführungsformen an dieses Kohlenstoffatom gebunden ist. Wenn die Gruppe CyC an die Brücke der Formeln (1) bis (6) bzw. die bevorzugten Ausführungsformen gebunden ist, so erfolgt die Bindung bevorzugt über die mit "o" markierte Position der oben abgebildeten Formeln, so dass dann bevorzugt das mit "o" markierte Symbol X für C steht. Die oben abgebildeten Strukturen, die kein mit "o" markiertes Symbol X enthalten, sind bevorzugt nicht direkt an die Brücke der Formeln (1) bis (6) bzw. die bevorzugten Ausführungsformen gebunden, da eine solche Bindung an die Brücke aus sterischen Gründen nicht vorteilhaft ist. Solche Gruppen CyC sind bevorzugt nur in (L-1) bzw. als untere Gruppe in (L-4) gebunden.

Bevorzugt stehen insgesamt maximal zwei Symbole X in CyC für N, besonders bevorzugt steht maximal ein Symbol X in CyC für N, ganz besonders bevorzugt stehen alle Symbole X für CR, mit der Maßgabe, dass, wenn die Brücke der Formeln (1) bis (6) bzw. die bevorzugten Ausführungsformen an CyC gebunden ist, ein Symbol X für C steht und die Brücke der Formeln (1) bis (6) bzw. die bevorzugten Ausführungsformen an dieses Kohlenstoffatom gebunden ist.

Besonders bevorzugte Gruppen CyC sind die Gruppen der folgenden Formeln (CyC-1a) bis (CyC-20a), 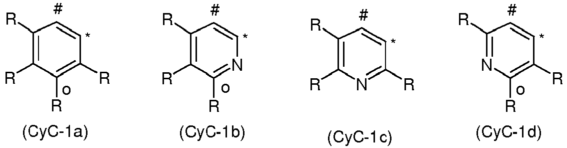 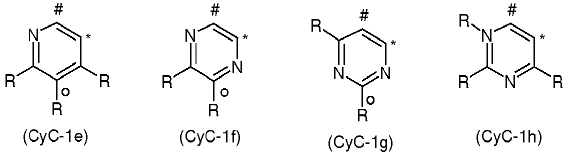 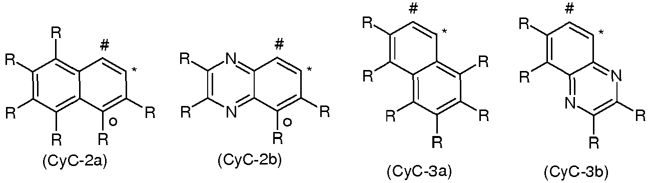 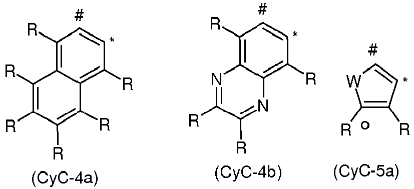 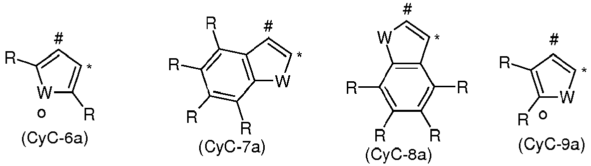 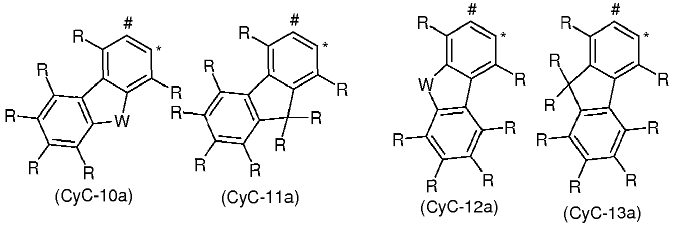 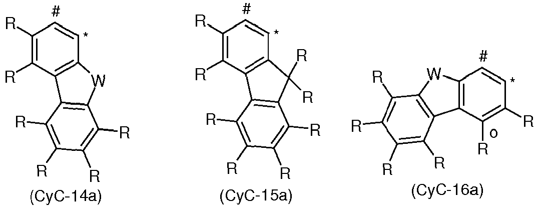 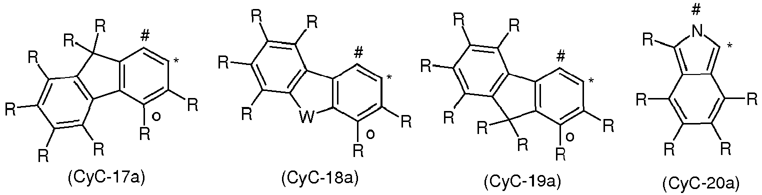 wobei die verwendeten Symbole die oben genannten Bedeutungen aufweisen und, wenn die Brücke der Formel (1) bis (6) bzw. die bevorzugten Ausführungsformen an CyC gebunden ist, ein Rest R nicht vorhanden ist und die Brücke der Formel (1) bis (6) bzw. die bevorzugten Ausführungsformen an das entsprechende Kohlenstoffatom gebunden ist. Wenn die Gruppe CyC an die Brücke der Formeln (1) bis (6) bzw. die bevorzugten Ausführungsformen gebunden ist, so erfolgt die Bindung bevorzugt über die mit "o" markierte Position der oben abgebildeten Formeln, so dass dann bevorzugt in dieser Position der Rest R nicht vorhanden ist. Die oben abgebildeten Strukturen, die kein mit "o" markiertes Kohlenstoffatom enthalten, sind bevorzugt nicht direkt an die Brücke der Formeln (1) bis (6) bzw. die bevorzugten Ausführungsformen gebunden.

Bevorzugte Gruppen unter den Gruppen (CyC-1) bis (CyC-19) sind die Gruppen (CyC-1), (CyC-3), (CyC-8), (CyC-10), (CyC-12), (CyC-13) und (CyC-16), und besonders bevorzugt sind die Gruppen (CyC-1a), (CyC-3a), (CyC-8a), (CyC-10a), (CyC-12a), (CyC-13a) und (CyC-16a).

In einer weiteren bevorzugten Ausführungsform der Erfindung ist CyD eine Heteroarylgruppe mit 5 bis 13 aromatischen Ringatomen, besonders bevorzugt mit 6 bis 10 aromatischen Ringatomen, welche über ein neutrales Stickstoffatom oder über ein Carben-Kohlenstoffatom an das Metall koordiniert und welche mit einem oder mehreren Resten R substituiert sein kann und welche über eine kovalente Bindung mit CyC verbunden ist.

Bevorzugte Ausführungsformen der Gruppe CyD sind die Strukturen der folgenden Formeln (CyD-1) bis (CyD-14), 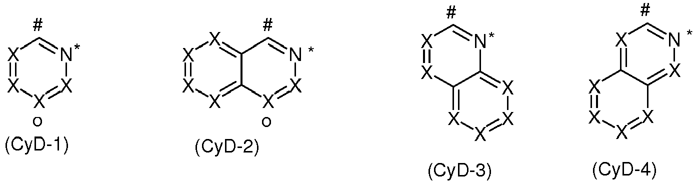 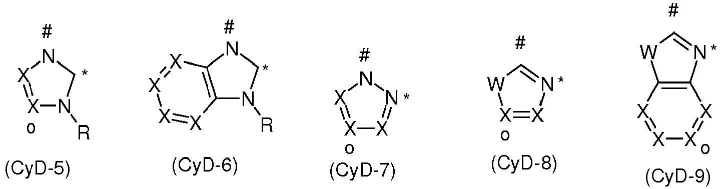 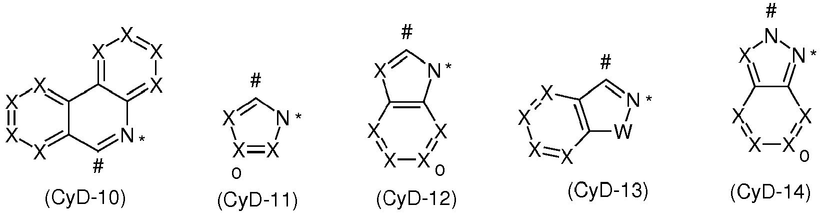 wobei die Gruppe jeweils an der durch # gekennzeichneten Position an CyC in (L-1) bzw. (L-2) bzw. an CyD in (L-3) bindet und an der durch * gekennzeichneten Position an das Metall koordiniert, wobei X, W und R die oben genannten Bedeutungen aufweisen, mit der Maßgabe, dass, wenn die Brücke der Formeln (1) bis (6) bzw. die bevorzugten Ausführungsformen an CyD gebunden ist, ein Symbol X für C steht und die Brücke der Formeln (1) bis (6) bzw. die bevorzugten Ausführungsformen an dieses Kohlenstoffatom gebunden ist. Wenn die Gruppe CyD an die Brücke der Formeln (1) bis (6) bzw. die bevorzugten Ausführungsformen gebunden ist, so erfolgt die Bindung bevorzugt über die mit "o" markierte Position der oben abgebildeten Formeln, so dass dann bevorzugt das mit "o" markierte Symbol X für C steht. Die oben abgebildeten Strukturen, die kein mit "o" markiertes Symbol X enthalten, sind bevorzugt nicht direkt an die Brücke der Formeln (1) bis (6) bzw. die bevorzugten Ausführungsformen gebunden, da eine solche Bindung an die Brücke aus sterischen Gründen nicht vorteilhaft ist. Solche Gruppen CyD sind bevorzugt nur in (L-2) bzw. als untere Gruppe in (L-3) gebunden.

Dabei koordinieren die Gruppen (CyD-1) bis (CyD-4), (CyD-7) bis (CyD-10), (CyD-13) und (CyD-14) über ein neutrales Stickstoffatom, (CyD-5) und (CyD-6) über ein Carben-Kohlenstoffatom und (CyD-11) und (CyD-12) über ein anionisches Stickstoffatom an das Metall.

Bevorzugt stehen insgesamt maximal zwei Symbole X in CyD für N, besonders bevorzugt steht maximal ein Symbol X in CyD für N, insbesondere bevorzugt stehen alle Symbole X für CR, mit der Maßgabe, dass, wenn die Brücke der Formeln (1) bis (6) bzw. die bevorzugten Ausführungsformen an CyD gebunden ist, ein Symbol X für C steht und die Brücke der Formeln (1) bis (6) bzw. die bevorzugten Ausführungsformen an dieses Kohlenstoffatom gebunden ist.

Besonders bevorzugte Gruppen CyD sind die Gruppen der folgenden Formeln (CyD-1a) bis (CyD-14b), 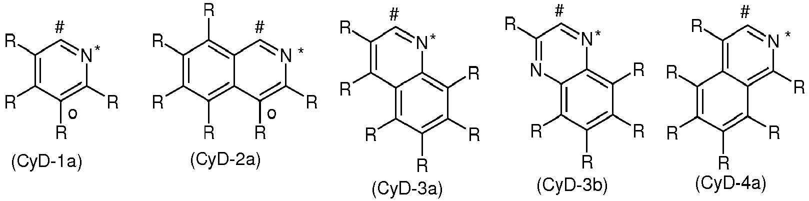 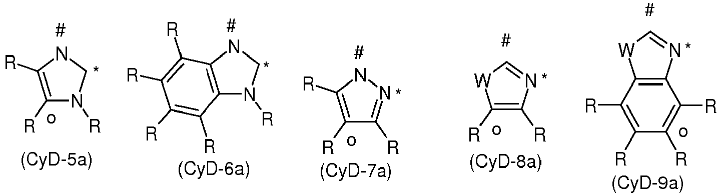 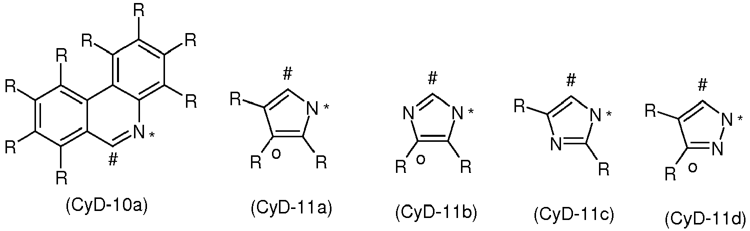 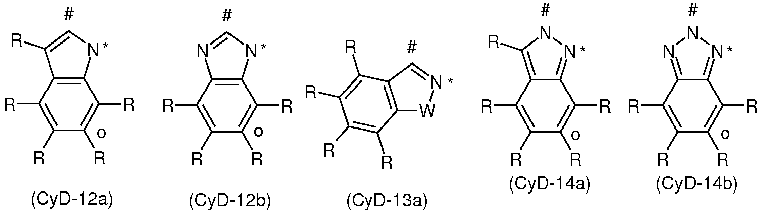 wobei die verwendeten Symbole die oben genannten Bedeutungen aufweisen und, wenn die Brücke der Formeln (1) bis (6) bzw. die bevorzugten Ausführungsformen an CyD gebunden ist, ein Rest R nicht vorhanden ist und die Brücke der Formel (1) bis (6) bzw. die bevorzugten Ausführungsformen an das entsprechende Kohlenstoffatom gebunden ist. Wenn die Gruppe CyD an die Brücke der Formeln (1) bis (6) bzw. die bevorzugten Ausführungsformen gebunden ist, so erfolgt die Bindung bevorzugt über die mit "o" markierte Position der oben abgebildeten Formeln, so dass dann bevorzugt in dieser Position der Rest R nicht vorhanden ist. Die oben abgebildeten Strukturen, die kein mit "o" markiertes Kohlenstoffatom enthalten, sind bevorzugt nicht direkt an die Brücke der Formeln (1) bis (6) bzw. die bevorzugten Ausführungsformen gebunden.

Bevorzugte Gruppen unter den Gruppen (CyD-1) bis (CyD-10) sind die Gruppen (CyD-1), (CyD-2), (CyD-3), (CyD-4), (CyD-5) und (CyD-6), insbesondere (CyD-1), (CyD-2) und (CyD-3), und besonders bevorzugt sind die Gruppen (CyD-1a), (CyD-2a), (CyD-3a), (CyD-4a), (CyD-5a) und (CyD-6a), insbesondere (CyD-1a), (CyD-2a) und (CyD-3a).

In einer bevorzugten Ausführungsform der vorliegenden Erfindung ist CyC eine Aryl- oder Heteroarylgruppe mit 6 bis 13 aromatischen Ringatomen, und gleichzeitig ist CyD eine Heteroarylgruppe mit 5 bis 13 aromatischen Ringatomen. Besonders bevorzugt ist CyC eine Aryl- oder Heteroarylgruppe mit 6 bis 10 aromatischen Ringatomen, und gleichzeitig ist CyD eine Heteroarylgruppe mit 5 bis 10 aromatischen Ringatomen. Ganz besonders bevorzugt ist CyC eine Aryl- oder Heteroarylgruppe mit 6 aromatischen Ringatomen und CyD eine Heteroarylgruppe mit 6 bis 10 aromatischen Ringatomen. Dabei können CyC und CyD mit einem oder mehreren Resten R substituiert sein.

Die oben genannten bevorzugten Gruppen (CyC-1) bis (CyC-20) und (CyD-1) bis (CyD-14) können in den Teilliganden der Formeln (L-1) und(L-2) beliebig miteinander kombiniert werden, sofern mindestens eine der Gruppen CyC bzw. CyD eine geeignete Anknüpfungsstelle an die Brücke der Formel (1) bis (6) bzw. die bevorzugten Ausführungsformen aufweist, wobei geeignete Anknüpfungsstellen in den oben genannten Formeln mit "o" gekennzeichnet sind.

Insbesondere bevorzugt ist es, wenn die oben als besonders bevorzugt genannten Gruppen CyC und CyD, also die Gruppen der Formeln (CyC-1a) bis (CyC-20a) und die Gruppen der Formeln (CyD1-a) bis (CyD-14b) miteinander kombiniert werden, sofern mindestens eine dieser Gruppen eine geeignete Anknüpfungsstelle an die Brücke der Formel (1) bis (6) bzw. die bevorzugten Ausführungsformen aufweist, wobei geeignete Anknüpfungsstellen in den oben genannten Formeln mit "o" gekennzeichnet sind. Kombinationen, in denen weder CyC noch CyD eine solche geeignete Anknüpfungsstelle für die Brücke der Formel (1) bis (6) bzw. die bevorzugten Ausführungsformen aufweist, sind daher nicht bevorzugt.

Ganz besonders bevorzugt ist es, wenn eine der Gruppen (CyC-1), (CyC-3), (CyC-8), (CyC-10), (CyC-12), (CyC-13) und (CyC-16), und insbesondere die Gruppen (CyC-1a), (CyC-3a), (CyC-8a), (CyC-10a), (CyC-12a), (CyC-13a) und (CyC-16a), mit einer der Gruppen (CyD-1), (CyD-2) und (CyD-3), und insbesondere mit einer der Gruppen (CyD-1a), (CyD-2a) und (CyD-3a), kombiniert wird.

Bevorzugte Teilliganden (L-1) sind die Strukturen der folgenden Formeln (L-1-1) und (L-1-2), und bevorzugte Teilliganden (L-2) sind die Strukturen der folgenden Formeln (L-2-1) bis (L-2-3), 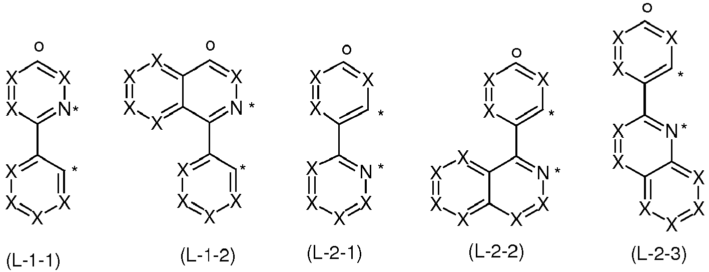 wobei die verwendeten Symbole die oben genannten Bedeutungen aufweisen und "o" die Position der Bindung an die Brücke der Formel (1) bis (6) bzw. die bevorzugten Ausführungsformen darstellt.

Besonders bevorzugte Teilliganden (L-1) sind die Strukturen der folgenden Formeln (L-1-1a) und (L-1-2b), und besonders bevorzugte Teilliganden (L-2) sind die Strukturen der folgenden Formeln (L-2-1a) bis (L-2-3a), 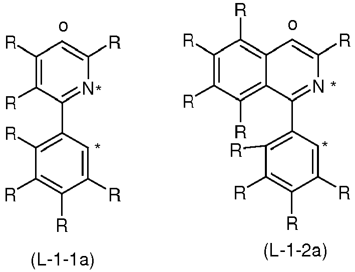 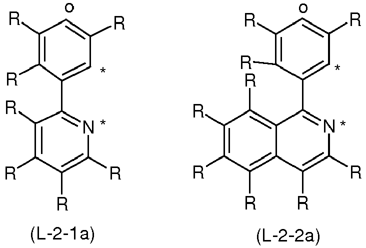 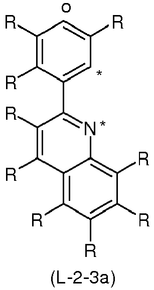 wobei die verwendeten Symbole die oben genannten Bedeutungen aufweisen und "o" die Position der Bindung an die Brücke der Formel (1) bis (6) bzw. die bevorzugten Ausführungsformen darstellt.

Ebenso können die oben genannten bevorzugten Gruppen CyD in den Teilliganden der Formel (L-3) beliebig miteinander kombiniert werden, wobei es bevorzugt ist, eine neutrale Gruppe CyD, also eine Gruppe (CyD-1) bis (CyD-10), (CyD-13) oder (CyD-14), mit einer anionischen Gruppe CyD, also einer Gruppe (CyD-11) oder (CyD-12), zu kombinieren, sofern mindestens eine der bevorzugten Gruppen CyD eine geeignete Anknüpfungsstelle an die Brücke der Formel (1) bis (6) bzw. die bevorzugten Ausführungsformen aufweist, wobei geeignete Anknüpfungsstellen in den oben genannten Formeln mit "o" gekennzeichnet sind.

Ebenso können die oben genannten bevorzugten Gruppen CyC in den Teilliganden der Formel (L-4) beliebig miteinander kombiniert werden, sofern mindestens eine der bevorzugten Gruppen CyC eine geeignete Anknüpfungsstelle an die Brücke der Formel (1) bis (6) bzw. die bevorzugten Ausführungsformen aufweist, wobei geeignete Anknüpfungsstellen in den oben genannten Formeln mit "o" gekennzeichnet sind.

Wenn zwei Reste R, von denen einer an CyC und der andere an CyD in den Formeln (L-1) und (L-2) gebunden sind bzw. von denen einer an die eine Gruppe CyD und der andere an die andere Gruppe CyD in Formel (L-3) gebunden sind bzw. von denen einer an die eine Gruppe CyC und der andere an die andere Gruppe CyC in Formel (L-4) gebunden sind, miteinander ein Ringsystem bilden, können sich überbrückte Teilliganden und beispielsweise auch Teilliganden ergeben, die insgesamt eine einzige größere Heteroarylgruppe darstellen, wie beispielsweise Benzo[h]chinolin, etc.. Die Ringbildung zwischen den Substituenten an CyC und CyD in den Formeln (L-1) und (L-2) bzw. zwischen den Substituenten an den beiden Gruppen CyD in Formel (L-3) bzw. zwischen den Substituenten an den beiden Gruppen (CyC) in Formel (L-4) erfolgt dabei bevorzugt durch eine Gruppe gemäß einer der folgenden Formeln (24) bis (33), 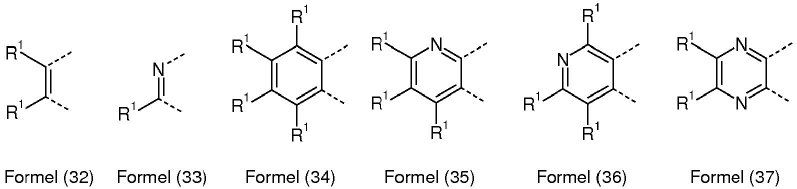 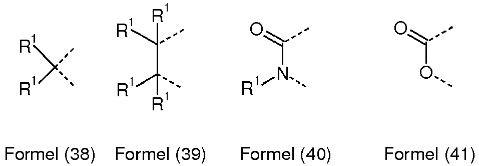 wobei R¹ die oben genannten Bedeutungen aufweist und die gestrichelten Bindungen die Bindungen an CyC bzw. CyD andeuten. Dabei können die unsymmetrischen der oben genannten Gruppen in jeder der beiden Möglichkeiten eingebaut werden, beispielsweise kann bei der Gruppe der Formel (41) das Sauerstoffatom an die Gruppe CyC und die Carbonylgruppe an die Gruppe CyD binden, oder das Sauerstoffatom kann an die Gruppe CyD und die Carbonylgruppe an die Gruppe CyC binden.

Dabei ist die Gruppe der Formel (38) besonders dann bevorzugt, wenn sich dadurch die Ringbildung zu einem Sechsring ergibt, wie beispielsweise unten durch die Formeln (L-23) und (L-24) dargestellt.

Bevorzugte Liganden, die durch Ringbildung zweier Reste R an den unterschiedlichen Cyclen entstehen, sind die im Folgenden aufgeführten Strukturen der Formeln (L-5) bis (L-32), 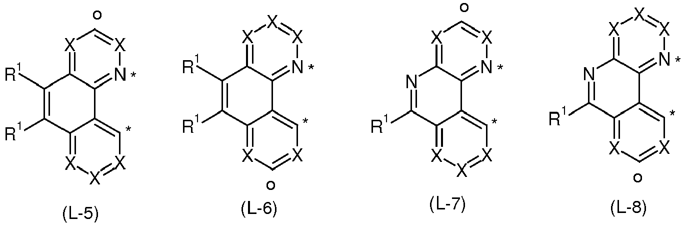 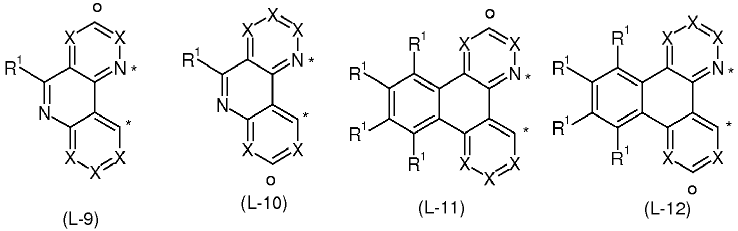 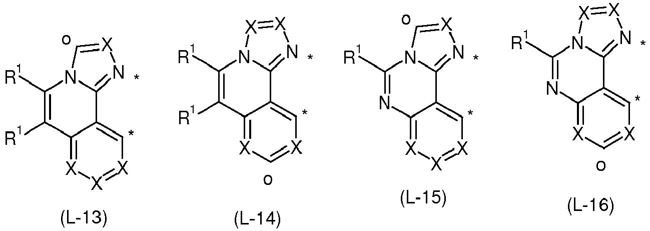 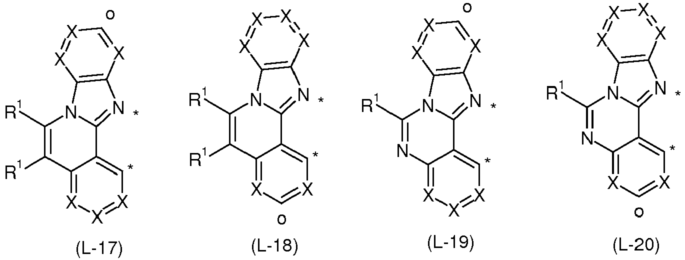 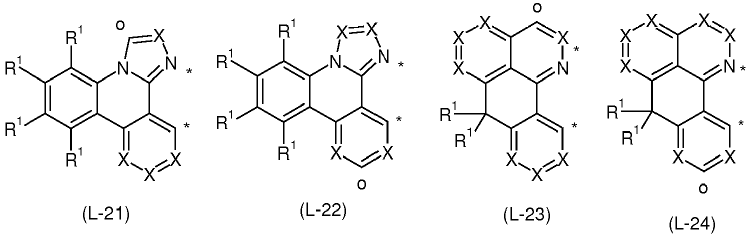 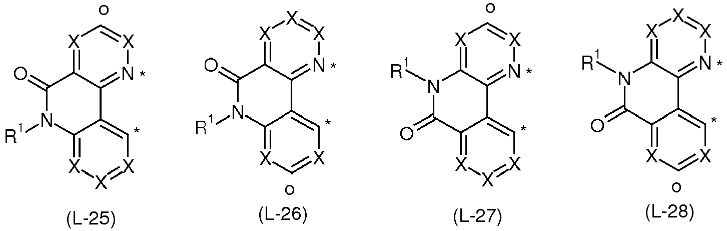 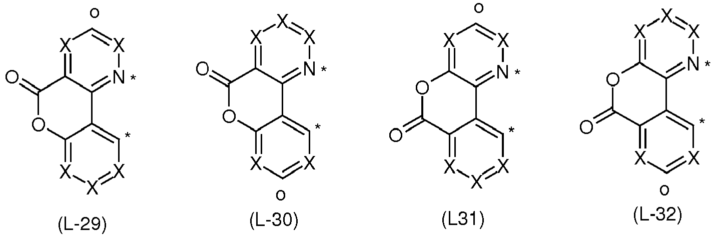 wobei die verwendeten Symbole die oben genannten Bedeutungen aufweisen und "o" die Position angibt, an denen dieser Teilligand mit der Gruppe der Formel (1) bis (6) bzw. den bevorzugten Ausführungsformen verknüpft ist.

In einer bevorzugten Ausführungsform der Teilliganden der Formeln (L-5) bis (L-32) steht insgesamt ein Symbol X für N und die anderen Symbole X stehen für CR, oder alle Symbole X stehen für CR. Besonders bevorzugt stehen alle Symbole X für CR.

In einer weiteren Ausführungsform der Erfindung ist es bevorzugt, falls in den Gruppen (CyC-1) bis (CyC-20) oder (CyD-1) bis (CyD-14) oder in den Teilliganden (L-5) bis (L-32) eines der Atome X für N steht, wenn benachbart zu diesem Stickstoffatom eine Gruppe R als Substituent gebunden ist, welche ungleich Wasserstoff oder Deuterium ist. Dies gilt analog für die bevorzugten Strukturen (CyC-1a) bis (CyC-20a) oder (CyD-1a) bis (CyD-14b), in denen bevorzugt benachbart zu einem nicht koordinierenden Stickstoffatom eine Gruppe R als Substituent gebunden ist, welche ungleich Wasserstoff oder Deuterium ist

Dabei ist dieser Substituent R bevorzugt eine Gruppe, ausgewählt aus CF₃, OCF₃, Alkyl- oder Alkoxygruppen mit 1 bis 10 C-Atomen, insbesondere verzweigten oder cyclischen Alkyl- oder Alkoxygruppen mit 3 bis 10 C-Atomen, einer Dialkylaminogruppe mit 2 bis 10 C-Atomen, aromatischen bzw. heteroaromatischen Ringsystemen oder Aralkyl- bzw. Heteroaralkylgruppen. Es handelt sich bei diesen Gruppen um sterisch anspruchsvolle Gruppen. Weiterhin bevorzugt kann dieser Rest R auch mit einem benachbarten Rest R einen Cyclus bilden.

Ein weiterer geeigneter bidentater Teilligand für Metallkomplexe, in denen das Metall ein Übergangsmetall ist, ist ein Teilligand der folgenden Formel (L-33) oder (L-34), 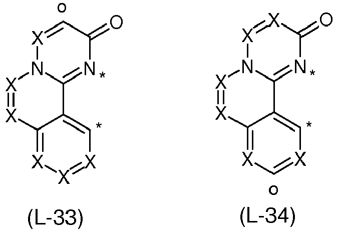 wobei R die oben genannten Bedeutungen aufweist, * die Position der Koordination an das Metall darstellt, "o" die Position der Verknüpfung des Teilliganden mit der Gruppe der Formeln (1) bis (6) bzw. den bevorzugten Ausführungsformen darstellt und für die weiteren verwendeten Symbole gilt:
- X: ist bei jedem Auftreten gleich oder verschieden CR oder N mit der Maßgabe, dass maximal ein Symbol X pro Cyclus für N steht und weiterhin mit der Maßgabe, dass ein Symbol X für C steht und an dieses Kohlenstoffatom die Gruppe der Formel (1) bis (6) bzw. die bevorzugten Ausführungsformen gebunden sind.

Wenn zwei Reste R, die in den Teilliganden (L-33) bzw. (L-34) an benachbarten Kohlenstoffatomen gebunden sind, miteinander einen aromatischen Cyclus bilden, so ist dieser zusammen mit den beiden benachbarten Kohlenstoffatomen bevorzugt eine Struktur der folgenden Formel (42), 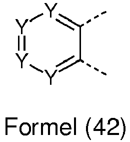 wobei die gestrichelten Bindungen die Verknüpfung dieser Gruppe im Teilliganden symbolisieren und Y gleich oder verschieden bei jedem Auftreten für CR¹ oder N steht und bevorzugt maximal ein Symbol Y für N steht.

In einer bevorzugten Ausführungsform des Teilliganden (L-33) bzw. (L-34) ist maximal eine Gruppe der Formel (42) vorhanden. Es handelt sich also bevorzugt um Teilliganden der folgenden Formeln (L-35) bis (L-40), 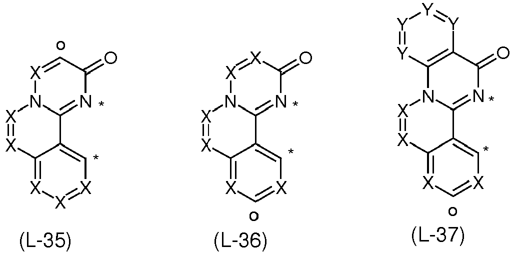 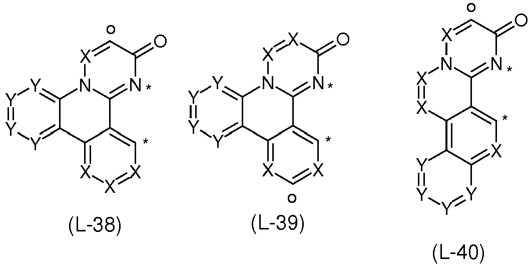 wobei X bei jedem Auftreten gleich oder verschieden für CR oder N steht, jedoch die Reste R nicht miteinander ein aromatisches oder heteroaromatisches Ringsystem bilden und die weiteren Symbole die oben genannten Bedeutungen aufweisen.

In einer bevorzugten Ausführungsform der Erfindung stehen im Teilliganden der Formeln (L-33) bis (L-40)insgesamt 0, 1 oder 2 der Symbole X und, falls vorhanden, Y für N. Besonders bevorzugt stehen insgesamt 0 oder 1 der Symbole X und, falls vorhanden, Y für N.

Bevorzugte Ausführungsformen der Formeln (L-35) bis (L-40) sind die Strukturen der folgenden Formeln (L-35a) bis (L-40f), 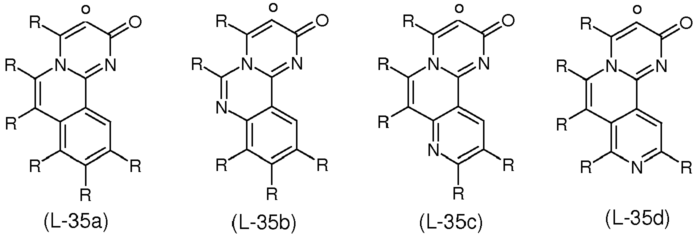 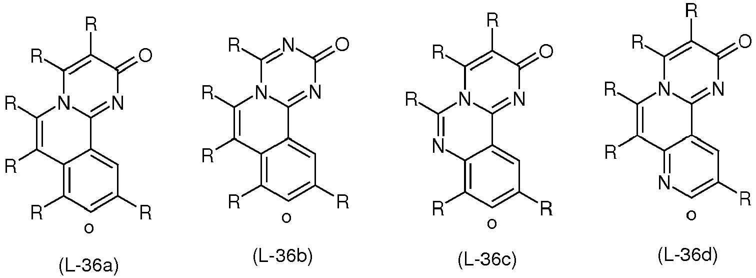 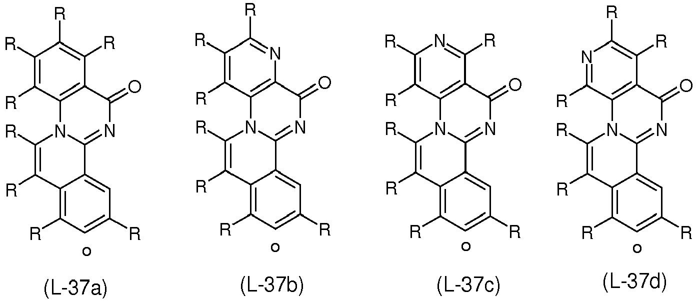 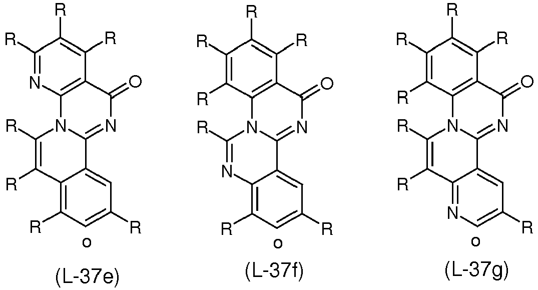 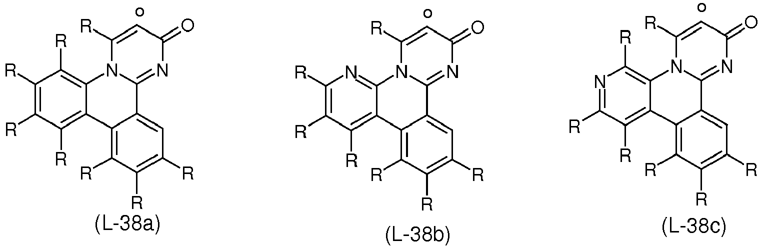 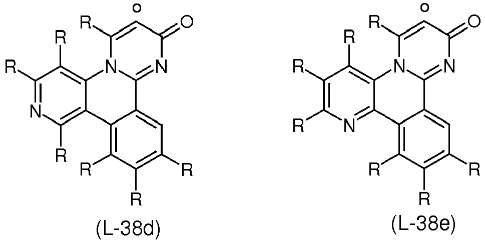 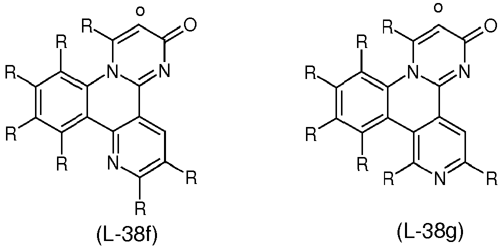 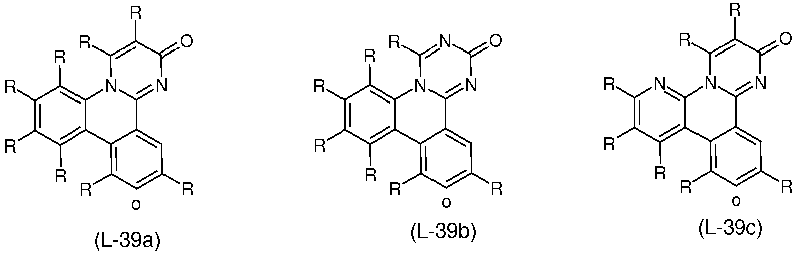 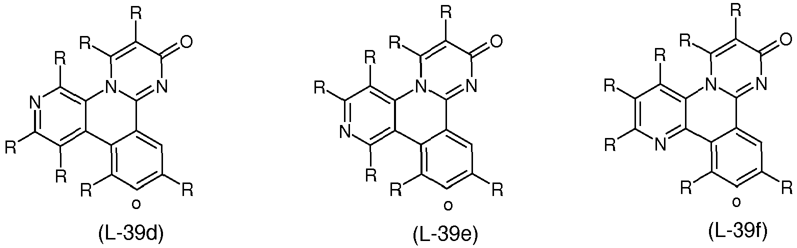 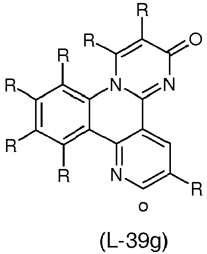 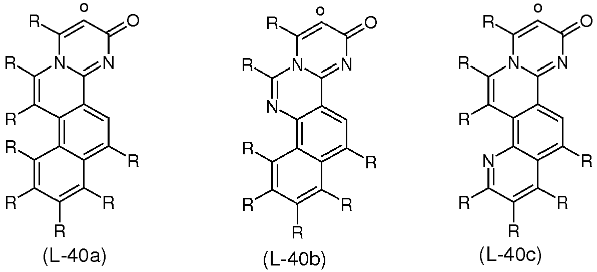 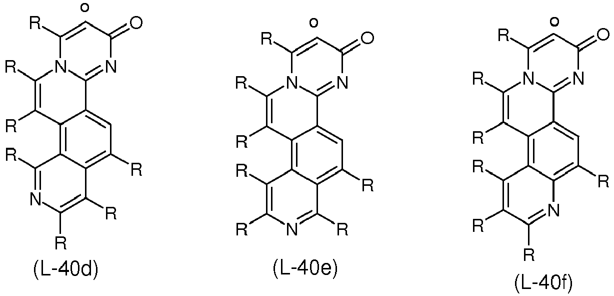 wobei die verwendeten Symbole die oben genannten Bedeutungen aufweisen und "o" die Position der Verknüpfung mit der Gruppe der Formeln (1) bis (6) bzw. den bevorzugten Ausführungsformen anzeigt.

In einer bevorzugten Ausführungsform der Erfindung steht die Gruppe X, die in ortho-Position zur Koordination an das Metall vorliegt, für CR. Dabei ist in dieser Rest R, der in ortho-Position zur Koordination an das Metall gebunden ist, bevorzugt ausgewählt aus der Gruppe bestehend aus H, D, F und Methyl.

In einer weiteren Ausführungsform der Erfindung ist es bevorzugt, falls eines der Atome X oder, wenn vorhanden, Y für N steht, wenn benachbart zu diesem Stickstoffatom eine Gruppe R als Substituent gebunden ist, welche ungleich Wasserstoff oder Deuterium ist.

Dabei ist dieser Substituent R bevorzugt eine Gruppe, ausgewählt aus CF₃, OCF₃, Alkyl- oder Alkoxygruppen mit 1 bis 10 C-Atomen, insbesondere verzweigten oder cyclischen Alkyl- oder Alkoxygruppen mit 3 bis 10 C-Atomen, einer Dialkylaminogruppe mit 2 bis 10 C-Atomen, aromatischen bzw. heteroaromatischen Ringsystemen oder Aralkyl- bzw. Heteroaralkylgruppen. Es handelt sich bei diesen Gruppen um sterisch anspruchsvolle Gruppen. Weiterhin bevorzugt kann dieser Rest R auch mit einem benachbarten Rest R einen Cyclus bilden.

Wenn das Metall des erfindungsgemäßen Komplexes für ein Hauptgruppenmetall, insbesondere für Al oder Ga, steht, so ist bevorzugt mindestens einer der bidentaten Teilliganden bei jedem Auftreten, bevorzugt mindestens zwei der bidentaten Teilliganden, besonders bevorzugt alle drei bidentaten Teilliganden, gleich oder verschieden ausgewählt aus den Teilliganden der folgenden Formeln (L-41) bis (L-45), 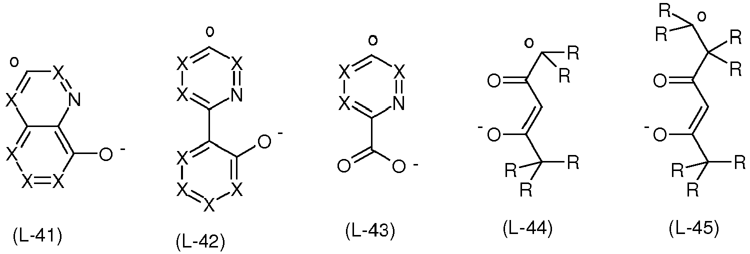 wobei die Teilliganden (L-41) bis (L-43) jeweils über das explizit eingezeichnete Stickstoffatom und das negativ geladene Sauerstoffatom und der Teilligand (L-44) über die beiden Sauerstoffatome an das Metall koordinieren, X die oben genannten Bedeutungen aufweist und "o" die Position angibt, über die der Teilligand mit der Gruppe der Formel (1) bis (6) bzw. den bevorzugten Ausführungsformen verknüpft ist.

Diese Teilliganden können auch bevorzugt sein für Übergangsmetalle in Kombination mit zwei Teilliganden, welche über ein Kohlenstoffatom und ein Stickstoffatom oder über zwei Kohlenstoffatome an das Metall koordinieren, insbesondere die Teilliganden (L-1) bis (L-40).

In den Teilliganden der Formeln (L-41) bis (L-43) stehen bevorzugt maximal zwei Symbole X für N, besonders bevorzugt maximal ein Symbol X. Ganz besonders bevorzugt stehen alle Symbole X für CR. Bevorzugte Teilliganden der Formeln (L-41) bis (L-43) sind daher die Teilliganden der folgenden Formeln (L-41a) bis (L-43a), 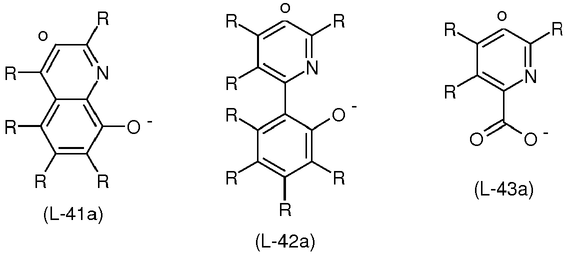 wobei die verwendeten Symbole die oben genannten Bedeutungen aufweisen und "o" die Position angibt, über die der Teilligand mit der Gruppe der Formel (1) bis (6) bzw. den bevorzugten Ausführungsformen verknüpft ist.

Besonders bevorzugt steht in diesen Formeln R für Wasserstoff, wobei "o" die Position angibt, über die der Teilligand mit der Gruppe der Formel (1) bis (6) bzw. den bevorzugten Ausführungsformen verknüpft ist, so dass es sich um die Strukturen der folgenden Formeln (L-41b) bis (L-43b) handelt, 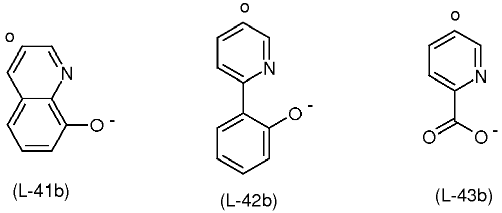 wobei die verwendeten Symbole die oben genannten Bedeutungen aufweisen.

Im Folgenden werden bevorzugte Substituenten beschrieben, wie sie an den oben beschriebenen Teilliganden vorliegen können. Diese Substituenten können weiterhin auch als Substituenten an der Gruppe X³ vorliegen. Insbesondere ist es auch bevorzugt, wenn die nachfolgend beschriebenen aliphatischen bzw. heteroaliphatischen Ringstrukturen an den Gruppen X³ vorliegen.

In einer bevorzugten Ausführungsform der Erfindung enthält der erfindungsgemäße Metallkomplex zwei Substituenten R, die an benachbarte Kohlenstoffatome gebunden sind und die miteinander einen aliphatischen oder heteroaliphatischen Ring gemäß einer der nachfolgend beschriebenen Formeln bilden. Dabei können die beiden Substituenten R, die diesen aliphatischen Ring bilden, an einem oder mehreren der bidentaten Teilliganden vorliegen. Ebenso können die beiden Substituenten R an einer oder an mehreren der Gruppen X³ vorliegen. Der aliphatische bzw. heteroaliphatische Ring, der durch die Ringbildung von zwei Substituenten R miteinander gebildet wird, wird bevorzugt durch eine der folgenden Formeln (43) bis (49) beschrieben, 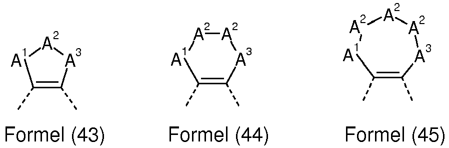 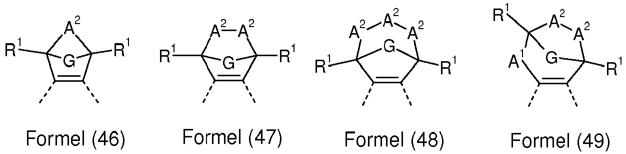 wobei R¹ und R² die oben genannten Bedeutungen aufweisen, die gestrichelten Bindungen die Verknüpfung der beiden Kohlenstoffatome im Liganden andeuten und weiterhin gilt:
- A¹, A³: ist gleich oder verschieden bei jedem Auftreten C(R³)₂, O, S, NR³ oder C(=O);
- A²: ist gleich oder verschieden bei jedem Auftreten C(R¹)₂, O, S, NR³ oder C(=O);
- G: ist eine Alkylengruppe mit 1, 2 oder 3 C-Atomen, welche mit einem oder mehreren Resten R² substituiert sein kann, -CR²=CR²- oder eine ortho-verknüpfte Arylen- oder Heteroarylengruppe mit 5 bis 14 aromatischen Ringatomen, welche durch einen oder mehrere Reste R² substituiert sein kann;
- R³: ist gleich oder verschieden bei jedem Auftreten H, D, F, eine geradkettige Alkyl- oder Alkoxygruppe mit 1 bis 10 C-Atomen, eine verzweigte oder cyclische Alkyl- oder Alkoxygruppe mit 3 bis 10 C-Atomen, wobei die Alkyl- oder Alkoxygruppe jeweils mit einem oder mehreren Resten R² substituiert sein kann, wobei eine oder mehrere nicht benachbarte CH₂-Gruppen durch R²C=CR², C=C, Si(R²)₂, C=O, NR², O, S oder CONR² ersetzt sein können, oder ein aromatisches oder heteroaromatisches Ringsystem mit 5 bis 24 aromatischen Ringatomen, das jeweils durch einen oder mehrere Reste R² substituiert sein kann, oder eine Aryloxy- oder Heteroaryloxygruppe mit 5 bis 24 aromatischen Ringatomen, die durch einen oder mehrere Reste R² substituiert sein kann; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden;
mit der Maßgabe, dass in diesen Gruppen nicht zwei Heteroatome direkt aneinander gebunden sind und nicht zwei Gruppen C=O direkt aneinander gebunden sind.

In einer bevorzugten Ausführungsform der Erfindung ist R³ ungleich H oder D.

In den oben abgebildeten Strukturen der Formeln (43) bis (49) sowie den weiteren als bevorzugt genannten Ausführungsformen dieser Strukturen wird formal eine Doppelbindung zwischen den zwei Kohlenstoffatomen abgebildet. Dies stellt eine Vereinfachung der chemischen Struktur dar, wenn diese beiden Kohlenstoffatome in ein aromatisches oder heteroaromatisches System eingebunden sind und somit die Bindung zwischen diesen beiden Kohlenstoffatomen formal zwischen dem Bindungsgrad einer Einfachbindung und dem einer Doppelbindung liegt. Das Einzeichnen der formalen Doppelbindung ist somit nicht limitierend für die Struktur auszulegen, sondern es ist für den Fachmann offensichtlich, dass es sich hier um eine aromatische Bindung handelt.

Wenn benachbarte Reste in den erfindungsgemäßen Strukturen ein aliphatisches Ringsystem bilden, dann ist es bevorzugt, wenn dieses keine aziden benzylischen Protonen aufweist. Unter benzylischen Protonen werden Protonen verstanden, die an ein Kohlenstoffatom binden, welches direkt an den Liganden gebunden ist. Dies kann dadurch erreicht werden, dass die Kohlenstoffatome des aliphatischen Ringsystems, die direkt an eine Aryl- oder Heteroarylgruppe binden, vollständig substituiert sind und keine Wasserstoffatome gebunden enthalten. So wird die Abwesenheit von aziden benzylischen Protonen in den Formeln (43) bis (45) dadurch erreicht, dass A¹ und A³, wenn diese für C(R³)₂ stehen, so definiert sind, dass R³ ungleich Wasserstoff ist. Dies kann weiterhin auch dadurch erreicht werden, dass die Kohlenstoffatome des aliphatischen Ringsystems, die direkt an eine Aryl- oder Heteroarylgruppe binden, die Brückenköpfe einer bi- oder polycyclischen Struktur sind. Die an Brückenkopfkohlenstoffatome gebundenen Protonen sind aufgrund der räumlichen Struktur des Bi- oder Polycyclus wesentlich weniger azide als benzylische Protonen an Kohlenstoffatomen, die nicht in einer bi- oder polycyclischen Struktur gebunden sind, und werden im Sinne der vorliegenden Erfindung als nicht-azide Protonen angesehen. So wird die Abwesenheit von aziden benzylischen Protonen in den Formeln (46) bis (49) dadurch erreicht, dass es sich dabei um eine bicyclische Struktur handelt, wodurch R¹, wenn es für H steht, deutlich weniger azide als benzylische Protonen, da das korrespondierende Anion der bicyclischen Struktur nicht mesomeriestabilisiert ist. Auch wenn R¹ in Formeln (46) bis (49) für H steht, handelt es sich dabei daher um ein nicht-azides Proton im Sinne der vorliegenden Anmeldung.

In einer bevorzugten Ausführungsform der Struktur gemäß Formel (43) bis (49) steht maximal eine der Gruppen A¹, A² und A³ für ein Heteroatom, insbesondere für O oder NR³, und die anderen Gruppen stehen für C(R³)₂ bzw. C(R¹)₂ oder A¹ und A³ stehen gleich oder verschieden bei jedem Auftreten für O oder NR³ und A² steht für C(R¹)₂. In einer besonders bevorzugten Ausführungsform der Erfindung stehen A¹ und A³ gleich oder verschieden bei jedem Auftreten für C(R³)₂ und A² steht für C(R¹)₂ und besonders bevorzugt für C(R³)₂ oder CH₂.

Bevorzugte Ausführungsformen der Formel (43) sind somit die Strukturen der Formel (43-A), (43-B), (43-C) und (43-D), und eine besonders bevorzugte Ausführungsform der Formel (43-A) sind die Strukturen der Formel (43-E) und (43-F), 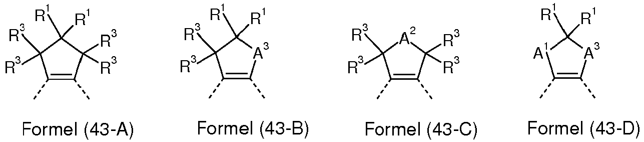 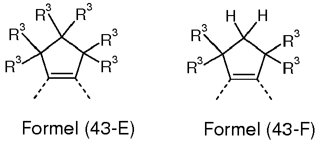 wobei R¹ und R³ die oben genannten Bedeutungen aufweisen und A¹, A² und A³ gleich oder verschieden bei jedem Auftreten für O oder NR³ steht.

Bevorzugte Ausführungsformen der Formel (44) sind die Strukturen der folgenden Formeln (44-A) bis (44-F), 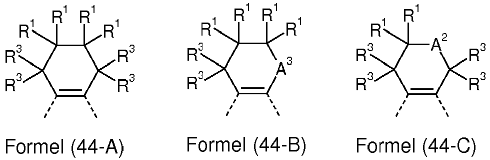 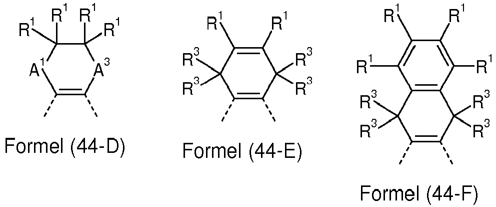 wobei R¹ und R³ die oben genannten Bedeutungen aufweisen und A¹, A² und A³ gleich oder verschieden bei jedem Auftreten für O oder NR³ steht.

Bevorzugte Ausführungsformen der Formel (45) sind die Strukturen der folgenden Formeln (45-A) bis (45-E), 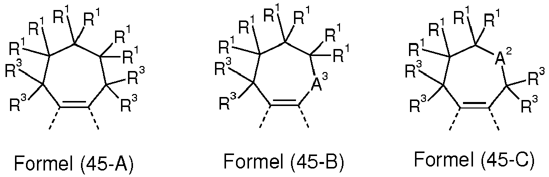 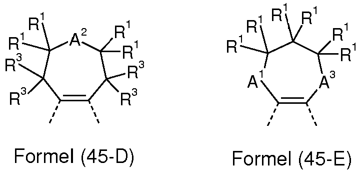 wobei R¹ und R³ die oben genannten Bedeutungen aufweisen und A¹, A² und A³ gleich oder verschieden bei jedem Auftreten für O oder NR³ steht.

In einer bevorzugten Ausführungsform der Struktur gemäß Formel (46) stehen die Reste R¹, die an den Brückenkopf gebunden sind, für H, D, F oder CH₃. Weiterhin bevorzugt steht A² für C(R¹)₂ oder O, und besonders bevorzugt für C(R³)₂. Bevorzugte Ausführungsformen der Formel (46) sind somit Strukturen der Formel (46-A) und (46-B), und eine besonders bevorzugte Ausführungsform der Formel (46) ist eine Struktur der Formel (46-C), 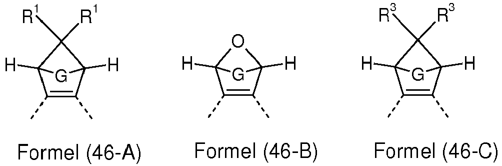 wobei die verwendeten Symbole die oben genannten Bedeutungen aufweisen.

In einer bevorzugten Ausführungsform der Struktur gemäß Formel (47), (48) und (49) stehen die Reste R¹, die an den Brückenkopf gebunden sind, für H, D, F oder CH₃. Weiterhin bevorzugt steht A² für C(R¹)₂. Bevorzugte Ausführungsformen der Formel (47), (48) und (49) sind somit die Strukturen der Formeln (47-A), (48-A) und (49-A), 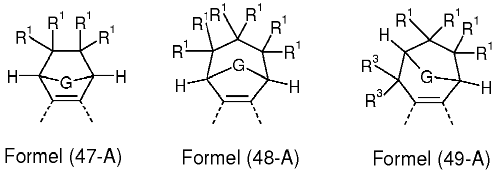 wobei die verwendeten Symbole die oben genannten Bedeutungen aufweisen.

Weiterhin bevorzugt steht die Gruppe G in den Formeln (46), (46-A), (46-B), (46-C), (47), (47-A), (48), (48-A), (49) und (49-A) für eine 1,2-Ethylengruppe, welche mit einem oder mehreren Resten R² substituiert sein kann, wobei R² bevorzugt gleich oder verschieden bei jedem Auftreten für H oder eine Alkylgruppe mit 1 bis 4 C-Atomen steht, oder eine ortho-Arylengruppe mit 6 bis 10 C-Atomen, welche mit einem oder mehreren Resten R² substituiert sein kann, bevorzugt aber unsubstituiert ist, insbesondere eine ortho-Phenylengruppe, welche mit einem oder mehreren Resten R² substituiert sein kann, bevorzugt aber unsubstituiert ist.

In einer weiteren bevorzugten Ausführungsform der Erfindung steht R³ in den Gruppen der Formeln (43) bis (49) und in den bevorzugten Ausführungsformen gleich oder verschieden bei jedem Auftreten für F, eine geradkettige Alkylgruppe mit 1 bis 10 C-Atomen oder eine verzweigte oder cyclische Alkylgruppe mit 3 bis 20 C-Atomen, wobei jeweils eine oder mehrere nicht benachbarte CH₂-Gruppen durch R²C=CR² ersetzt sein können und ein oder mehrere H-Atome durch D oder F ersetzt sein können, oder ein aromatisches oder heteroaromatisches Ringsystem mit 5 bis 14 aromatischen Ringatomen, das jeweils durch einen oder mehrere Reste R² substituiert sein kann; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden.

In einer besonders bevorzugten Ausführungsform der Erfindung steht R³ in den Gruppen der Formeln (43) bis (49) und in den bevorzugten Ausführungsformen gleich oder verschieden bei jedem Auftreten für F, eine geradkettige Alkylgruppe mit 1 bis 3 C-Atomen, insbesondere Methyl, oder ein aromatisches oder heteroaromatisches Ringsystem mit 5 bis 12 aromatischen Ringatomen, das jeweils durch einen oder mehrere Reste R² substituiert sein kann, bevorzugt aber unsubstituiert ist; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden.

Beispiele für besonders geeignete Gruppen der Formel (43) sind die im Folgenden aufgeführten Strukturen:

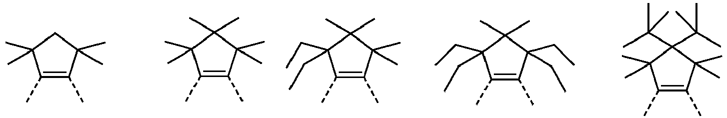

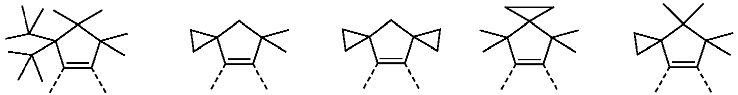

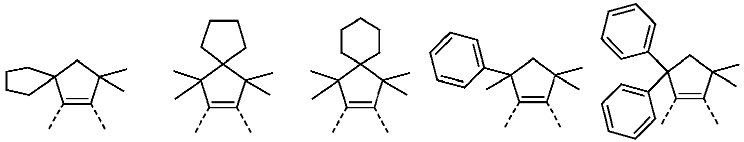

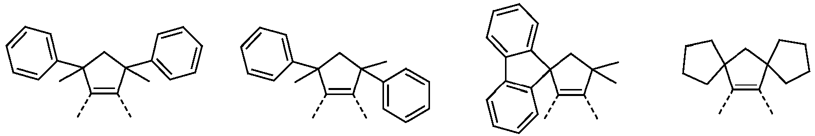

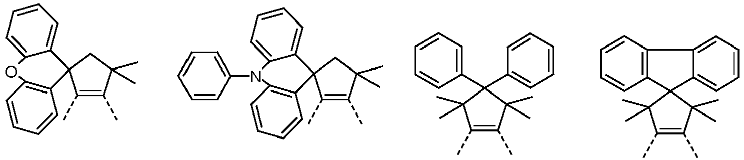

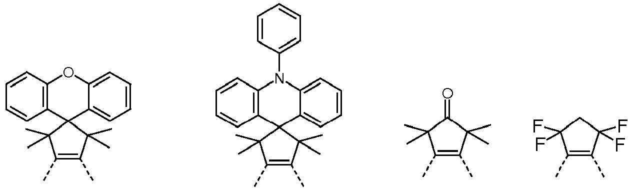

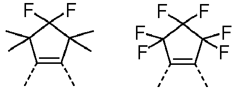

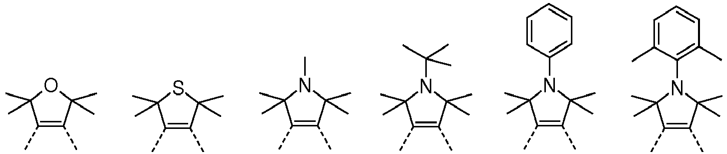

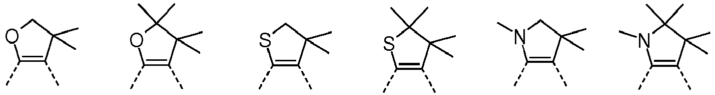

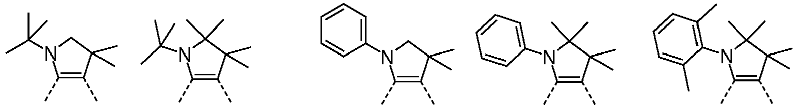

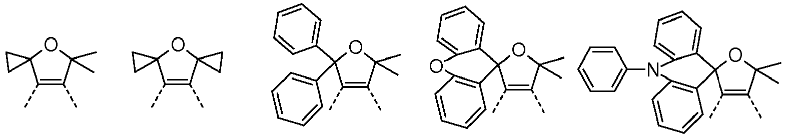

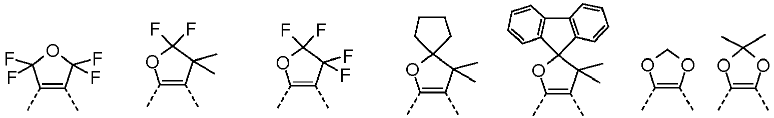

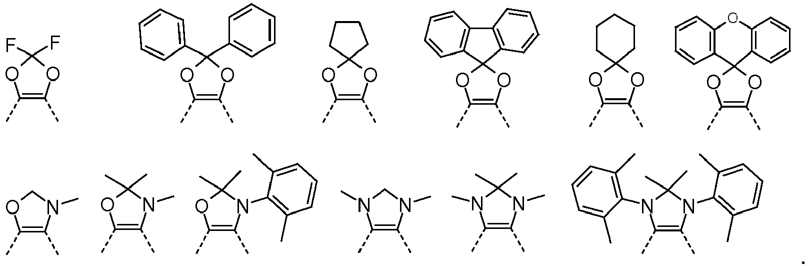

Beispiele für besonders geeignete Gruppen der Formel (43) sind die im Folgenden aufgeführten Strukturen:

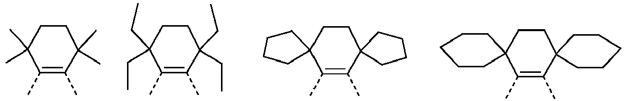

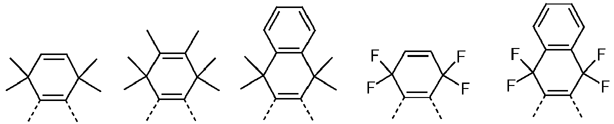

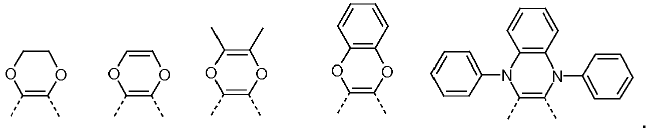

Beispiele für besonders geeignete Gruppen der Formel (45), (48) und (49) sind die im Folgenden aufgeführten Strukturen:

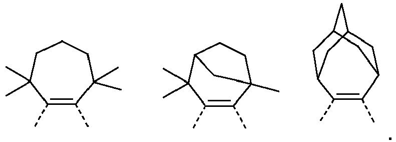

Beispiele für besonders geeignete Gruppen der Formel (46) sind die im Folgenden aufgeführten Strukturen:

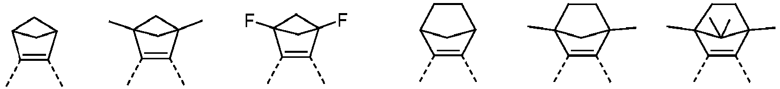

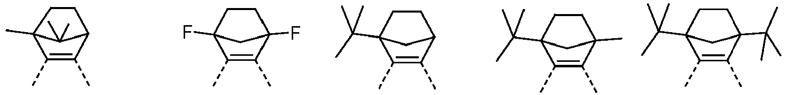

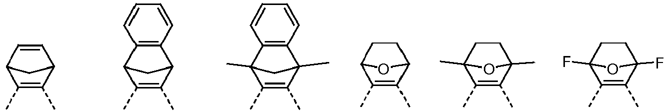

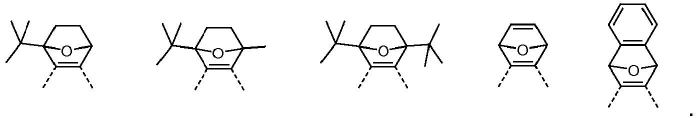

Beispiele für besonders geeignete Gruppen der Formel (47) sind die im Folgenden aufgeführten Strukturen:

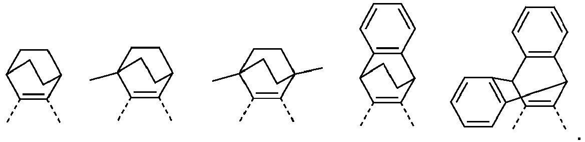

Wenn in den bidentaten Teilliganden Reste R gebunden sind, so sind diese Reste R bei jedem Auftreten gleich oder verschieden bevorzugt ausgewählt aus der Gruppe bestehend aus H, D, F, Br, I, N(R¹)₂, CN, Si(R¹)₃, B(OR¹)₂, C(=O)R¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl oder Alkenylgruppe jeweils mit einem oder mehreren Resten R¹ substituiert sein kann, oder einem aromatischen oder heteroaromatischen Ringsystem mit 5 bis 30 aromatischen Ringatomen, das jeweils durch einen oder mehrere Reste R¹ substituiert sein kann; dabei können zwei benachbarte Rest R oder R mit R¹ auch miteinander ein mono- oder polycyclisches, aliphatisches oder aromatisches Ringsystem bilden. Besonders bevorzugt sind diese Reste R bei jedem Auftreten gleich oder verschieden ausgewählt aus der Gruppe bestehend aus H, D, F, N(R¹)₂, einer geradkettigen Alkylgruppe mit 1 bis 6 C-Atomen, insbesondere mit 1 bis 4 C-Atomen, oder einer verzweigten oder cyclischen Alkylgruppe mit 3 bis 10 C-Atomen, insbesondere mit 3 bis 6 C-Atomen, wobei ein oder mehrere H-Atome durch D oder F ersetzt sein können, oder einem aromatischen oder heteroaromatischen Ringsystem mit 5 bis 24 aromatischen Ringatomen, insbesondere mit 6 bis 13 aromatischen Ringatomen, das jeweils durch einen oder mehrere Reste R¹ substituiert sein kann; dabei können zwei benachbarte Reste R oder R mit R¹ auch miteinander ein mono- oder polycyclisches, aliphatisches oder aromatisches Ringsystem bilden.

Bevorzugte Reste R¹, die an R gebunden sind, sind bei jedem Auftreten gleich oder verschieden H, D, F, N(R²)₂, CN, eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkylgruppe jeweils mit einem oder mehreren Resten R² substituiert sein kann, oder ein aromatisches oder heteroaromatisches Ringsystem mit 5 bis 24 aromatischen Ringatomen, das jeweils durch einen oder mehrere Reste R² substituiert sein kann; dabei können zwei oder mehrere benachbarte Reste R¹ miteinander ein mono- oder polycyclisches, aliphatisches Ringsystem bilden. Besonders bevorzugte Reste R¹, die an R gebunden sind, sind bei jedem Auftreten gleich oder verschieden H, F, CN, eine geradkettige Alkylgruppe mit 1 bis 5 C-Atomen oder eine verzweigte oder cyclische Alkylgruppe mit 3 bis 5 C-Atomen, die jeweils mit einem oder mehreren Resten R² substituiert sein kann, oder ein aromatisches oder heteroaromatisches Ringsystem mit 5 bis 13 aromatischen Ringatomen, das jeweils durch einen oder mehrere Reste R² substituiert sein kann; dabei können zwei oder mehrere benachbarte Reste R¹ miteinander ein mono- oder polycyclisches, aliphatisches Ringsystem bilden.

Bevorzugte Reste R² sind bei jedem Auftreten gleich oder verschieden H, F oder ein aliphatischer Kohlenwasserstoffrest mit 1 bis 5 C-Atomen oder ein aromatischer Kohlenwasserstoffrest mit 6 bis 12 C-Atomen; dabei können zwei oder mehrere Substituenten R² auch miteinander ein mono- oder polycyclisches, aliphatisches Ringsystem bilden.

Bei den erfindungsgemäßen Metallkomplexen kann es sich abhängig von der Konfiguration der Brücke um chirale Strukturen handeln. Wenn zusätzlich auch der tripodale Ligand der Komplexe chiral ist, ist die Bildung von Diastereomeren und mehreren Enantiomerenpaaren möglich. Die erfindungsgemäßen Komplexe umfassen dann sowohl die Mischungen der verschiedenen Diastereomere bzw. die entsprechenden Racemate wie auch die einzelnen isolierten Diastereomere bzw. Enantiomere.

Werden in der ortho-Metallierung C₃- bzw. C₃ᵥ-symmetrische Liganden eingesetzt, fällt üblicherweise ein racemisches Gemisch der C₃-symmetrischen Komplexe, also des Δ- und des Λ-Enantiomers, an. Diese können durch gängige Methoden (Chromatographie an chiralen Materialien / Säulen oder Racemattrennung durch Kristallisation) getrennt werden. Dies ist im folgenden Schema am Beispiel eines C₃-symmetrischen Liganden, der drei Phenylpyridin-Teilliganden trägt, gezeigt und gilt in analoger Form auch für alle anderen C₃- bzw. C₃ᵥ-symmetrischen Liganden.

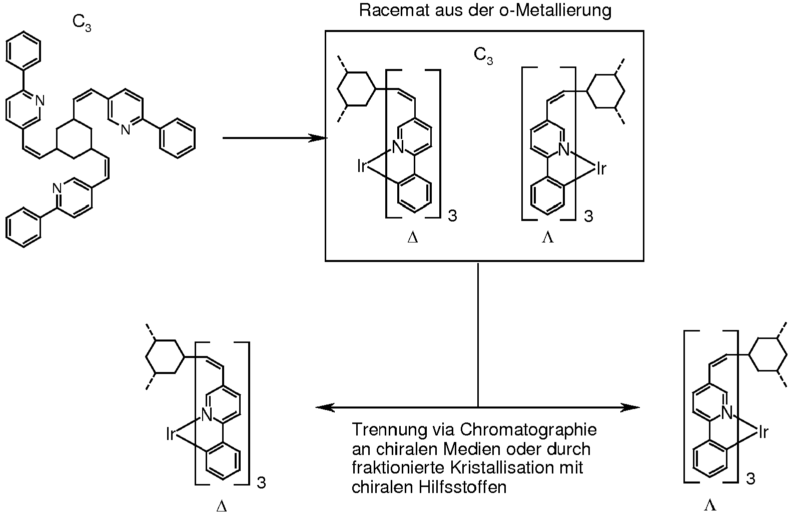

Die Racemattrennung via fraktionierter Kristallisation von diastereomeren Salzpaaren kann nach üblichen Methoden erfolgen. Hierzu bietet es sich an, die neutralen Ir(III)-Komplexe zu oxidieren (z. B. mit Peroxiden, H₂O₂ oder elektrochemisch), die so erzeugten kationischen Ir(IV)-Komplexe mit dem Salz einer enantiomerenreinen, monoanionischen Base (chirale Base) zu versetzen, die so erzeugten diasteromeren Salze durch fraktionierte Kristallisation zu trennen und diese dann mit Hilfe eines Reduktionsmittels (z. B. Zink, Hydrazinhydrat, Ascorbinsäure, etc.) zu den enantiomerenreinen neutralen Komplex zu reduzieren, wie im Folgenden schematisch dargestellt.

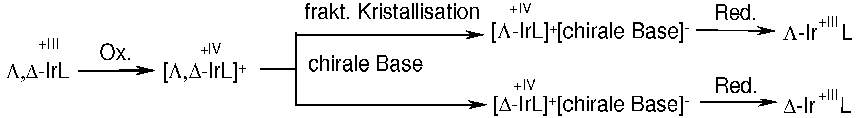

Daneben ist eine enantiomerenreine bzw. enantiomerenanreichernde Synthese durch Komplexierung in einem chiralen Medium (z. B. R- oder S-1,1-Binaphthol) möglich.

Analoge Verfahren können auch mit Komplexen Cₛ-symmetrischer Liganden durchgeführt werden.

Werden in der Komplexierung C₁-symmetrische Liganden eingesetzt, fällt üblicherweise ein Diastereomerengemisch der Komplexe an, das durch gängige Methoden (Chromatographie, Kristallisation) getrennt werden kann.

Enantiomerenreine C₃-symmetrische Komplexe können auch gezielt synthetisiert werden. Dazu wird ein enantiomerenreiner, C₃-symmetrischer Ligand dargestellt, komplexiert, das erhaltene Diasteroemerengemisch wird getrennt, und anschließend wird die chirale Gruppe abgespalten.

Die oben genannten bevorzugten Ausführungsformen sind beliebig miteinander kombinierbar. In einer besonders bevorzugten Ausführungsform der Erfindung gelten die oben genannten bevorzugten Ausführungsformen gleichzeitig.

Die erfindungsgemäßen Metallkomplexe sind prinzipiell durch verschiedene Verfahren darstellbar. Generell wird hierzu ein Metallsalz mit dem entsprechenden freien Liganden umgesetzt.

Daher ist ein weiterer Gegenstand der vorliegenden Erfindung ein Verfahren zur Herstellung der erfindungsgemäßen Metallkomplexe durch Umsetzung der entsprechenden freien Liganden mit Metallalkoholaten der Formel (50), mit Metallketoketonaten der Formel (51), mit Metallhalogeniden der Formel (52) oder mit Metallcarboxylaten der Formel (53), 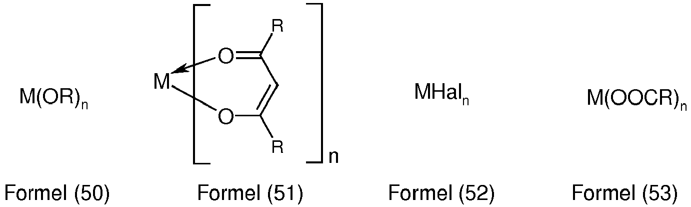 wobei M für das Metall des erfindungsgemäßen Metallkomplexes, der synthetisiert wird, steht, n für die Wertigkeit des Metalls M steht, R die oben angegebenen Bedeutungen hat, Hal = F, Cl, Br oder I ist und die Metalledukte auch als die entsprechenden Hydrate vorliegen können. Dabei steht R bevorzugt für Gruppen gemäß R², besonders bevorzugt für eine Alkylgruppe mit 1 bis 4 C-Atomen.

Es können ebenfalls Metallverbindungen, insbesondere Iridiumverbindungen, die sowohl Alkoholat- und/oder Halogenid- und/oder Hydroxy- wie auch Ketoketonatreste tragen, verwendet werden. Diese Verbindungen können auch geladen sein. Entsprechende Iridiumverbindungen, die als Edukte besonders geeignet sind, sind in WO 2004/085449 offenbart. Besonders geeignet sind [IrCl₂(acac)₂]⁻, beispielsweise Na[IrCl₂(acac)₂], Metallkomplexe mit Acetylacetonat-Derivaten als Ligand, beispielsweise Ir(acac)₃ oder Tris(2,2,6,6-Tetramethylheptan-3,5-dionato)iridium, und IrCl₃·xH₂O, wobei x üblicherweise für eine Zahl zwischen 2 und 4 steht. Die Synthese der Komplexe wird bevorzugt durchgeführt wie in WO 2002/060910 und in WO 2004/085449 beschrieben. Dabei kann die Synthese beispielsweise auch thermisch, photochemisch und/oder durch Mikrowellenstrahlung aktiviert werden. Weiterhin kann die Synthese auch im Autoklaven bei erhöhtem Druck und/oder erhöhter Temperatur durchgeführt werden.

Die Reaktionen können ohne Zusatz von Lösemitteln oder Schmelzhilfen in einer Schmelze der entsprechenden zu o-metallierenden Liganden durchgeführt werden. Gegebenenfalls können Lösemittel oder Schmelzhilfen zugesetzt werden. Geeignete Lösemittel sind protische oder aprotische Lösemittel, wie aliphatische und / oder aromatische Alkohole (Methanol, Ethanol, iso-Propanol, t-Butanol, etc.), Oligo- und Polyalkohole (Ethylenglykol, 1,2-Propandiol, Glycerin, etc.), Alkoholether (Ethoxyethanol, Diethylenglykol, Triethylenglycol, Polyethylenglykol, etc.), Ether (Di- und Triethylenglykoldimethylether, Diphenylether, etc.), aromatische, heteroaromatische und oder aliphatische Kohlenwasserstoffe (Toluol, Xylol, Mesitylen, Chlorbenzol, Pyridin, Lutidin, Chinolin, iso-Chinolin, Tridecan, Hexadecan, etc.), Amide (DMF, DMAC, etc.), Lactame (NMP), Sulfoxide (DMSO) oder Sulfone (Dimethylsulfon, Sulfolan, etc.). Geeignete Schmelzhilfen sind Verbindungen, die bei Rautemperatur fest vorliegen, jedoch beim Erwärmen der Reaktionsmischung schmelzen und die Reaktanden lösen, so dass eine homogene Schmelze entsteht. Besonders geeignet sind Biphenyl, m-Terphenyl, Triphenylen, R- oder S-Binaphthol oder auch das entsprechende Racemat, 1,2-, 1,3-, 1,4-Bis-phenoxybenzol, Triphenylphosphinoxid, 18-Krone-6, Phenol, 1-Naphthol, Hydrochinon, etc.. Dabei ist die Verwendung von Hydrochinon besonders bevorzugt.

Durch diese Verfahren, gegebenenfalls gefolgt von Aufreinigung, wie z. B. Umkristallisation oder Sublimation, lassen sich die erfindungsgemäßen Verbindungen gemäß Formel (1) in hoher Reinheit, bevorzugt mehr als 99 % (bestimmt mittels ¹H-NMR und/oder HPLC) erhalten.

Die erfindungsgemäßen Metallkomplexe können auch durch geeignete Substitution, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, löslich gemacht werden. Insbesondere auch die Verwendung von ankondensierten aliphatischen Gruppen, wie sie beispielsweise durch die oben offenbarten Formeln (43) bis (49) dargestellt werden, führt zu einer deutlichen Verbesserung der Löslichkeit der Metallkomplexe. Solche Verbindungen sind dann in gängigen organischen Lösemitteln, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren.

Die erfindungsgemäßen Metallkomplexe können auch mit einem Polymer gemischt werden. Ebenso ist es möglich, diese Metallkomplexe kovalent in ein Polymer einzubauen. Dies ist insbesondere möglich mit Verbindungen, welche mit reaktiven Abgangsgruppen, wie Brom, lod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Metallkomplexe, wobei statt eines oder mehrerer Wasserstoffatome und/oder Substituenten ein oder mehrere Bindungen des erfindungsgemäßen Metallkomplexes zum Polymer, Oligomer oder Dendrimer vorhanden sind. Je nach Verknüpfung des erfindungsgemäßen Metallkomplexes bildet diese daher eine Seitenkette des Oligomers oder Polymers oder ist in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Metallkomplexe in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die erfindungsgemäßen Metallkomplexe zu 0.01 bis 99.9 mol%, bevorzugt 5 bis 90 mol%, besonders bevorzugt 5 bis 50 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Für die Verarbeitung der erfindungsgemäßen Metallkomplexe aus flüssiger Phase, beispielsweise durch Spin-Coating oder durch Druckverfahren, sind Formulierungen der erfindungsgemäßen Metallkomplexe erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend mindestens einen erfindungsgemäßen Metallkomplex bzw. mindestens ein erfindungsgemäßes Oligomer, Polymer oder Dendrimer und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Der oben beschriebene erfindungsgemäße Metallkomplex bzw. die oben aufgeführten bevorzugten Ausführungsformen können in der elektronischen Vorrichtung als aktive Komponente oder als Sauerstoff-Sensibilisatoren verwendet werden. Ein weiterer Gegenstand der vorliegenden Erfindung ist somit die Verwendung einer erfindungsgemäßen Verbindung in einer elektronischen Vorrichtung oder als Sauerstoff-Sensibilisator. Nochmals ein weiterer Gegenstand der vorliegenden Erfindung ist eine elektronische Vorrichtung enthaltend mindestens eine erfindungsgemäße Verbindung.

Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens einen erfindungsgemäßen Metallkomplex enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), wobei hierunter sowohl rein organische Solarzellen wie auch farbstoffsensibilisierte Solarzellen verstanden werden, organischen optischen Detektoren, organischen Photorezeptoren, organischen Feld-Quench-Devices (O-FQDs), lichtemittierenden elektrochemischen Zellen (LECs), Sauerstoff-Sensoren oder organischen Laserdioden (O-Laser), enthaltend in mindestens einer Schicht mindestens einen erfindungsgemäßen Metallkomplex. Besonders bevorzugt sind organische Elektrolumineszenzvorrichtungen. Dies gilt insbesondere, wenn das Metall Iridium oder Aluminium ist. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäßen Verbindungen zeigen besonders gute Eigenschaften als Emissionsmaterial in organischen Elektrolumineszenzvorrichtungen. Eine bevorzugte Ausführungsform der Erfindung sind daher organische Elektrolumineszenzvorrichtungen. Weiterhin können die erfindungsgemäßen Verbindungen zur Erzeugung von Singulett-Sauerstoff oder in der Photokatalyse eingesetzt werden. Insbesondere wenn das Metall Ruthenium ist, ist der Einsatz als Photosensibilisator in einer farbstoffsensibilisierten Solarzelle ("Grätzel-Zelle") bevorzugt.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoO₃ oder WO₃ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren. Eine weitere Ausführungsform für weiß emittierende OLEDs sind Tandem-OLEDs. Weiß emittierende organische Elektrolumineszenzvorrichtungen können für Beleuchtungsanwendungen oder mit Farbfilter auch für Vollfarb-Displays verwendet werden.

In einer bevorzugten Ausführungsform der Erfindung enthält die organische Elektrolumineszenzvorrichtung den erfindungsgemäßen Metallkomplex als emittierende Verbindung in einer oder mehreren emittierenden Schichten.

Wenn der erfindungsgemäße Metallkomplex als emittierende Verbindung in einer emittierenden Schicht eingesetzt wird, wird er bevorzugt in Kombination mit einem oder mehreren Matrixmaterialien eingesetzt. Die Mischung aus dem erfindungsgemäßen Metallkomplex und dem Matrixmaterial enthält zwischen 0.1 und 99 Vol.-%, vorzugsweise zwischen 1 und 90 Vol.-%, besonders bevorzugt zwischen 3 und 40 Vol.-%, insbesondere zwischen 5 und 15 Vol.-% des erfindungsgemäßen Metallkomplexes bezogen auf die Gesamtmischung aus Emitter und Matrixmaterial. Entsprechend enthält die Mischung zwischen 99.9 und 1 Vol.-%, vorzugsweise zwischen 99 und 10 Vol.-%, besonders bevorzugt zwischen 97 und 60 Vol.-%, insbesondere zwischen 95 und 85 Vol.-% des Matrixmaterials bezogen auf die Gesamtmischung aus Emitter und Matrixmaterial.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015 oder WO 2015/169412, oder verbrückte Carbazolderivate, z. B. gemäß US 2009/0136779, WO 2010/050778, WO 2011/042107 oder WO 2011/088877.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Eine bevorzugte Kombination ist beispielsweise die Verwendung eines aromatischen Ketons, eines Triazin-Derivats oder eines Phosphinoxid-Derivats mit einem Triarylamin-Derivat oder einem Carbazol-Derivat als gemischte Matrix für den erfindungsgemäßen Metallkomplex. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben. Ebenso bevorzugt ist die Verwendung von zwei elektronentransportierenden Matrixmaterialien, beispielsweise Triazinderivaten und Lactamderivaten, wie z. B. in WO 2014/094964 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum. So können beispielsweise die erfindungsgemäßen Metallkomplexe als Co-Matrix für längerwellig emittierende Triplettemitter, beispielsweise für grün oder rot emittierende Triplettemitter, eingesetzt werden. Dabei kann es auch bevorzugt sein, wenn sowohl der kürzerwellig wie auch der längerwellig emittierende Metallkomplex eine erfindungsgemäße Verbindung ist.

Die erfindungsgemäßen Metallkomplexe lassen sich auch in anderen Funktionen in der elektronischen Vorrichtung einsetzen, beispielsweise als Lochtransportmaterial oder p-Dotand in einer Lochinjektions- oder -transportschicht, als Ladungserzeugungsmaterial, als Elektronenblockiermaterial, als Lochblockiermaterial oder als Elektronentransportmaterial oder n-Dotand, beispielsweise in einer Elektronentransportschicht, je nach Wahl des Metalls und genauer Struktur des Liganden. Wenn es sich bei dem erfindungsgemäßen Metallkomplex um einen Aluminiumkomplex handelt, so wird dieser bevorzugt in einer Elektronentransportschicht oder einer Lochblockierschicht eingesetzt. Ebenso lassen sich die erfindungsgemäßen Metallkomplexe als Matrixmaterial für andere phosphoreszierende Metallkomplexe in einer emittierenden Schicht einsetzen.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoO₃ oder WO₃, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer oder noch höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend einen erfindungsgemäßen Metallkomplex und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend Verbindungen gemäß Formel (1) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Die erfindungsgemäßen Metallkomplexe lassen sich in sehr hoher Ausbeute und sehr hoher Reinheit bei außergewöhnlich kurzen Reaktionszeiten und vergleichsweise geringen Reaktionstemperaturen synthetisieren.
2. Die erfindungsgemäßen Metallkomplexe weisen eine hervorragende thermische Stabilität auf, was sich auch bei der Sublimation der Komplexe zeigt.
3. Die erfindungsgemäßen Metallkomplexe zeigen weder thermisch noch photochemisch fac/mer- bzw. mer/fac-Isomerisierung, was zu Vorteilen in der Anwendung dieser Komplexe führt.
4. Die erfindungsgemäßen Metallkomplexe weisen teilweise ein sehr schmales Emissionsspektrum auf, was zu einer hohen Farbreinheit der Emission führt, wie sie insbesondere für Displayanwendungen wünschenswert ist.
5. Organische Elektrolumineszenzvorrichtungen enthaltend die erfindungsgemäßen Metallkomplexe als emittierende Materialien weisen eine sehr gute Lebensdauer auf.
6. Organische Elektrolumineszenzvorrichtungen enthaltend die erfindungsgemäßen Metallkomplexe als emittierende Materialien weisen eine hervorragende Effizienz auf.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße elektronische Vorrichtungen herstellen und somit die Erfindung im gesamten beanspruchten Bereich ausführen.

### Beispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Liganden mit Imineinheiten werden nachfolgend so bildlich bezüglich ihrer Konformation an der Iminbindung dargestellt, wie sie im Metallkomplex vorliegen, unabhängig davon, ob sie aus der Synthese als cis-Form, trans-Form oder als Gemisch anfallen.

### 1. Darstellung der organischen Synthone:

### Beispiel S1:

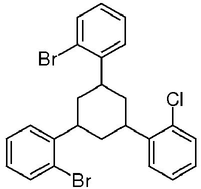

a) 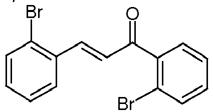 Darstellung gemäß G. Markopoulos et al., Angew. Chem., Int. Ed., 2012, 51, 12884.
b) 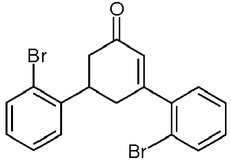 Durchführung nach JP 2000-169400. Eine Lösung von 36.6 g (100 mmol) 1,3-Bis-(2-bromphenyl)-2-propen-1-on [126824-93-9], Stufe a) in 300 ml trockenem Aceton wird portionsweise mit 5.7 g [105 mmol] Natriummethanolat versetzt und dann 12 h bei 40 °C gerührt. Man entfernt das Lösungsmittel im Vakuum, nimmt den Rückstand in Ethylacetat auf, wäscht dreimal mit je 200 ml Wasser, zweimal mit je 200 ml ges. Kochsalzlösung und trocknet über Magnesiumsulfat. Das nach Entfernen das Lösungsmittels im Vakuum erhaltene Öl wird Flash-chromatographiert (Torrent CombiFlash, Fa. Axel Semrau). Ausbeute: 17.9 g (44 mmol), 44 %. Reinheit: ca. 97 %-ig n. ¹H-NMR.
c) 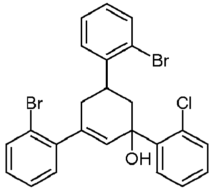 Zu einer Lösung von 2-Chlor-phenylmagnesiumbromid (200 mmol) [36692-27-0] in 200 ml Di-n-butylether fügt man bei 0 °C 2.4 g (2.4 mmol) wasserfreies Kupfer(I)chlorid [7758-89-6] zu und rührt 30 min. nach. Dann tropft man eine Lösung von 40.6 g (100 mmol) Stufe b) in 200 ml Toluol während 30 min. zu und rührt 5 h bei 0 °C nach. Man quencht die Reaktionsmischung durch vorsichtige Zugabe von 100 ml Wasser und dann 220 ml 1N Salzsäure. Man trennt die organische Phase ab, wäscht diese zweimal mit je 200 ml Wasser, einmal mit 200 ml gesättigter Natriumhydrogerncarbonat-Lösung, einmal mit 200 ml ges. Kochsalzlösung und trocknet über Magnesiumsulfat. Das nach Entfernen das Lösungsmittels im Vakuum erhaltene Öl wird mit Toluol über Kieselgel filtriert. Das so erhaltene Rohprodukt wird ohne weitere Reinigung weiter umgesetzt. Ausbeute: 49.8 g (96 mmol), 96 %. Reinheit: ca. 90-95 %-ig n. ¹H-NMR.
d) 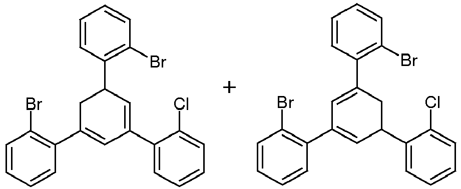 Eine auf 0 °C gekühlte Lösung von 51.9 g (100 mmol) Stufe c) in 500 ml Dichlormethan (DCM) wird mit 1.0 ml Trifluormethansulfonsäure und dann portionsweise mit 50 g Phosphorpentoxid versetzt. Man lässt auf Raumtemperatur erwärmen und rührt 2 h nach. Man dekantiert vom Phosphorpentoxid ab, schlämmt dieses in 200 ml DCM auf und dekantiert erneut ab. Die vereinigten DCM-Phasen werden zweimal mit Wasser und einmal mit ges. Kochsalzlösung gewaschen und über Magnesiumsulfat getrocknet. Das nach Entfernen des Lösungsmittels im Vakuum erhaltene Wachs wird Flash-chromatographiert (Torrent CombiFlash, Fa. Axel Semrau). Ausbeute: 31.5 g (63 mmol), 63 %, Isomerengemisch. Reinheit: ca. 90-95 %-ig n. ¹H-NMR.
e)

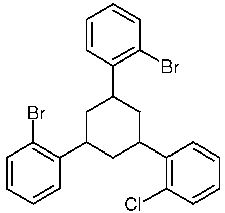

Ein Gemisch aus 25.0 g (50 mmol) Stufe d), 2 g Pd/C (10 %), 200 ml Methanol und 300 ml Ethylacetat wird im Rührautoklaven mit 3 bar Wasserstoff beaufschlagt und bei 30 °C bis zur beendeten Wasserstoffaufnahme hydriert. Man filtriert über ein mit Ethylacetat vorgeschlämmtes Celite-Bett ab, engt das Filtrat zur Trockene ein. Das so erhaltene Öl wird Flash-chromatographiert (Torrent CombiFlash, Fa. Axel Semrau). Ausbeute: 17.2 g (34 mmol), 68 %. Reinheit: ca. 95 %-ig n. ¹H-NMR, cis,cis-Isomer.

Analog können folgende Verbindungen hergestellt werden.

| | | | |
|---|---|---|---|
| Bsp. | Edukte sofern von S1 abweichend | Produkt | Ausbeute a) bis e) |
| S2 | 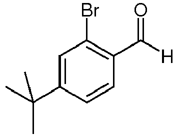 | 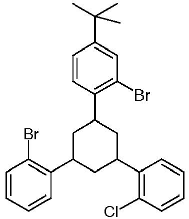 | 21 % |
| | 246139-77-5 | | |
| S3 | 246139-77-5 | 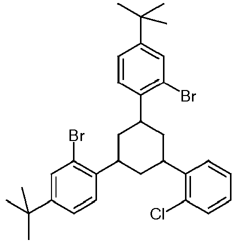 | 19 % |
| | 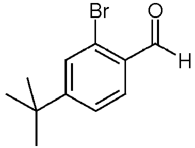 | | |
| | 147438-85-5 | | |
| S4 | 246139-77-5 147438-85-5 | 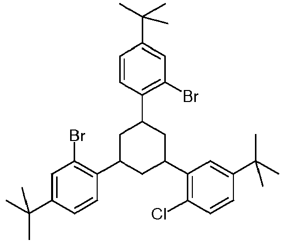 | 14% |
| | 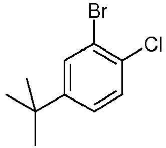 | | |

### Beispiel S5:

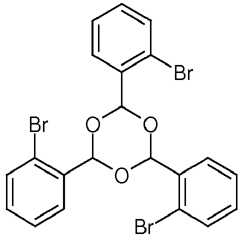

Eine auf 40 °C temperierte, gut gerührte Schmelze von 18.5 g (100 mmol) 2-Brom-benzaldehyd wird mit 801 mg (10 mmol) nanoskaligem Zinkoxid versetzt. Nach 16 h versetzt man die Reaktionsmischung mit 100 ml Toluol, filtriert vom Zinkoxid über Celite ab, entfernt das Toluol komplett im Vakuum und kristallisiert das so erhaltene Wachs aus Aceton um. Ausbeute: 6.3 g (34 mmol), 34 %. Reinheit: ca. 95 %-ig n. ¹H-NMR, cis,cis-Isomer.

### Beispiel S6:

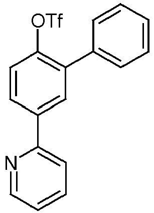

### a) S6a

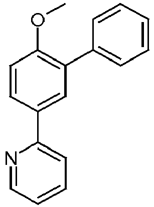

Ein Gemisch aus 22.6 g (100 mmol) (6-Methoxy-[1,1'-biphenyl]-3-yl)boronsäure [459423-16-6], 16.6 g (105 mmol) 2-Brompyridin [109-04-6], 21.2 g (200 mmol) Natriumcarbonat, 1.2 g (1 mmol) Tetrakis-triphenylphosphinopalladium [14221-01-3], 300 ml Toluol, 100 mol Ethanol, 300 ml Wasser wird unter gutem Rühren 18 h unter Rückfluss erhitzt. Nach Erkalten trennt man die org. Phase ab, wäscht diese zweimal mit je 300 ml Wasser und einmal mit 300 ml ges. NaCl-Lösung und trocknet über Magnesiumsulfat. Das nach Einengen der org. Phase erhaltene Öl wird am Ölpumpenvakuum bei 80 °C getrocknet und ohne weitere Reinigung umgesetzt. Ausbeute: 25.6 g (98 mmol), 98 %; Reinheit: ca. 95 % n. ¹H-NMR.

### b) S6b

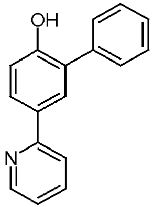

Ein Gemisch aus 26.1 g (100 mmol) 5-(2-Pyridyl)-[1,1'-biphenyl]-2-ol S6a und 81.9 g (700 mmol) Pyridiniumhydrochlorid werden 3 h auf 190 °C erhitzt. Nach Erkalten gießt man die Reaktionsmischung in 500 ml Wasser ein, extrahiert fünfmal mit je 200 ml Dichlormethan, wäscht die org. Phase zweimal mit 200 ml Wasser und einmal mit 200 ml ges. NaCl-Lösung, entfernt das Lösungsmittel im Vakuum, gibt zur azeotropen Trocknung 300 ml Toluol zu und destilliert dieses im Vakuum komplett ab. Das so erhaltene zähe Öl wird ohne weitere Reinigung umgesetzt. Ausbeute: 21.0 g (85 mmol), 85 %; Reinheit: ca. 95 % n. ¹H-NMR.

### c) S6

Eine auf 0 °C gekühlte Lösung von 24.7 g (100 mmol) S6b in einem Gemisch aus 300 ml Dichlormethan und 80 ml Pyridin wird unter gutem Rühren tropfenweise mit 34 ml (200 mmol) Trifluormethansulfonsäureanhydrid [358-23-6] versetzt. Man lässt die Reaktionsmischung auf RT erwärmen, rührt 16 h nach, gießt unter Rühren auf 1000 ml Eiswasser und extrahiert dieses dann dreimal mit 300 ml Dichlormethan. Die vereinigten org. Phasen werden zweimal mit je 300 ml Eiswasser und einmal mit 500 ml ges. NaCl-Lösung gewaschen und dann über Natriumsulfat getrocknet. Das nach Entfernen des Dichlormethans im Vakuum verbliebene Wachs wird aus Acetonitril umkristallisiert. Ausbeute: 32.6 g (86 mmol), 86 %; Reinheit: ca. 95 % n. ¹H-NMR.

Analog kann S7 erhalten werden, wobei anstelle von 2-Brompyridin 2-Brom-4-tert-butylpyridin [50488-34-1] eingesetzt wird:

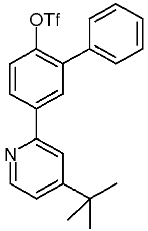

### Beispiel S10: 5-Brom-2-[1,1,2,2,3,3-hexamethyl-indan-5-yl]-pyridin

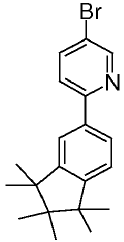

Ein Gemisch aus 164.2 g (500 mmol) 2-(1,1,2,2,3,3-Hexamethyl-indan-5-yl)-4,4,5,5-tetramethyl-[1,3,2]dioxa-borolan [152418-16-9] (analog können Boronsäuren eingesetzt werden), 142.0 g (500 mmol) 5-Brom-2-iod-pyridin [223463-13-6], 159.0 g (1.5 mol) Natriumcarbonat, 5.8 g (5 mmol) Tetrakis(triphenylphosphino)-palladium(0), 700 ml Toluol, 300 ml Ethanol und 700 ml Wasser wird unter gutem Rühren 16 h unter Rückfluss erhitzt. Nach dem Abkühlen werden 1000 ml Toluol zugegeben, die organische Phase wird abgetrennt, und die wässrige Phase wird mit 300 ml Toluol nachextrahiert. Die vereinigten organischen Phasen werden einmal mit 500 ml gesättigter Kochsalzlösung gewaschen. Nach Trocknen der organischen Phase über Natriumsulfat und Entfernen des Lösemittels im Vakuum kristallisiert man das Rohprodukt zweimal aus ca. 300 ml EtOH um. Ausbeute: 130.8 g (365 mmol), 73 %. Reinheit: ca. 95 % n. ¹H-NMR.

Analog kann die folgende Verbindung dargestellt werden:

| Bsp. | Boronsäure/-ester Pyridin | Produkt | Ausbeute |
|---|---|---|---|
| S11 | 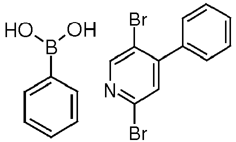 | 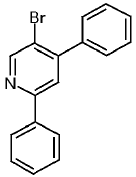 | 73 % |
| | 98-80-6 / 1381937-40-1 | | |

### Beispiel S20:

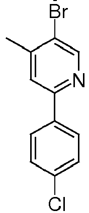

Ein Gemisch aus 25.1 g (100 mmol) 2,5-Dibrom-4-methylpyridin [3430-26-0], 15.6 g (100 mmol) 4-Chlorphenylboronsäure [1679-18-1], 27.6 g (200 mmol) Kaliumcarbonat, 1.57 g (6 mmol) Triphenylphosphin [603-35-0], 676 mg (3 mmol) Palladium(II)acetat [3375-31-3], 200 g Glaskugeln (3 mm Durchmesser), 200 ml Acetonitril und 100 ml Ethanol wird 48 h unter Rückfluss erhitzt. Nach Erkalten entfernt man die Lösemittel im Vakuum, gibt 500 ml Toluol zu, wäscht zweimal mit je 300 ml Wasser, einmal mit 200 ml ges. Kochsalzlösung, trocknet über Magnesiumsulfat, filtriert über ein vorgeschlämmtes Kieselgelbett ab und wäscht dieses mit 300 ml Toluol nach. Nach Entfernen des Toluols im Vakuum kristallisiert man einmal aus Methanol/Ethanol (1:1 vv) und einmal aus n-Heptan um. Ausbeute: 17.3 g (61 mmol), 61 %. Reinheit: ca. 95 %-ig n. ¹H-NMR.

### Beispiel S21:

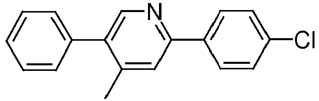

Ein Gemisch aus 28.3 g (100 mmol) S20, 12.8 g (105 mmol) Phenylboronsäure, 31.8 g (300 mmol) Natriumcarbonat, 787 mg (3 mmol) Triphenylphosphin, 225 mg (1 mmol) Palladium(II)acetat, 300 ml Toluol, 150 ml Ethanol und 300 ml Wasser wird 48 h unter Rückfluss erhitzt. Nach Erkalten wird mit 300 ml Toluol erweitert, die org. Phase wird abgetrennt, einmal mit 300 ml Wasser, einmal mit 200 ml ges. Kochsalzlösung gewaschen und über Magnesiumsulfat getrocknet. Nach Entfernen das Lösungsmittels wird der Rückstand an Kieselgel (Toluol/Ethylacetat, 9:1 vv) chromatographiert. Ausbeute: 17.1 g (61 mmol), 61 %. Reinheit: ca. 97 %-ig n. ¹H-NMR.

Analog können die folgenden Verbindungen synthetisiert werden:

| Bsp. | Boronester | Produkt | Ausbeute |
|---|---|---|---|
| S22 | 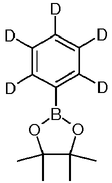 | 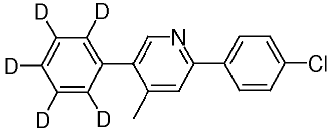 | 56 % |
| | 245043-33-8 | | |
| S23 | 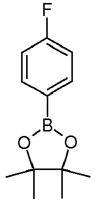 | 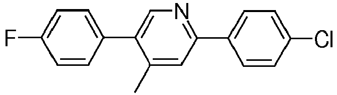 | 61 % |
| | 214360-58-4 | | |
| S24 | 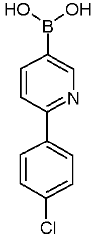 | 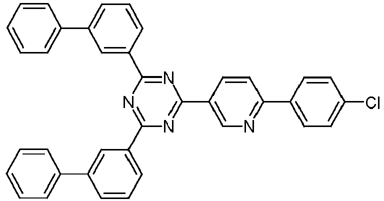 | 70 % |
| | 1264513-60-1 | | |
| | 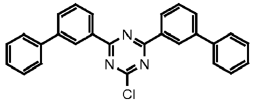 | | |
| | 1205748-61-3 | | |

### Beispiel S30: 2-[1,1,2,2,3,3-Hexamethyl-indan-5-yl]-5-(4,4,5,5-tetra-methyl-[1,3,2]dioxaborolan-2-yl)-pyridin

### Variante A:

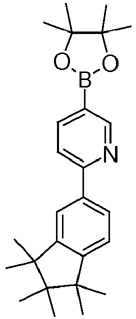

Ein Gemisch aus 35.8 g (100 mmol) S10, 25.4 g (100 mmol) Bis(pinacolato)diboran [73183-34-3], 49.1 g (500 mmol) Kaliumacetat, 1.5 g (2 mmol) 1,1-Bis(diphenylphosphino)ferrocendichlorpalladium(II)-Komplex mit DCM [95464-05-4], 200 g Glaskugeln (3 mm Durchmesser), 700 ml 1,4-Dioxan und 700 ml Toluol wird 16 h unter Rückfluss erhitzt. Nach Abkühlen wird die Suspension über ein Celite-Bett filtriert und das Lösemittel im Vakuum entfernt. Der schwarze Rückstand wird mit 1000 ml heißem Cyclohexan digeriert, es wird noch heiß über ein Celite-Bett abfiltriert, dann auf ca. 200 ml eingeengt, wobei das Produkt zu kristallisieren beginnt. Die Kristallisation wird über Nacht im Kühlschrank vervollständigt, die Kristalle werden abfiltriert und mit wenig n-Heptan gewaschen. Aus der Mutterlauge kann eine zweite Produktfraktion gewonnen werden. Ausbeute: 31.6 g (78 mmol), 78 %. Reinheit: ca. 95 % ig n. ¹H-NMR.

### Variante B: Umsetzung von Arylchloriden

Wie Variante A, jedoch wird an Stelle von 1,1-Bis(diphenylphosphino)-ferrocendichlorpalladium(II)-Komplex mit DCM 1.5 mmol SPhos [657408-07-6] und 1.0 mmol Palladium(II)acetat eingesetzt.

Analog können folgende Verbindungen dargestellt werden, wobei zur Reinigung anstelle von n-Heptan auch Cyclohexan, Toluol, Acetonitril, Ethylacetat bzw. Gemische der genannten Lösungsmittel verwendet werden können:

| Bsp. | Bromid/Triflat - Variante A Chlorid - Variante B | Produkt | Ausbeute |
|---|---|---|---|
| S31 | 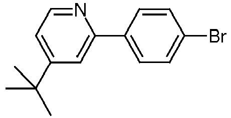 | 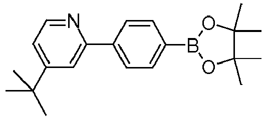 | 88% |
| | 1246851-70-6 | | |
| S32 | 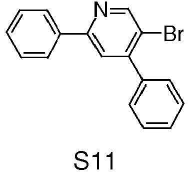 | 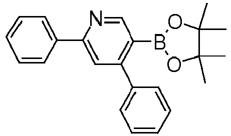 | 70% |
| S33 | 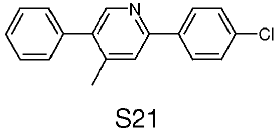 | 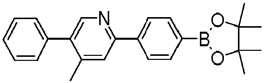 | 86% |
| S34 | 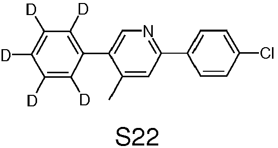 | 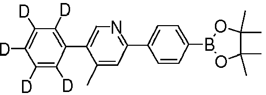 | 79% |
| S35 | 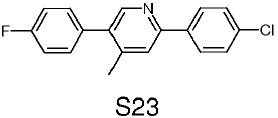 | 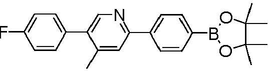 | 77% |
| S36 | 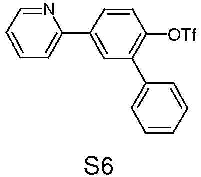 | 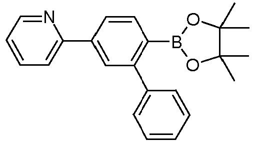 | 64% |
| S37 | 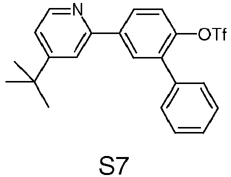 | 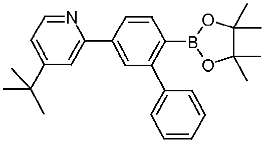 | 69 % |
| S38 | 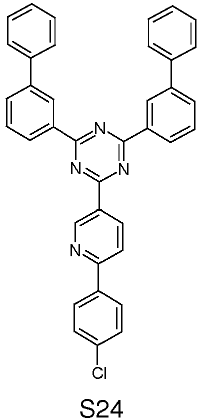 | 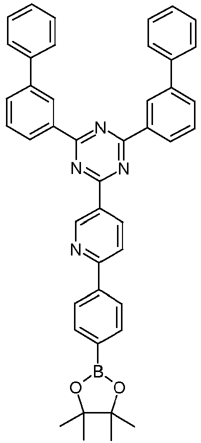 | 74% |
| S39 | 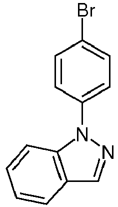 | 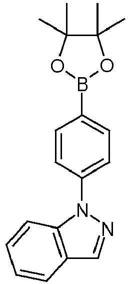 | 83% |
| | 838820-83-0 | | |
| S40 | 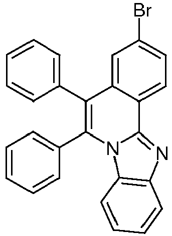 | 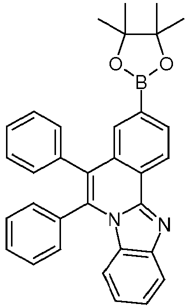 | 80% |
| | 1447946-51-1 | | |
| S41 | 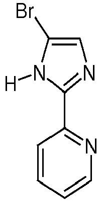 | 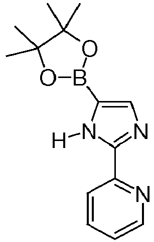 | 36% |
| | 71048-48-1 | | |
| S42 | 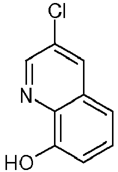 | 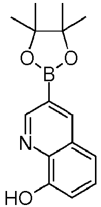 | 48% |
| | 102878-83-1 | | |
| S43 | 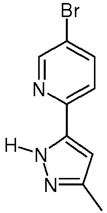 | 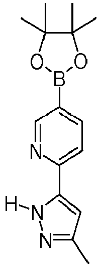 | 46% |
| | 1239480-83-1 | | |

### Beispiel S100:

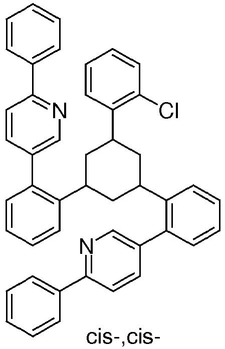

Ein Gemisch aus 54.5 g (100 mmol) S1, 59.0 g (210 mmol) 2-Phenyl-5-(4,4,5,5-tetramethyl-[1,3,2]dioxaborolan-2-yl)-pyridin [879291-27-7], 127.4 g (600 mmol) Trikaliumphosphat, 1.57 g (6 mmol) Triphenylphosphin und 449 mg (2 mmol) Palladium(II)acetat in 750 ml Toluol, 300 ml Dioxan und 500 ml Wasser wird 30 h unter Rückfluss erhitzt. Nach Erkalten trennt man org. Phase ab, wäscht diese zweimal mit je 300 ml Wasser, einmal mit 300 ml gesättigter Kochsalzlösung und trocknet über Magnesiumsulfat. Man filtriert vom Magnesiumsulfat über ein mit Toluol vorgeschlämmtes Celite-Bett ab, engt das Filtrat im Vakuum zur Trockene ein und kristallisiert den verbliebenen Schaum aus Acetonitril/Ethylacetat um. Ausbeute: 41.8 g (64 mmol), 64 %. Reinheit: ca. 95 % ig n. ¹H-NMR.

Analog können folgende Verbindungen dargestellt werden:

| Bsp. | Edukte | Produkt | Ausbeute |
|---|---|---|---|
| S101 | S1 | 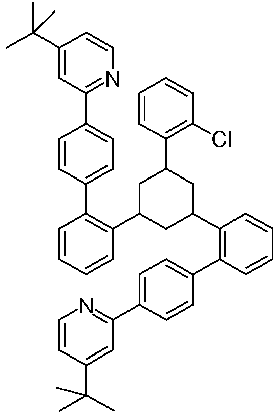 | 68 % |
| | S31 | | |
| S102 | S3 | 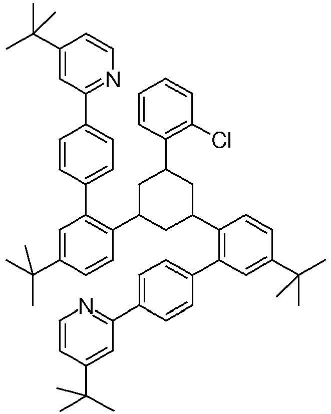 | 60 % |
| | S31 | | |
| S103 | S3 | 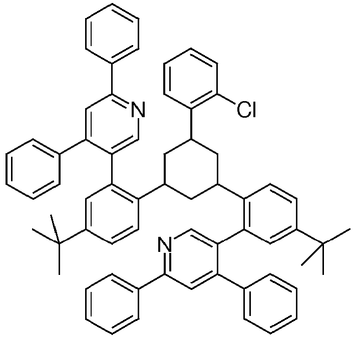 | 60% |
| | S32 | | |
| S104 | S3 | 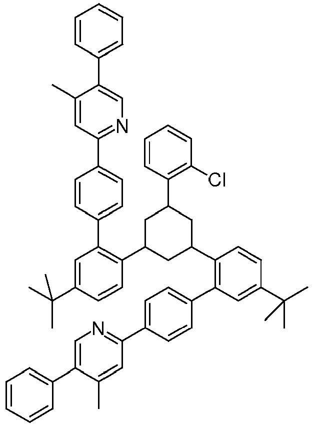 | 69 % |
| | S33 | | |
| S105 | S3 | 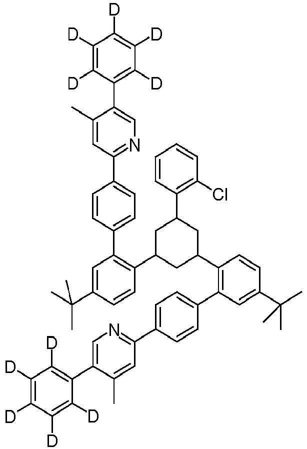 | 64 % |
| | S34 | | |
| S106 | S4 | 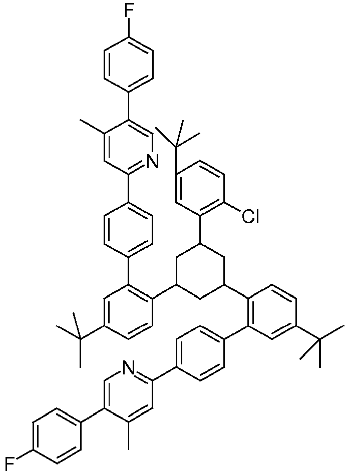 | 61 % |
| | S35 | | |
| S107 | S3 | 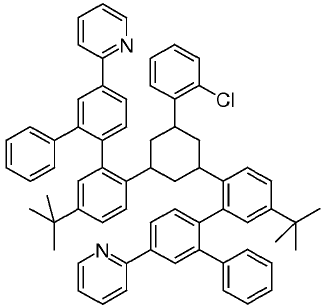 | 63 % |
| | S36 | | |
| S108 | S3 | 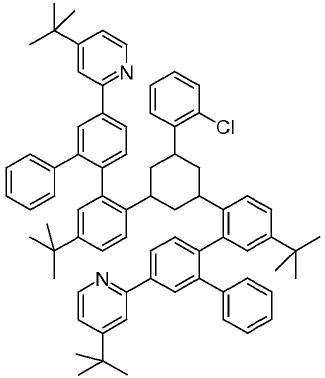 | 60 % |
| | S37 | | |
| S109 | S3 | 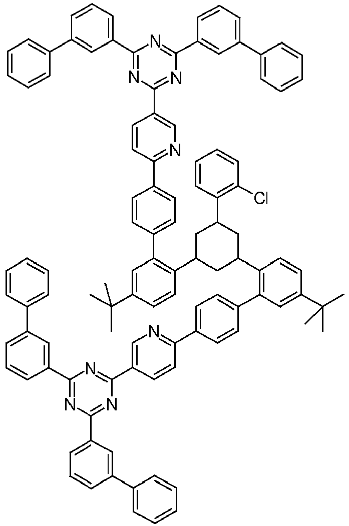 | 66 % |
| | S38 | | |

### Beispiel S200:

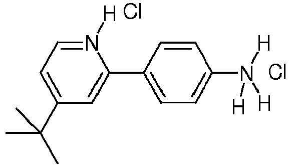

Ein Gemisch aus 29.0 g (100 mmol) S31, 20.2 g (200 mmol) Pivaloylamid, 97.8 g (300 mmol) Cäsiumcarbonat, 1157 mg (2 mmol) Xanthphos, 449 mg (2 mmol) Palladium(II)acetat und 500 ml Dioxan und 200 g Glaskugeln (3 mm Durchmesser) wird 12 h bei 100 °C gerührt. Man entfernt das Dioxan weitgehend im Vakuum, nimmt den Rückstand in 500 ml Wasser und 500 ml Ethylacetat auf, wäscht die org. Phase zweimal mit 300 ml Wasser und einmal mit 300 ml ges. Kochsalzlösung und trocknet dann über Magnesiumsulfat. Man filtriert vom Trockenmittel über ein mit Ethylacetat vorgeschlämmtes Celite-Bett ab und engt das Filtrat zur Trockene ein. Man nimmt den öligen Rückstand in 200 ml Dioxan auf, fügt 50 ml konz. HCl zu und kocht die Lösung 12 h unter Rückfluss, destilliert dann das Dioxan weitgehend ab, wobei das Produkt auskristallisiert. Nach Absaugen und Waschen mit eiskaltem Wasser wird das Produkt im Vakuum getrocknet. Ausbeute: 19.0 g (63 mmol), 63 %. Reinheit: ca. 95 % ig n. ¹H-NMR.

Analog können folgende Verbindungen dargestellt werden:

| Bsp. | Edukt | Produkt | Aus-beute |
|---|---|---|---|
| S201 | S33 | 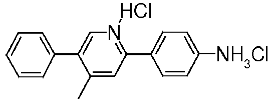 | 66% |
| S202 | S37 | 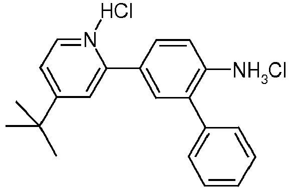 | 60 % |
| S203 | S38 | 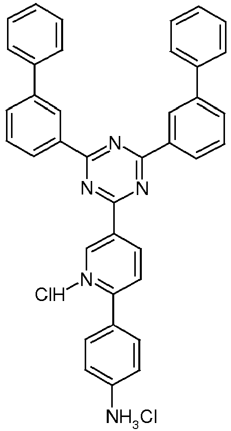 | 60 % |
| S204 | S39 | 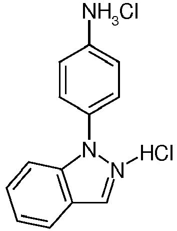 | 67% |

### Beispiel S300:

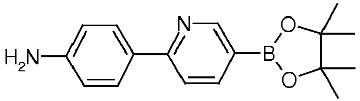

Ein Gemisch von 24.9 g (100 mmol) 2-(4-Aminophenyl)-5-brompyridin [1264652-77-8], 26.7 g (105 mmol) Bis(pinacolato)diboran [73183-34-3], 29.5 g (300 mmol) Kaliumacetat, wasserfrei, 561 mg (2 mmol) Tricyclohexylphosphin, 224 mg (1 mmol) Palladium(II)acetat und 500 ml Dioxan wird 16 h bei 90 °C gerührt. Nach Entfernen des Lösungsmittels im Vakuum wird der Rückstand in 500 ml Ethylacetat aufgenommen, über ein Celite-Bett filtriert, das Filtrat wird bis zur beginnenden Kristallisation im Vakuum eingeengt und abschließend noch tropfenweise mit ca. 100 ml Methanol versetzt, um die Kristallisation zu vervollständigen. Ausbeute: 20.1 g (68 mmol), 68 %; Reinheit: ca. 95 %ig nach ¹H-NMR.

Analog können die folgenden Verbindungen synthetisiert werden:

| Bsp. | Edukt | Produkt | Ausbeute |
|---|---|---|---|
| S301 | 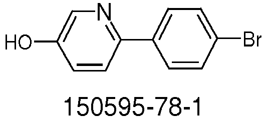 | 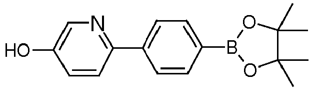 | 63 % |
| S302 | 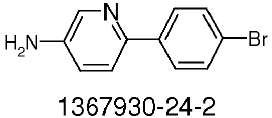 | 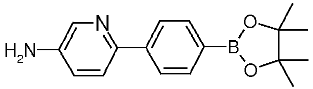 | 58 % |

### 2. Darstellung der hexadentaten Liganden L:

### Beispiel L1:

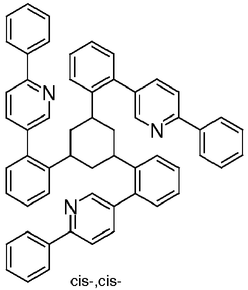

Ein Gemisch aus 50.5 g (100 mmol) S1, 98.4 g (350 mmol) 2-Phenyl-5-(4,4,5,5-tetramethyl-[1,3,2]dioxaborolan-2-yl)-pyridin [879291-27-7], 106.0 g (1 mol) Natriumcarbonat, 2.1 g (5 mmol) SPhos [657408-07-6], 674 mg (3 mmol) Palladium(II)acetat, 750 ml Toluol, 200 ml Dioxan und 500 ml Wasser wird unter sehr gutem Rühren 24 h auf 70 °C erhitzt. Man lässt erkalten, trennt die wässrige Phase ab und engt die organische Phase zur Tockene ein. Man nimmt den braunen Schaum nach Einengen der organischen Phase aus der Suzuki-Kupplung in 300 ml Dichlormethan:Ethylacetat (1:1, vv) auf und filtriert über ein mit Dichlormethan: Ethylacetat (1:1, vv) vorgeschlämmtes Kieselgelbett (Durchmesser 15 cm, Länge 20 cm), um braune Anteile zu entfernen. Nach Einengen wird der verbliebene Schaum aus 300 ml Ethylacetat unter Zusatz von 300 ml Methanol in der Siedehitze und dann ein zweites Mal aus 250 ml reinem Ethylacetat umkristallisiert und anschließend im Hochvakuum (p ca. 10⁻⁵ mbar, T 260 °C) Kugelrohr-sublimiert. Ausbeute: 45.6 g (59 mmol), 59 %. Reinheit: ca. 99.7 % ig n. ¹H-NMR, cis,cis-Isomer.

Analog können folgende Verbindungen dargestellt werden, wobei die Reinigung auch chromatographisch (z.B. Torrent CombiFlash der Fa. Axel Semrau) erfolgen kann:

| Bsp. | Bromid Boronester | Produkt | Ausbeute |
|---|---|---|---|
| L2 | S3 | 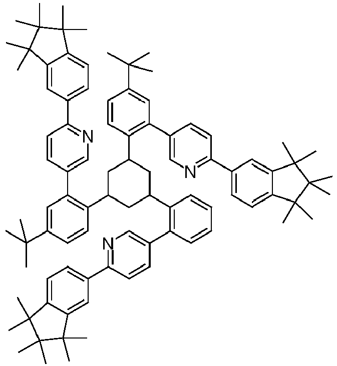 | 59 % |
| | S30 | | |
| L3 | S2 | 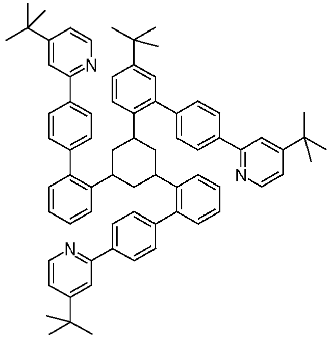 | 65 % |
| | S31 | | |
| L4 | S4 | 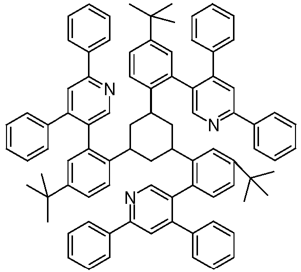 | 60 % |
| | S32 | | |
| L5 | S3 | 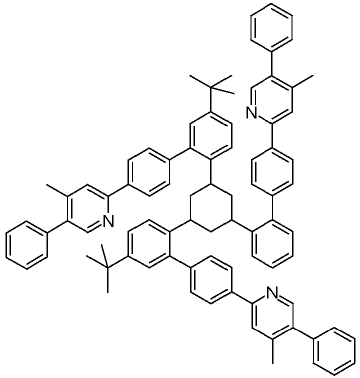 | 63 % |
| | S33 | | |
| L6 | S3 | 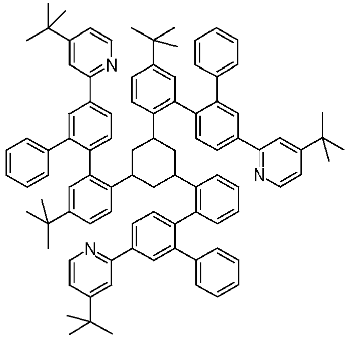 | 58 % |
| | S37 | | |
| L7 | S3 | 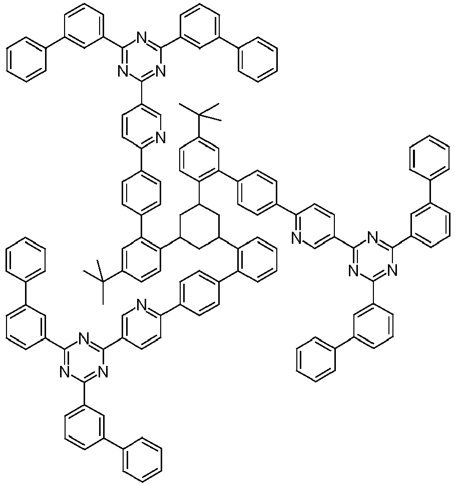 | 57% |
| | S38 | | |
| L8 | S3 1383803-71-1 | 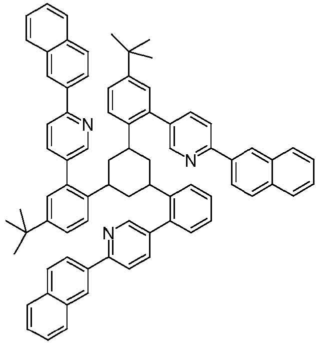 | 60 % |
| L9 | S1 1310383-27-7 | 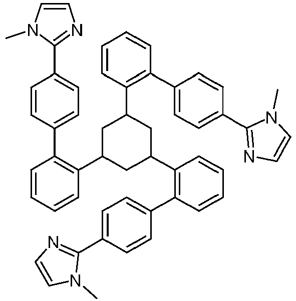 | 55 % |
| L10 | S3 1146340-38-6 | 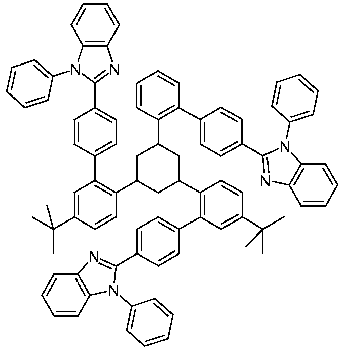 | 62 % |
| L11 | S3 1228267-13-7 | 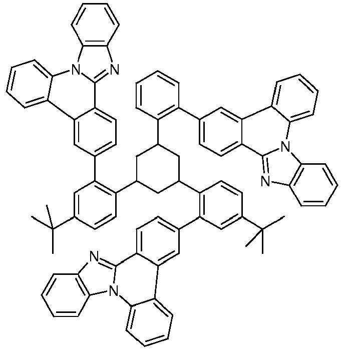 | 65 % |
| L12 | S3 | 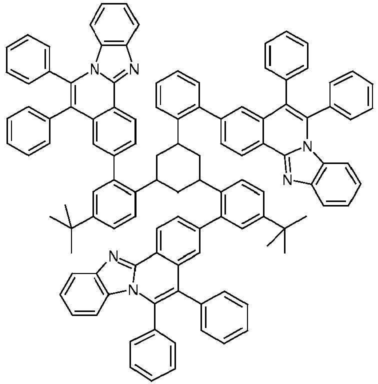 | 67% |
| | S40 | | |
| L13 | S3 1312478-63-9 | 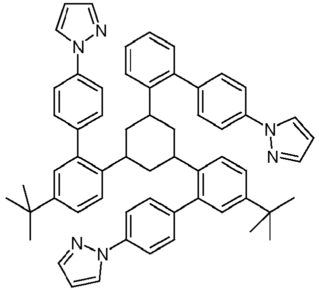 | 58 % |
| L14 | S3 | 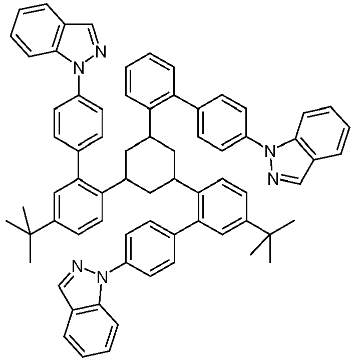 | 61 % |
| | S39 | | |
| L15 | S5 | 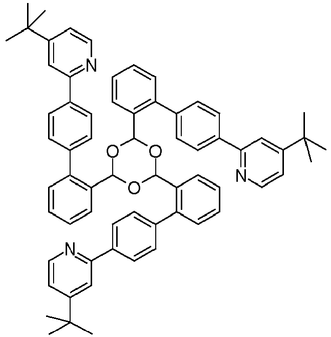 | 49 % |
| | S31 | | |
| L16 | S1 [562098-24-2] | 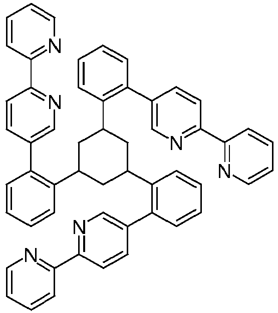 | 38 % |
| L17 | S1 | 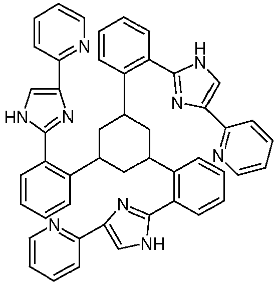 | 33 % |
| | S41 | | |
| | | Vor Aufarbeitung mit Eisessig auf pH = 7 einstellen | |
| L18 | S2 | 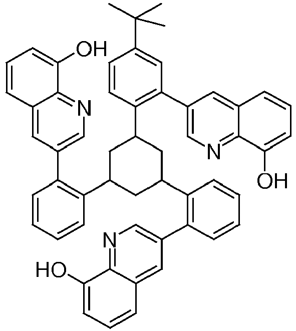 | 41 % |
| | S42 | | |
| | | Vor Aufarbeitung mit Eisessig auf pH = 7 einstellen | |
| L19 | S3 S43 | 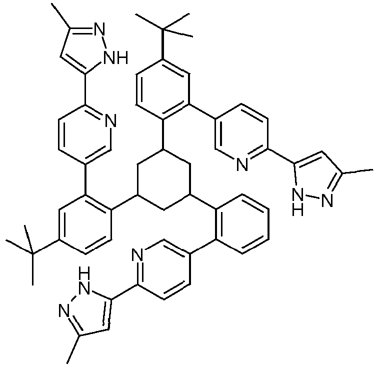 | 45 % |
| | | Vor Aufarbeitung mit Eisessig auf pH = 7 einstellen | |
| L20 | S3 [913836-11-0] | 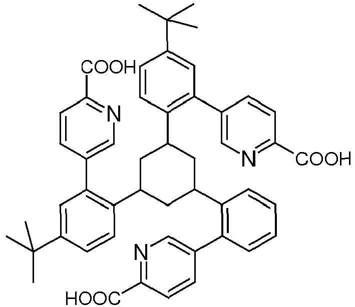 | 29 % |
| | | Vor Aufarbeitung mit Eisessig auf pH = 6 einstellen | |

### Beispiel L100:

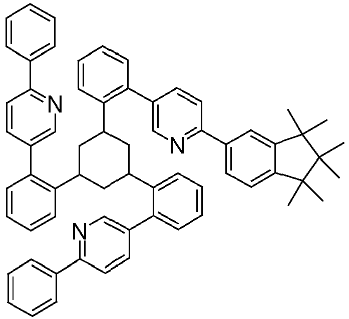

Ein Gemisch aus 65.3 g (100 mmol) S100, 42.5 g (105 mmol) S30, 63.7 g (300 mmol) Trikaliumphosphat, 1.23 g (3 mmol) SPhos [657408-07-6], 449 mg (2 mmol) Palladium(II)acetat, 500 ml Toluol, 300 ml Dioxan und 300 ml Wasser wird 6 h unter Rückfluss erhitzt. Nach Abkühlen wird die org. Phase abgetrennt, zweimal mit 300 ml Wasser und einmal mit 200 ml ges. Kochsalzlösung gewaschen, über Magnesiumsulfat getrocknet und dann über ein mit Toluol vorgeschlämmtes Celite-Bett filtriert, welches mit Toluol nachgewaschen wird. Das Filtrat wird zur Trockene eingeengt, und der Rückstand wird anschließend zweimal aus Ethylacetat/Methanol umkristallisiert. Ausbeute: 56.5 g (63 mmol), 63 %. Reinheit: ca. 97 % n. ¹H-NMR.

Analog können die folgenden Verbindungen synthetisiert werden:

| Bsp. | Boronester Bromid | Produkt | Ausbeute |
|---|---|---|---|
| L101 | S100 | 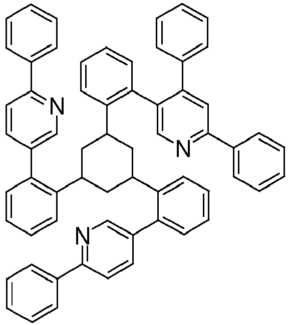 | 60 % |
| | S32 | | |
| L102 | S101 | 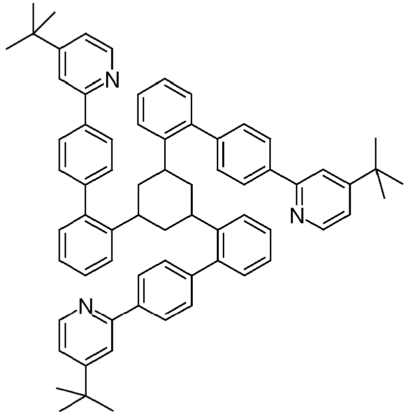 | 83 % |
| | S31 | | |
| L103 | S101 | 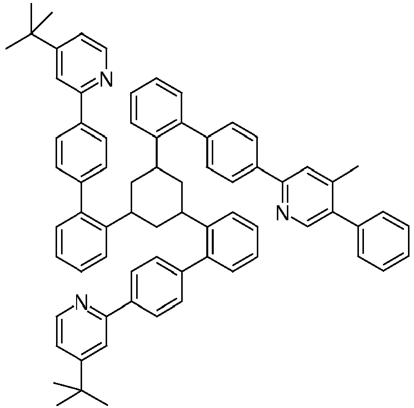 | 66 % |
| | S33 | | |
| L104 | S101 | 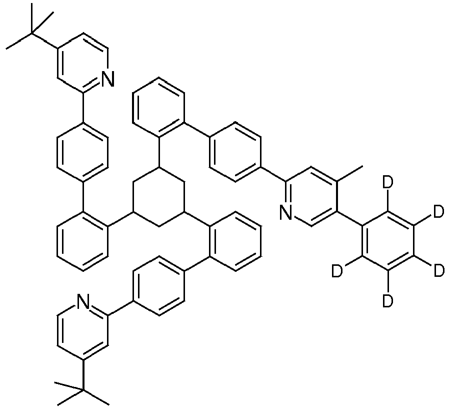 | 63 % |
| | S34 | | |
| L105 | S101 | 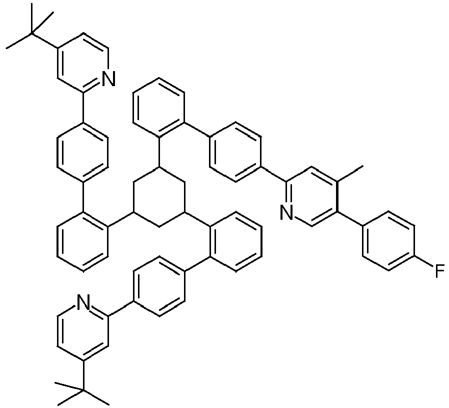 | 60 % |
| | S35 | | |
| L106 | S101 | 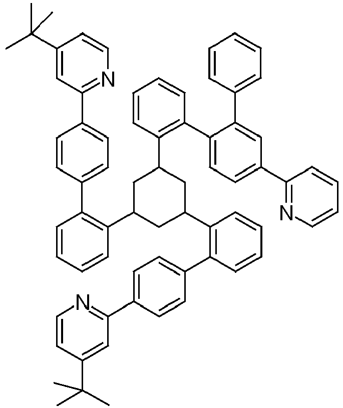 | 67% |
| | S36 | | |
| L107 | S101 | 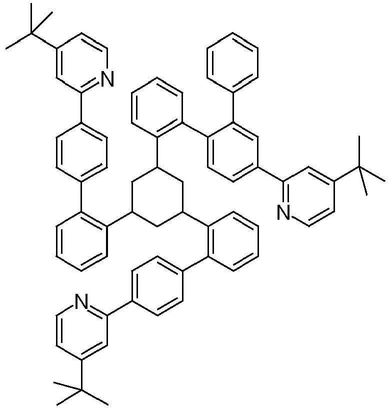 | 58 % |
| | S37 | | |
| L108 | S101 | 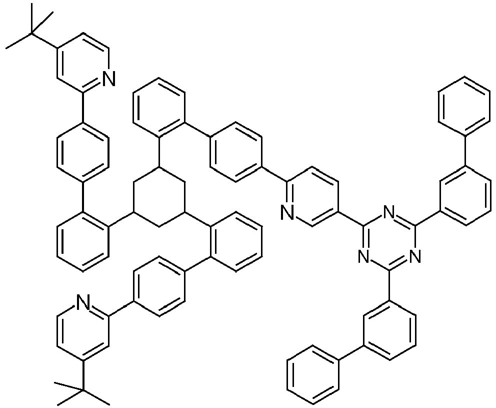 | 70 % |
| | S38 | | |
| L109 | S102 | 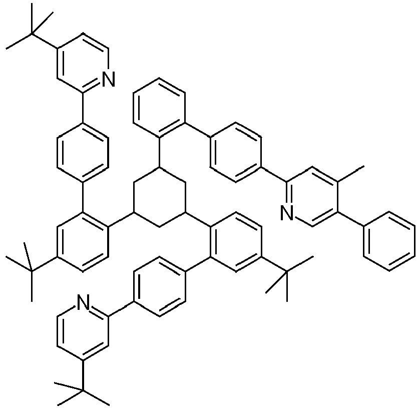 | 63 % |
| | S33 | | |
| L110 | S104 | 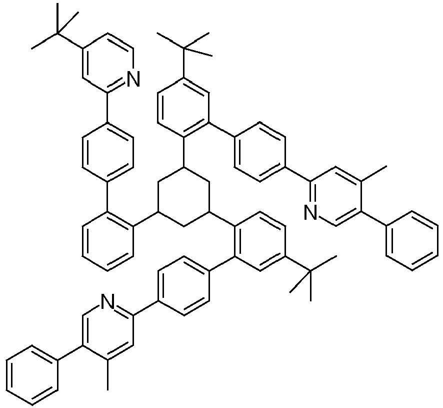 | 65 % |
| | S31 | | |
| L111 | S105 | 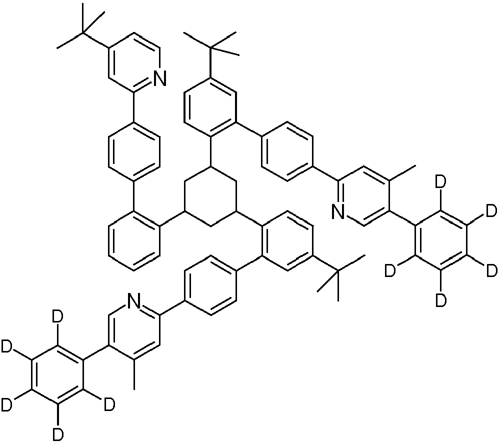 | 68 % |
| | S31 | | |
| L112 | S106 | 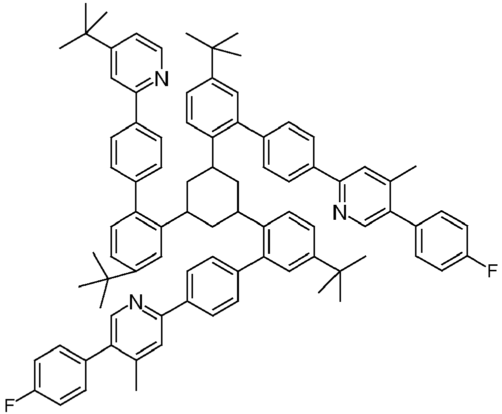 | 62 % |
| | S31 | | |
| L113 | S107 | 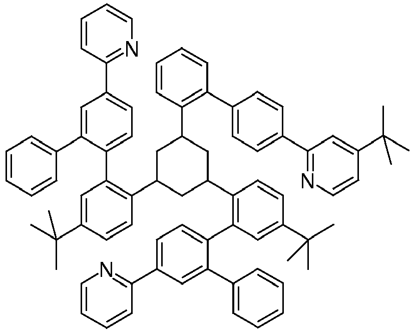 | 54% |
| | S31 | | |
| L114 | S108 | 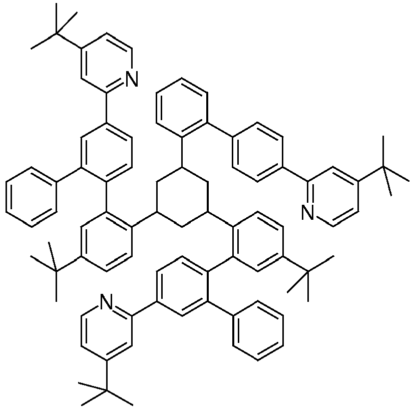 | 57% |
| | S31 | | |
| L115 | S109 | 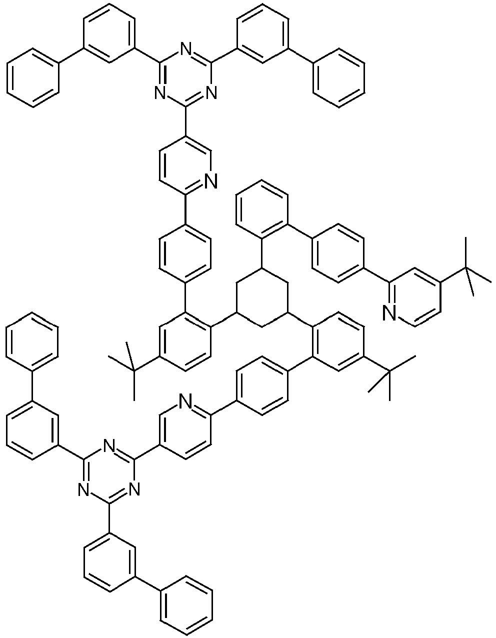 | 69 % |
| | S31 | | |
| L116 | S103 | 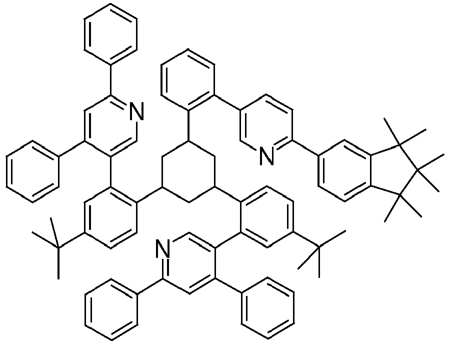 | 70 % |
| | S30 | | |
| L117 | S101 1383803-71-1 | 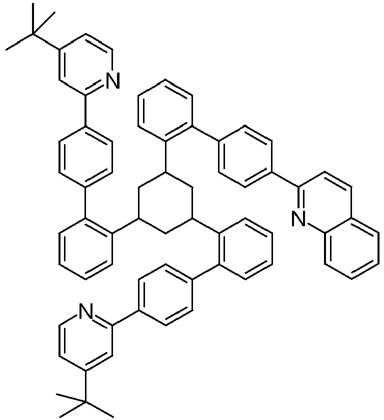 | 60 % |
| L118 | S101 1848992-66-4 | 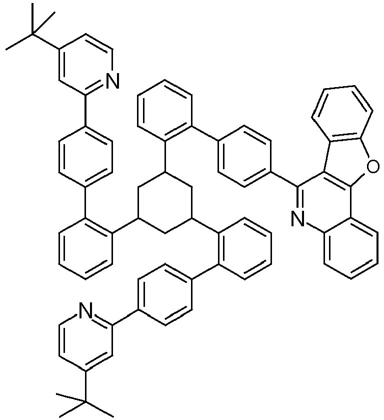 | 70 % |
| L119 | S101 1310383-27-7 | 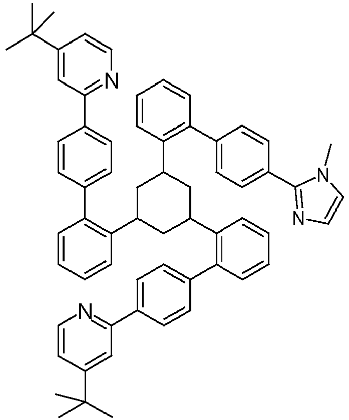 | 65 % |
| L120 | S102 1146340-38-6 | 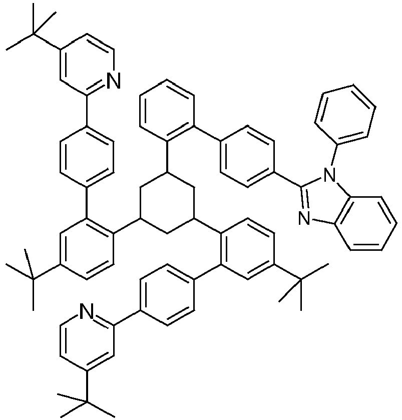 | 71 % |
| L121 | S102 1228267-13-7 | 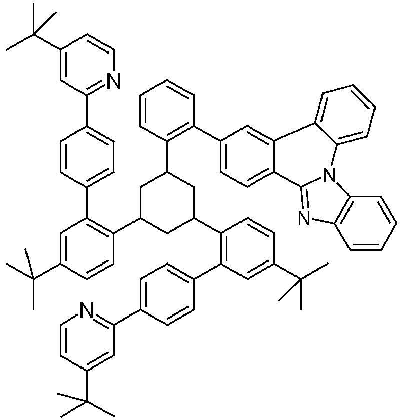 | 73 % |
| L122 | S101 S40 | 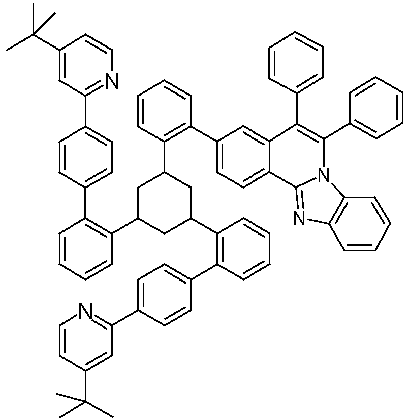 | 67% |
| L123 | S101 1312478-63-9 | 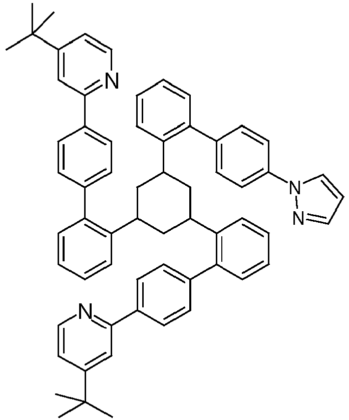 | 60 % |
| L124 | S101 S39 | 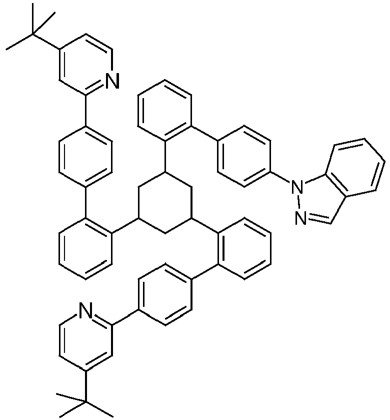 | 65 % |

### Beispiel L200:

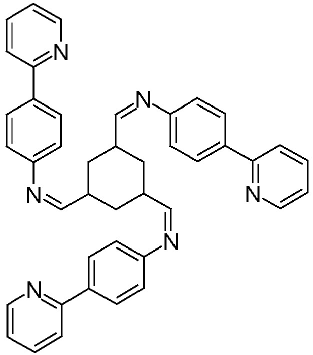

### Variante A, für Aldehyde:

Durchführung analog J. G. Muntaner et al., Org. & Biomol. Chem., 2014, 12, 286. Eine Lösung von 24.3 g (100 mmol) 4-(2-Pyridyl)anilinium-dihydrochlorid [856849-12-2] in 200 ml Ethanol wird mit 97 ml einer 2N Natrium-ethanolat-Lösung in Ethanol versetzt. Dann gibt man 5.1 g (30 mmol) cis-cis-1,3,5-Cyclohexantricarboxaldehyd [107354-37-0] zu und erhitzt das Gemisch 3 h unter Rückfluss. Anschließend destilliert man das Ethanol fast bis zur Trockene ab, nimmt den öligen Rückstand in 300 ml DCM auf, filtriert von unlöslichen Anteilen über ein mit DCM vorgeschlämmtes Celite-Bett ab, entfernt das DCM im Vakuum und kristallisiert das Rohprodukt aus Acetonitril/Cyclohexan um. Ausbeute: 14.4 g (23 mmol), 69 %. Reinheit: ca. 97 % n. ¹H-NMR.

### Beispiel L201:

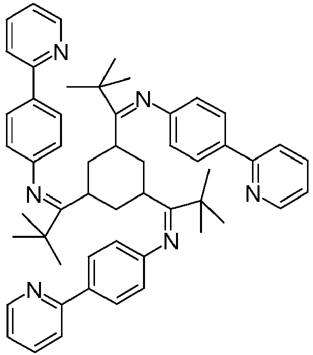

### Variante B, für Ketone:

Durchführung analog P. Sulmon et al., Synthesis 1985, 192.
Eine Suspension von 24.3 g (100 mmol) 4-(2-Pyridyl)anilinium-dihydrochlorid in 200 ml Diethylether wird mit drei Tropfen Methanol und dann portionsweise mit 8.0 g (200 mmol) Natriumhydrid, 60 Gew.-%ige Dispersion in Mineralöl versetzt (Vorsicht: Wasserstoffentwicklung!). Nach 3 h bei Raumtemperatur ist die Wasserstoffentwicklung beendet. Man fügt 10.1 g (30 mmol) cis,cis-1,1',1"-(1,3,5-Cyclohexantriyl)tris[2,2-dimethyl-1-propanon] [98013-15-1] zu und kühlt die Reaktionsmischung im Eis/ Kochsalz-Bad auf 0 °C. Dann tropft man 95 ml 1N Titantetrachlorid-Lösung in DCM zu, rührt 2 h nach, lässt auf Raumtemperatur erwärmen und erhitzt dann 18 h unter Rückfluss. Nach Erkalten saugt man vom ausgefallenen Feststoff ab, wäscht dreimal mit 100 ml DCM nach, engt das Filtrat zur Trockene ein, nimmt den öligen Rückstand in 300 ml DCM auf, wäscht dreimal mit je 100 ml 2N wässriger KOH-Lösung und trocknet dann über Magnesiumsulfat. Man entfernt das DCM im Vakuum und chromatographiert den Rückstand mit Cyclohexan:Ethylacetat:Triethylamin (90:9:1, vv) an Kieselgel (desaktiviert mit Triethylamin). Ausbeute: 5.6 g (7 mmol), 23 %. Reinheit: ca. 97 % n. ¹H-NMR.

Analog können die folgenden Verbindungen synthetisiert werden:

| Bsp. | Carbonyl-Komponente Amin | Produkt Variante | Ausbeute |
|---|---|---|---|
| L202 | 187805-79-4 S200 | 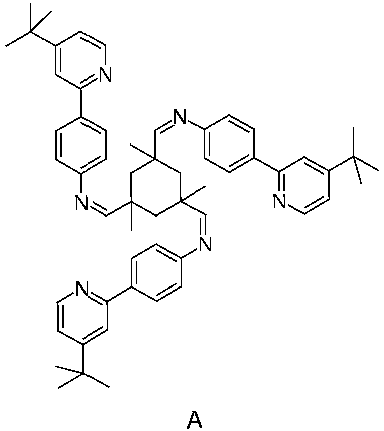 | 54% |
| L203 | 187805-79-4 S201 | 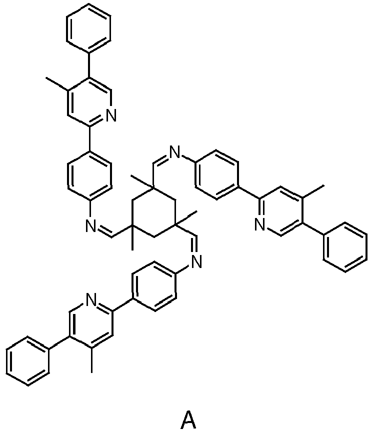 | 57% |
| L204 | 107354-37-0 S202 | 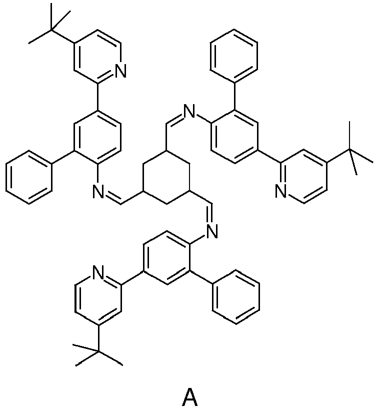 | 69 % |
| L205 | 98013-04-8 S203 | 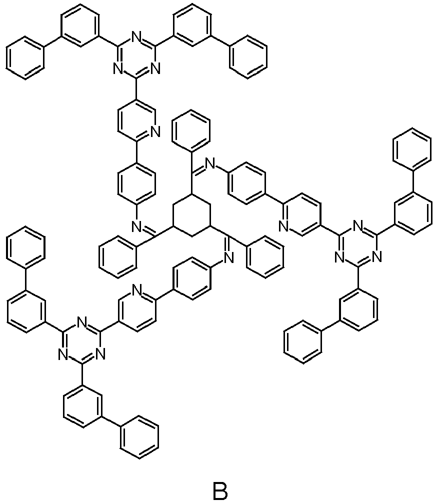 | 38 % |
| L206 | 98013-15-1 S204 | 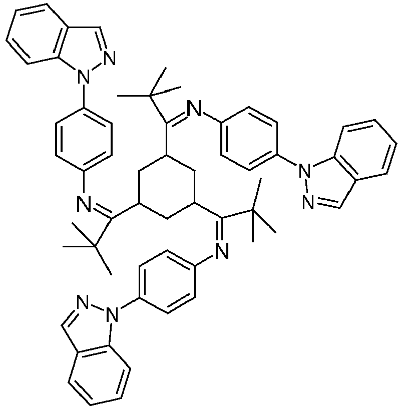 | 25 % |
| | | B | |
| L207 | 187805-79-4 1246767-56-5 | 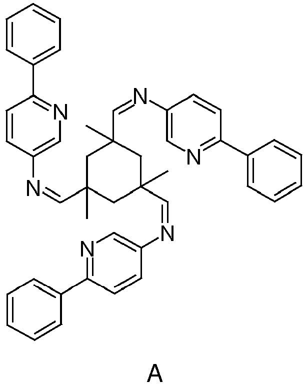 | 54 % |
| L208 | 187805-79-4 52090-60-5 | 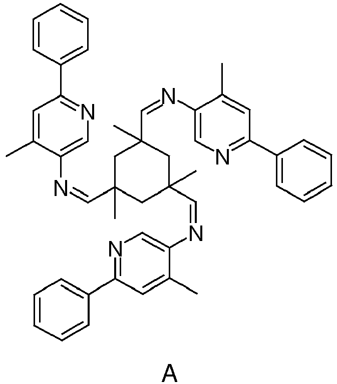 | 49 % |
| L209 | 98013-15-1 66728-99-2 | 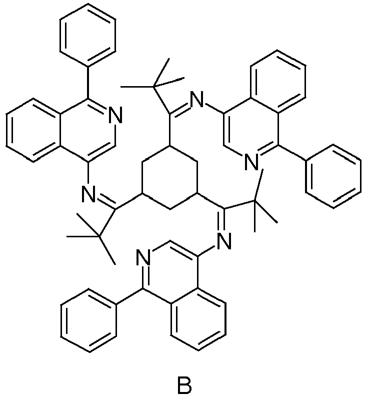 | 27% |
| L210 | 98013-15-1 1110656-27-3 | 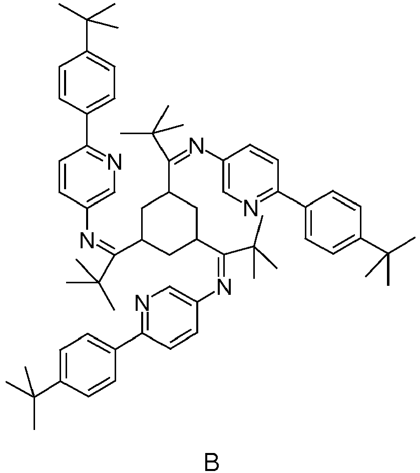 | 24% |
| L211 | 107354-37-0 1357165-91-3 | 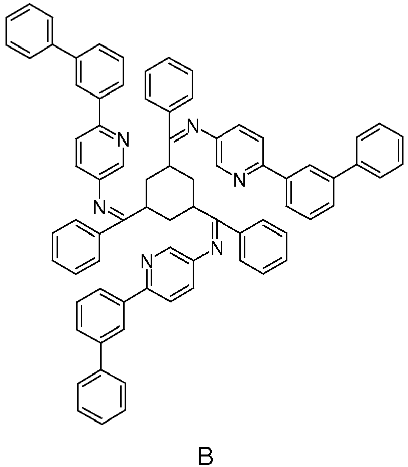 | 71 % |

### Beispiel L300:

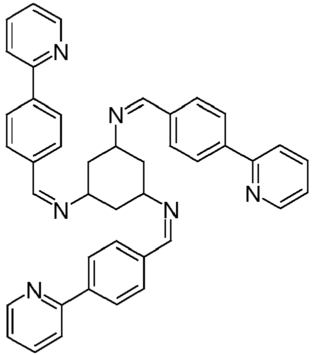

Ein Gemisch aus 3.9 g (30 mmol) cis,cis-1,3,5-Triaminocyclohexan [26150-46-9], 18.3 g (100 mmol) 4-(2-Pyridinyl)benzaldehyd [127406-56-8], 951 mg (5 mmol) 4-Toluolsulfonsäure-Monohydrat [6192-52-5] und 300 ml Mesitylen wird bis zur beendeten Wasserabscheidung unter Rückfluss erhitzt. Nach Erkalten entfernt man das Mesitylen im Vakuum und chromatographiert den Rückstand mit Cyclohexan:Ethylacetat:Triethylamin (90:9:1, vv) an Kieselgel (desaktiviert mit Triethylamin). Ausbeute: 15.0 g (24 mmol), 88 %. Reinheit: ca. 97 % n. ¹H-NMR.

Analog können die folgenden Verbindungen synthetisiert werden:

| Bsp. | Carbonyl-Komponente Amin | Produkt Variante | Ausbeute |
|---|---|---|---|
| L301 | 52199-29-8 478978-03-9 | 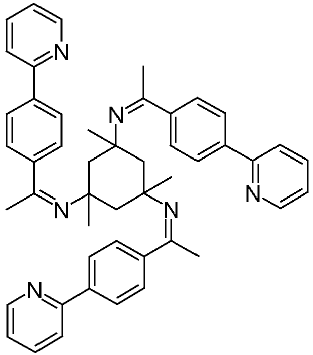 | 64% |
| L302 | 221910-24-3 478978-03-9 | 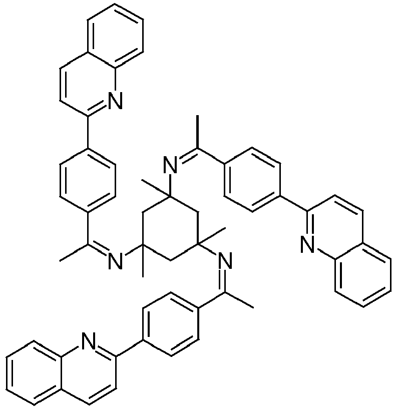 | 60 % |
| L303 | 1138735-13-3 478978-03-9 | 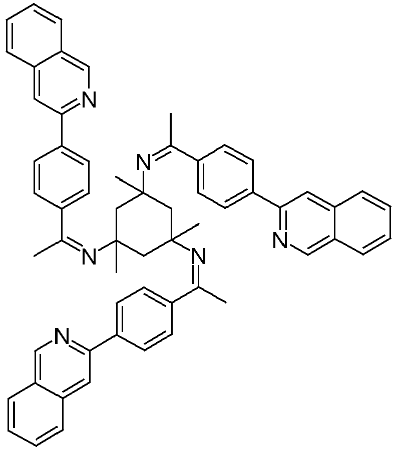 | 58 % |
| L304 | 1107640-93-6 582312-14-9 | 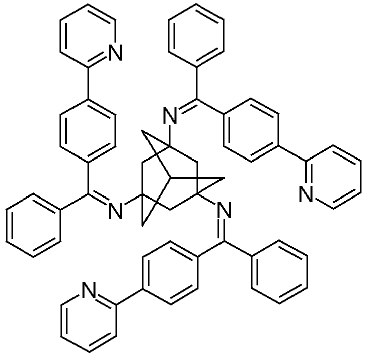 | 53 % |
| L305 | 1094356-84-9 478978-03-9 | 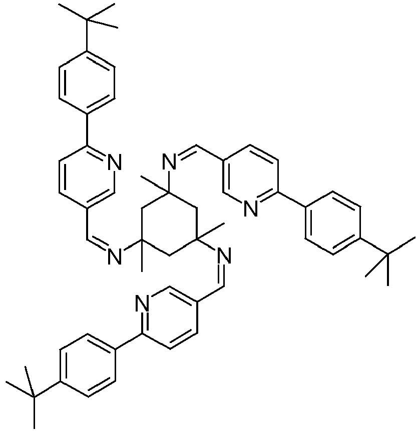 | 55 % |
| L306 | 1401797-64-5 582312-14-9 | 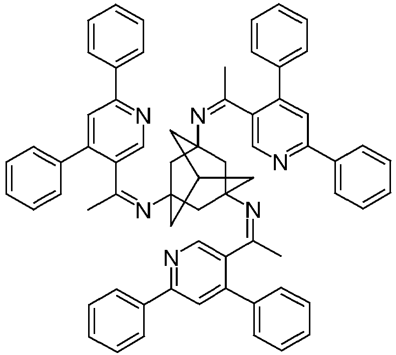 | 57% |
| L307 | 64869-17-6 582312-14-9 | 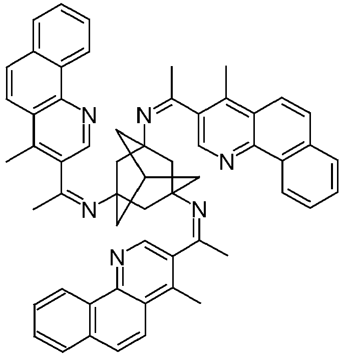 | 47% |
| L308 | 30091-51-1 478978-03-9 | 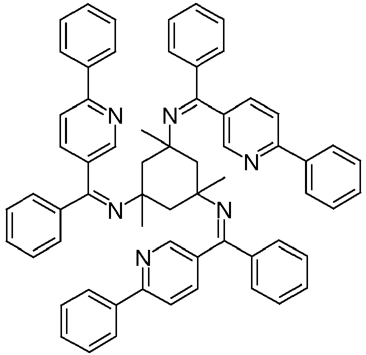 | 59 % |
| L309 | 1252578-97-4 478978-03-9 | 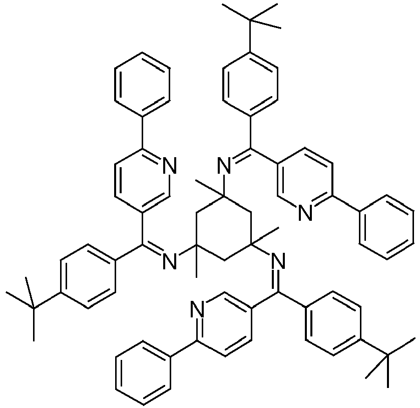 | 56 % |

### Beispiel L400:

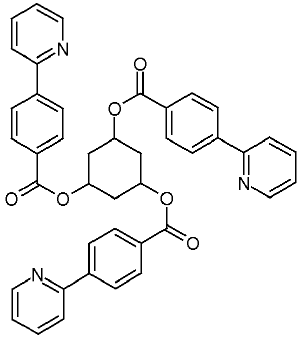

Eine gut gerührte Lösung von 4.0 g (30 mmol) cis,cis-1,3,5-Cyclohexan-triol [50409-12-6] in 100 ml Dichlormethan wird mit 28 ml Triethylamin und dann tropfenweise mit einer Lösung von 21.8 g [100 mmol) 4-(2-Pyridinyl)-benzoylchlorid [190850-37-4] in 100 ml Dichlormethan versetzt und 12 h unter Rückfluss gerührt. Nach Erkalten entfernt man die flüchtigen Bestandteile im Vakuum, rührt den Rückstand mit 300 ml heißem Methanol aus, saugt vom Produkt ab, wäscht dieses dreimal mit je 50 ml Methanol und kristallisiert abschließend aus Ethylacetat/Methanol um. Ausbeute: 14.0 g (21 mmol), 69 %. Reinheit: ca. 97 % n. ¹H-NMR.

Analog können folgende Verbindungen dargestellt werden, wobei die Reinigung der Rohprodukte durch Kugelrohrdestillation, Umkristallisation oder Chromatographie erfolgen kann. Wird ein Gemisch aus Alkoholen, Aminen oder Säurechloriden eingesetzt, können neben den symmetrischen, durch chromatographische Trennung (CombiFlash Torrent, Fa. Axel Semrau GmbH&Co KG), auch Liganden mit verschiedenen zweizähnigen Teilliganden erhalten werden.

| Bsp. | Edukt | Produkt | Ausbeute |
|---|---|---|---|
| L401 | 50409-12-6 | 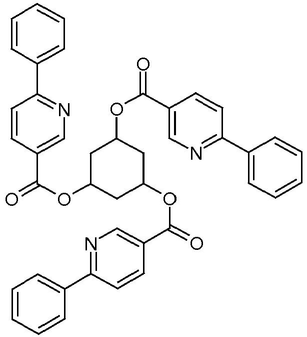 | 75 % |
| | 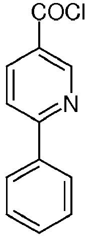 | | |
| | 257876-10-3 | | |
| L402 | 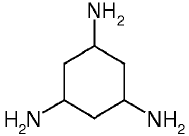 | 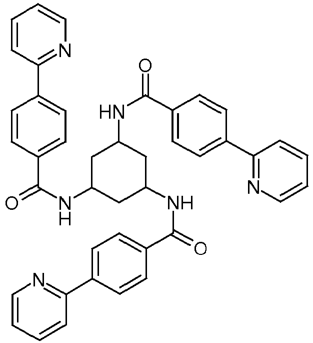 | 68 % |
| | 26150-46-9 | | |
| | 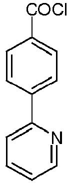 | | |
| | 190850-37-4 | | |
| L403 | 26150-46-9 | 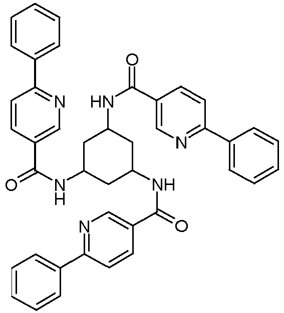 | 70 % |
| | 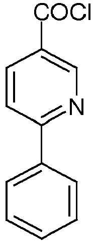 | | |
| | 257876-10-3 | | |
| L404 | 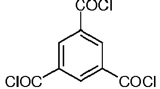 | 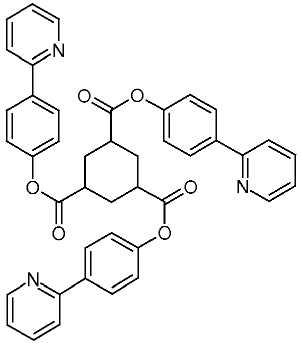 | 69 % |
| | 147365-19-3 | | |
| | 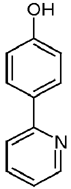 | | |
| | 51035-40-6 | | |
| L405 | 147365-19-3 | 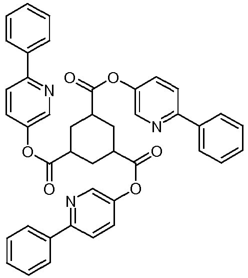 | 68 % |
| | 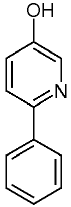 | | |
| | 66131-77-9 | | |
| L406 | 147365-19-3 | 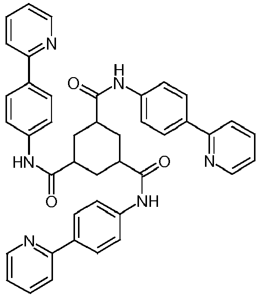 | 70 % |
| | 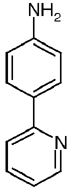 | | |
| | 18471-73-3 | | |
| L407 | 147365-19-3 | 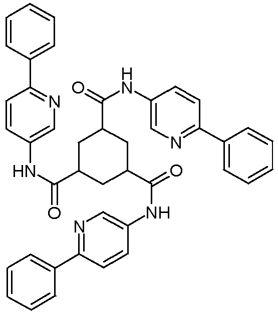 | 77 % |
| | 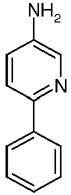 | | |
| | 126370-67-0 | | |
| L408 | 50409-12-6 | 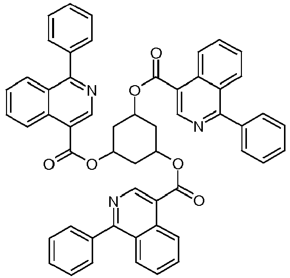 | 73% |
| | 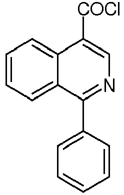 | | |
| | 90828-20-2 | | |
| L409 | 26150-46-9 | 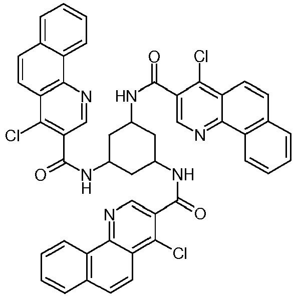 | 71 % |
| | 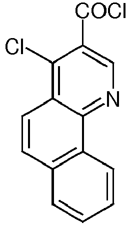 | | |
| | 37041-29-5 | | |
| L410 | 50409-12-6 | 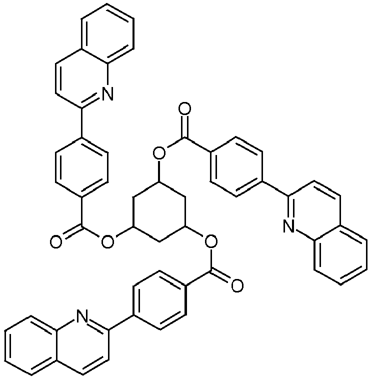 | 73 % |
| | 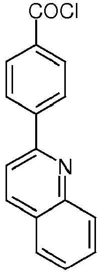 | | |
| | 854167-98-9 | | |
| L411 | 26150-46-9 | 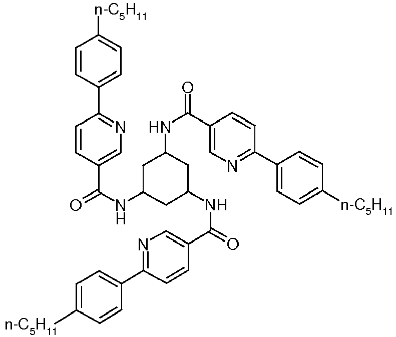 | 64% |
| | 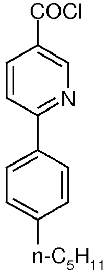 | | |
| | 111647-50-8 | | |
| L412 | 147365-19-3 | 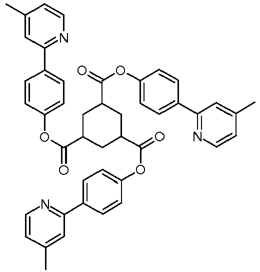 | 69 % |
| | 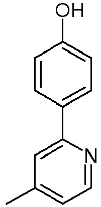 | | |
| | 53164-95-7 | | |
| L413 | 147365-19-3 | 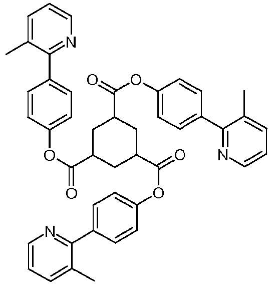 | 66 % |
| | 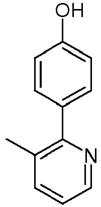 | | |
| | 371201-06-8 | | |
| L414 | 147365-19-3 | 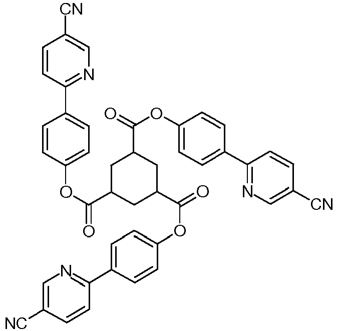 | 64% |
| | 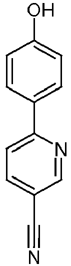 | | |
| | 149353-76-4 | | |
| L415 | 147365-19-3 | 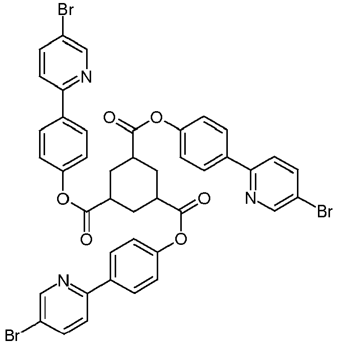 | 65 % |
| | 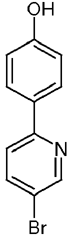 | | |
| | 1032825-10-7 | | |
| L416 | 147365-19-3 | 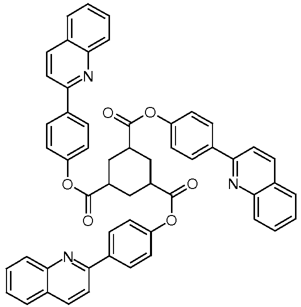 | 68 % |
| | 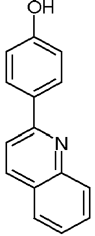 | | |
| | 30696-03-8 | | |
| L417 | 147365-19-3 | 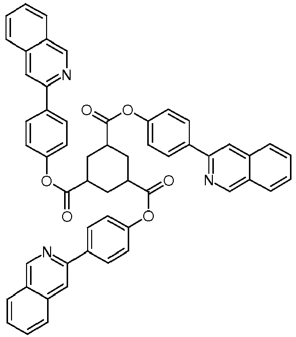 | 66 % |
| | 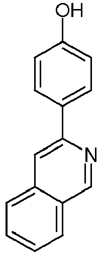 | | |
| | 884500-88-3 | | |
| L418 | 147365-19-3 | 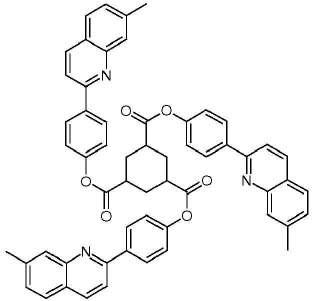 | 69 % |
| | 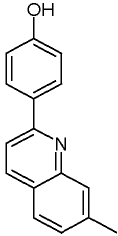 | | |
| | 855839-55-3 | | |
| L419 | 147365-19-3 | 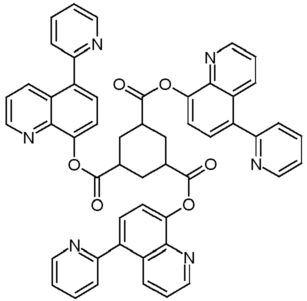 | 68 % |
| | 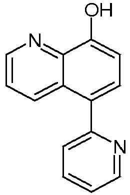 | | |
| | 775344-00-8 | | |
| L420 | 147365-19-3 | 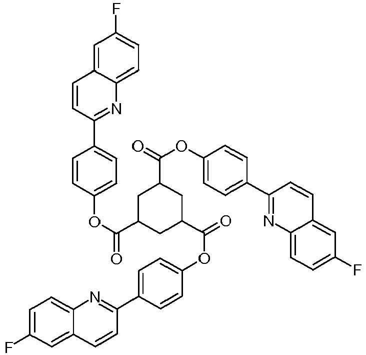 | 70 % |
| | 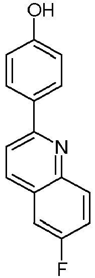 | | |
| | 54231-47-9 | | |
| L421 | 147365-19-3 | 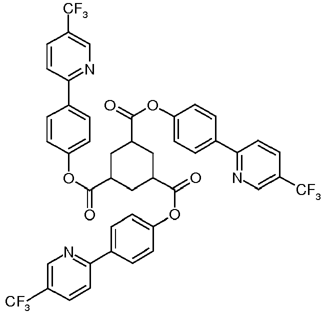 | 59 % |
| | 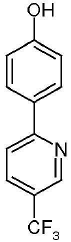 | | |
| | 139218-75-0 | | |
| L422 | 147365-19-3 | 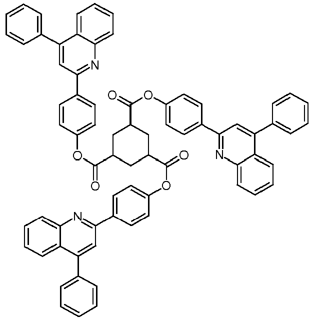 | 63 % |
| | 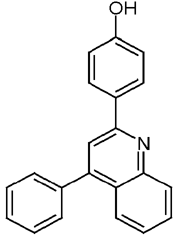 | | |
| | 1087269-19-9 | | |
| L423 | 147365-19-3 | 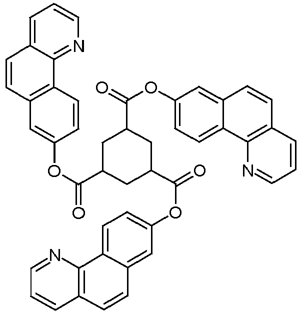 | 70 % |
| | 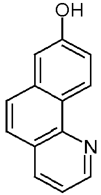 | | |
| | 57442-05-4 | | |
| L424 | 147365-19-3 | 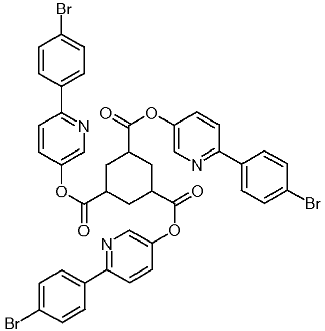 | 65 % |
| | 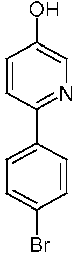 | | |
| | 150595-78-1 | | |
| L425 | 147365-19-3 | 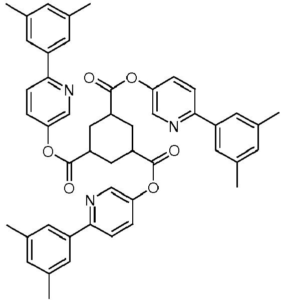 | 67% |
| | 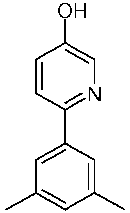 | | |
| | 1261970-83-5 | | |
| L426 | 147365-19-3 | 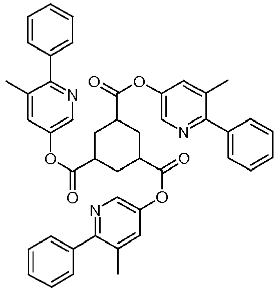 | 68 % |
| | 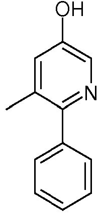 | | |
| | 942134-44-3 | | |
| L427 | 147365-19-3 | 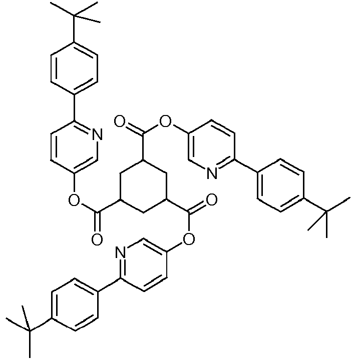 | 65 % |
| | 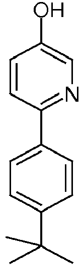 | | |
| | 906101-30-2 | | |
| L428 | 147365-19-3 | 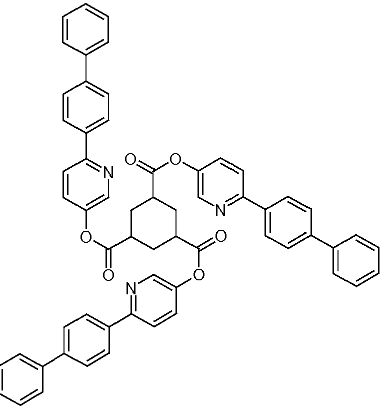 | 71 % |
| | 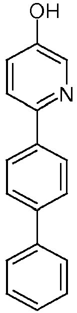 | | |
| | 1551357-67-5 | | |
| L429 | 147365-19-3 | 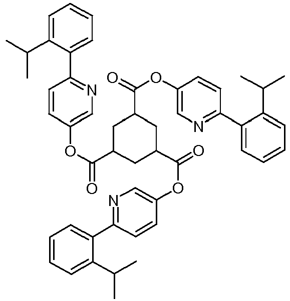 | 53 % |
| | 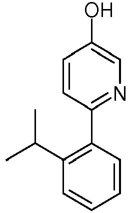 | | |
| | 1876770-00-1 | | |
| L430 | 147365-19-3 | 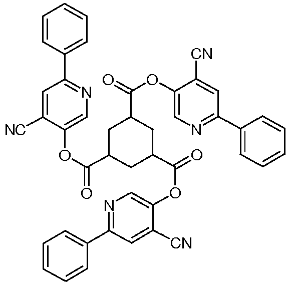 | 68 % |
| | 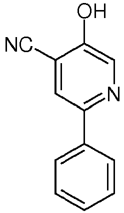 | | |
| | 1903525-21-2 | | |
| L431 | 147365-19-3 | 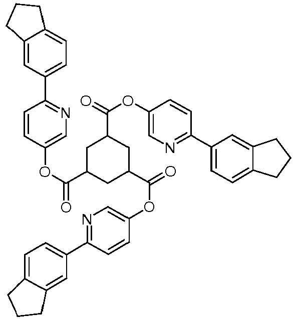 | 69 % |
| | 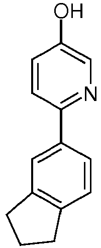 | | |
| | 1698352-04-3 | | |
| L432 | 147365-19-3 | 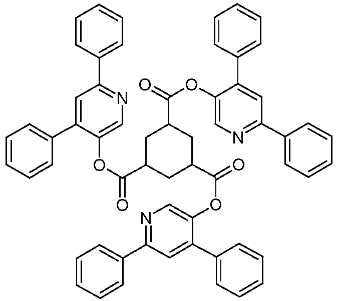 | 70 % |
| | 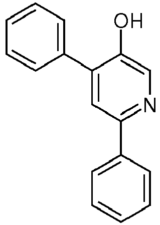 | | |
| | 76570-31-5 | | |
| L433 | 147365-19-3 | 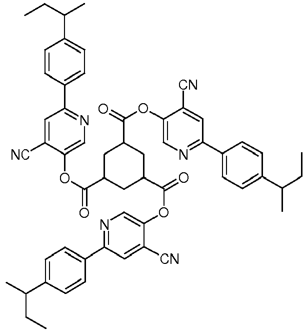 | 58 % |
| | 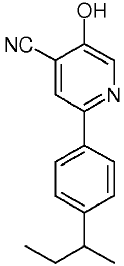 | | |
| | 1894503-17-3 | | |
| L434 | 147365-19-3 | 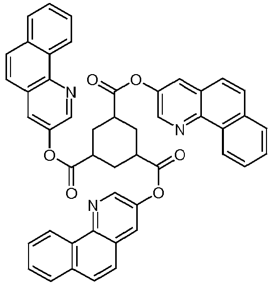 | 68 % |
| | 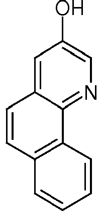 | | |
| | 91804-13-6 | | |
| L435 | 147365-19-3 | 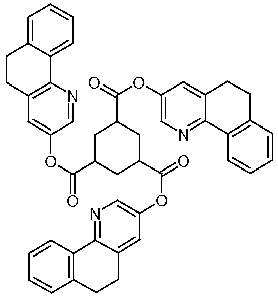 | 69 % |
| | 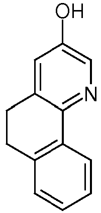 | | |
| | 84731-41-9 | | |
| L436 | 147365-19-3 | 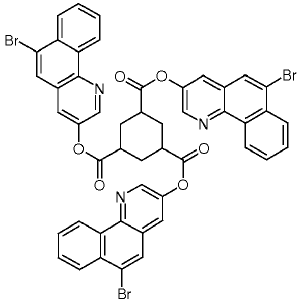 | 67% |
| | 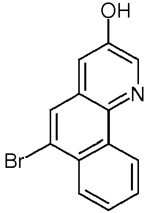 | | |
| | 1702400-65-4 | | |
| L437 | 147365-19-3 | 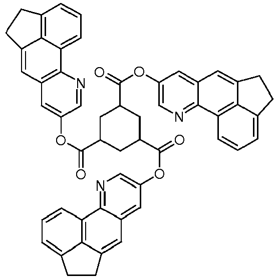 | 65 % |
| | 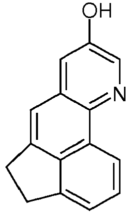 | | |
| | 66404-96-4 | | |
| L438 | 147365-19-3 | 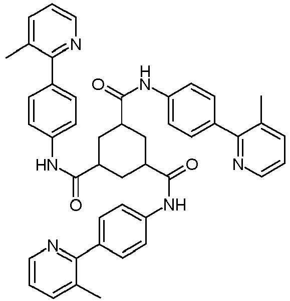 | 73 % |
| | 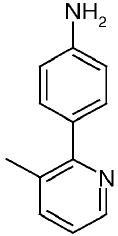 | | |
| | 885955-74-8 | | |
| L439 | 147365-19-3 | 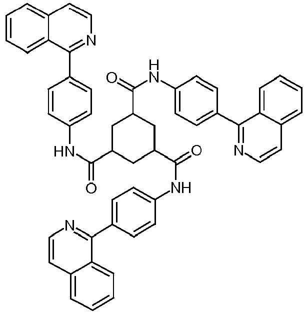 | 79 % |
| | 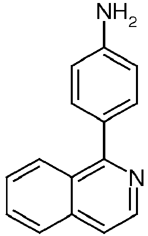 | | |
| | 58992-84-0 | | |
| L440 | 147365-19-3 | 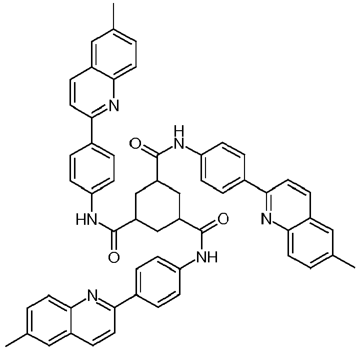 | 73 % |
| | 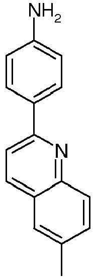 | | |
| | 1224953-47-2 | | |
| L441 | 147365-19-3 | 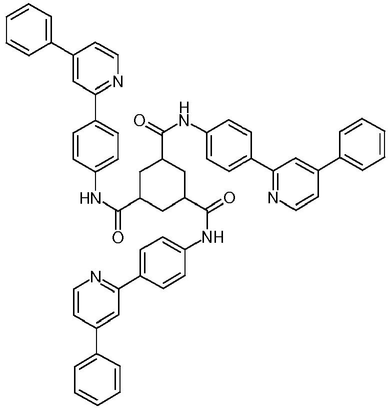 | 70 % |
| | 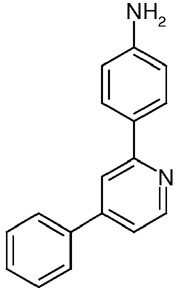 | | |
| | 1351665-31-0 | | |
| L442 | 147365-19-3 | 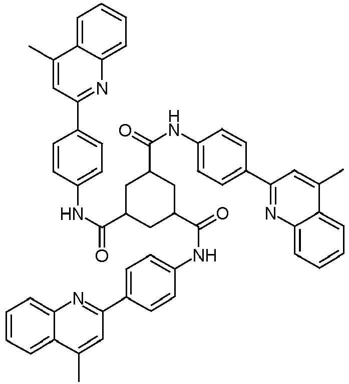 | 74% |
| | 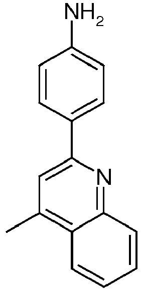 | | |
| | 580-38-1 | | |
| L443 | 147365-19-3 | 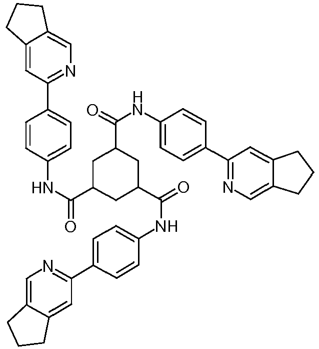 | 69 % |
| | 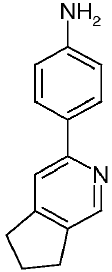 | | |
| | 1798331-49-3 | | |
| L444 | 147365-19-3 | 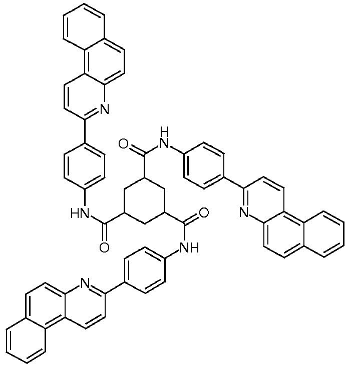 | 72 % |
| | 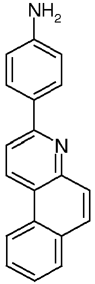 | | |
| | 94211-88-8 | | |
| L445 | 147365-19-3 | 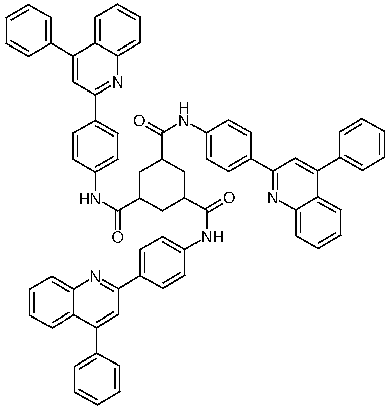 | 74% |
| | 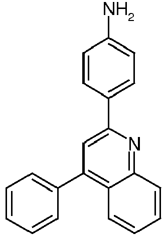 | | |
| | 530086-92-1 | | |
| L446 | 147365-19-3 | 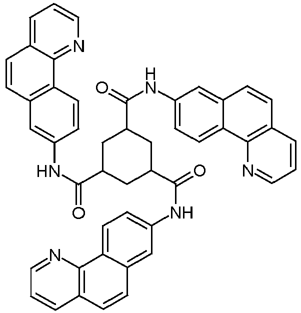 | 71 % |
| | 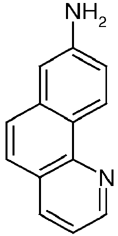 | | |
| | 344285-96-7 | | |
| L447 | 147365-19-3 | 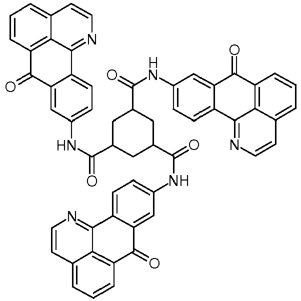 | 72 % |
| | 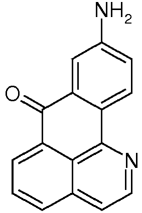 | | |
| | 13102354-6 | | |
| L448 | 147365-19-3 | 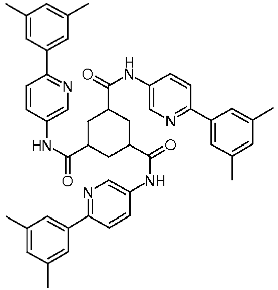 | 70 % |
| | 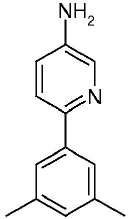 | | |
| | 1159407-94-9 | | |
| L449 | 147365-19-3 | 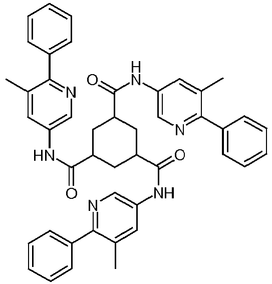 | 69 % |
| | 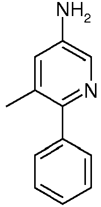 | | |
| | 126370-67-0 | | |
| L450 | 147365-19-3 | 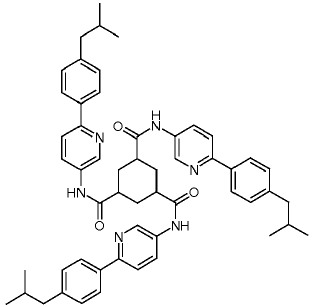 | 65 % |
| | 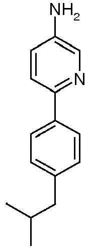 | | |
| | 1554504-03-8 | | |
| L451 | 147365-19-3 | 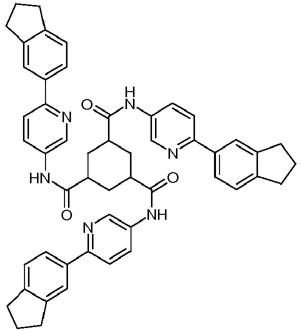 | 71 % |
| | 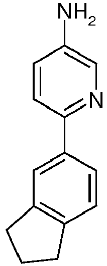 | | |
| | 1551869-82-9 | | |
| L452 | 147365-19-3 | 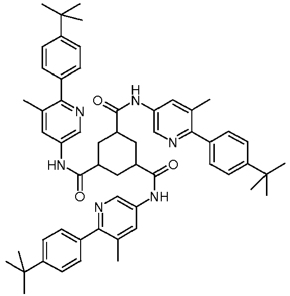 | 67% |
| | 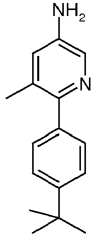 | | |
| | 1192165-48-2 | | |
| L453 | 147365-19-3 | 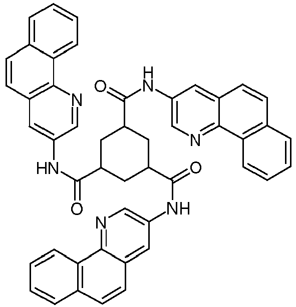 | 70 % |
| | 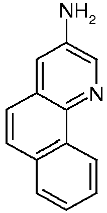 | | |
| | 151585-47-6 | | |
| L454 | 147365-19-3 | 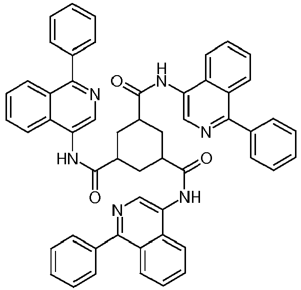 | 67% |
| | 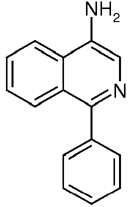 | | |
| | 66728-99-2 | | |
| L455 | 147365-19-3 | 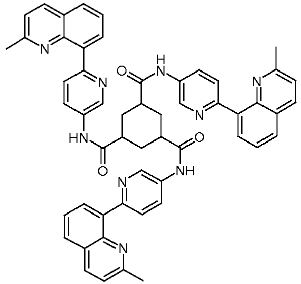 | 74% |
| | 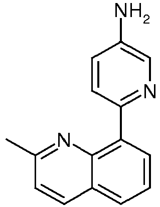 | | |
| | 1357166-67-6 | | |
| L456 | 147365-19-3 | 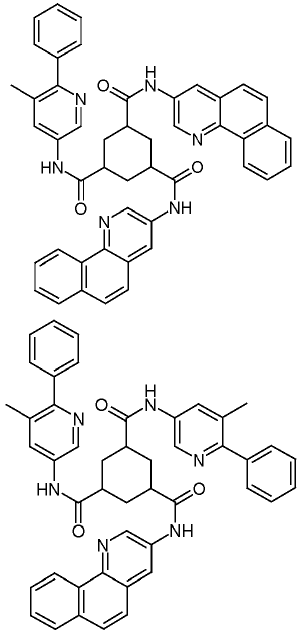 | 19 % |
| | 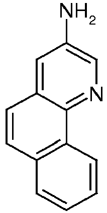 | | |
| | 151585-47-6 | | |
| L457 | 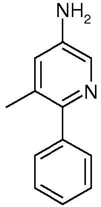 | | 16 % |
| | 126370-67-0 | | |
| L458 | 147365-19-3 | 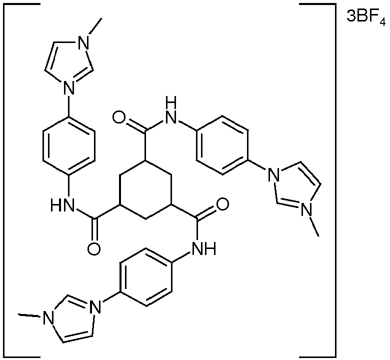 | 28 % |
| | 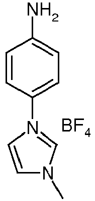 | | |
| | 1870003-70-5 Zugabe als DMF-Lösung | | |
| L459 | 147365-19-3 | 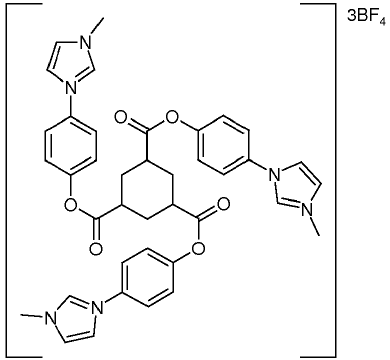 | 33 % |
| | 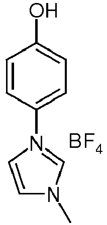 | | |
| | 1870003-72-7 Zugabe als DMF-Lösung | | |
| L460 | 147365-19-3 | 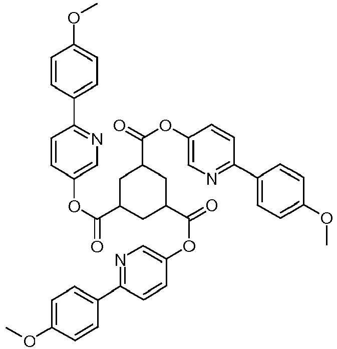 | 70 % |
| | 1255636-82-8 | | |

### Beispiel L500:

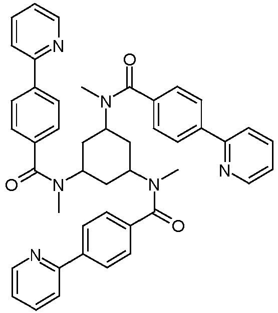

Eine Suspension von 6.7 g (10 mmol) L402 in 150 ml Dimethylacetamid wird portionsweise mit 1.2 g (50 mmol) Natriumhydrid versetzt und 30 min. bei Raumtemperatur gerührt. Dann gibt man 2.1 ml (33 mmol) Methyliodid [74-88-4] zu und erwärmt für 16 h auf 60 °C. Man fügt tropfenweise 20 ml konz. Ammoniaklösung zu, rührt 30 min. nach, entfernt das Lösungsmittel weitgehend im Vakuum, nimmt den Rückstand in 300 ml Dichlormethan auf, wäscht einmal mit 200 ml 5 Gew.-%igem Ammoniakwasser, zweimal mit je 100 ml Wasser, einmal mit 100 ml ges. Kochsalzlösung und trocknet dann über Magnesiumsulfat. Man entfernt das Dichlormethan im Vakuum und kristallisiert das Rohprodukt aus Ethylacetat/Methanol um. Ausbeute: 5.0 g (7.0 mmol), 70 %. Reinheit: ca. 97 % n. ¹H-NMR.

Analog können folgende Verbindungen dargestellt werden, wobei anstelle von Methyliodid die angegebenen Elektrophile verwendet wird. Bei Verwendung sekundärer Alkylhalogenide wird 60 mmol NaH und 60 mmol des sekundären Alkylierungsmittels verwendet. Die Reinigung der Rohprodukte kann durch Kugelrohrdestillation, Umkristallisation oder Chromatographie erfolgen.

| Bsp. | Edukte | Produkt | Ausbeute |
|---|---|---|---|
| L501 | L403 74-88-4 | 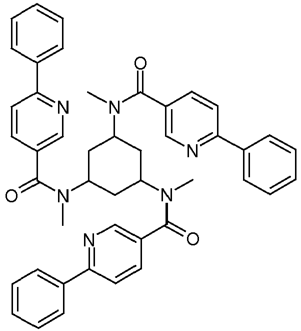 | 72 % |
| L502 | L406 74-88-4 | 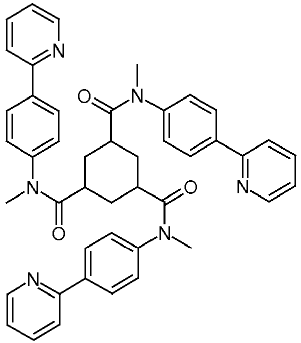 | 76 % |
| L503 | L407 74-88-4 | 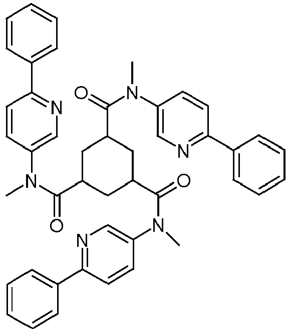 | 71 % |
| L504 | L409 74-88-4 | 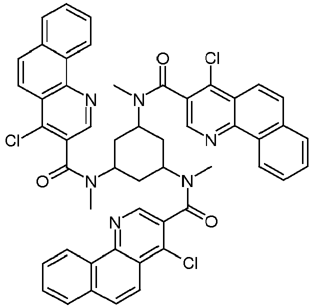 | 68 % |
| L505 | L411 865-50-9 | 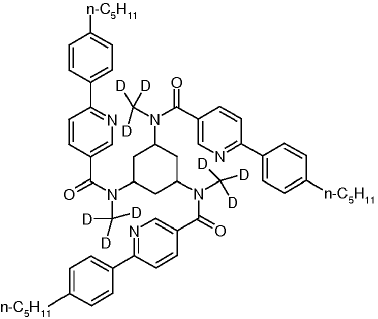 | 66 % |
| L506 | L438 71162-19-1 | 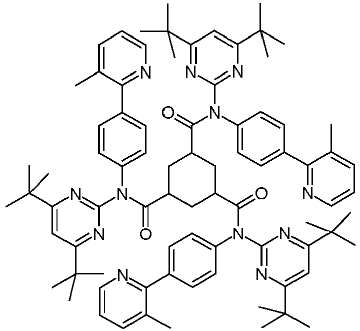 | 39 % |
| L507 | L439 29394-58-9 | 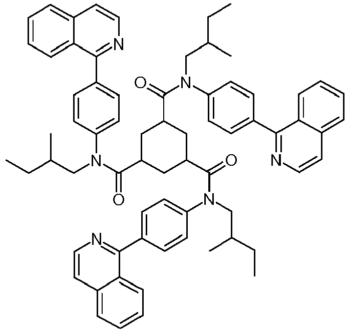 | 59 % |
| L508 | L440 75-03-6 | 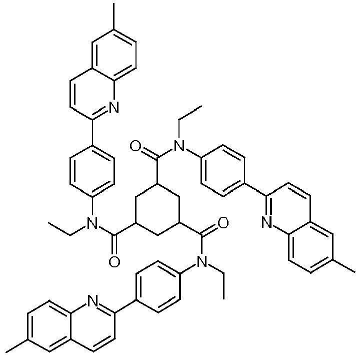 | 70 % |
| L509 | L441 15501-33-4 | 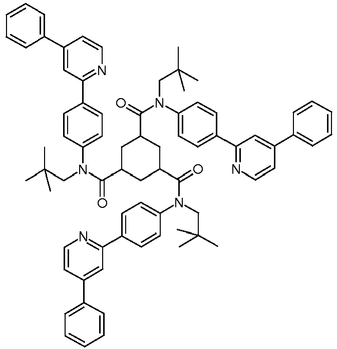 | 71 % |
| L510 | L442 74-88-4 24424-99-5 | 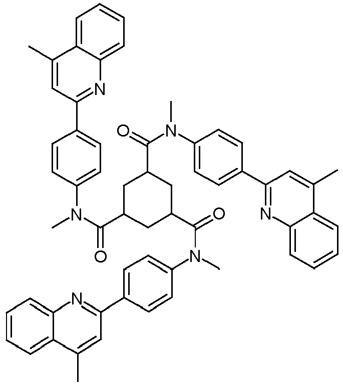 | 73 % |
| L511 | L443 24424-99-5 | 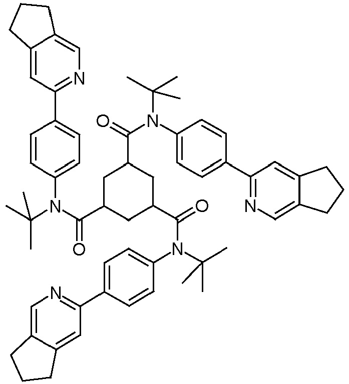 | 69 % |
| L512 | L444 865-50-9 | 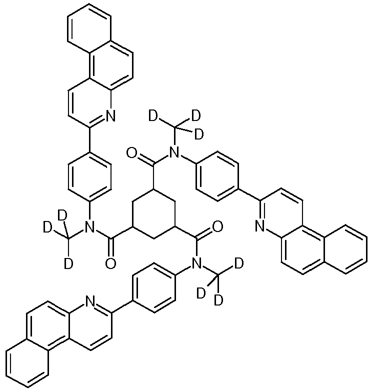 | 68 % |
| L513 | L445 75-26-3 | 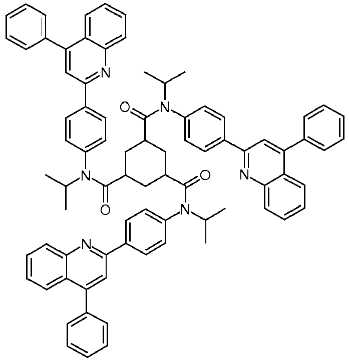 | 42 % |
| L514 | L447 513-38-2 | 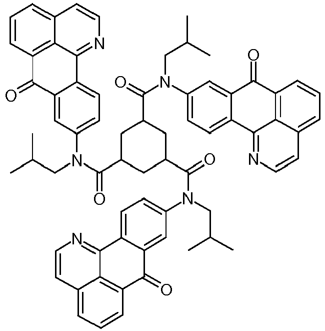 | 65 % |
| L515 | L450 15501-33-4 | 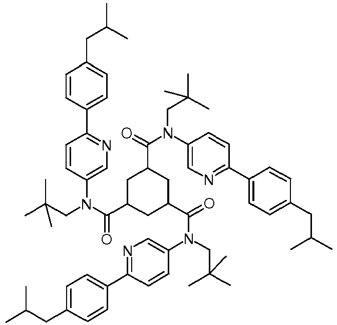 | 63 % |
| L516 | L451 620-05-3 | 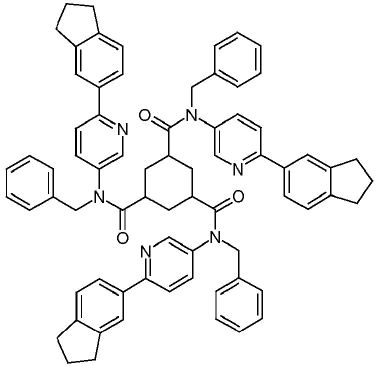 | 70 % |
| L517 | L453 15501-33-4 | 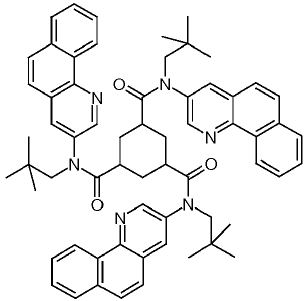 | 61 % |
| L518 | L454 15501-33-4 | 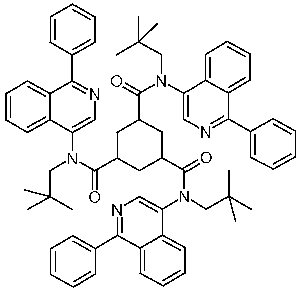 | 68 % |
| L519 | L455 74-88-4 | 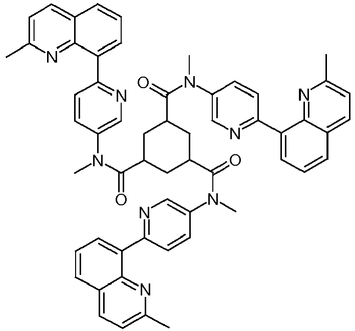 | 61 % |
| L520 | L456 75-77-4 | 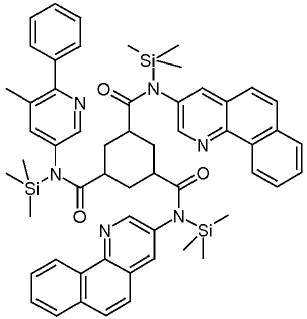 | 41% |
| L521 | L457 15501-33-4 | 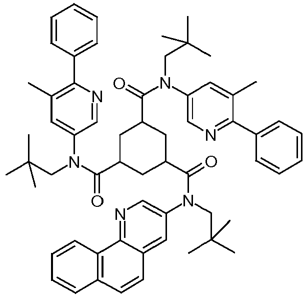 | 72 % |
| L522 | L458 74-88-4 Base Cs₂CO₃ Lösungsmittel Aceton | 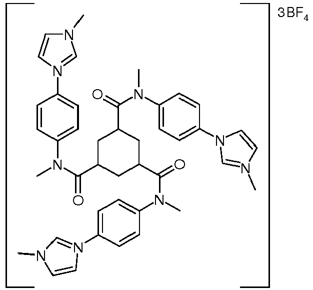 | 40 % |

### Beispiel L600:

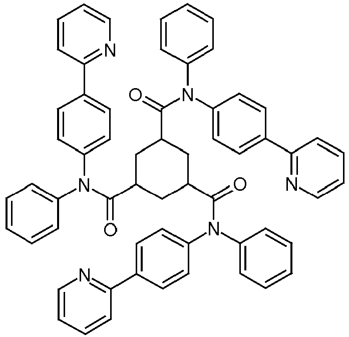

Ein Gemisch aus 6.7 g (10 mmol) L406, 4.5 ml (40 mmol) lodbenzol [591-50-4], 12.7 g (60 mmol) Trikaliumphosphat, 292 mg (1.5 mmol) Kupfer(I)-iodid, 553 mg (3 mmol) 2,2,6,6-Tetramethyl-3,5-heptandion [1118-71-4], 50 g Glaskugeln (3 mm Durchmesser) und 150 ml o-Xylol wird 24 h auf 130 °C erhitzt. Nach Erkalten entfernt man das Lösungsmittel im Vakuum, nimmt den Rückstand in 500 ml Dichlormethan auf, filtriert von den Salzen über ein vorgeschlämmtes Celite-Bett ab, wäscht das Filtrat dreimal mit 100 ml 5 Gew.-%iger. Ammoniak-Lösung und einmal mit 100 ml Wasser und trocknet dann über Magnesiumsulfat. Das nach Entfernen des Lösungsmittels erhaltene Rohprodukt wird aus Ethylacetat/Methanol umkristallisiert. Ausbeute: 6.5 g (7.2 mmol), 72 %. Reinheit: ca. 97 % n. ¹H-NMR.

Analog können folgende Verbindungen dargestellt werden. Die Reinigung der Rohprodukte kann durch Kugelrohrdestillation, Umkristallisation oder Chromatographie erfolgen kann.

| Bsp. | Edukte | Produkt | Ausbeute |
|---|---|---|---|
| L601 | L446 37055-53-1 | 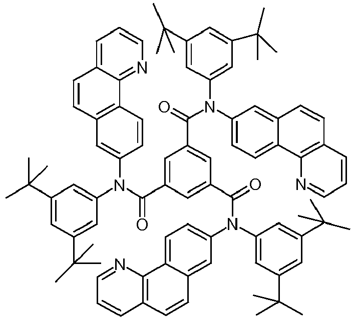 | 51 % |
| L602 | L448 20442-79-9 | 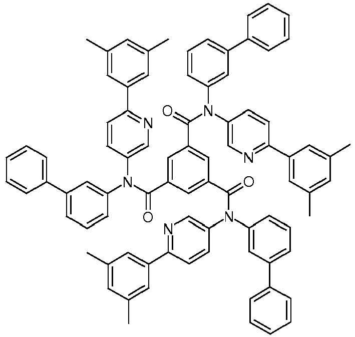 | 56 % |
| L603 | L449 857784-97-5 | 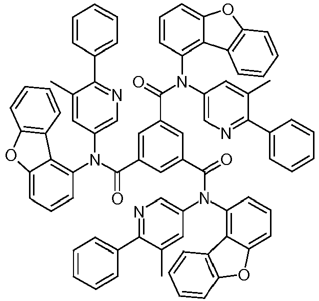 | 33 % |
| L604 | L452 1643766-87-3 | 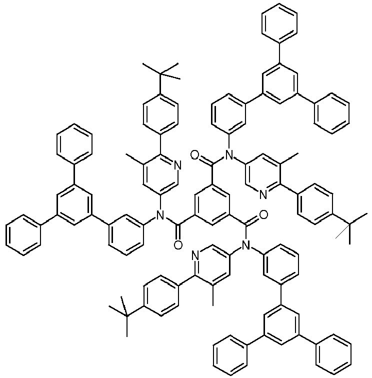 | 61 % |

### Beispiel L700:

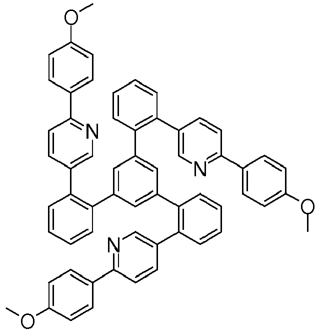

Ein gut gerührtes Gemisch aus 16.3 g (30 mmol) 1,3,5-Tris(2-bromphenyl)benzol [380626-56-2], 31.1 g (100 mmol) 2-(4-Methoxyphenyl)-5-(4,4,5,5-tetramethyl-[1,3,2]dioxa-borolan-2-yl)-pyridin [1374263-53-2], 42.5 g (200 mol) Trikaliumphosphat, 534 mg (1.3 mmol) SPhos [657408-07-6], 224 mg (1.0 mmol) Palladium(II)acetat, 300 ml Toluol, 100 ml Dioxan und 300 ml Wasser wird 16 h unter Rückfluss erhitzt. Nach Erkalten, trennt man die wässrige Phase ab und engt die organische Phase zur Tockene ein. Man nimmt den braunen Schaum in 300 ml Ethylacetat auf und filtriert über ein mit Ethylacatat vorgeschlämmtes Kieselgelbett (Durchmesser 15 cm, Länge 20 cm), um braune Anteile zu entfernen. Nach Einengen auf 100 ml wird die warme Lösung unter sehr gutem Rühren tropfenwiese mit 300 ml Methanol versetzt, wobei ein beiger Feststoff auskristallisiert. Der Feststoff wird abgesaugt, zweimal mit je 100 ml Methanol gewaschen und im Vakuum getrocknet. Ausbeute: 20.5 g (24 mmol), 80 %. Reinheit: ca. 95 %ig nach ¹H-NMR.

Analog können folgende Verbindungen dargestellt werden.

| Bsp. | Edukte | Produkt | Ausbeute |
|---|---|---|---|
| L701 | S300 | 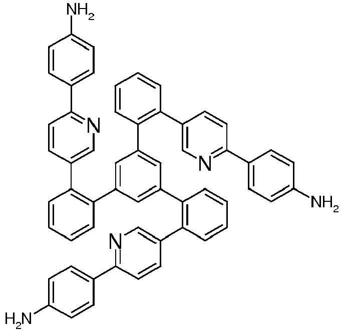 | 54% |
| L702 | S301 | 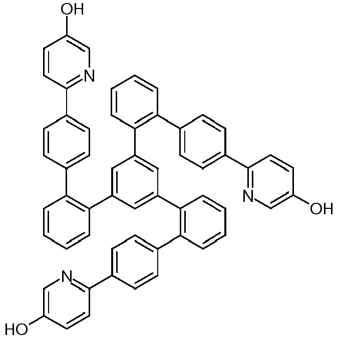 | 57% |
| L703 | S302 | 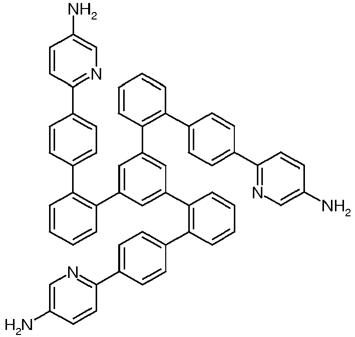 | 49 % |

### 3. Darstellung der Metallkomplexe:

### Beispiel Ir(L1):

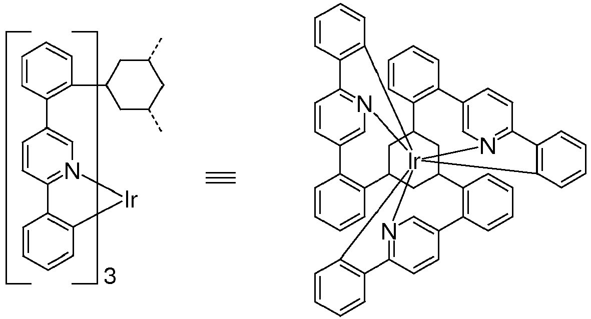

Ein Gemisch aus 7.72 g (10 mmol) des Liganden L1, 4.90 g (10 mmol) Tris-acetylacetonato-iridium(III) [15635-87-7] und 100 g Hydrochinon [123-31-9] werden in einem 500 mL Zweihalsrundkolben mit einem glasummantelten Magnetkern vorgelegt. Der Kolben wird mit einem Wasserabscheider (für Medien geringerer Dichte als Wasser) und einem Luftkühler mit Argonüberlagerung versehen. Der Kolben wird in einer Metallheizschale platziert. Die Apparatur wird über die Argonüberlagerung von oben her 15 min. mit Argon gespült, wobei man das Argon aus dem Seitenhals des Zweihalskolbens ausströmen lässt. Über den Seitenhals des Zweihalskolbens führt man ein glasummanteltes Pt-100 Thermoelement in den Kolben ein und platziert das Ende kurz oberhalb des Magnetrührkerns. Dann wird die Apparatur mit mehreren lockeren Wicklungen von Haushaltsaluminiumfolie thermisch isoliert, wobei die Isolation bis zur Mitte des Steigrohres des Wasserabscheiders geführt wird. Dann wird die Apparatur schnell mit einem Laborheizrührwerk auf 250 - 260 °C, gemessen am Pt-100 Thermofühler, der in die aufgeschmolzene, gerührte Reaktionsmischung eintaucht, erhitzt. Während der nächsten 1.5 h wird das Reaktionsgemisch bei 250 - 260 °C gehalten, wobei wenig Kondensat abdestilliert und sich im Wasserabscheider sammelt. Man lässt die Reaktionsmischung auf 190 °C abkühlen, tropft 50 ml Ethylenglykol zu, lässt auf ca. 70 °C abkühlen und tropft dann 250 ml Methanol zu. Nach Erkalten wird die so erhaltene beige Suspension über eine Umkehrfritte filtriert, der beige Feststoff wird dreimal mit 50 ml Methanol gewaschen und dann im Vakuum getrocknet. Rohausbeute: quantitativ. Der so erhaltene Feststoff wird in 1000 ml Dichlormethan gelöst und über ca. 800 g mit Dichlormethan vorgeschlämmtes Kieselgel (Säulendurchmesser ca. 18 cm) unter Luft- und Lichtausschluss filtriert, wobei dunkle Anteile am Start liegen bleiben. Die Kernfraktion wird herausgeschnitten, am Rotationsverdampfer weitgehend eingeengt, wobei gleichzeitig kontinuierlich MeOH bis zur Kristallisation zugetropft wird. Nach Absaugen, Waschen mit wenig MeOH und Trocknen im Vakuum erfolgt die weitere Reinigung des gelben Produkts durch fünfmalige kontinuierliche Heißextraktion mit DCM (Vorlagemenge jeweils ca. 150 ml, Extraktionshülse: Standard Soxhletthülsen aus Cellulose der Fa. Whatman) unter sorgfältigem Luft- und Lichtausschluss. Ausbeute: 7.03 g (7.3 mmol), 73 %. Reinheit: > 99.9 % nach HPLC.

Analog können folgende Verbindungen dargestellt werden:

| Bsp. | Ligand | Produkt Reaktionszeit* Reaktionstemperatur* Extraktionsmittel* | Ausbeute |
|---|---|---|---|
| Rh(L1) | L1 | 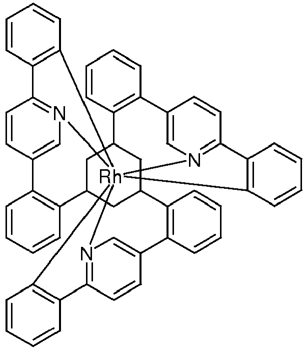 | 36 % |
| | | Rh(L1 ) Verwendung von Rh(acac)₃ [14284-92-5] | |
| Ir(L2) | L2 | Ir(L2) Acetonitril | 57% |
| Ir(L3) | L3 | 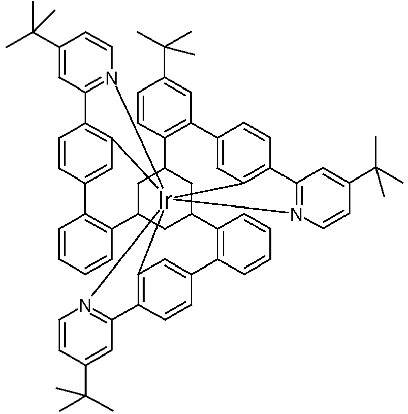 | 54% |
| | | Ir(L3) Ethylacetat | |
| Ir(L4) | L4 | Ir(L4) Butylacetat | 59 % |
| Ir(L5) | L5 | Ir(L5) Toluol | 60 % |
| Ir(L6) | L6 | Ir(L6) Toluol | 61 % |
| Ir(L7) | L7 | 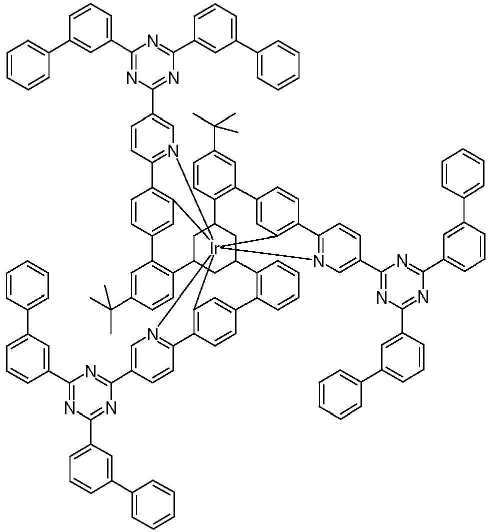 | 57% |
| | | Ir(L7) Toluol | |
| Ir(L8) | L8 | Ir(L8) Toluol | 27% |
| Ir(L9) | L9 | 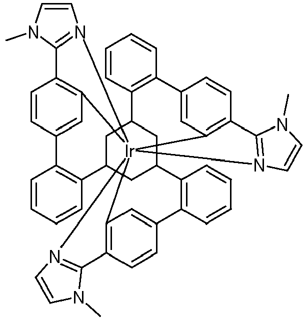 | 63 % |
| | | Ir(L9) Butylacetat | |
| Ir(L10) | L10 | Ir(L10) o-Xylol | 65 % |
| Ir(L11) | L11 | Ir(L11) 265 °C / 2 h | 53 % |
| Ir(L12) | L12 | Ir(L12) 265 °C / 2 h | 57% |
| Ir(L13) | L13 | Ir(L13) Toluol | 51 % |
| Ir(L14) | L14 | Ir(L14) Toluol | 39 % |
| Ir(L15) | L15 | 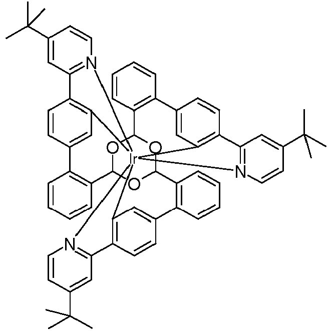 | 24% |
| | | Ir(L15) | |
| Ir(L100) | L100 | 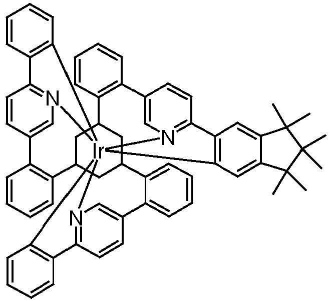 | 57% |
| | | Ir(L100) | |
| Ir(L101) | L101 | Ir(L101) DCM | 62 % |
| Ir(L102) | L102 | Ir(L102) DCM | 65 % |
| Ir(L103) | L103 | 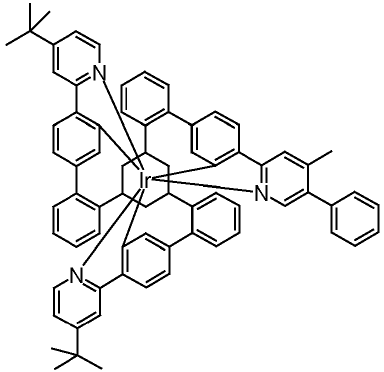 | 61 % |
| | | Ir(L103) | |
| Ir(L104) | L104 | Ir(L104) | 58 % |
| Ir(L105) | L105 | Ir(L105) | 61 % |
| Ir(L106) | L106 | Ir(L106) | 65 % |
| Ir(L107) | L107 | Ir(L107) | 65 % |
| Ir(L108) | L108 | Ir(L108) o-Xylol | 57% |
| Ir(L109) | L109 | Ir(L109) | 70 % |
| Ir(L110) | L110 | Ir(L110) | 63 % |
| Ir(L111) | L111 | Ir(L111) | 60 % |
| Ir(L112) | L112 | Ir(L112) | 62 % |
| Ir(L113) | L113 | Ir(L113) | 66 % |
| Ir(L114) | L114 | Ir(L114) | 58 % |
| Ir(L115) | L115 | Ir(L115) o-Xylol | 55 % |
| Ir(L116) | L116 | Ir(L116) | 60 % |
| Ir(L117) | L117 | Ir(L117) | 69 % |
| Ir(L118) | L118 | Ir(L118) o-Xylol | 55 % |
| Ir(L119) | L119 | Ir(L119) | |
| Ir(L120) | L120 | Ir(L120) | 61 % |
| Ir(L121) | L121 | Ir(L121) DCM | 54% |
| Ir(L122) | L122 | Ir(L122) DCM | 56 % |
| Ir(L123) | L123 | Ir(L123) | 70 % |
| Ir(L124) | L124 | Ir(L124) | 67% |
| Ir(L200) | L200 | 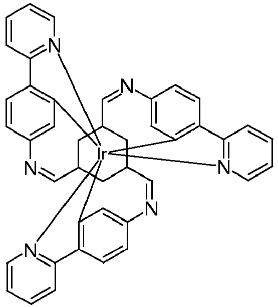 | 23 % |
| | | Ir(L200) DCM | |
| Ir(L201) | L201 | 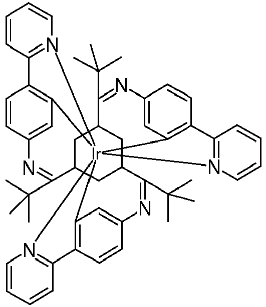 | 34% |
| | | Ir(L201) | |
| Ir(L202) | L202 | Ir(L202) | 37% |
| Ir(L203) | L203 | Ir(L203) | 35 % |
| Ir(L204) | L204 | Ir(L204) | 28 % |
| Ir(L205) | L205 | Ir(L205) | 40 % |
| Ir(L206) | L206 | 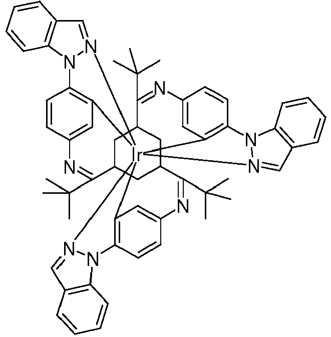 | 29 % |
| | | Ir(L206) | |
| Ir(L207) | L207 | Ir(L207) | 33 % |
| Ir(L208) | L208 | Ir(L208) | 36 % |
| Ir(L209) | L209 | Ir(L209) | 29 % |
| Ir(L210) | L210 | Ir(L210) | 32 % |
| Ir(L211) | L211 | Ir(L211) | 39 % |
| Ir(L300) | L300 | 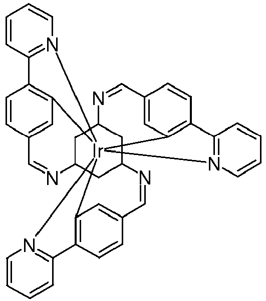 | 27% |
| | | Ir(L300) | |
| Ir(L301) | L301 | Ir(L301) | 35 % |
| Ir(L302) | L302 | Ir(L302) | 14% |
| Ir(L303) | L303 | Ir(L303) | 28 % |
| Ir(L304) | L304 | Ir(L304) | 38 % |
| Ir(L305) | L305 | Ir(L305) | 35 % |
| Ir(L306) | L306 | 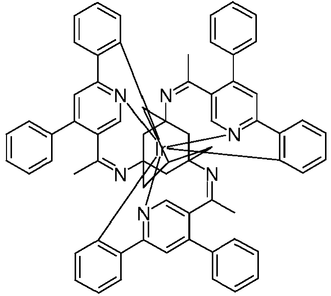 | 38 % |
| | | Ir(L306) | |
| Ir(L307) | L307 | Ir(L307) 265 °C / 2h | 24% |
| Ir(L308) | L308 | Ir(L308) | 33 % |
| Ir(L309) | L309 | Ir(L309) | 31 % |
| Ir(L400) | L400 | 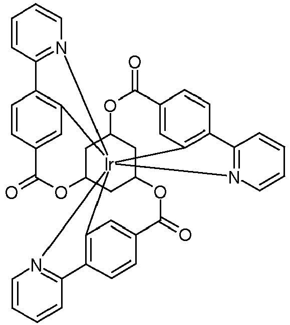 | 33 % |
| | | Ir(L400) | |
| Ir(L401) | IrL401 | Ir(L401) | 29 % |
| Ir(L404) | IrL404 | 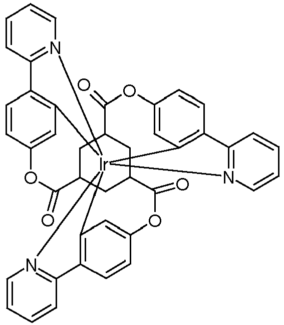 | 31 % |
| | | Ir(L404) | |
| Ir(L405) | IrL405 | Ir(L405) | 30 % |
| Ir(L408) | IrL408 | Ir(L408) | 28 % |
| Ir(L410) | IrL410 | Ir(L410) | 23 % |
| Ir(L411) | IrL411 | 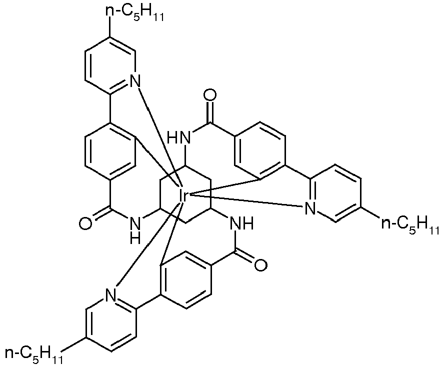 | 13 % |
| | | Ir(L411) Benzoesäu remethylester | |
| Ir(L412) | IrL412 | Ir(L412) | 35 % |
| Ir(L413) | IrL413 | Ir(L413) | 34% |
| Ir(L414) | IrL414 | Ir(L414) | 24% |
| Ir(L415) | IrL415 | Ir(L415) | 29 % |
| Ir(L416) | IrL416 | Ir(L416) | 21 % |
| Ir(L417) | IrL417 | Ir(L417) | 33 % |
| Ir(L418) | IrL418 | Ir(L418) | 24% |
| Ir(L419) | IrL419 | Ir(L419) | 30 % |
| Ir(L420) | IrL420 | Ir(L420) | 24 % |
| Ir(L421) | IrL421 | Ir(L421) | 19 % |
| Ir(L422) | IrL422 | Ir(L422) | 23 % |
| Ir(L423) | IrL423 | Ir(L423) | 25 % |
| | | 2.5 h | |
| Ir(L424) | IrL424 | Ir(L424) | 29 % |
| Ir(L425) | IrL425 | Ir(L425) | 18 % |
| Ir(L426) | IrL426 | Ir(L426) | 23 % |
| Ir(L427) | IrL427 | Ir(L427) | 31 % |
| Ir(L428) | IrL428 | Ir(L428) | 36 % |
| Ir(L429) | IrL429 | Ir(L429) | 22 % |
| Ir(L430) | IrL430 | Ir(L430) | 21 % |
| Ir(L431) | IrL431 | Ir(L431) | 31 % |
| Ir(L432) | IrL432 | Ir(L432) | 33 % |
| Ir(L433) | IrL433 | Ir(L433) | 23 % |
| Ir(L434) | IrL434 | Ir(L434) | 24% |
| | | 2.5 h | |
| Ir(L435) | IrL435 | Ir(L435) | 30 % |
| Ir(L436) | IrL436 | Ir(L436) | 21 % |
| Ir(L437) | IrL437 | Ir(L437) | 19 % |
| Ir(L459) | IrL459 | Ir(L459) | 17% |
| | | Zusatz von 33 mmol NaO-t-Bu | |
| | | 250 °C | |
| | | 2 h | |
| | | Toluol | |
| Ir(L460) | IrL460 | Ir(L460) | 51 % |
| Ir(L500) | L500 | 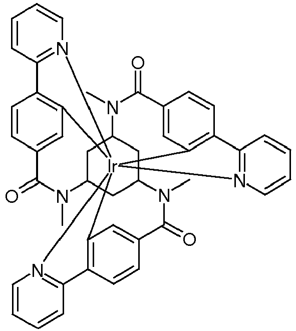 | 54% |
| | | Ir(L500) | |
| Ir(L501) | L501 | Ir(L501) | 49 % |
| Ir(L502) | L502 | Ir(L502) | 55 % |
| Ir(L503) | L503 | Ir(L503) | 50 % |
| Ir(L504) | L504 | Ir(L504) | 36 % |
| Ir(L505) | L505 | Ir(L505) | 48 % |
| Ir(L506) | L506 | Ir(L506) | 50 % |
| Ir(L507) | L507 | Ir(L507) | 52 % |
| Ir(L508) | L508 | Ir(L508) | 33 % |
| Ir(L509) | L59 | Ir(L509) | 46 % |
| Ir(L510) | L510 | Ir(L510) | 30 % |
| Ir(L511) | L511 | Ir(L511) | 53 % |
| Ir(L512) | L512 | Ir(L512) | 26 % |
| Ir(L513) | L513 | Ir(L513) | 32 % |
| Ir(L514) | L514 | Ir(L514) | 50 % |
| Ir(L515) | L515 | Ir(L515) | 51 % |
| Ir(L516) | L516 | Ir(L516) | 56 % |
| Ir(L517) | L517 | Ir(L517) | 38 % |
| Ir(L518) | L518 | Ir(L518) | 50 % |
| Ir(L519) | L519 | Ir(L519) | 54% |
| Ir(L520) | L520 | Ir(L520) | 19 % |
| Ir(L521) | L521 | Ir(L521) | 49 % |
| Ir(L522) | L522 | Ir(L522) | 17 % |
| Ir(L600) | L600 | 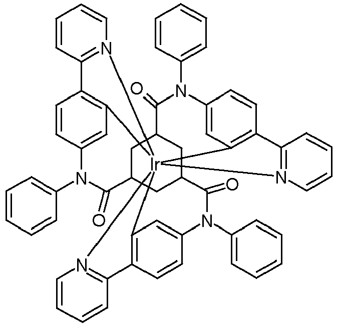 | 54% |
| | | Ir(L600) | |
| Ir(L601) | L601 | Ir(L601) | 23 % |
| Ir(L602) | L602 | Ir(L602) | 19 % |
| Ir(L603) | L603 | Ir(L603) | 56 % |
| Ir(L700) | L(700) | 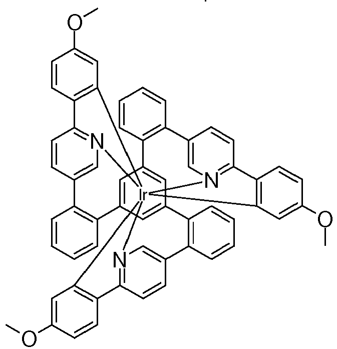 | 85 % |
| | | Ir(L700) | |
| | | 250 °C | |
| | | 1.5 h | |
| | | 1 x Heißextraktion des Rohprodukts mit DCM | |
| Ir(L701 ) | L(701) | Ir(L701 ) | 56 % |
| | | wie Ir(L700) | |
| Ir(L702) | L(702) | Ir(L702) | 49 % |
| | | wie Ir(L700) | |
| Ir(L703) | L(703) | Ir(L703) | 46 % |
| | | wie Ir(L700) | |

| | | | |
|---|---|---|---|
| *: Angabe sofern abweichend von allgemeiner Vorschrift | | | |

### Metallkomplexe des Liganden L16:

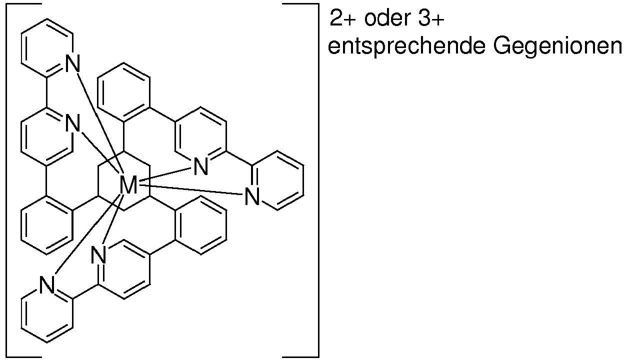

Eine Lösung von 769 mg (1 mmol) L16 in 10 ml DMSO wird bei 75 °C tropfenweise mit einer auf 75 °C temperierten Lösung von 1 mmol des entsprechenden Metallsalzes in 15 ml EtOH bzw. EtOH-Wasser (1:1 vv) versetzt und 10 h nachgerührt. Gegebenenfalls wird unter Zusatz von 6 mmol des entsprechenden Salzes (KPF₆, (NH₄)PF₆, KBF₄, etc.) in 10 ml EtOH oder EtOH/Wasser (1:1, vv) ein Anionenaustausch durchgeführt. Nach Erkalten wird der mikrokristalline Niederschlag abgesaugt, mit kaltem MeOH gewaschen und im Vakuum getrocknet. Die Reinigung kann durch Umkristallisation aus Acetonitril / Methanol erfolgen.

Analog können folgende Verbindungen dargestellt werden:

| Bsp. | Ligand Metallsalz | Produkt | Ausbeute |
|---|---|---|---|
| M1 | L16 Fe(ClO₄)₂ | [Fe(L 16)](ClO₄)₂ | 56 % |
| M2 | L16 Fe(ClO₄)₃ | [Fe(L 16)](ClO₄)₃ | 64% |
| M3 | L16 Ru(ClO₄)₃ | [Ru(L16)](ClO₄)₃ | 71 % |
| M4 | L16 Os(ClO₄)₂ | [Os(L16)](ClO₄)₂ | 52 % |
| M5 | L16 Co(ClO₄)₃ | [Co(L16)](ClO₄)₃ | 43 % |
| M6 | L16 RhCl₃ x H₂O KPF₆ | [Rh(L16)](PF₆)₃ | 50 % |
| M7 | L16 IrCl₃ x H₂O KPF₆ | [Ir(L16)](PF₆)₃ | 55 % |
| M8 | L16 ZnCl₂ KPF₆ | [Zn(L16)] (PF₆)₂ | 68 % |

### Metallkomplexe des Liganden L17:

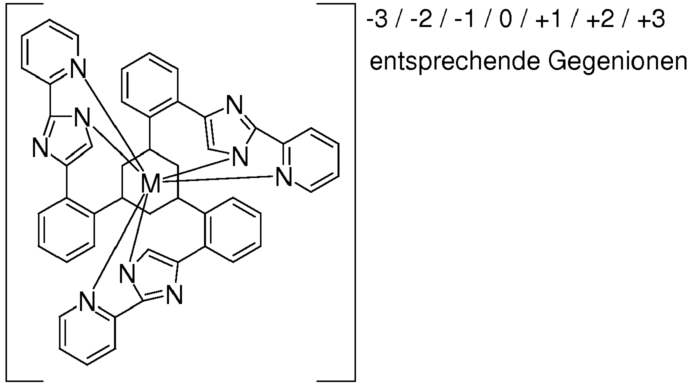

Eine Lösung von 736 mg (1 mmol) L17 und 643 mg (6 mmol) 2,6-Dimethyl-pyridin in 10 ml DMSO wird bei 75 °C tropfenweise mit einer auf 75 °C temperierten Lösung von 1 mmol des entsprechenden Metallsalzes in 15 ml EtOH bzw. EtOH-Wasser (1:1 vv) versetzt und 10 h nachgerührt. Gegebenenfalls wird unter Zusatz von 6 mmol des entsprechenden Salzes (KPF₆, (NH₄)PF₆, KBF₄, etc.) in 10 ml EtOH oder EtOH/Wasser (1:1, vv) ein Anionenaustausch durchgeführt. Nach Erkalten wird der mikrokristalline Niederschlag abgesaugt, mit kaltem MeOH gewaschen und im Vakuum getrocknet. Die Reinigung kann durch Umkristallisation aus Acetonitril / Methanol erfolgen.

Analog können folgende Verbindungen dargestellt werden:

| Bsp. | Ligand Metallsalz | Produkt | Ausbeute |
|---|---|---|---|
| M100 | L17 FeBr₃ Hydrat | Fe(L17) | 70 % |
| M101 | L17 | NH₄[Ru(L17)] | 54% |
| | [Ru(NH₃)₆]Cl₂ kein 2,6-Dimethylpyridin | | |
| M102 | L17 RuCl₃ Hydrat | Ru(L17) | 66 % |
| M103 | L17 OsCl₃ Hydrat | Os(L17) | 58 % |
| M104 | L17 RhCl₃ Hydrat | Rh(L17) | 41 % |
| M105 | L17 IrCl₃ Hydrat | Ir(L17) | 67% |
| M106 | L17 (NH₄)₂[PtCl₆] als Feststoff zugesetzt NH₄PF₆ | [Pt(L17)](PF₆) | 71 % |

### Metallkomplexe des Liganden L18:

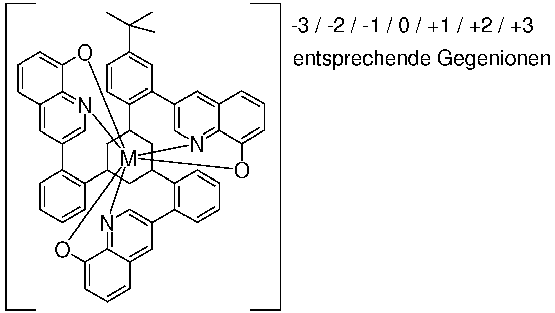

Eine Lösung von 736 mg (1 mmol) L18, 643 mg (6 mmol) 2,6-Dimethylpyridin in 10 ml DMSO wird bei 75 °C tropfenweise mit einer auf 75 °C temperierten Lösung von 1 mmol des entsprechenden Metallsalzes in 20 ml EtOH bzw. EtOH-Wasser (1:1 vv) versetzt und 10 h nachgerührt. Gegebenenfalls wird unter Zusatz von 6 mmol des entsprechenden Salzes (KPF₆, (NH₄)PF₆, KBF₄, etc.) in 10 ml EtOH oder EtOH/Wasser (1:1, vv) ein Anionenaustausch durchgeführt. Nach Erkalten wird der mikrokristalline Niederschlag abgesaugt, mit kaltem MeOH gewaschen und im Vakuum getrocknet. Die Reinigung kann durch Umkristallisation aus Acetonitril / Methanol erfolgen.

### Analog können folgende Verbindungen dargestellt werden:

| Bsp. | Ligand Metallsalz | Produkt | Ausbeute |
|---|---|---|---|
| M200 | L18 AlCl₃ | Al(L18) | 74% |
| M201 | L18 GaCl₃ | Ga(L18) | 77% |
| M202 | L18 InCl₃ | In(L18) | 80 % |
| M203 | L18 LaCl₃ | La(L18) | 46 % |
| M204 | L18 CeCl₃ | Ce(L18) | 40 % |
| M205 | L18 FeCl₃ | Fe(L18) | 88 % |
| M206 | L18 RuCl₃ | Ru(L18) | 90 % |

### Metallkomplexe des Liganden L19:

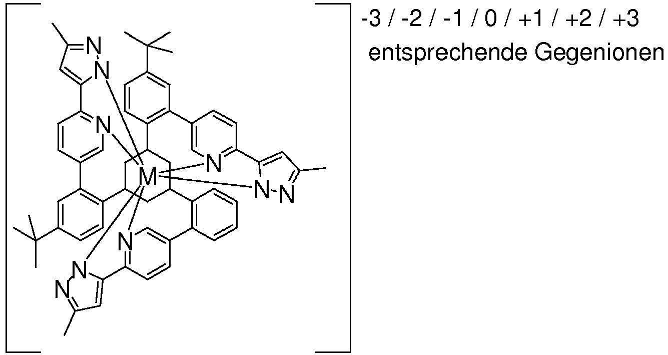

Eine Lösung von 778 mg (1 mmol) L19, 643 mg (6 mmol) 2,6-Dimethylpyridin in 10 ml DMSO wird bei 80 °C tropfenweise mit einer auf 75 °C temperierten Lösung von 1 mmol des entsprechenden Metallsalzes in 20 ml EtOH bzw. EtOH-Wasser (1:1 vv) versetzt und 12 h nachgerührt. Gegebenenfalls wird unter Zusatz von 6 mmol des entsprechenden Salzes (KPF₆, (NH₄)PF₆, KBF₄, etc.) in 10 ml EtOH oder EtOH/Wasser (1:1, vv) ein Anionenaustausch durchgeführt. Nach Erkalten wird der mikrokristalline Niederschlag abgesaugt, mit kaltem MeOH gewaschen und im Vakuum getrocknet. Die Reinigung kann durch Umkristallisation aus Acetonitril / Methanol oder durch Heißextraktion und anschließende fraktionierte Sublimation erfolgen.

Analog können folgende Verbindungen dargestellt werden:

| Bsp. | Ligand Metallsalz | Produkt | Ausbeute |
|---|---|---|---|
| M300 | L19 GaCl₃ | Ga(L19) | 67% |
| M301 | L19 InCl₃ | In(L19) | 63 % |
| M302 | L19 IrCl₃ Hydrat | Ir(L19) | 66 % |
| M303 | L19 LaCl₃ | La(L19) | 48 % |
| M304 | L19 FeCl₃ | Fe(L19) | 83 % |
| M305 | L19 IrCl₃ Hydrat | Ir(L19) | 79 % |
| M306 | L19 RuCl₃ | Ru(L19) | 80 % |

### Metallkomplexe des Liganden L20:

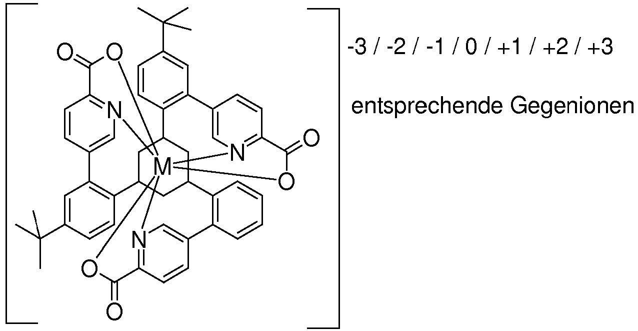

Eine Lösung von 736 mg (1 mmol) L20, 643 mg (6 mmol) 2,6-Dimethylpyridin in 10 ml DMSO wird bei 75 °C tropfenweise mit einer auf 75 °C temperierten Lösung von 1 mmol des entsprechenden Metallsalzes in 15 ml EtOH bzw. EtOH-Wasser (1:1 vv) versetzt und 12 h nachgerührt. Gegebenenfalls wird unter Zusatz von 6 mmol des entsprechenden Salzes (KPF₆, (NH₄)PF₆, KBF₄, etc.) in 10 ml EtOH oder EtOH/Wasser (1:1, vv) ein Anionenaustausch durchgeführt. Nach Erkalten wird der mikrokristalline Niederschlag abgesaugt, mit kaltem MeOH gewaschen und im Vakuum getrocknet. Die Reinigung kann durch Umkristallisation aus Acetonitril / Methanol erfolgen.

Analog können folgende Verbindungen dargestellt werden:

| Bsp. | Ligand Metallsalz | Produkt | Ausbeute |
|---|---|---|---|
| M400 | L20 AlCl₃ | Al(L20) | 72 % |
| M401 | L20 GaCl₃ | Ga(L20) | 68 % |
| M402 | L20 LaCl₃ | La(L20) | 55 % |
| M403 | L20 CeCl₃ | Ce(L20) | 51 % |
| M404 | L20 FeCl₃ | Fe(L20) | 78 % |
| M405 | L20 RuCl₃ | Ru(L20) | 83 % |
| M406 | L20 IrCl₃ Hydrat | Ir(L20) | 77% |

### 4. Funktionalisierung der Metallkomplexe:

### 4.1 Halogenierung der Iridium-Komplexe:

Eine Lösung bzw. Suspension von 10 mmol eines Komplexes, der in para-Position zum Iridium A x C-H-Gruppen (mit A = 1, 2, 3) trägt, in 500 ml bis 2000 ml Dichlormethan, je nach Löslichkeit der Metallkomplexe, wird unter Licht- und Luftausschluss bei -30 bis +30 °C mit A x 10.5 mmol N-Halogen-succinimid (Halogen: Cl, Br, I) versetzt und 20 h gerührt. In DCM schlecht lösliche Komplexe können auch in anderen Lösungsmitteln (TCE, THF, DMF, Chlorbenzol, etc.) und bei erhöhter Temperatur umgesetzt werden. Anschließend wird das Lösungsmittel im Vakuum weitgehend entfernt. Der Rückstand wird mit 100 ml Methanol und 1 ml Hydrazinhydrat versetzt, kurz gerührt, der Feststoff wird abgesaugt, dreimal mit 30 ml Methanol gewaschen und dann im Vakuum getrocknet. Man erhält so die in para-Position zum Iridium bromierten Iridiumkomplexe. Komplexe mit einem HOMO (CV) von ca. -5.1 bis -5.0 eV und betragsmäßig kleiner neigen zur Oxidation (Ir(III) > Ir(IV)), wobei das Oxidationsmittel Brom, freigesetzt aus NBS, ist. Diese Oxidationsreaktion ist durch eine deutliche Grünfärbung der ansonsten gelben bis roten Lösungen / Suspensionen der Emitter zu erkennen. In solchen Fällen wird ein weiteres Äquivalent NBS zugesetzt. Zur Aufarbeitung setzt man 100 - 500 ml Methanol und 2 ml Hydrazin Hydrat als Reduktionsmittel zu, wodurch sich die grünen Lösungen / Suspension gelb verfärben (Reduktion Ir(IV) > Ir(III). Dann zieht man im Vakuum das Lösungsmittel weitgehend ab, versetzt mit 300 ml Methanol, saugt den Feststoff ab, wäscht dreimal mit je 100 ml Methanol und trocknet im Vakuum.

Unterstöchiometrische Bromierungen, z.B. Mono- und Dibromierungen von Komplexen mit 3 C-H-Gruppen para-Position zum Iridium, verlaufen meist weniger selektiv als die stöchiometrischen Bromierungen. Die Rohprodukte dieser Bromierungen können chromatographisch (CombiFlash Torrent der Fa. A. Semrau) getrennt werden.

### Beispiel Ir(L1-3Br):

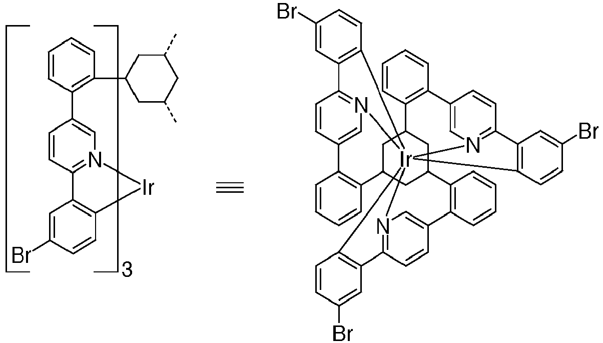

Eine bei 0 °C gerührte Suspension von 9.6 g (10 mmol) Ir(L1) in 500 ml Dichlormethan (DCM) wird auf einmal mit 5.6 g (31.5 mmol) N-Bromsuccinimid versetzt und dann weitere 6 h bei Raumtemperatur gerührt. Nach Entfernen von ca. 400 ml des DCMs im Vakuum wird die gelbe Suspension mit einem Gemisch aus 100 ml Methanol und 1 ml Hydrazinhydrat versetzt, der Feststoff wird abgesaugt, dreimal mit ca. 30 ml Methanol gewaschen und dann im Vakuum getrocknet. Ausbeute: 11.2 g (9.5 mmol) 93 %; Reinheit: > 99.0 %ig nach NMR.

Analog können folgende Komplexe dargestellt werden:

| Bsp. | Edukt > bromierter Komplex Bedingungen | Ausbeute |
|---|---|---|
| Tribromierung | | |
| Ir(L3-3Br) | 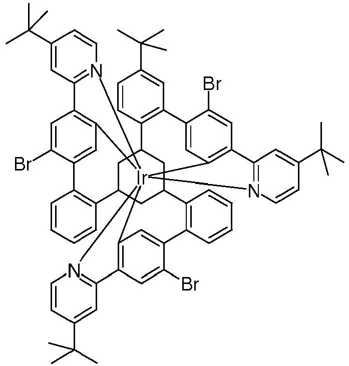 | 93 % |
| | Ir(L3) > Ir(L3-3Br) | |
| Ir(L5-3Br) | 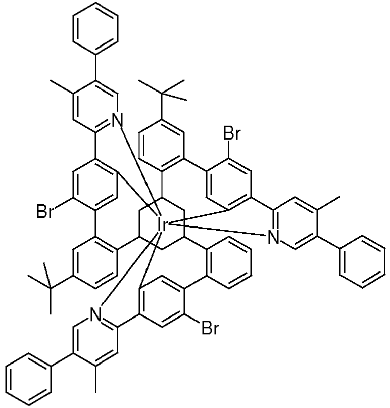 | 95 % |
| | Ir(L5) > Ir(L5-3Br) | |
| Ir(L7-3Br) | 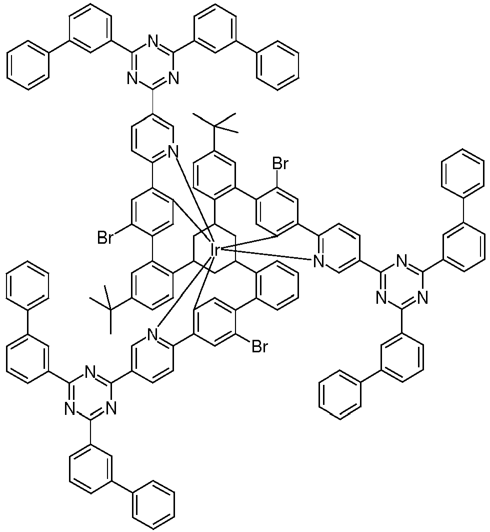 | 87 % |
| | Ir(L7) > Ir(L7-3Br) | |
| Ir(L9-3Br) | 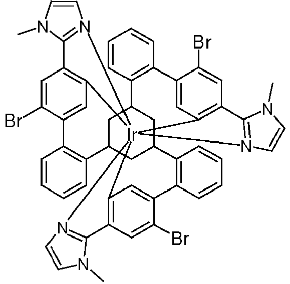 | 84 % |
| | Ir(L9) > Ir(L9-3Br) | |
| Ir(L101-3Br) | 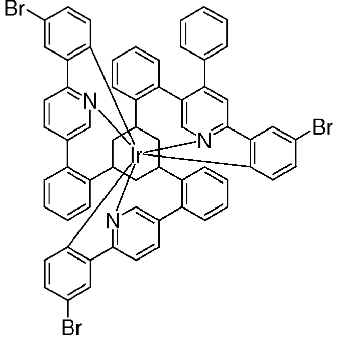 | 93 % |
| | Ir(L101) > Ir(L101-3Br) | |
| Ir(L102-3Br) | 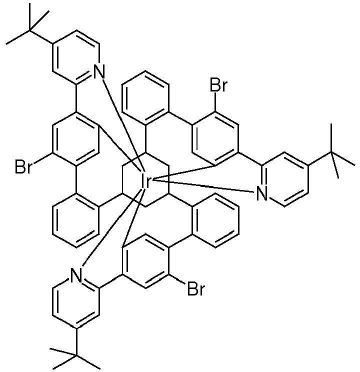 | 95 % |
| | Ir(L102) > Ir(L102-3Br) | |
| Ir(L103-3Br) | 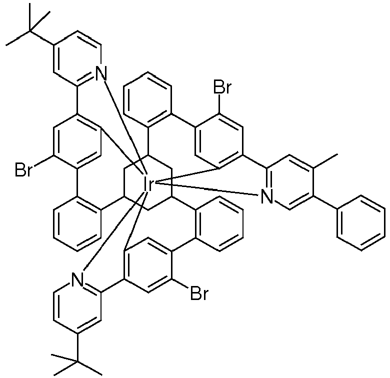 | 90 % |
| | Ir(L103) > Ir(L103-3Br) | |
| Ir(L108-3Br) | 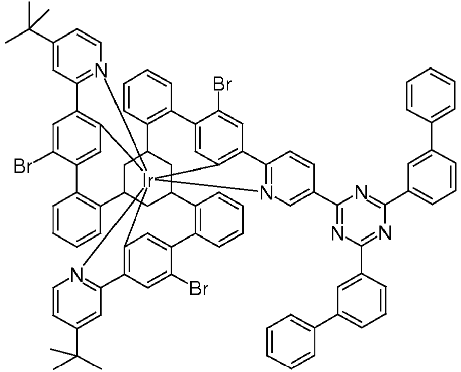 | 88 % |
| | Ir(L108) > Ir(L108-3Br) | |
| Ir(L109-3Br) | 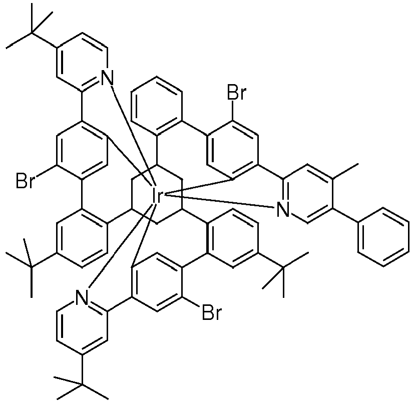 | 89 % |
| | Ir(L109) > Ir(L109-3Br) | |
| Ir(L110-3Br) | 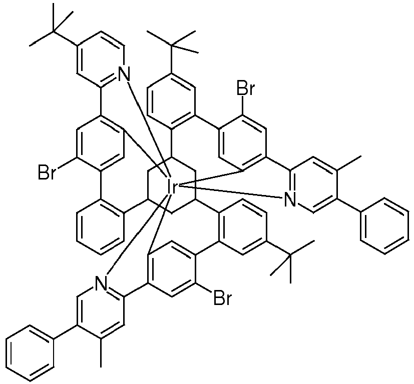 | 90 % |
| | Ir(L110) > Ir(L110-3Br) | |
| Ir(L111-3Br) | 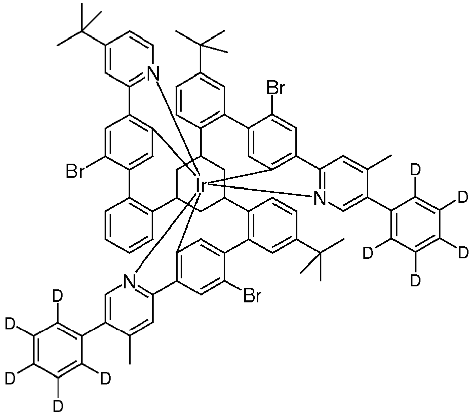 | 93 % |
| | Ir(L111) > Ir(L111-3Br) | |
| Ir(L112-3Br) | 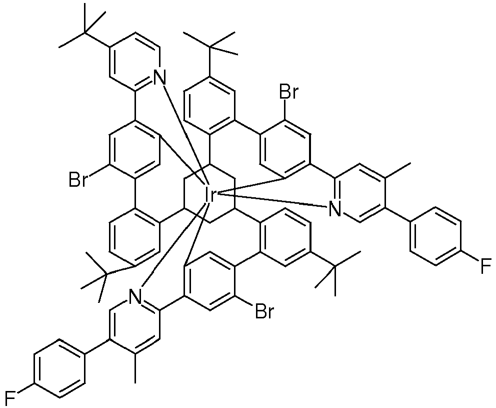 | 87% |
| | Ir(L112) > Ir(L112-3Br) | |
| Ir(L115-3Br) | 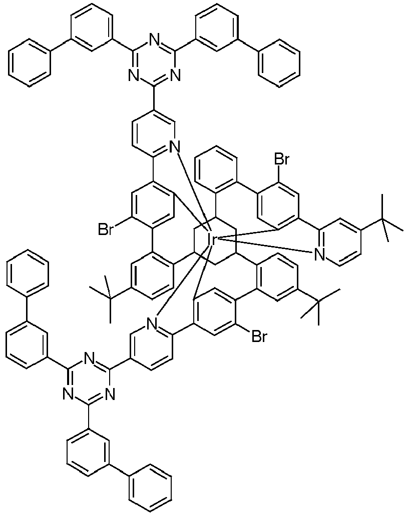 | 84% |
| | Ir(L115) > Ir(L115-3Br) | |
| Ir(L117-3Br) | 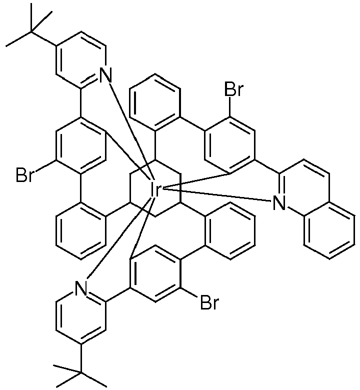 | 91 % |
| | Ir(L117) > Ir(L117-3Br) | |
| Ir(L120-3Br) | 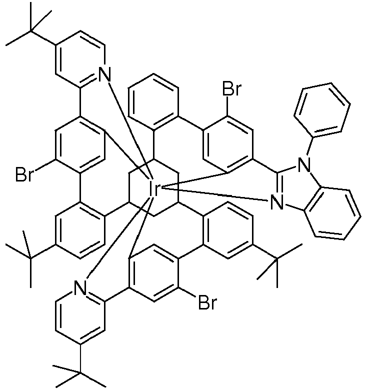 | 85 % |
| | Ir(L120) > Ir(L120-3Br) | |
| Ir(L123-3Br) | 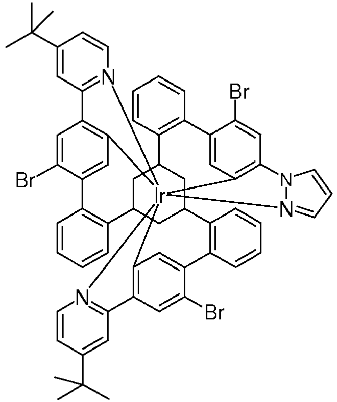 | 83 % |
| | Ir(L123) > Ir(L123-3Br) | |
| Ir(L201-3Br) | 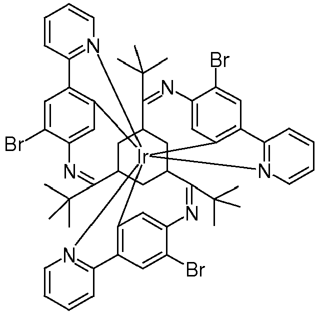 | 92 % |
| | Ir(L201) > Ir(L201-3Br) | |
| Ir(L203-3Br) | 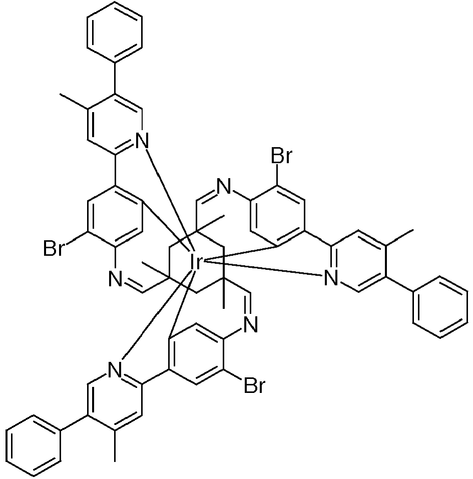 | 90 % |
| | Ir(L203) > Ir(L203-3Br) | |
| Ir(L208-3Br) | 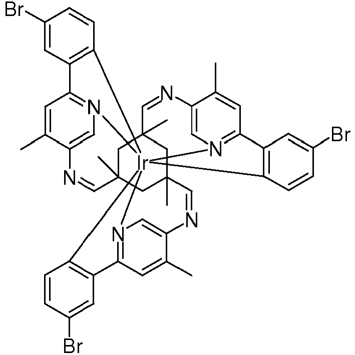 | 88 % |
| | Ir(L208) > Ir(L208-3Br) | |
| Ir(L301-3Br) | 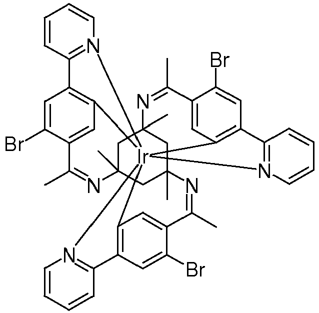 | 85 % |
| | Ir(L301) > Ir(L301-3Br) | |
| Ir(L306-3Br) | 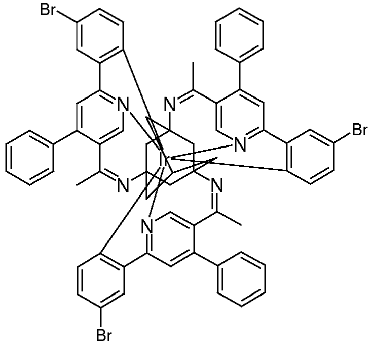 | 86 % |
| | Ir(L306) > Ir(L306-3Br) | |
| Ir(L400-3Br) | 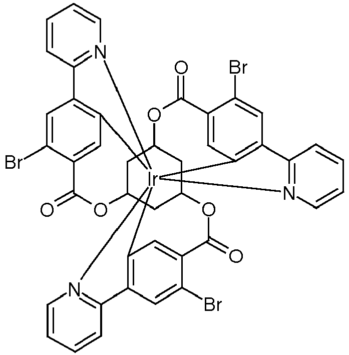 | 76 % |
| | Ir(L400) > Ir(L400-3Br) | |
| Ir(L404-3Br) | 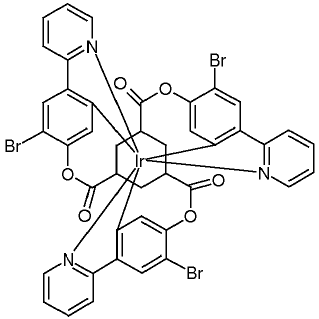 | 90 % |
| | Ir(L404) > Ir(L404-3Br) | |
| Ir(L405-3Br) | 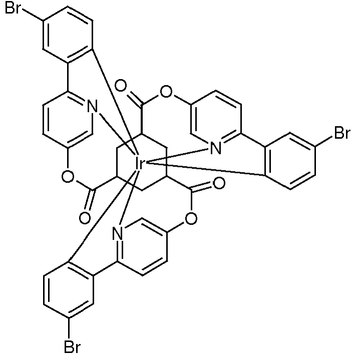 | 90 % |
| | Ir(L405) > Ir(L405-3Br) | |
| Ir(L500-3Br) | 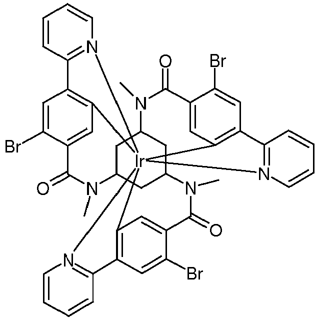 | 86% |
| | Ir(L500) > Ir(L500-3Br) | |
| Ir(L503-3Br) | 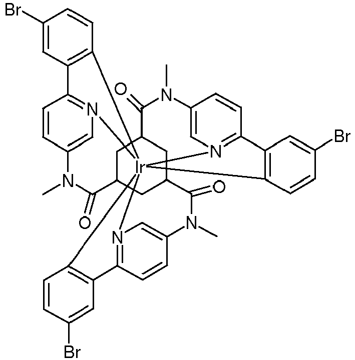 | 93 % |
| | Ir(L503) > Ir(L503-3Br) | |

| Dibromierung | | |
|---|---|---|
| Ir(L100-2Br) | 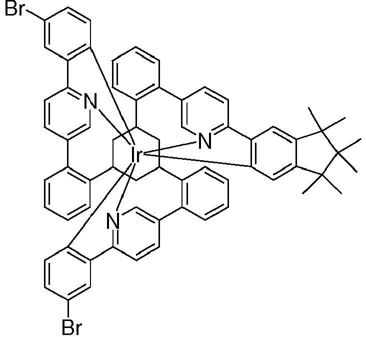 | 95 % |
| | Ir(L100) > Ir(L100-2Br) | |
| Ir(L102-2Br) | 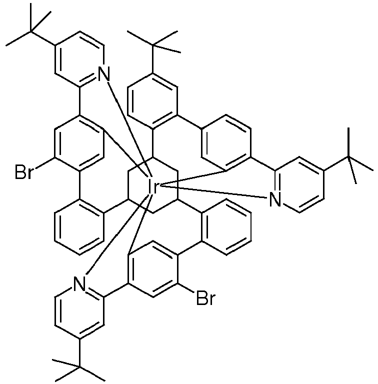 | 26 % |
| | Chromatographische Reinigung Ir(L102) > Ir(L102-2Br) | |
| Ir(L106-2Br) | 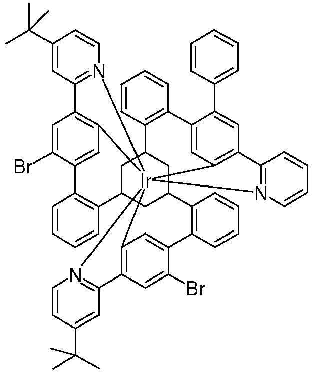 | 94% |
| | Ir(L106) > Ir(L106-2Br) | |
| Ir(L107-2Br) | 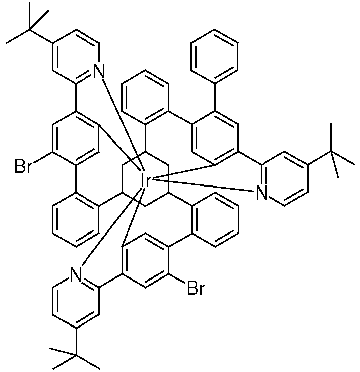 | 96 % |
| | Ir(L107) > Ir(L107-2Br) | |
| Ir(L116-2Br) | 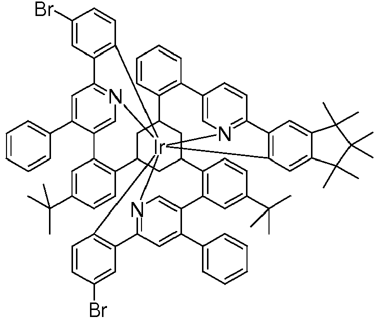 | 96 % |
| | Ir(L116) > Ir(L116-2Br) | |
| Ir(L121-2Br) | 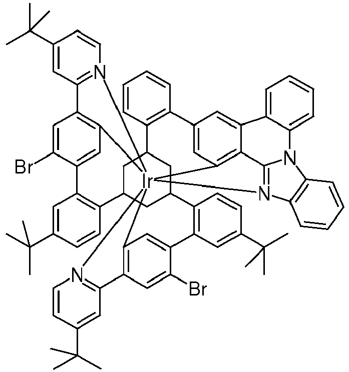 | 89 % |
| | Ir(L121) > Ir(L121-2Br) | |

| Monobromierung | | |
|---|---|---|
| Ir(L102-1 Br) | 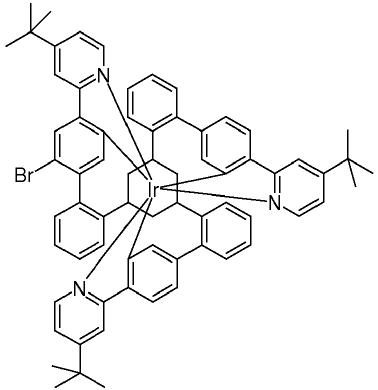 | 57% |
| | Ir(L102) > Ir(L102-1 Br) Chromatographische Reinigung | |
| Ir(L113-Br) | 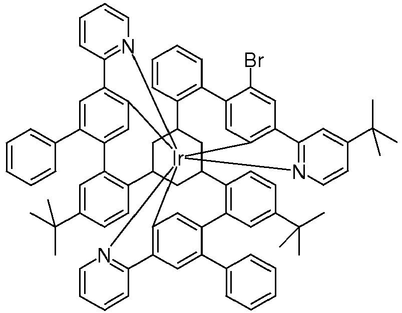 | 92 % |
| | Ir(L113) > Ir(L113-1 Br) | |
| Ir(L114-Br) | 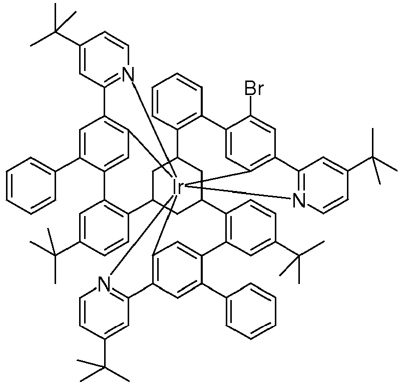 | 94% |
| | Ir(L114) > Ir(L114-1 Br) | |

### 4.2 Borylierung der Metallkomplexe mit Bromfunktion:

Ein Gemisch von 10 mmol des bromierten Komplexes, 12 mmol Bis-(pinacolato)diboran [73183-34-3] pro Brom-Funktion, 30 mmol Kaliumacetat, wasserfrei pro Bromfunktion, 0.2 mmol Tricyclohexylphosphin, 0.1 mmol Palladium(II)acetat (Variante A) oder 0.2 mmol dppfPdCl₂ ^{∗} CH₂Cl₂ [95464-05-4] (Variante B) und 300 ml Lösungsmittel (Dioxan, DMSO, NMP, Toluol, etc.) wird 4-16 h bei 80-160 °C gerührt. Nach Entfernen des Lösungsmittels im Vakuum wird der Rückstand in 300 ml Dichlormethan, THF oder Ethylacetat aufgenommen, über ein Celite-Bett filtriert, das Filtrat wird bis zur beginnenden Kristallisation im Vakuum eingeengt und abschließend noch tropfenweise mit ca. 100 ml Methanol versetzt, um die Kristallisation zu vervollständigen. Die Verbindungen können aus Dichlormethan, Ethylacetat oder THF unter Zusatz von Methanol umkristallisiert werden oder an Kieselgel chromatographiert werden.

### Synthese von Ir(L1-3BE) - Variante B:

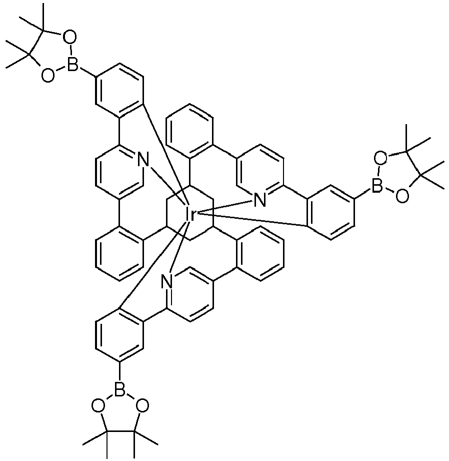

Einsatz von 12.0 g (10 mmol) Ir(L1-3Br) und 9.1 g (36 mmol) Bis(pinacolato)diboran [73183-34-3], Dioxan/Toluol 1:1 vv, 120 °C, 16 h, aufnehmen und Celite-Filtration in THF, Umkristallisation aus THF:Methanol. Ausbeute: 7.9 g (5.9 mmol), 59 %; Reinheit: ca. 99.8 %ig nach HPLC.

Analog können folgende Verbindungen dargestellt werden:

| Bsp. | Produkt Edukt / Variante | Ausbeute |
|---|---|---|
| Triborylierung | | |
| Ir(L3-3BE) | 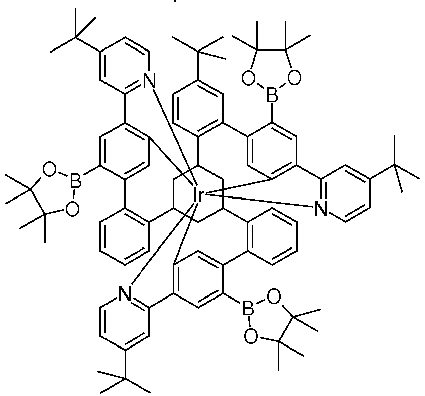 | 55 % |
| | Ir(L3-3Br) > Ir(L3-3BE) / B | |
| Ir(L5-3BE) | 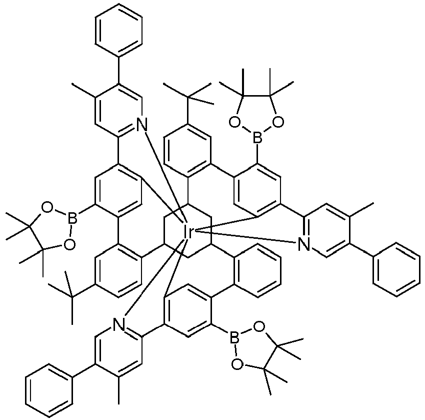 | 52 % |
| | Ir(L5-3Br) > Ir(L5-3BE) / B | |
| Diborylierung | | |
| Ir(L107-2BE) | 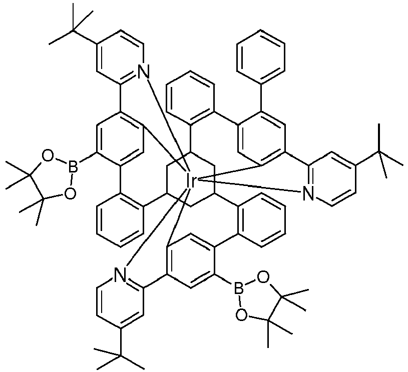 | 66 % |
| | Ir(L107-2Br) > Ir(L107-2BE) / B | |
| Monoborylierung | | |
| Ir(L114-BE) | 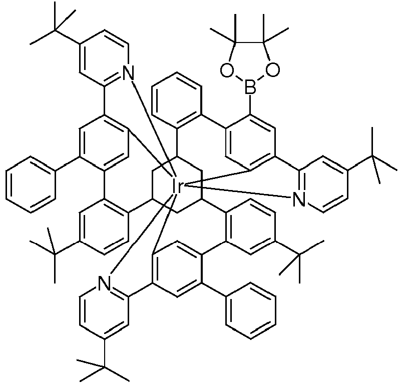 | 81 % |
| | Ir(L114-1 Br) > Ir(L114-1 BE) / B | |

### 4.3 Suzukikupplung an den halogenierten Metallkomplexen

### Variante A, zweiphasige Reaktionsmischung:

Eine Suspension von 10 mmol eines bromierten Komplexes, 12-20 mmol Boronsäure bzw. Boronsäureester pro Br-Funktion und 40 - 80 mmol Trikaliumphosphat in einem Gemisch aus 300 ml Toluol, 100 ml Dioxan und 300 ml Wasser wird mit 0.6 mmol Tri-o-tolylphosphin und dann mit 0.1 mmol Palladium(II)acetat versetzt und 16 h unter Rückfluss erhitzt. Nach Erkalten gibt man 500 ml Wasser und 200 ml Toluol zu, trennt die wässrige Phase ab, wäscht die organische Phase dreimal mit 200 ml Wasser, einmal mit 200 ml gesättigter Kochsalzlösung und trocknet über Magnesiumsulfat. Man filtriert über ein Celite-Bett ab, wäscht dieses mit Toluol nach, entfernt das Toluol fast vollständig im Vakuum, gibt 300 ml Methanol zu, saugt vom ausgefallenen Rohprodukt ab, wäscht dieses dreimal mit je 50 ml Methanol und trocknet im Vakuum. Das Rohprodukt wird an Kieselgel gesäult. Die weitere Reiningung kann durch Chromatographie, Umkristallisaton oder Heißextraktion erfolgen. Der Metallkomplex kann gegebenenfalls abschließend getempert oder sublimiert werden. Das Tempern erfolgt im Hochvakuum (p ca 10⁻⁴ Pa 10⁻⁶ mbar)) im Temperaturbereich von ca. 200 - 300 °C. Die Sublimation erfolgt bei geeigneten sublimationsfähigen Komplexen im Hochvakuum (p ca 10⁻⁴ Pa (10⁻⁶ mbar)) im Temperaturbereich von ca. 300 - 400 °C, wobei die Sublimation bevorzugt in Form einer fraktionierten Sublimation durchgeführt wird.

### Variante B, einphasige Reaktionsmischung:

Eine Suspension von 10 mmol eines bromierten Komplexes, 12-20 mmol Boronsäure bzw. Boronsäureester pro Br-Funktion und 60 - 100 mmol der Base (Kaliumfluorid, Trikaliumphosphat (wasserfrei oder Monohydrat oder Trihydrat), Kaliumcarbonat, Cäsiumcarbonat etc.) und 100 g Glaskugeln (3 mm Durchmesser) in 100 ml - 500 ml eines aprotischen Lösungsmittels (THF, Dioxan, Xylol, Mesitylen, Dimethylacetamid, NMP, DMSO, etc.) wird mit 0.6 mmol Tri-o-tolylphosphin und dann mit 0.1 mmol Palladium(II)-acetat oder 0.3 mmol Tetrakis(triphenylphosphino)palladium(0) versetzt und 1 - 24 h unter Erwärmen (80 -130 °C) gerührt. Alternativ können andere Phosphine wie Triphenylphosphin, Tri-tert-butylphosphin, SPhos, XPhos, RuPhos, XanthPhos, etc. eingesetzt werden, wobei bei diesen Phosphinen das bevorzugte Phosphin:Palladium-Verhältnis 3:1 bis 1.2:1 beträgt. Man entfernt das Lösungsmittel im Vakuum, nimmt das Produkt in einem geeigneten Lösungsmittel (Toluol, Dichlormethan, Ethylacetat, etc.) auf und reinigt wie unter Variante A beschrieben.

### Synthese von Ir100:

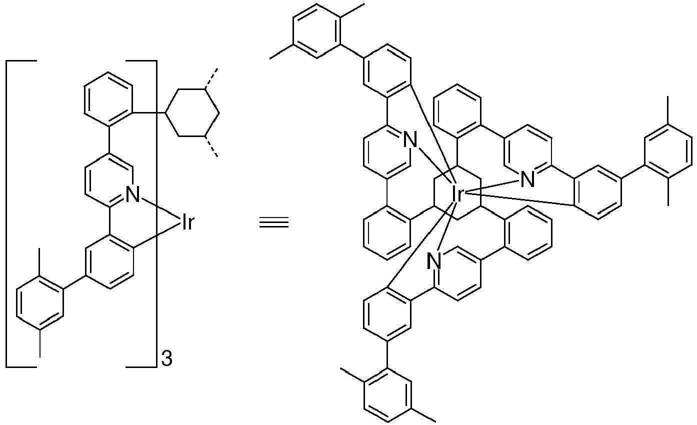

### Variante A:

Einsatz von 12.0 g (10.0 mmol) Ir(L1-3Br) und 9.0 g (60.0 mmol) 2,5-Dimethylphenylboronsäure [85199-06-0], 17.7 g (60 mmol) Trikaliumphosphat (wasserfrei), 183 mg (0.6 mmol) Tri-o-tolylphosphin [6163-58-2], 23 mg (0.1 mmol) Palladium(II)acetat, 300 ml Toluol, 100 ml Dioxan und 300 ml Wasser, Rückfluss, 16 h. Zweimalige chromatographische Trennung an Kieselgel mit Toluol/Ethylacetat (9:1, vv), anschließend zweimalige Heißextraktion mit Toluol unter Zusatz von 0.5 ml Hydrazinhydrat, dann fünfmalige Heißextraktion mit Butylacetat. Ausbeute: 6.9 g (5.4 mmol), 54 %; Reinheit: ca. 99.9 %ig nach HPLC.

### Variante B:

Einsatz von 12.0 g (10.0 mmol) Ir(L1-3Br) und 9.0 g (60.0 mmol) 2,5-Dimethylphenylboronsäurepinakolester [356570-53-1], 17.7 g (60 mmol) Trikaliumphosphat-Monohydrat, 347 mg (0.3 mmol) Tetrakis-triphenylphosphino-palladium(0), 300 ml DMSO, 90 °C, 24 h. Reinigung wie unter Variante A beschrieben. Ausbeute: 7.3 g (5.7 mmol), 57 %; Reinheit: ca. 99.8 %ig nach HPLC.

Analog können folgende Verbindungen dargestellt werden:

| Bsp. | Bromid / Boronsäure / Variante Produkt Heißextraktionsmittel | Ausbeute |
|---|---|---|
| Ir101 | Ir(L1-3Br) / 5122-95-2 / A | 57% |
| | 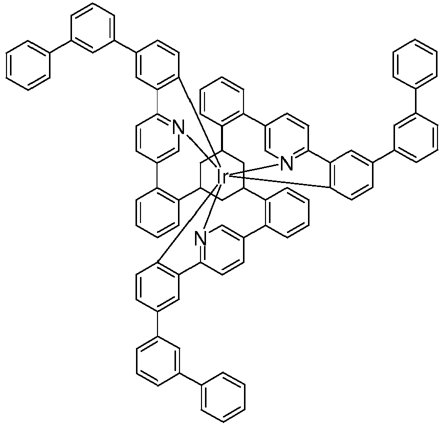 | |
| | Butylacetat, dann Toluol | |
| Ir102 | Ir(L1-3Br) / 1233200-59-3 / A | 59 % |
| | 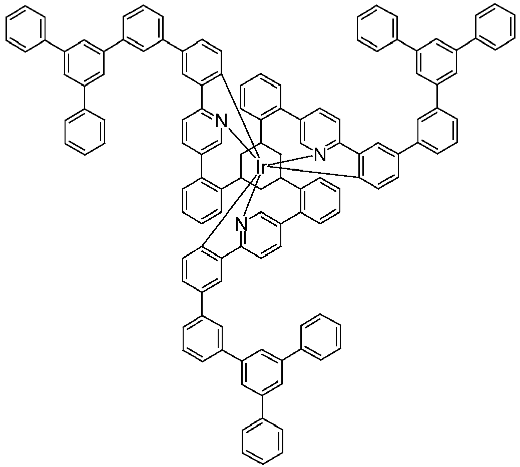 | |
| | Butylacetat | |
| Ir103 | Ir(L3-3Br) / 98-80-6 / B | 64% |
| | 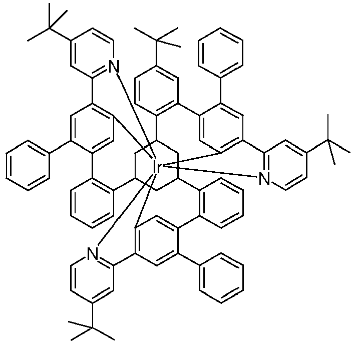 | |
| | Toluol | |
| Ir104 | Ir(L3-3Br) / 560132-24-3 / B | 51 % |
| | 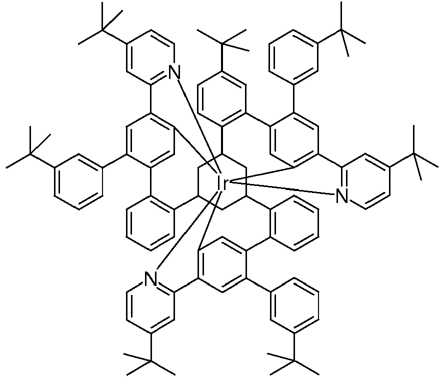 | |
| | Ethylacetat / Acetonitril | |
| Ir105 | Ir(L3-3Br) / 197223-39-5 / B | 55 % |
| | 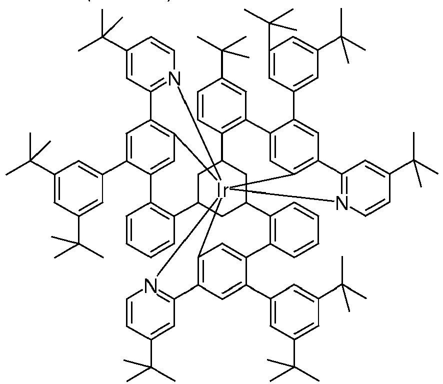 | |
| | Ethylacetat / Acetonitril | |
| Ir106 | Ir(L3-3Br) / 177171-16-3 / B | 58 % |
| | 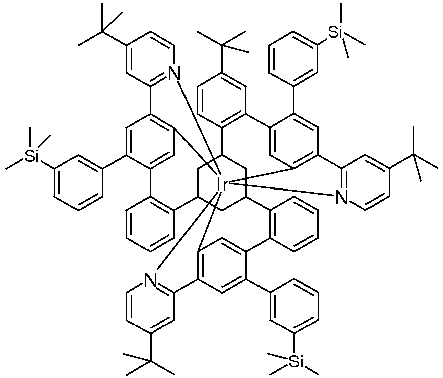 | |
| | Ethylacetat | |
| Ir107 | Ir(L3-3Br) / 915230-75-0 / B | 63 % |
| | 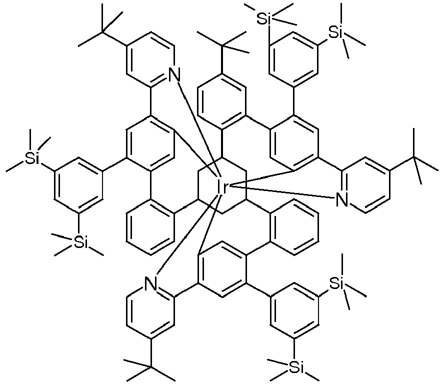 | |
| | Cyclohexan | |
| Ir108 | Ir(L5-3Br) / 162607-19-4 / A | 67% |
| | 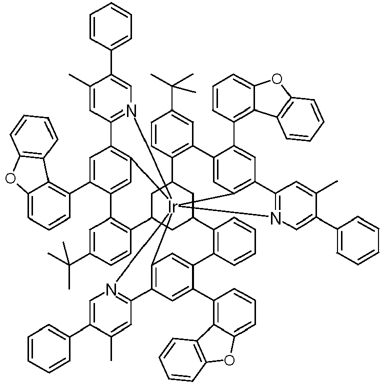 | |
| | Toluol | |
| Ir109 | Ir(L7-3Br) / 100124-06-9 / A | 60 % |
| | 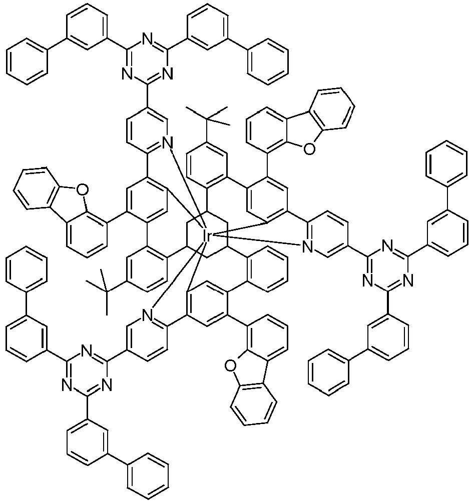 | |
| | Toluol | |
| Ir110 | Ir(L9-3Br) / 1392146-23-4 / B | 59 % |
| | 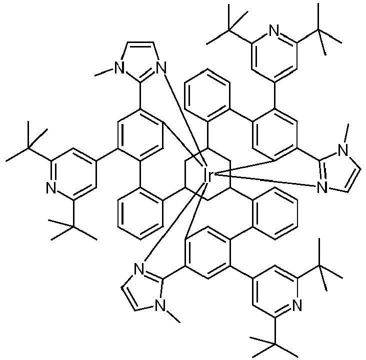 | |
| | Ethylacetat / Acetonitril | |
| Ir111 | Ir(L101-3Br) / 854952-58-2 / B | 65 % |
| | 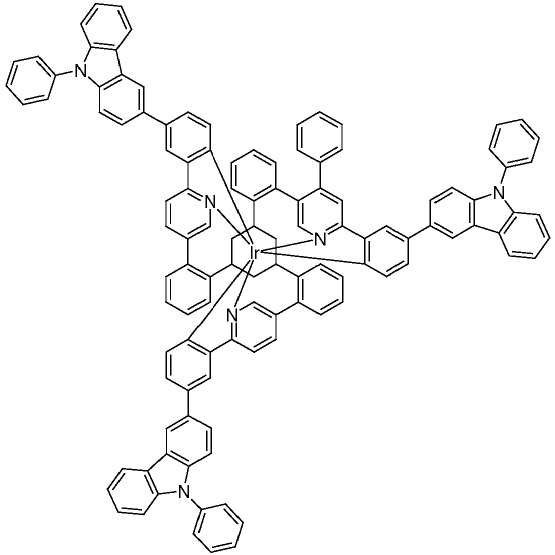 | |
| | Toluol | |
| Ir112 | 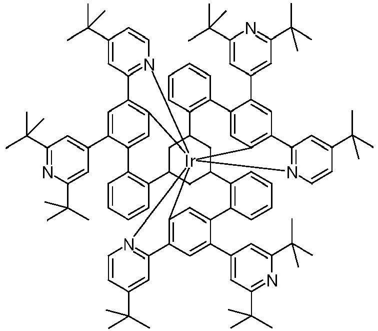 Ir(L102-3Br) / 1392146-23-4 / B | 60 % |
| | Toluol | |
| Ir113 | Ir(L102-3Br) / 1313018-07-3 / B | 67% |
| | 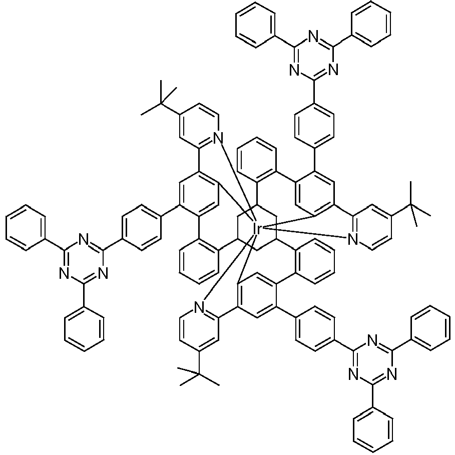 | |
| | Toluol | |
| Ir114 | Ir(L103-3Br) / 1809075-56-6 / B | 58 % |
| | 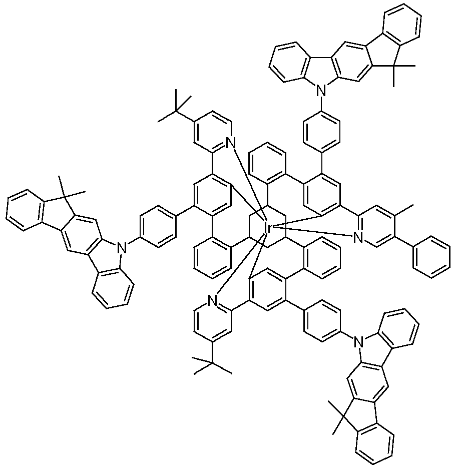 | |
| | o-Xylol | |
| Ir115 | Ir(L108-3Br) / 1562418-16-9 / A | 49 % |
| | 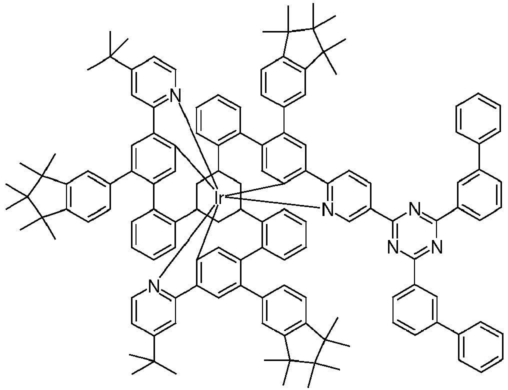 | |
| | Ethylacetat / Acetonitril | |
| Ir116 | Ir(L109-3Br) / 1680179-22-9 / B | 66 % |
| | 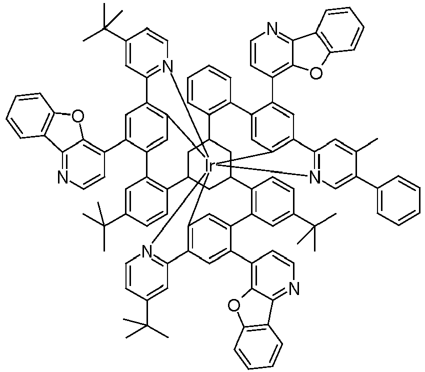 | |
| | Toluol | |
| Ir117 | Ir(L110-3Br) / 1345508-82-8 / B | 60 % |
| | 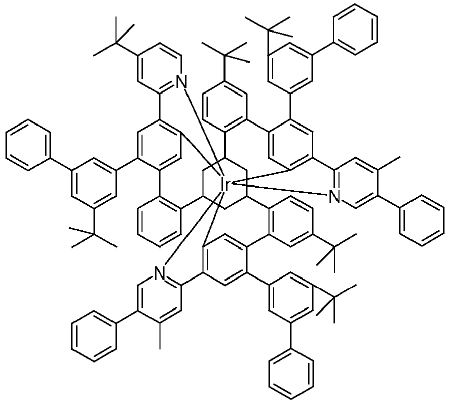 | |
| | Toluol | |
| Ir118 | Ir(L111-3Br) / 5122-95-2 / B | 63 % |
| | 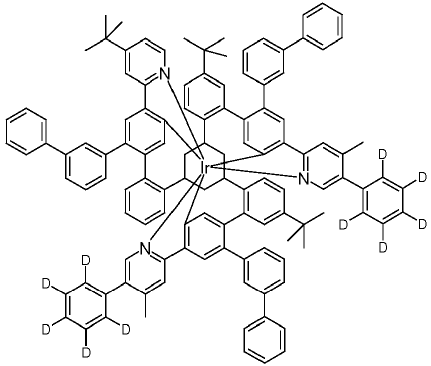 | |
| | Toluol | |
| Ir119 | Ir(L112-3Br) / 123324-71-0 / B | 61 % |
| | 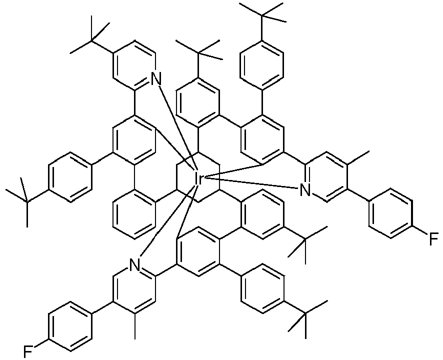 | |
| | Butylacetat dann Toluol | |
| Ir120 | Ir(L115-3Br) / 701261-35-0 / B | 65 % |
| | 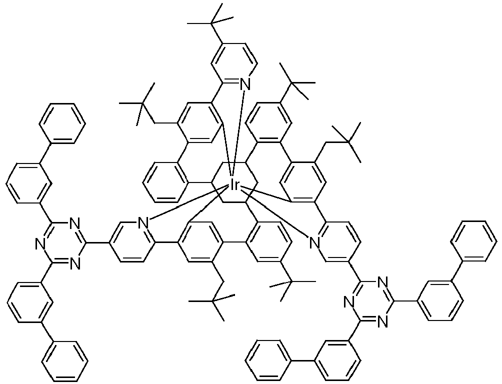 | |
| | Toluol | |
| Ir121 | Ir(L117-3Br) / 84110-40-7 / B | 47% |
| | 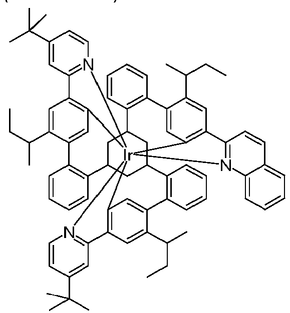 Ethylacetat | |
| Ir122 | Ir(L120-3Br) / 1269508-31-7 / B | 54% |
| | 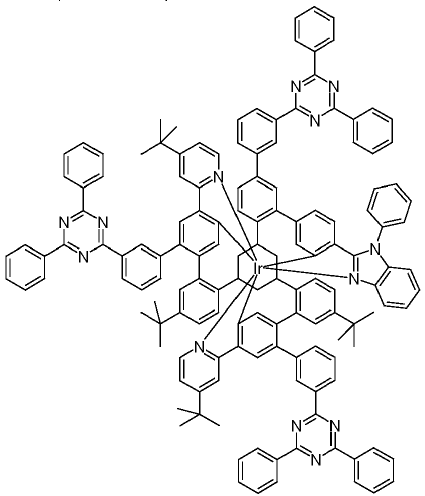 | |
| | Toluol | |
| Ir123 | Ir(L123-3Br) / 98-80-6 / B | 59 % |
| | 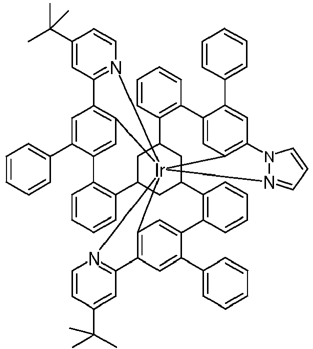 | |
| | o-Xylol | |
| Ir124 | Ir(L201-3Br) / 51067-38-0 / A | 47% |
| | 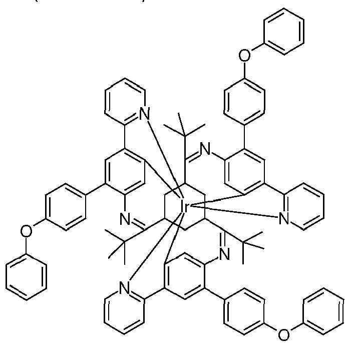 Toluol | |
| Ir125 | Ir(L203-3Br) / 4688-76-0 / B | 57% |
| | 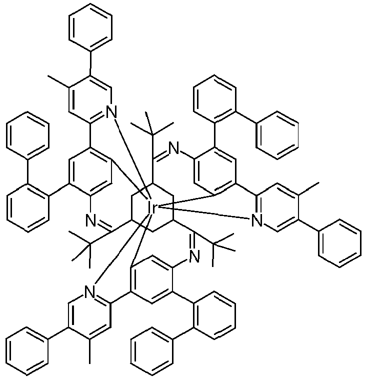 | |
| | Toluol | |
| Ir126 | Ir(L208-3Br) / 1245943-60-5 / B | 50 % |
| | 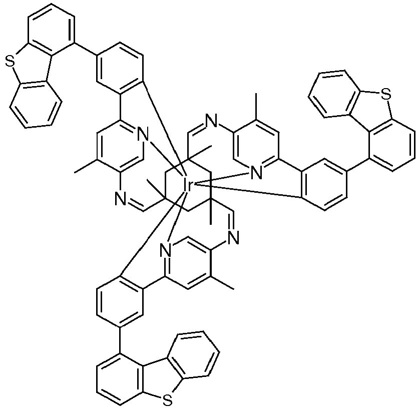 p-Xylol | |
| Ir127 | Ir(L301-3Br) / 400607-32-1 / B | 62 % |
| | 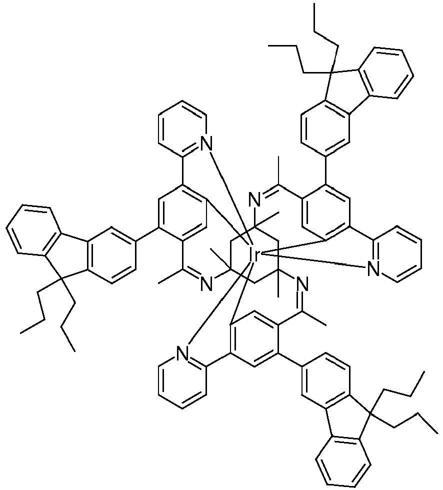 | |
| | Toluol | |
| Ir128 | Ir(L306-3Br) / 1421789-05-0 / B | 60 % |
| | 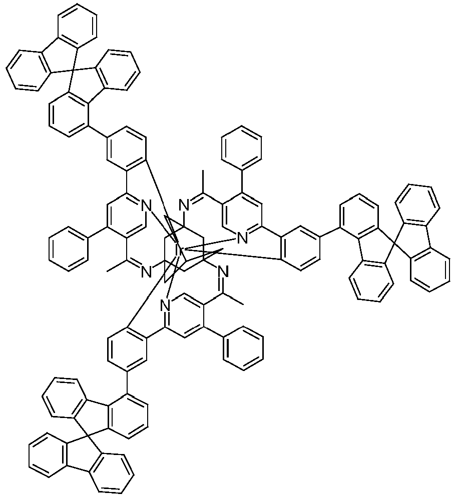 | |
| | o-Xylol | |
| Ir129 | Ir(L100-2Br) / 1233200-59-3 / B | 65 % |
| | 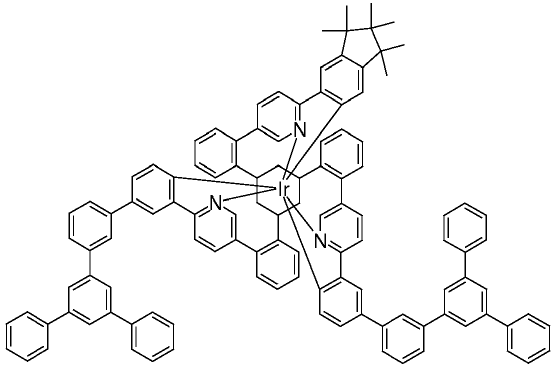 | |
| | Toluol | |
| Ir130 | Ir(L102-2Br) / 197223-39-5 / B | 66 % |
| | 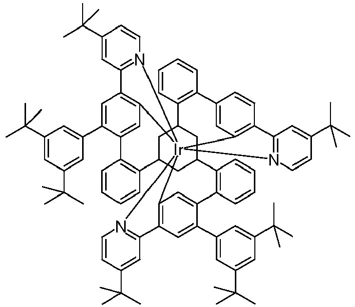 | |
| | Butylacetat | |
| Ir131 | Ir(L106-2Br) / 5122-95-2 / B | 70 % |
| | 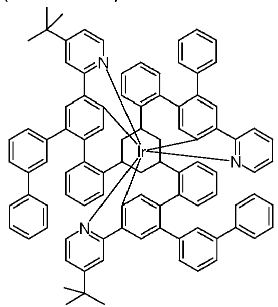 Toluol | |
| Ir132 | Ir(L107-2Br) / 786071-96-0 | 68 % |
| | 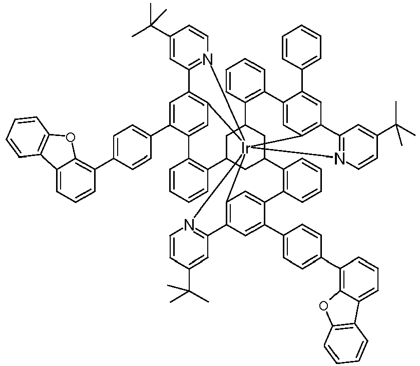 Toluol | |
| Ir133 | Ir(L116-2Br) / 1416814-68-0 / B | 67% |
| | 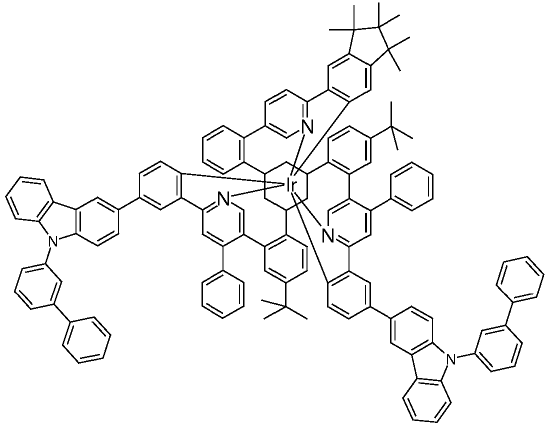 | |
| | Butylacetat | |
| Ir134 | Ir(L121-2Br) / 1423-26-3 / B | 63 % |
| | 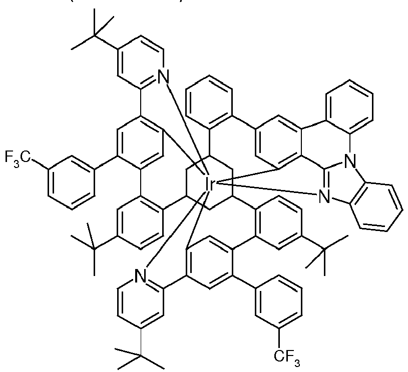 | |
| | Butylacetat | |
| Ir135 | Ir(L102-1 Br) / 1565126-29-5 / B | 65 % |
| | 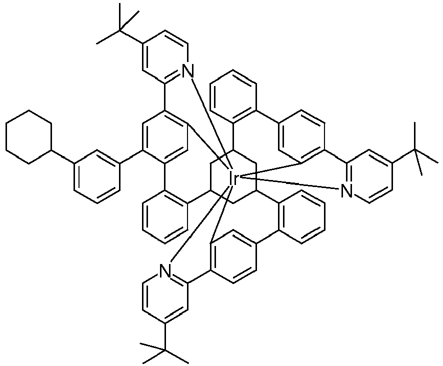 | |
| | Toluol | |
| Ir136 | Ir(L113-Br) / 1801624-63-4 / B | 62 % |
| | 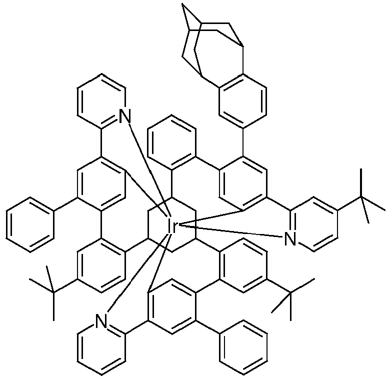 | |
| | Butylacetat | |
| Ir137 | / 1000869-26-0 / B | 71 % |
| | 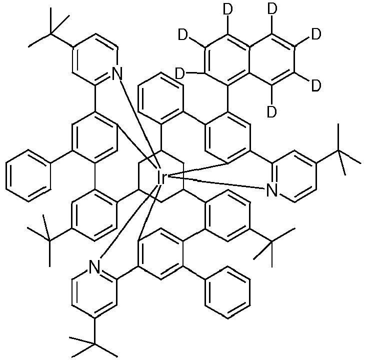 Toluol | |
| Ir138 | Ir(L400-3Br) /5122-95-2 / B | 58 % |
| | 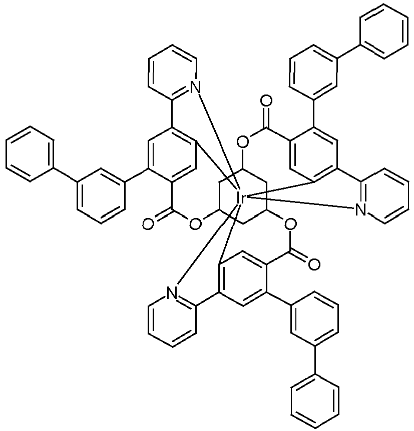 Toluol | |
| Ir139 | Ir(L404-3Br) / 84110-40-7 / B | 47% |
| | 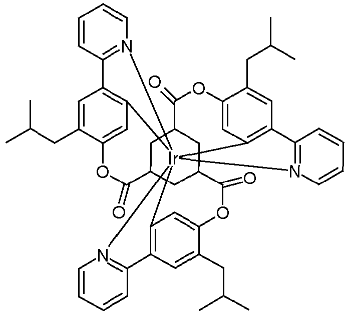 | |
| Ir140 | Ir(L405-3Br) / 1056113-44-0 / B | 54% |
| | 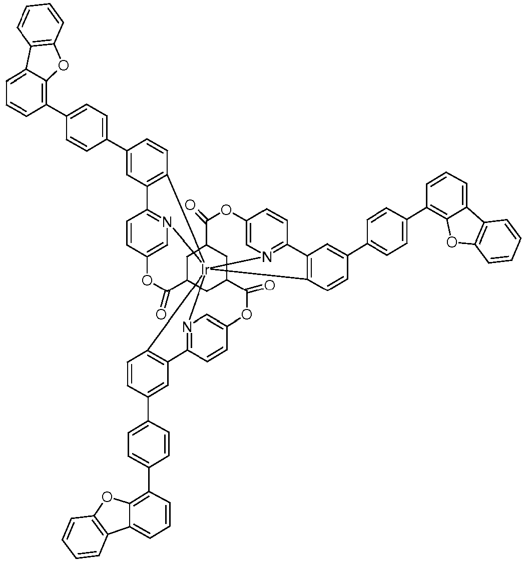 | |
| | Toluol | |
| Ir141 | Ir(L500-3Br) / 1801285-73-3 / B | 49 % |
| | 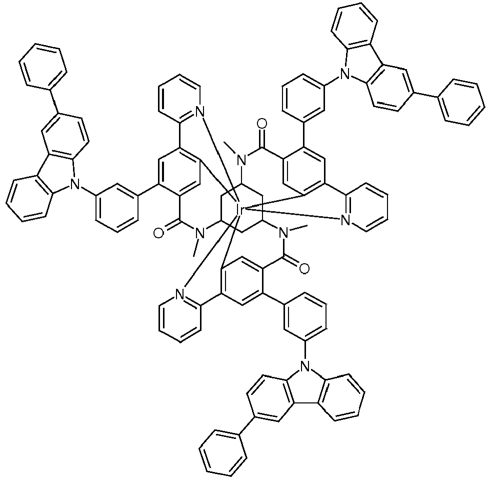 | |
| 1r142 | Ir(L503-3Br) / 1345508-82-8 / B | 52 % |
| | 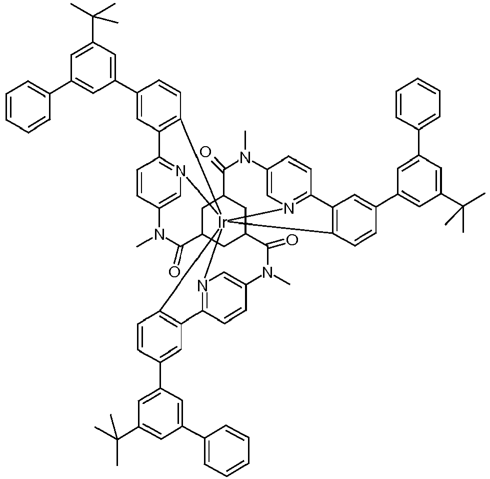 | |

### 4.4 Buchwald-Kupplung an den Ir-Komplexen

### Variante A:

Ein Gemisch aus 10 mmol des bromierten Komplexes, 12-20 mmol des Diarylamins oder Carbazols pro Brom-Funktion, 1.1 molare Menge an Natrium-tert-butylat pro eingesetztem Amin bzw. 80 mmol Trikaliumphosphat (wasserfrei) bei Carbazolen, 100 g Glaskugeln (3 mm Durchmesser) und 300 - 500 ml Toluol bzw. o-Xylol bei Carbazolen wird mit 0.4 mmol Tri-tert-butylphosphin und dann mit 0.3 mmol Palladium(II)acetat versetzt und unter gutem Rühren 16 - 30 h unter Rückfluss erhitzt. Nach Erkalten gibt man 500 ml Wasser zu, trennt die wässrige Phase ab, wäscht die organische Phase zweimal mit 200 ml Wasser, einmal mit 200 ml gesättigter Kochsalzlösung und trocknet über Magnesiumsulfat. Man filtriert über ein Celite-Bett ab, wäscht dieses mit Toluol bzw. o-Xylol nach, entfernt das Lösungsmittel fast vollständig im Vakuum, gibt 300 ml Ethanol zu, saugt vom ausgefallenen Rohprodukt ab, wäscht dieses dreimal mit je 50 ml EtOH und trocknet im Vakuum. Das Rohprodukt wird an Kieselgel chromatographisch und / oder durch Heißextraktion gereinigt. Der Metallkomplex wird abschließend getempert oder sublimiert. Das Tempern erfolgt im Hochvakuum (p ca 10⁻⁴ Pa (10⁻⁶ mbar)) im Temperaturbereich von ca. 200 - 300 °C. Die Sublimation erfolgt im Hochvakuum (p ca 10⁻⁴ Pa (10⁻⁶ mbar)) im Temperaturbereich von ca. 300 - 400 °C, wobei die Sublimation bevorzugt in Form einer fraktionierten Sublimation durchgeführt wird.

### Variante B:

Ein Gemisch aus 10 mmol des bromierten Komplexes, 12-20 mmol des Diarylamins oder Carbazols pro Brom-Funktion, 30 mmol Kaliumcarbonat und 30 mmol Natriumsulfat pro pro Brom-Funktion, 10 mmol Kupferiodid Brom-Funktion, 50 g Glaskugeln (3 mm Durchmesser) und 150 ml Nitrobenzol wird unter gutem Rühren 16 - 30 h auf 200 °C erhitzt. Nach Erkalten auf 100 °C entfernt man das Nitrobenzol weitgehend im Vakuum, gibt 300 ml MeOH zu, filtriert vom ausgefallenen Produkt und den Salzen ab, wäscht diese mit 50 ml Methanol nach und trocknet im Vakuum. Man nimmt den Rückstand in 300 ml Dichlormethan auf, filtriert über ein mit Dichlormethan vorgeschlämmtes Kieselgelbett von den Salzen ab, entfernt das Dichlormethan im Vakuum und chromatographiert erneut an Kieselgel.

### Synthese von Ir200:

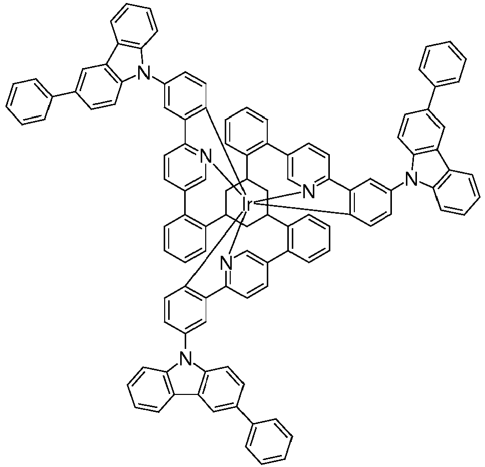

### Variante A:

Einsatz von 12.0 g (10 mmol) Ir(L1-3Br) und 9.7 g (40 mmol) 3-Phenylcarbazol [103012-26-6]. Dreimalige Chromatographie mit DCM an Kieselgel, tempern. Ausbeute: 6.3 g (3.7 mmol), 37 %; Reinheit: ca. 99.8 %ig nach HPLC.

### Variante B:

Einsatz von 12.0 g (10 mmol) Ir(L1-3Br) und 9.7 g (40 mmol) 3-Phenylcarbazol [103012-26-6]. Dreimalige Chromatographie mit DCM an Kieselgel, tempern. Ausbeute: 7.5 g (4.4 mmol), 44 %; Reinheit: ca. 99.7 %ig nach HPLC.

Analog können folgende Verbindungen dargestellt werden:

| Bsp. | Edukt / Amin oder Carbazol Produkt Heißextraktionsmittel | Ausbeute |
|---|---|---|
| Ir201 | Ir(L102-3Br) / 1257220-47-5 | 30 % |
| | 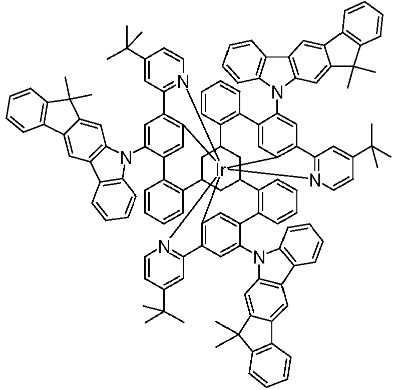 | |
| | Toluol | |
| Ir202 | Ir(L301-3Br) / 1421789-16-3 | 38 % |
| | 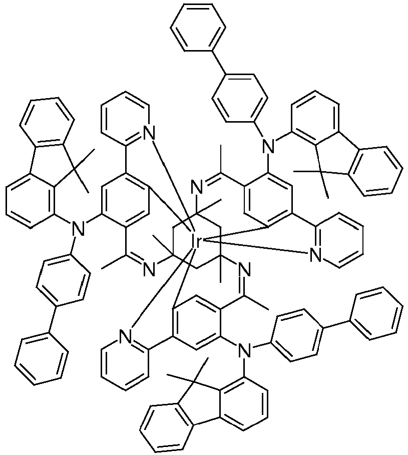 | |
| | Toluol | |
| Ir203 | Ir(L114-Br) / 103012-26-6 | 69 % |
| | 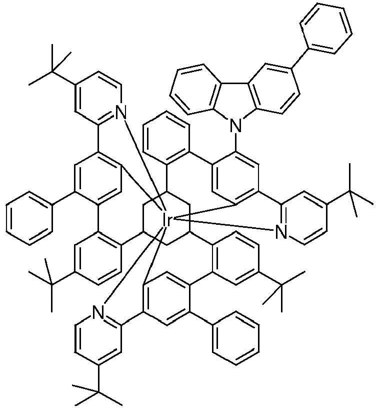 | |
| | Toluol | |

### 4.5 Cyanierung der Iridium-Komplexe:

Ein Gemisch aus 10 mmol des bromierten Komplexes, 13 mmol Kupfer(I)-cyanid pro Brom-Funktion und 300 ml NMP wird 20 h bei 180 °C gerührt. Nach Erkalten entfernt man das Lösungsmittel im Vakuum, nimmt den Rückstand in 500 ml Dichlormethan auf, filtriert über Celite von den Kupfersalzen ab, engt das Dichlormethan im Vakuum fast bis zur Trockene ein, gibt 100 ml Ethanol zu, saugt vom ausgefallenen Feststoff ab, wäscht diesen zweimal mit je 50 ml Ethanol und trocknet im Vakuum. Das Rohprodukt wird durch Chromatographie und / oder Heißextraktion gereinigt. Das Tempern erfolgt im Hochvakuum (p ca 10⁻⁴ Pa (10⁻⁶ mbar)) im Temperaturbereich von ca. 200 - 300 °C. Die Sublimation erfolgt im Hochvakuum (p ca 10⁻⁴ Pa (10⁻⁶ mbar)) im Temperaturbereich von ca. 300 - 400 °C, wobei die Sublimation bevorzugt in Form einer fraktionierten Sublimation durchgeführt wird.

### Synthese von Ir300:

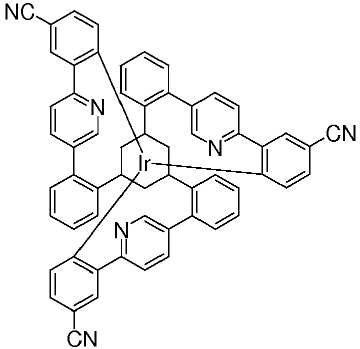

Einsatz von 12.0 g (10 mmol) Ir(L1-3Br) und 3.5 g (39) mmol) Kupfer(I)-cyanid. Zweimalige Chromatographie an Kieselgel mit Dichlormethan, Heißextraktion mit DCM, Sublimation. Ausbeute: 4.9 g (4.7 mmol), 47 %; Reinheit: ca. 99.9 %ig nach HPLC.

Analog können folgende Verbindungen dargestellt werden:

| Bsp. | Edukt Cyanierungsprodukt | |
|---|---|---|
| Ir301 | Ir(L123-3Br) | 51 % |
| | 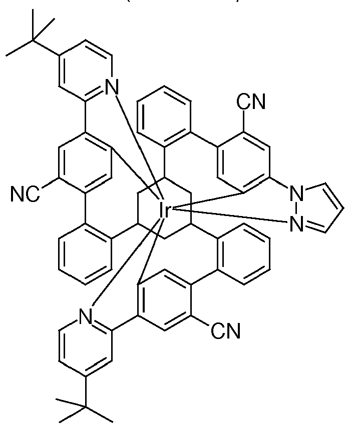 | |
| Ir302 | Ir(L121-2Br) | 64% |
| | 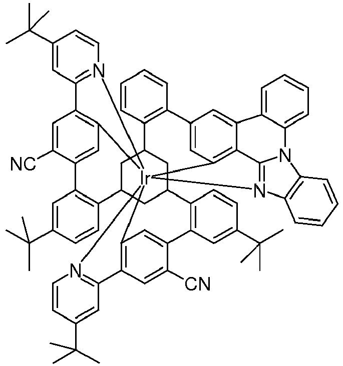 | |
| Ir303 | Ir(L208-3Br) | 47 % |
| | 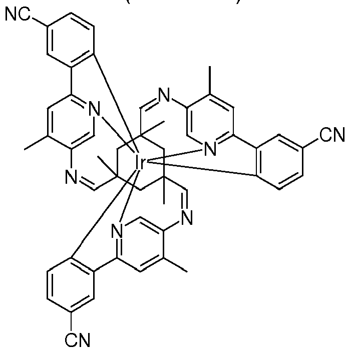 | |

### 4.6 Suzuki-Kupplung an den borylierten Iridium-Komplexen: Variante A, zweiphasige Reaktionsmischung:

Eine Suspension von 10 mmol eines borylierten Komplexes, 12-20 mmol Arylbromid pro (RO)₂B-Funktion und 80 mmol Trikaliumphosphat in einem Gemisch aus 300 ml Toluol, 100 ml Dioxan und 300 ml Wasser wird mit 0.6 mmol Tri-o-tolylphosphin und dann mit 0.1 mmol Palladium(II)acetat versetzt und 16 h unter Rückfluss erhitzt. Nach Erkalten gibt man 500 ml Wasser und 200 ml Toluol zu, trennt die wässrige Phase ab, wäscht die organische Phase dreimal mit 200 ml Wasser, einmal mit 200 ml gesättigter Kochsalzlösung und trocknet über Magnesiumsulfat. Man filtriert über ein Celite-Bett ab, wäscht dieses mit Toluol nach, entfernt das Toluol fast vollständig im Vakuum, gibt 300 ml Methanol zu, saugt vom ausgefallenen Rohprodukt ab, wäscht dieses dreimal mit je 50 ml Methanol und trocknet im Vakuum. Das Rohprodukt wird zweimal an Kieselgel gesäult und / oder durch Heißextraktion gereinigt. Der Metallkomplex wird abschließend getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 300 - 400 °C, wobei die Sublimation bevorzugt in Form einer fraktionierten Sublimation durchgeführt wird.

### Variante B, einphasige Reaktionsmischung:

Eine Suspension von 10 mmol eines borylierten Komplexes, 12-20 mmol Arylbromid pro (RO)₂B-Funktion und 60 - 100 mmol der Base (Kaliumfluorid, Trikaliumphosphat (wasserfrei, Monohydrat oder Trihydrat), Kaliumcarbonat, Cäsiumcarbonat etc.) und 100 g Glaskugeln (3 mm Durchmesser) in 100 ml - 500 ml eines aprotischen Lösungsmittels (THF, Dioxan, Xylol, Mesitylen, Dimethylacetamid, NMP, DMSO, etc.) wird mit 0.6 mmol Tri-o-tolylphosphin und dann mit 0.1 mmol Palladium(II)acetat oder 0.3 mmol Tetrakis-triphenylphosphino-palladium(O) versetzt und 1 - 24 h unter Rückfluss erhitzt. Alternativ können andere Phosphine wie Triphenylphosphin, Tri-tert-butylphosphin, SPhos, XPhos, RuPhos, XanthPhos, etc. eingesetzt werden, wobei bei diesen Phosphinen das bevorzugte Phosphin:Palladium Verhältnis 3:1 bis 1.2:1 beträgt. Man entfernt das Lösungsmittel im Vakuum, nimmt das Produkt in einem geeigneten Lösungsmittel (Toluol, Dichlormethan, Ethylacetat, etc.) auf und reinigt wie unter Variante A beschrieben.

### Synthese von Ir400:

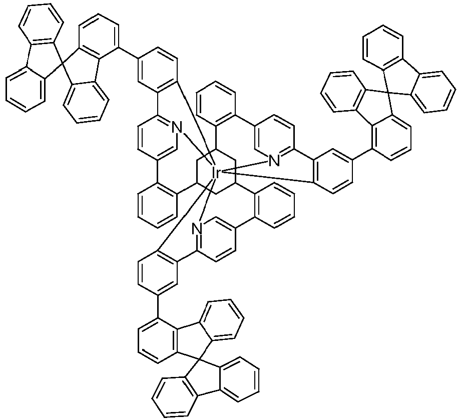

### Variante A:

Einsatz von 13.4 g (10.0 mmol) Ir(L1-3BE) und 7.4 g (40.0 mmol) 9,9'-Spirobifluoren-4-boronsäurepinacolester [1161009-88-6], 17.7 g (60 mmol) Trikaliumphosphat (wasserfrei), 183 mg (0.6 mmol) Tri-o-tolylphosphin [6163-58-2], 23 mg (0.1 mmol) Palladium(II)acetat, 300 ml Toluol, 100 ml Dioxan und 300 ml Wasser, 100 °C, 16 h. Zweimalige chromatographische Trennung an Kieselgel mit Toluol/Ethylacetat (9:1, vv), dreimalige Heißextraktion mit o-Xylol. Ausbeute: 10.9 g (5.7 mmol),57 %; Reinheit: ca. 99.9 %ig nach HPLC.

Analog können folgende Verbindungen dargestellt werden:

| Bsp. | Edukte / Variante Produkt Heißextraktionsmittel | Ausbeute |
|---|---|---|
| Ir401 | Ir(L3-3BE) / 1613576-58-1 / A | 48 % |
| | 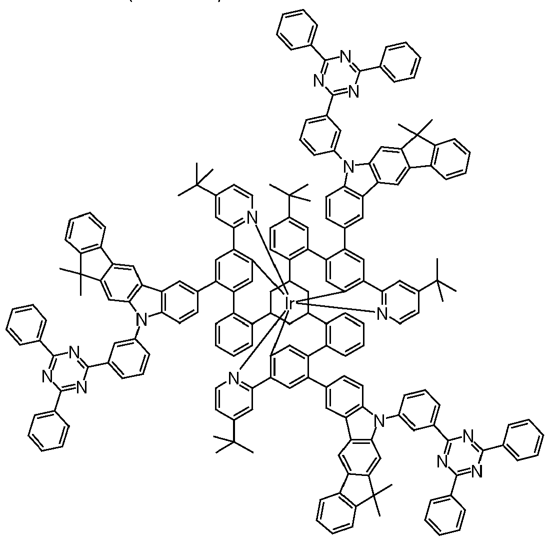 | |
| | Toluol | |
| Ir402 | Ir(L5-3BE) / 3842-55-5 / B | 37% |
| | 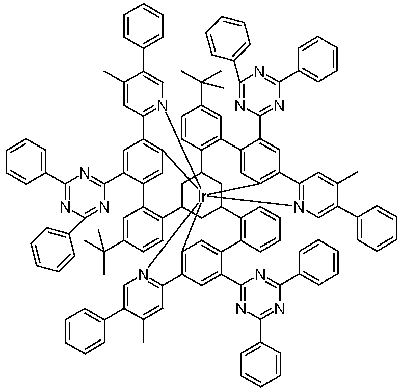 Toluol | |
| Ir403 | Ir(L107-2BE) / 50548-45-3 / B / PPh₃ : Pd(ac)₂ 3:1 / K₃PO₄ ^{∗} H₂O / DMSO / 90 °C / 18 h | 41 % |
| | 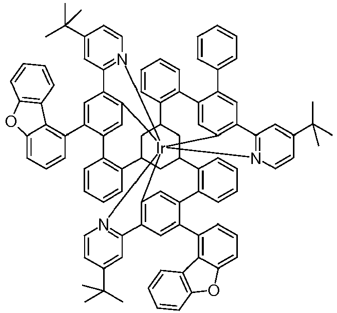 | |
| | Toluol | |

### 4.7 Alkylierung an Iridium-Komplexen:

Eine Suspension von 10 mmol des Komplexes in 1500 ml THF wird mit 50 ml einer frisch bereiteten LDA-Lösung, 1 molar in THF versetzt und 24 h bei 25 °C nachgerührt. Dann gibt man unter gutem Rühren auf ein Mal 200 mmol des Alkylierungsmittels zu, wobei flüssige Alkylierungsmittel ohne Verdünnug, feste als Lösung in THF zugegeben werden. Man rührt 60 min. bei Raumtemperatur nach, entfernt das THF im Vakuum und chromatographiert den Rückstand an Kieselgel. Die weitere Reiningung kann durch Heißextraktion - wie oben beschrieben - erfolgen. Der Metallkomplex wird abschließend getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca 10⁻⁴ Pa (10⁻⁶ mbar)) im

Temperaturbereich von ca. 300 - 400 °C, wobei die Sublimation bevorzugt in Form einer fraktionierten Sublimation durchgeführt wird.

### Synthese von Ir500:

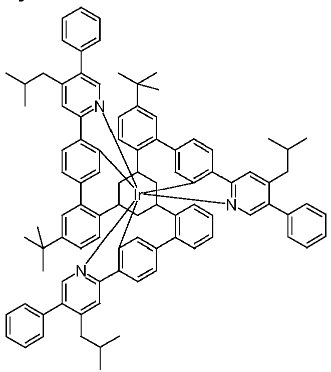

Einsatz von 13.4 g (10.0 mmol) Ir(L5) und 21.7 ml (200 mmol) 1-Brom-2-methyl-propan [78-77-3]. Zweimalige chromatographische Trennung an Kieselgel mit Toluol, anschließend fünfmalige Heißextraktion mit Ethylacetat / Acetonitril. Ausbeute: 4.6 g (3.1 mmol) 31 %; Reinheit: ca. 99.7 %ig nach HPLC.

Analog können folgende Verbindungen dargestellt werden:

| Bsp. | Edukt / Alkylierungsmittel Produkt | Ausbeute |
|---|---|---|
| Ir501 | Ir(L103) / 1.5 eq LDA / 6 eq 78-77-3 | 42 % |
| | 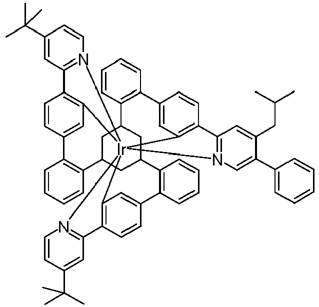 | |
| Ir502 | Ir(L104) / 1.5 eq LDA / 6 eq 108-85-0 | 29 % |
| | 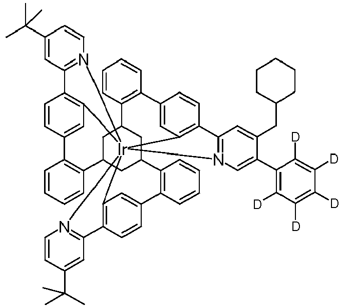 | |
| Ir503 | Ir(L105) / 1.5 eq LDA / 6 eq 630-17-1 | 35 % |
| | 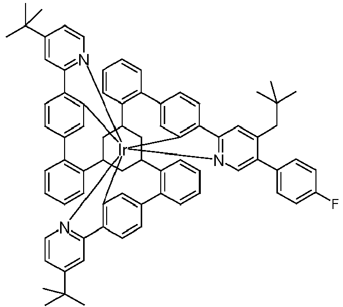 | |
| Ir504 | Ir(L110) / 3 eq LDA / 9 eq 74-83-9 | 32 % |
| | 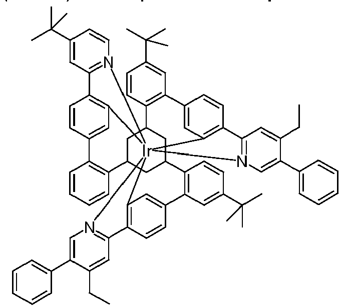 | |
| Ir505 | Ir(L203) / 5 eq LDA / 20 eq 78-77-3 | 27 % |
| | 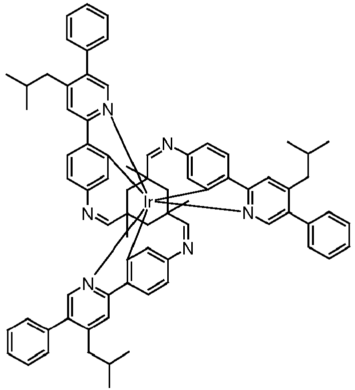 | |

### 4.8 Deuterierung von Ir-Komplexen:

### Beispiel: Ir(L5-D9)

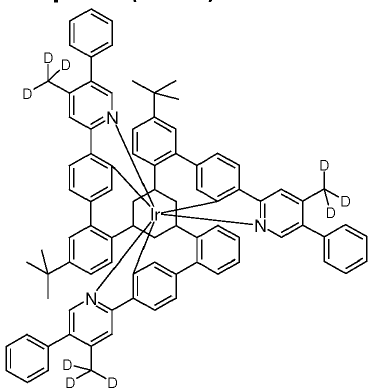

Ein Gemisch von 1.34 g (1.0 mmol) Ir(L5), 24 mg (1.0 mmol) Natriumhydrid, 3 ml Methanol-D4 und 30 ml DMSO-D6 wird 18 h auf 80 °C erhitzt. Nach Erkalten gibt man 1.0 ml DCI 5 M in D₂O zu, rührt kurz nach und tropft dann 80 ml Methanol zu. Man saugt vom ausgefallenen Feststoff ab, wäscht diesen dreimal mit je 10 ml Methanol, trocknet im Vakuum und chromatographiert den Rückstand mit DCM an Kieselgel. Ausbeute: 1.14 g (0.84 mmol), 84 %, Deuterierungsgrad > 90 %.

Analog können folgende Verbindungen dargestellt werden:

| Bsp. | Edukt / Produkt | Ausbeute |
|---|---|---|
| Ir(L103-D3) | Ir(L103) / 0.3 mmol NaH | 90 % |
| | 2 ml Methanol-D4 / 10 ml DMSO-D6 | |
| | 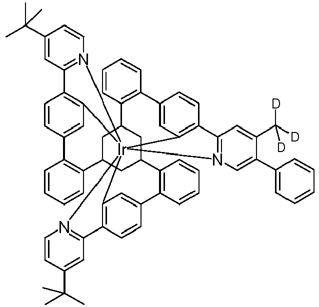 | |
| Ir(L104-D3) | Ir(L104) / 0.3 mmol NaH | 87% |
| | 2 ml Methanol-D4 / 10 ml DMSO-D6 | |
| | 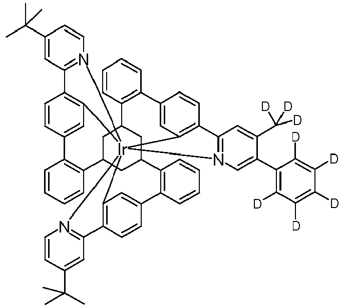 | |
| Ir(L105-D3) | Ir(L105) / 0.3 mmol NaH | 92 % |
| | 2 ml Methanol-D4 / 10 ml DMSO-D6 | |
| | 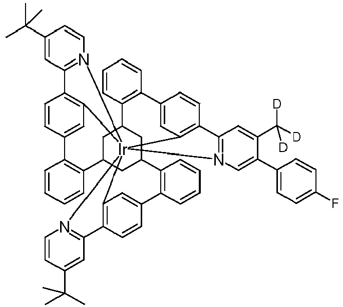 | |
| Ir(110-D6) | Ir(L110)) / 0.6 mmol NaH | 90 % |
| | 2 ml Methanol-D4 / 20 ml DMSO-D6 | |
| | 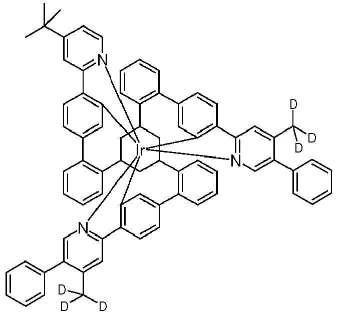 | |
| Ir(L203-D9) | Ir(L203) / 1.0 mmol NaH | 93 % |
| | 4 ml Methanol-D4 / 30 ml DMSO-D6 | |
| | 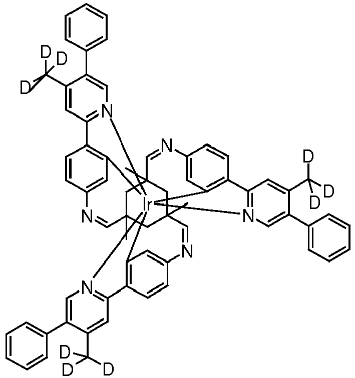 | |

### 4.9 Trennung der Δ- und Λ-Enantiomeren der Metallkomplexe mittels Chromatographie an chiralen Säulen:

Die Δ- und Λ-Enantiomeren der Komplexe können mittels analytischer und/oder präparativer Chromatographie an chiralen Säulen nach laborüblichen Methoden getrennt werden, z.B. Trennung von Ir105 an ChiralPak AZ-H (Fa. Chiral Technologies INC.) mit n-Hexan/Ethanol (90:10), Retentionszeiten 13.4 min. bzw. 16.8 min.

### 5. Polymere enthaltend die Metallkomplexe:

### Allgemeine Polymerisationsvorschrift für die Bromide bzw. Boronsäure-Derivate als polymerisierbare Gruppe, Suzuki-Polymerisation Variante A - Zweiphasiges Reaktionsgemisch:

Die Monomere (Bromide und Boronsäuren bzw. Boronsäureester, Reinheit nach HPLC > 99.8 % ig) werden in der in der Tabelle angegebenen Zusammensetzung in einer Gesamtkonzentration von ca. 100 mmol/L in einem Gemisch aus 2 Volumenteilen Toluol : 6 Volumenteilen Dioxan : 1 Volumenteil Wasser gelöst bzw. suspendiert. Dann gibt man 2 mol Äquivalente Trikaliumphosphat pro eingesetzter Br-Funktionalität zu, rührt 5 min. nach, fügt dann 0.03 bis 0.003 mol Äquivalente Tri-ortho-tolylphosphin und dann 0.005 bis 0.0005 mol Äquivalente Palladium(II)acetat (Verhältnis Phosphin zu Pd bevorzugt 6:1) pro eingesetzter Br-Funktionalität zu und erhitzt unter sehr gutem Rühren 2-3 h unter Rückfluss. Falls die Viskosität der Mischung zu stark ansteigt, kann mit einem Gemisch aus 2 Volumenteilen Toluol : 3 Volumenteilen Dioxan verdünnt werden. Nach insgesamt 4-6 h Reaktionszeit fügt man zum End-capping 0.05 mol Äquivalente pro eingesetzter Boronsäure-Funktionalität eines Monobromaromaten und dann 30 min. danach 0.05 mol Äquivalente pro eingesetzter Br-Funktionalität einer Monoboronsäure bzw. eines Monoboronsäureesters zu und kocht weitere 1 h nach. Nach Erkalten verdünnt man mit 300 ml Toluol, trennt die wässrige Phase ab, wäscht die organische Phase zweimal mit je 300 ml Wasser, trocknet über Magnesiumsulfat, filtriert über ein Celite-Bett ab, um Palladium zu entfernen und engt dann zur Trockene ein. Man löst das Rohpolymer in THF (Konzentration ca. 10 - 30 g/L) und lässt die Lösung unter sehr gutem Rühren langsam in das doppelte Volumen Methanol einlaufen. Das Polymer wird abgesaugt und dreimal mit Methanol gewaschen. Der Umfällvorgang wird fünfmal wiederholt, danach wird das Polymer im Vakuum bis zur Gewichtskonstanz bei 30 - 50 °C getrocknet.

### Variante B - Einphasiges Reaktionsgemisch:

Die Monomere (Bromide und Boronsäuren bzw. Boronsäureester, Reinheit nach HPLC > 99.8 % ig) werden in der in Tabelle angegebenen Zusammensetzung in einer Gesamtkonzentration von ca. 100 mmol/L in einem Lösemittel (THF, Dioxan, Xylol, Mesitylen, Dimethylacetamid, NMP, DMSO, etc.) gelöst bzw. suspendiert. Dann gibt man 3 mol Äquivalente Base (Kaliumfluorid, Trikaliumphosphat (wasserfrei, Monohydrat oder Trihydrat), Kaliumcarbonat, Cäsiumcarbonat etc. jeweils wasserfrei) pro Br-Funktionalität und das Gewichtsäquivalent Glaskugeln (3 mm Durchmesser) zu, rührt 5 min. nach, fügt dann 0.03 bis 0.003 mol Äquivalente Tri-ortho-tolylphosphin und dann 0.005 bis 0.0005 mol Äquivalente Palladium(II)acetat (Verhältnis Phosphin zu Pd bevorzugt 6:1) pro Br-Funktionalität zu und erhitzt unter sehr gutem Rühren 2-3 h unter Rückfluss. Alternativ können andere Phosphine wir Tri-tert-butylphosphin, SPhos, XPhos, RuPhos, XanthPhos, etc. eingesetzt werden, wobei bei diesen Phosphinen das bevorzugte Phosphin : Palladium Verhältnis 2:1 bis 1.3:1 beträgt. Nach insgesamt 4-12 h Reaktionszeit fügt man zum End-capping 0.05 mol Äquivalente eines Monobromaromaten und dann 30 min. danach 0.05 mol Äquivalente einer Monoboronsäure bzw. eines Monoboronsäureesters zu und kocht weiter 1 h nach. Man entfernt das Lösungsmittel weitgehend im Vakuum, nimmt den Rückstand in Toluol auf und reinigt das Polymer wie unter Variante A beschrieben.

### Monomere M / Endcapper E:

| | |
|---|---|
| 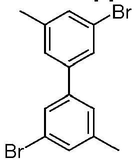 | 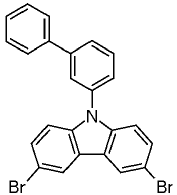 |
| 13974-84-0 M1 | 57103-20-5 M2 |
| 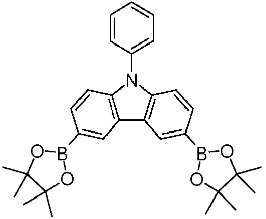 | 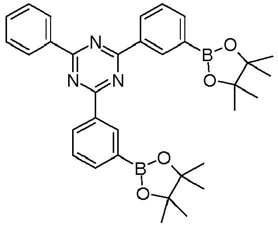 |
| 618442-57-2 M3 | 1238752-26-5 M4 |
| 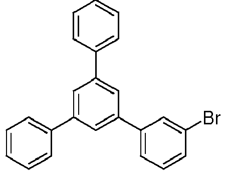 | 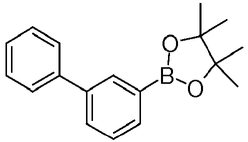 |
| 1233200-57-1 E1 | 912844-88-3 E2 |

### Polymere:

### Zusammensetzung der Polymere, mmol :

| **Polymer** | **M1** | **M2** | **M3** | **M4** | **Ir-Komplex** |
|---|---|---|---|---|---|
| P1 | --- | 30 | --- | 45 | Ir(L102-3Br) / 10 |
| P2 | 5 | 25 | --- | 40 | Ir(L107-2Br) / 10 |
| P3 | 10 | 40 | 25 | 20 | Ir(L107-2BE) / 5 |

### Molekulargewichte und Ausbeute der erfindungsgemäßen Polymere:

| Polymer | Mn [gmol⁻¹] | Polydispersität | Ausbeute |
|---|---|---|---|
| P1 | 200.000 | 5.3 | 70 % |
| P2 | 350.000 | 2.4 | 53 % |
| P3 | 240.000 | 2.2 | 57% |

### 6. Thermische und photophysikalische Eigenschaften sowie Oxidations- und Reduktionspotentiale

Tabelle 1 fasst die thermischen und photochemischen Eigenschaften sowie Oxidations- und Reduktionspotentiale der Vergleichsmaterialien IrPPy, Ir1 bis Ir3 (Strukturen s. Tabelle 5) und die ausgewählter erfindungsgemäßer Materialien zusammen. Die erfindungsgemäßen Verbindungen weisen eine im Vergleich zu den Materialien nach Stand der Technik verbesserte thermische Stabilität und Photostabilität auf. Während Materialien nach Stand der Technik nach siebentägiger thermischer Auslagerung bei 380 °C Braunverfärbungen und Veraschung zeigen und man im ¹H-NMR Nebenkomponenten im Bereich > 2 mol % nachweisen kann, sind die erfindungsgemäßen Komplexe unter diesen Bedingungen inert. Diese thermische Robustheit ist insbesondere für die Verarbeitung der Materialien im Hochvakuum (Vapor-Small-Molecule Devices) entscheidend. Außerdem weisen die erfindungsgemäßen Verbindungen eine sehr gute Photostabilität in wasserfreien C₆D₆-Lösung unter Bestrahlung mit Licht der Wellenlänge von ca. 455 nm auf. Insbesondere ist im Gegensatz zu Komplexen nach Stand der Technik, die zweizähnige Liganden enthalten, im ¹H-NMR keine facial-meridional Isomerisierung nachzuweisen. Wie Tabelle 1 entnommen werden kann, zeichnen sich die erfindungsgemäßen Verbindungen in Lösung durch durchweg sehr hohe PL-Quanteneffizienzen aus.

**Tabelle 1:**

| **Komplex** | **therm. Stab. Photo. Stab.** | **PL-max. FWHM** | **PLQE** | **HOMO LUMO** |
|---|---|---|---|---|
| **Vergleichsbeispiele, Strukturen s. Tabelle 5** | | | | |
| **IrPPy** | Zersetzung | 509 | 0.97 | --- |
| | Zersetzung | 67 | Toluol | --- |
| **Ir1** | --- | 513 | 0.97 | -5.09 |
| | --- | 60 | Toluol | -1.99 |
| **Ir2** | Zersetzung | 516 | 0.97 | -5.05 |
| | Zersetzung | 69 | Toluol | -1.71 |
| **Ir3** | Zersetzung | 510* | 0.76* | --- |
| | Zersetzung | --- | BuCN | --- |

| Erfindungsgemäße Beispiele | | | | |
|---|---|---|---|---|
| **Ir(L1)** | keine Zers. | 523 | 0.99 | -5.09 |
| | keine Zers. | 63 | Toluol | -2.01 |
| | | | 0.91 | |
| | | | MeCN | |
| **Ir(L6)** | keine Zers. | 520 | 0.96 | -5.02 |
| | keine Zers. | 56 | Toluol | -1.96 |
| **Ir(L103)** | keine Zers. | 528 | 0.95 | -5.04 |
| | keine Zers. | 67 | Toluol | -1.97 |
| **Ir(L400)** | keine Zers. | 495 | 0.97 | -5.02 |
| | keine Zers. | 57 | Toluol | -2.00 |
| **Ir(L404)** | keine Zers. | 552 | 0.94 | -5.26 |
| | keine Zers. | 62 | Toluol | -2.21 |
| **Ir(L500)** | keine Zers. | 512 | 0.96 | -5.03 |
| | keine Zers. | 61 | Toluol | -1.99 |

| | | | | |
|---|---|---|---|---|
| *: Daten aus G. St-Pierre et al., Dalton Trans, 2011, 40, 11726. | | | | |

### Legende:

### - Therm. Stab. (thermische Stabilität):

Auslagerung in unter Vakuum abgeschmolzenen Ampullen, 7 Tage bei 380 °C. Visuelle Begutachtung auf Farbveränderung / Braunverfärbung / Veraschung und Analyse mittels ¹H-NMR Spektroskopie.

### - Photo. Stab. (photochemische Stabilität):

Bestrahlung ca. 1 mmolarer Lösungen in wasserfreiem C₆D₆ (entgaste und abgeschmolzene NMR-Röhrchen) mit blauem Licht (ca. 455 nm, 1.2 W Lumispot der Fa. Dialight Corporation, USA) bei RT.

### - PL-max.:

Maximum des PL-Spektrums in [nm] einer entgasten ca. 10⁻⁵ molaren Lösung bei RT, Anregungswellenlänge 370 nm, Lösungsmittel s. Spalte PLQE.

### - FWHM:

Halbwertsbreite des PL-Spektrums in [nm] bei RT.

### - PLQE.:

Abs. Photolumineszenz-Quanteneffizienz einer entgasten, ca. 10⁻⁵ molaren Lösung im angegebenen Lösungsmittel bei RT.

### - HOMO, LUMO:

in [eV] vs. Vakuum, bestimmt in Dichlormethan-Lösung (Oxidation) bzw. THF (Reduktion) mit interner Ref. Ferrocen (- 4.8 eV vs. Vakuum).

### 7. Löslichkeit ausgewählter Komplexe bei 25 °C

Für die Verarbeitung der erfindungsgemäßen Komplexe aus Lösung (Spin-Coating, InkJet-Printing, Nozzle-Printing, Rakeln, etc.) werden langzeitstabile Lösungen mit Feststoffgehalten von ca. 5 mg/ml oder mehr benötigt.

**Tabelle 2: Löslichkeiten ausgewählter Komplexe**

| Komplex | Lösungsmittel | Löslichkeit |
|---|---|---|
| Ir(L4) | Toluol | > 10 mg/ml |
| Ir(L4) | 3-Phenoxytoluol | > 30 mg/ml |
| Ir(L5) | Toluol | > 5 mg/ml |
| Ir(L7) | Toluol | > 10 mg/ml |
| Ir(L107) | Toluol | > 10 mg/ml |
| Ir(L109) | Toluol | > 15 mg/ml |
| Ir(L115) | Toluol | > 15 mg/ml |
| Ir(L115) | Anisol | > 20 mg/ml |
| Ir(L115) | 3-Phenoxytoluol | > 25 mg/ml |
| Ir(L120) | Toluol | > 10 mg/ml |
| Ir(L120) | 3-Phenoxytoluol | > 20 mg/ml |
| Ir(138) | 3-Phenoxytoluol | > 25 mg/ml |
| Ir(141) | 3-Phenoxytoluol | > 35 mg/ml |
| Ir(142) | 3-Phenoxytoluol | > 30 mg/ml |

### Herstellung der OLEDs

### 1) Vakuum-prozessierte Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Glasplättchen, mit strukturiertem ITO (50 nm, Indium-Zinn-Oxid) bilden die Substrate, auf welche die OLEDs aufgebracht werden. Die OLEDs haben prinzipiell folgenden Schichtaufbau: Substrat / Lochtransportschicht 1 (HTL1) bestehend aus HTM dotiert mit 5 % NDP-9 (kommerziell erhältlich von der Fa. Novaled), 20 nm / Lochtransportschicht 2 (HTL2) / optionale Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet.

Zunächst werden vakuumprozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Co-Verdampfung in einem bestimmten Volumenanteil beigemischt wird. Eine Angabe wie M3:M2:Ir(L1) (55%:35%:10%) bedeutet hierbei, dass das Material M3 in einem Volumenanteil von 55%, M2 in einem Anteil von 35% und Ir(L1) in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 5 gezeigt.

Die OLEDs werden standardmäßig charakterisiert. Hierfür werden die Elektrolumineszenzspektren, die Stromeffizienz (gemessen in cd/A) und die Spannung (gemessen bei 1000 cd/m² in V) bestimmt aus Strom-Spannungs-Helligkeits-Kennlinien (IUL-Kennlinien). Für ausgewählte Versuche wird die Lebensdauer bestimmt. Als Lebensdauer wird die Zeit definiert, nach der die Leuchtdichte von einer bestimmten Startleuchtdichte aus auf einen gewissen Anteil abgesunken ist. Die Angabe LD50 bedeutet, dass es sich bei der genannten Lebensdauer um die Zeit handelt, bei der die Leuchtdichte auf 50% der Startleuchtdichte abgefallen ist, also von z.B. 1000 cd/m² auf 500 cd/m². Je nach Emissionsfarbe wurden unterschiedliche Starthelligkeiten gewählt. Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von 1000 cd/m² eine übliche Angabe.

### Verwendung von erfindungsgemäßen Verbindungen als Emittermaterialien in phosphoreszierenden OLEDs

Die erfindungsgemäßen Verbindungen lassen sich unter anderem als phosphoreszierende Emittermaterialien (Dotanden) in der Emissionsschicht in OLEDs und als Elektronentransportmaterial einsetzen. Als Vergleich gemäß dem Stand der Technik werden die Iridium-Verbindungen gemäß Tabelle 5 verwendet. Die Ergebnisse der OLEDs sind in Tabelle 2 zusammengefasst.

**Tabelle 1: Aufbau der OLEDs**

| **Bsp.** | **HTL2 Dicke** | **EBL Dicke** | **EML Dicke** | **HBL Dicke** | **ETL Dicke** |
|---|---|---|---|---|---|
| **grüne - gelbe Devices** | | | | | |
| Ref.-D1 | HTM | --- | M1:IrPPy (85%:15%) 30 nm | ETM1 | ETM1:ETM2 (50%:50%) 30 nm |
| | 40 nm | | | 10 nm | |
| Ref.-D2 | HTM | --- | M1:Ir2 (85%:15%) 30 nm | ETM1 | ETM1:ETM2 (50%:50%) 30 nm |
| | 40 nm | | | 10 nm | |
| Ref.-D3 | HTM | --- | M1:M3:Ir2 (60%:30%:10%) 30 nm | ETM1 | ETM1:ETM2 (50%:50%) 30 nm |
| | 40 nm | | | 10 nm | |
| Ref.-D4 | HTM | --- | M1:Ir3 (85%:15%) 30 nm | ETM1 | ETM1:ETM2 (50%:50%) 30 nm |
| | 40 nm | | | 10 nm | |
| Ref.-D5 | HTM | --- | M1:M3:Ir3 (60%:30%:10%) 30 nm | ETM1 | ETM1:ETM2 (50%:50%) 30 nm |
| | 40 nm | | | 10 nm | |
| D1 | HTM | --- | M1:Ir(L1) (85%:15%) 30 nm | ETM1 | ETM1:ETM2 (50%:50%) 30 nm |
| | 40 nm | | | 10 nm | |
| D2 | HTM | --- | M1:Ir(L3) (85%:15%) 30 nm | ETM1 | ETM1:ETM2 (50%:50%) 30 nm |
| | 40 nm | | | 10 nm | |
| D3 | HTM | --- | M1:Ir(L102) (85%:15%) 30 nm | ETM1 | ETM1:ETM2 (50%:50%) 30 nm |
| | 40 nm | | | 10 nm | |
| D4 | HTM | --- | M1:M3:Ir(L 1) (60%:30%:10%) 30 nm | ETM1 | ETM1:ETM2 (50%:50%) 30 nm |
| | 40 nm | | | 10 nm | |
| D5 | HTM | --- | M1:M3:Ir(L 102) (60%:30%:10%) 30 nm | ETM1 | ETM1:ETM2 (50%:50%) 30 nm |
| | 40 nm | | | 10 nm | |
| D6 | HTM | --- | M2:M3:Ir(L103) (60%:30%:10%) 30 nm | ETM1 | ETM1:ETM2 (50%:50%) 30 nm |
| | 40 nm | | | 10 nm | |
| D7 | HTM | --- | M1:M3:Ir(L 102) (60%:30%:10%) 30 nm | ETM1 | M200 |
| | 40 nm | | | 10 nm | 30 nm |
| D8 | HTM | --- | M1:M3:Ir(L400) (60%:30%:10%) 30 nm | ETM1 | ETM1:ETM2 (50%:50%) 30 nm |
| | 40 nm | | | 10 nm | |
| D9 | HTM | --- | M1:M3:Ir(L500) (60%:30%:10%) 30 nm | ETM1 | M200 |
| | 40 nm | | | 10 nm | 30 nm |

| **orange - rote Devices** | | | | | |
|---|---|---|---|---|---|
| D100 | HTM | --- | M1:Ir(L8) (90%:10%) 30 nm | ETM1 | ETM1:ETM2 (50%:50%) 30 nm |
| | 40 nm | | | 10 nm | |
| D100 | HTM | --- | M2:M3:Ir(L8) (50%:40%:10%) 30 nm | ETM1 | ETM1:ETM2 (50%:50%) 30 nm |
| | 40 nm | | | 10 nm | |
| D102 | HTM | --- | M2:M3:Ir(L404) (60%:30%:10%) 30 nm | ETM1 | ETM1:ETM2 (50%:50%) 30 nm |
| | 40 nm | | | 10 nm | |

**Tabelle 2: Ergebnisse der Vakuum-prozessierten OLEDs**

| **Bsp.** | **EQE (%) 1000 cd/m²** | **Spannung (V) 1000 cd/m²** | **CIE x/y 1000 cd/m²** | **LD50 (h) 1000 cd/m²** |
|---|---|---|---|---|
| **grüne - gelbe Devices** | | | | |
| Ref.-D1 | 15.7 | 2.8 | 0.33/0.62 | 60000 |
| Ref.-D2 | 18.6 | 2.9 | 0.35/0.61 | 200000 |
| Ref.-D3 | 18.8 | 2.9 | 0.35/0.61 | 330000 |
| Ref.-D4 | 18.7 | 3.0 | 0.34/0.62 | 180000 |
| Ref.-D5 | 18.6 | 3.0 | 0.34/0.62 | 270000 |
| D1 | 19.7 | 2.9 | 0.35/0.61 | 280000 |
| D2 | 19.3 | 2.9 | 0.34/0.62 | 260000 |
| D3 | 20.4 | 2.9 | 0.34/0.63 | 270000 |
| D4 | 19.5 | 2.9 | 0.35/0.61 | 370000 |
| D5 | 21.0 | 3.1 | 0.34/0.62 | 360000 |
| D6 | 20.2 | 3.0 | 0.37/0.61 | 390000 |
| D7 | 20.9 | 3.0 | 0.34/0.62 | 350000 |
| D8 | 19.8 | 2.9 | 0.22/0.61 | 260000 |
| D9 | 20.7 | 3.1 | 0.34/0.62 | 410000 |

| **orange - rote Devices** | | | | |
|---|---|---|---|---|
| D100 | 19.4 | 2.9 | 0.45/0.55 | 270000 |
| D101 | 19.7 | 2.9 | 0.46/0.54 | 380000 |
| D102 | 20.1 | 3.2 | 0.40/0.58 | 360000 |

### Lösungs-prozessierte Devices:

### A: Aus löslichen Funktionsmaterialien

Die erfindungsgemäßen Iridium-Komplexe können auch aus Lösung verarbeitet werden und führen dort zu prozesstechnisch wesentlich einfacheren OLEDs, im Vergleich zu den vakuumprozessierten OLEDs, mit dennoch guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 2004/037887). Der Aufbau setzt sich aus Substrat / ITO / Lochinjektionsschicht (60 nm) / Interlayer (20 nm) / Emissionsschicht (60 nm) / Lochblockierschicht (10 nm) / Elektronentransportschicht (40 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum eine 60 nm Lochinjektionsschicht durch Spin-Coating aufgebracht. Die benötigte Spinrate hängt vom Verdünnungsgrad und der spezifischen Spin-Coater-Geometrie ab. Um Restwasser aus der Schicht zu entfernen, werden die Substrate für 30 Minuten bei 200 °C auf einer Heizplatte ausgeheizt. Die verwendete Interlayer dient dem Lochtransport, in diesem Fall wird ein HL-X von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Triplettemitter zusammen mit den Matrixmaterialien in Toluol oder Chlorbenzol gelöst. Der typische Feststoffgehalt solcher Lösungen liegt zwischen 16 und 25 g/L, wenn, wie hier, die für eine Device typische Schichtdicke von 60 nm mittels Spincoating erzielt werden soll. Die lösungsprozessierten Devices vom Typ1 enthalten eine Emissionsschicht aus M4:M5:IrL (25%:55%:20%), die vom Typ2 enthalten eine Emissionsschicht aus M4:M5:IrLa:IrLb (30%:34%:30%:6%), d.h. sie enthalten zwei verschiedene Ir-Komplexe. Die Emissionsschicht wird in einer Inertgasatmosphäre, im vorliegenden Fall Argon, aufgeschleudert und 10 min bei 160 °C ausgeheizt. Darüber wird die Lochblockierschicht (10nm ETM1) und die Elektronentransportschicht (40nm ETM1 (50%) / ETM2 (50%)) aufgedampft (Aufdampfanlagen von Lesker o.a., typischer Aufdampfdruck 5 x 10⁻⁶ mbar). Zuletzt wird eine Kathode aus Aluminium (100 nm) (hochreines Metall von Aldrich) aufgedampft. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen.

**Tabelle 3: Ergebnisse mit aus Lösung prozessierten Materialien**

| **Bsp.** | **Emitter Device** | **EQE (%) 1000 cd/m²** | **Spannung (V) 1000 cd/m²** | **CIE x/y** | **LD50 (h) 1000 cd/m²** |
|---|---|---|---|---|---|
| **Grüne und Gelbe OLEDs** | | | | | |
| Sol-Ref.-D1 | Ir1 | 19.8 | 5.1 | 0.34/0.62 | 200000 |
| | Typ1 | | | | |
| Sol-D1 | Ir(L4) | 20.6 | 5.0 | 0.36/0.61 | 240000 |
| | Typ1 | | | | |
| Sol-D2 | Ir(L107) | 21.2 | 5.0 | 0.34/0.62 | 270000 |
| | Typ1 | | | | |
| Sol-D3 | Ir(L109) | 20.7 | 5.1 | 0.37 /0.60 | 280000 |
| | Typ1 | | | | |
| Sol-D4 | Ir(L120) | 20.7 | 5.2 | 0.35/0.61 | 260000 |
| | Typ1 | | | | |
| Sol-D5 | Ir139 | 18.8 | 5.3 | 0.24/0.62 | 180000 |
| | Typ1 | | | | |
| Sol-D6 | Ir142 | 19.9 | 5.1 | 0.33 /0.63 | 260000 |
| | Typ1 | | | | |

| **Orange und Rote OLEDs** | | | | | |
|---|---|---|---|---|---|
| Sol-D100 | Ir(L7) | 16.2 | 6.1 | 0.64/0.36 | 45000 |
| | Typ1 | | | | |
| Sol-D101 | Ir1 | 17.6 | 6.0 | 0.64/0.36 | 135000 |
| | Ir(L7) | | | | |
| | Typ2 | | | | |
| Sol-D102 | Ir(L5) | 18.0 | 6.1 | 0.64/0.36 | 190000 |
| | Ir(L7) | | | | |
| | Typ2 | | | | |
| Sol-D103 | Ir(L107) | 17.4 | 6.1 | 0.66/0.34 | 270000 |
| | Ir(L115) | | | | |
| | Typ2 | | | | |

### B: Aus polymeren Funktionsmaterialien:

Herstellung der OLEDs wie unter A beschrieben. Zur Herstellung der Emissionsschicht werden die erfindungsgemäßen Polymere in Toluol gelöst. Der typische Feststoffgehalt solcher Lösungen liegt zwischen 10 und 15 g/L, wenn, wie hier, die für eine Device typische Schichtdicke von 40 nm mittels Spincoating erzielt werden soll. Die genannten OLED-Beispiele sind noch nicht optimiert, Tabelle 4 fasst die erhaltenen Daten zusammen.

**Tabelle 4: Ergebnisse mit aus Lösung prozessierten Materialien**

| **Bsp.** | **Polymer** | **EQE (%) 1000 cd/m²** | **Spannung (V) 1000 cd/m²** | **CIE x/y 1000 cd/m²** |
|---|---|---|---|---|
| **Grüne OLEDs** | | | | |
| D-P1 | P1 | 19.8 | 4.1 | 0.35/0.61 |
| D-P2 | P2 | 20.3 | 4.4 | 0.36/0.60 |
| D-P3 | P3 | 20.1 | 4.3 | 0.36/0.60 |

**Tabelle 5: Strukturformeln der verwendeten Materialien**

| | |
|---|---|
| 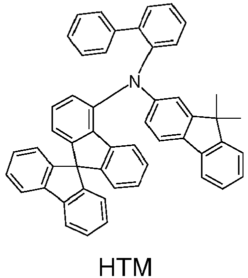 | 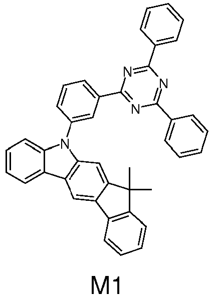 |
| 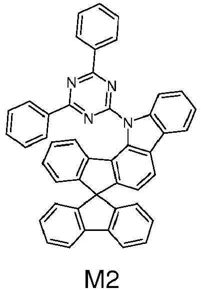 | 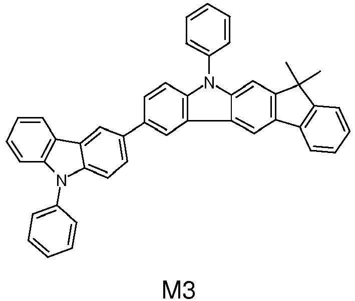 |
| 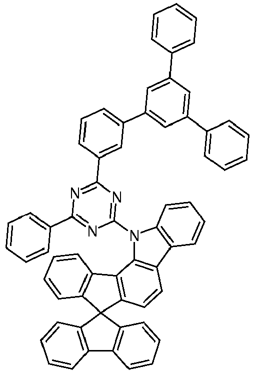 | 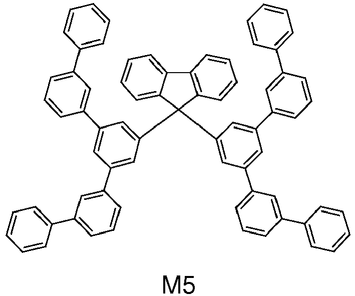 |
| 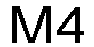 | |
| 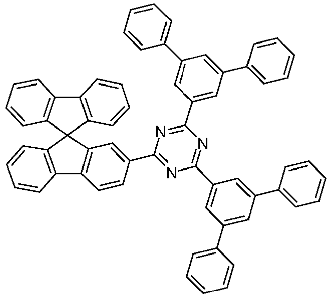 | 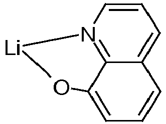 |
| 1233200-52-6 | 25387 -93-3 |
| ETM1 | ETM2 |
| 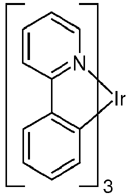 | 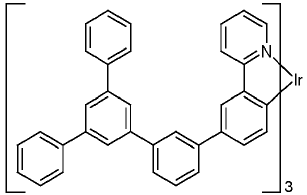 |
| 693794-98-8 | 1269508-30-6 |
| IrPPy | Ir1 |
| 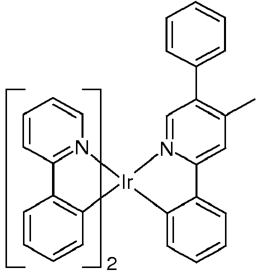 | 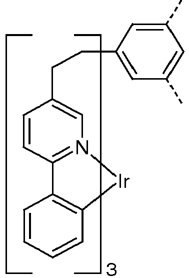 |
| 1215692-34-4 | 861806-70-4 |
| Ir2 | Ir3* |

| | |
|---|---|
| *: G. St-Pierre et al., Dalton Trans, 2011, 40, 11726. | |

## Patentansprüche

1. Monometallische Verbindung, enthaltend einen hexadentaten tripodalen Liganden, in dem drei bidentate Teilliganden, die gleich oder verschieden sein können, an ein Metall koordinieren und die drei bidentaten Teilliganden über eine Brücke der Formel (1) miteinander verknüpft sind: 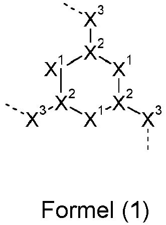 wobei die gestrichelte Bindung die Bindung der bidentaten Teilliganden an diese Struktur darstellt und für die verwendeten Symbole gilt:
X¹ ist bei jedem Auftreten gleich oder verschieden CR₂ oder O;
X² ist bei jedem Auftreten gleich oder verschieden CR, P=O, B oder Si, welches optional substituiert ist, mit der Maßgabe, dass für X² gleich P=O, B oder Si, welches optional substituiert ist, X¹ für O steht; dabei können die optional vorhandenen Substituenten an X¹ und X² jeweils und auch miteinander ein aliphatisches oder heteroaliphatisches Ringsystem bilden;
X³ ist bei jedem Auftreten gleich oder verschieden -CR=CR-, -CR=N-, -C(=O)-O-, -C(=O)-NR"-,-C(=O)-S-, -C(=S)-O-, -C(=S)-NR"-, -C(=S)-S-;
R ist bei jedem Auftreten gleich oder verschieden H, D, F, Cl, Br, I, N(R¹)₂, CN, NO₂, OR¹, SR¹, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei oder mehr Reste R, die an X¹ und/oder X² binden, auch miteinander ein aliphatisches oder heteroaliphatisches Ringsystem bilden; weiterhin können zwei Reste R für X³ = -CR=CR- auch miteinander ein aliphatisches, heteroaliphatisches, aromatisches oder heteroaromatisches Ringsystem bilden;
R" ist bei jedem Auftreten gleich oder verschieden H, D, eine geradkettige Alkylgruppe mit 1 bis 20 C-Atomen oder eine verzweigte oder cyclische Alkylgruppe mit 3 bis 20 C-Atomen oder eine Alkenylgruppe mit 2 bis 20 C-Atomen, wobei die Alkylgruppe bzw. Alkenylgruppe jeweils mit einem oder mehreren Resten R¹ substituiert sein kann und wobei eine oder mehrere nicht benachbarte CH₂-Gruppen durch Si(R¹)₂ ersetzt sein können, oder ein aromatisches oder heteroaromatisches Ringsystem mit 5 bis 40 aromatischen Ringatomen, das jeweils durch einen oder mehrere Reste R¹ substituiert sein kann;
R¹ ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können mehrere Substituenten R¹ auch miteinander ein aliphatisches, heteroaliphatisches, aromatisches oder heteroaromatisches Ringsystem bilden;
R² ist bei jedem Auftreten gleich oder verschieden H, D, F oder ein aliphatischer, aromatischer und/oder heteroaromatischer organischer Rest, insbesondere ein Kohlenwasserstoffrest, mit 1 bis 20 C-Atomen, in dem auch ein oder mehrere H-Atome durch F ersetzt sein können;
dabei können die drei bidentaten Liganden außer durch die Brücke der Formel (1) auch noch durch eine weitere Brücke zu einem Kryptat geschlossen sein.

2. Verbindung nach Anspruch 1, **dadurch gekennzeichnet, dass** die Struktur der Formel (1) ausgewählt ist aus den Strukturen der Formeln (2) bis (6), 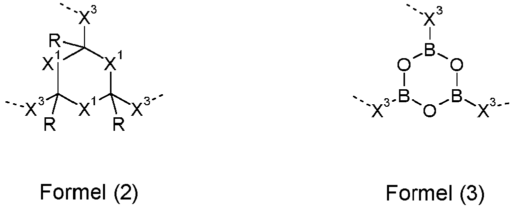 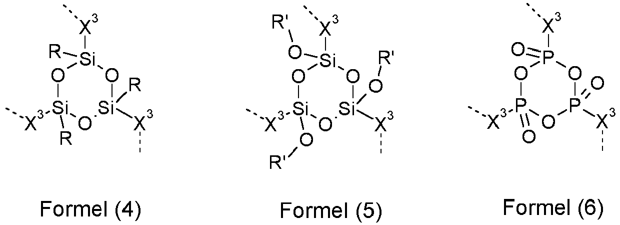 wobei die verwendeten Symbole die in Anspruch 1 genannten Bedeutungen aufweisen, wobei zusätzlich gilt:
R' ist bei jedem Auftreten gleich oder verschieden H, D, F, Cl, Br, I, N(R¹)₂, CN, NO₂, OR¹, SR¹, COOH, C(=O)N(R¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann.

3. Verbindung nach Anspruch 1 oder 2, **dadurch gekennzeichnet, dass** die Gruppe der Formel (1) ausgewählt ist aus den Strukturen der Formeln (2a) und (2b), 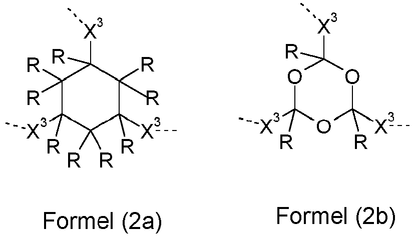 wobei die Symbole die in Ansprüche 1 und 2 aufgeführten Bedeutungen aufweisen.

4. Verbindung nach einem oder mehreren der Ansprüche 1 bis 3, **dadurch gekennzeichnet, dass** das Metall ausgewählt ist aus der Gruppe bestehend aus Aluminium, Gallium, Indium, Zinn, Chrom, Molybdän, Wolfram, Rhenium, Ruthenium, Osmium, Rhodium, Iridium, Eisen, Kobalt, Nickel, Palladium, Platin, Kupfer, Silber und Gold.

5. Verbindung nach einem oder mehreren der Ansprüche 1 bis 4, **dadurch gekennzeichnet, dass** die bidentaten Teilliganden jeweils monoanionisch sind und dass die drei bidentaten Teilliganden entweder gleich gewählt oder zwei der bidentaten Teilliganden gleich und der dritte bidentate Teilligand von den ersten beiden bidentaten Teilliganden verschieden gewählt ist und dass die koordinierenden Atome der bidentaten Teilliganden gleich oder verschieden bei jedem Auftreten ausgewählt sind aus C, N und/oder O.

6. Verbindung nach einem oder mehreren der Ansprüche 1 bis 5, **dadurch gekennzeichnet, dass** das Metall Ir(III) ist und zwei der bidentaten Teilliganden an das Iridium über jeweils ein Kohlenstoffatom und ein Stickstoffatom oder über zwei Kohlenstoffatome koordinieren und der dritte der bidentaten Teilliganden an das Iridium über ein Kohlenstoffatom und ein Stickstoffatom oder über zwei Kohlenstoffatome oder über zwei Stickstoffatome oder über ein Stickstoffatom und ein Sauerstoffatom oder über zwei Sauerstoffatome koordiniert.

7. Verbindung nach einem oder mehreren der Ansprüche 1 bis 6, **dadurch gekennzeichnet, dass** die bidentaten Teilliganden gleich oder verschieden bei jedem Auftreten ausgewählt sind aus den Strukturen der Formeln (L-1), (L-2), (L-3) oder (L-4), 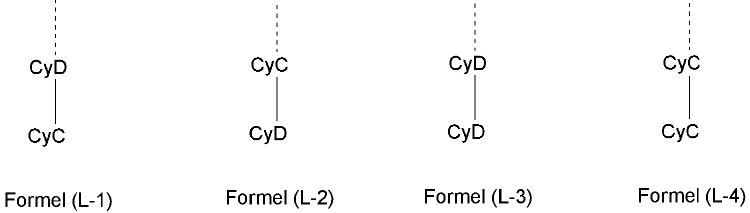 wobei die gestrichelte Bindung die Bindung des Teilliganden an die Brücke der Formel (1) darstellt und für die weiteren verwendeten Symbole gilt:
CyC ist gleich oder verschieden bei jedem Auftreten eine optional substituierte Aryl- oder Heteroarylgruppe mit 5 bis 14 aromatischen Ringatomen, welche über ein Kohlenstoffatom an das Metall koordiniert und welche über eine kovalente Bindung mit CyD verbunden ist;
CyD ist gleich oder verschieden bei jedem Auftreten eine optional substituierte Heteroarylgruppe mit 5 bis 14 aromatischen Ringatomen, welche über ein Stickstoffatom oder über ein Carben-Kohlenstoffatom an das Metall koordiniert und welche über eine kovalente Bindung mit CyC verbunden ist;
dabei können mehrere der optionalen Substituenten miteinander ein Ringsystem bilden.

8. Verbindung nach Anspruch 7, **dadurch gekennzeichnet, dass** CyC ausgewählt ist aus den Strukturen der Formeln (CyC-1) bis (CyC-20), 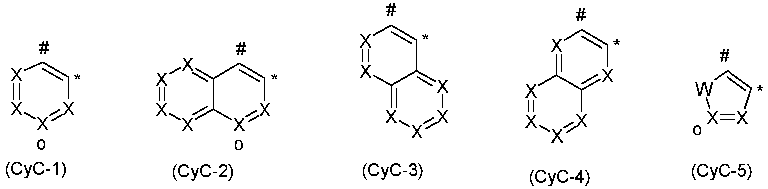 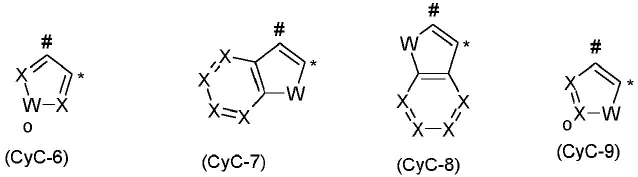 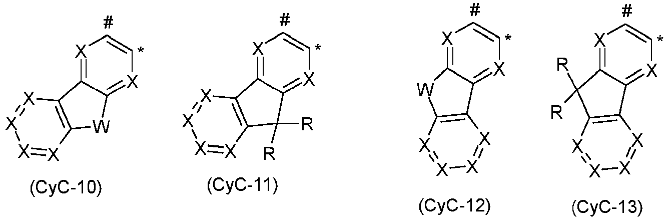 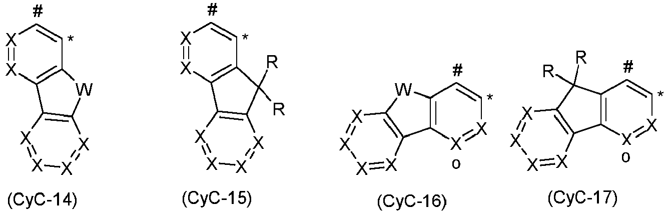 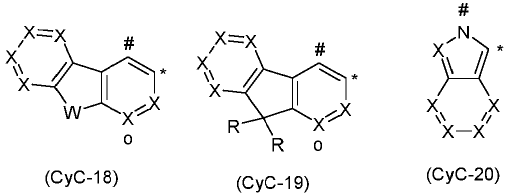 wobei die Gruppe jeweils an der durch # gekennzeichneten Position an CyD in (L-1) bzw. (L-2) bzw. an CyC in (L-4) bindet und an der durch * gekennzeichneten Position an das Metall koordiniert;
und dass CyD ausgewählt ist aus den Strukturen der Formeln (CyD-1) bis (CyD-14), 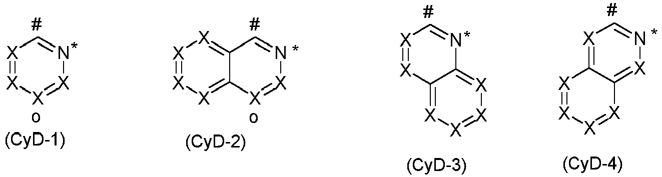 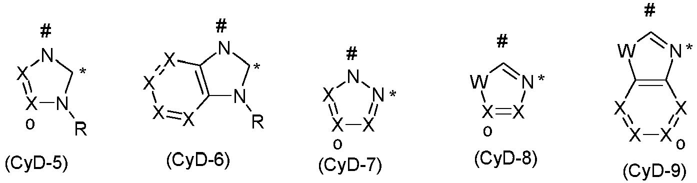 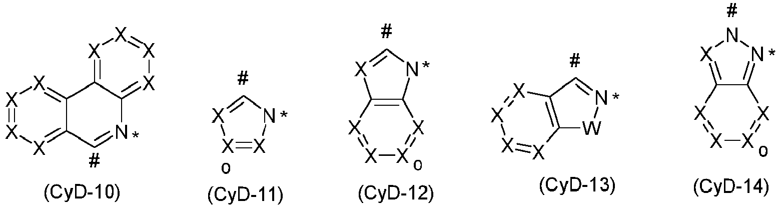 wobei die Gruppe jeweils an der durch # gekennzeichneten Position an CyC in (L-1) bzw. (L-2) bzw. an CyD in (L-3) bindet und an der durch * gekennzeichneten Position an das Metall koordiniert;
weiterhin gilt für die in CyC und CyD verwendeten Symbole:
R hat die in Anspruch 1 aufgeführten Bedeutungen, wobei zwei Reste R auch miteinander ein aliphatisches, heteroaliphatisches, aromatisches oder heteroaromatisches Ringsystem bilden können;
X ist bei jedem Auftreten gleich oder verschieden CR oder N mit der Maßgabe, dass maximal zwei Symbole X pro Cyclus für N stehen;
W ist bei jedem Auftreten gleich oder verschieden NR, O oder S;
dabei erfolgt die Bindung dieser Gruppen an die Brücke der Formel (1) über eine in den Formeln mit "o" markierte Position und das entsprechende Symbol X steht für C.

9. Verbindung nach einem oder mehreren der Ansprüche 1 bis 8, **dadurch gekennzeichnet, dass** die bidentaten Teilliganden ausgewählt sind aus den folgenden Strukturen, 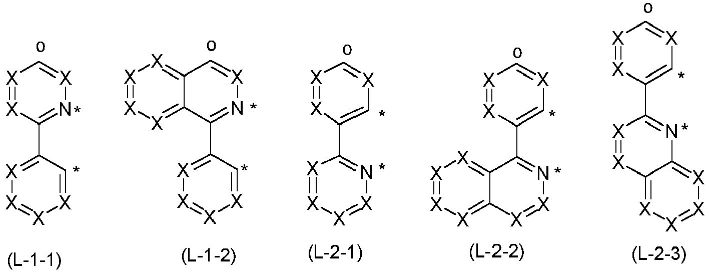 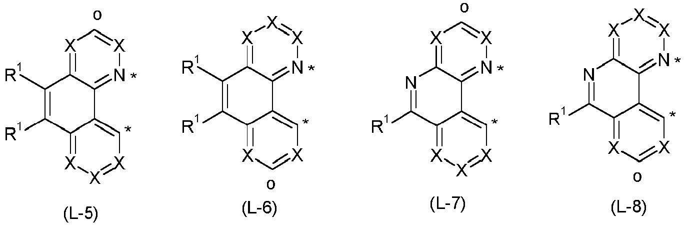 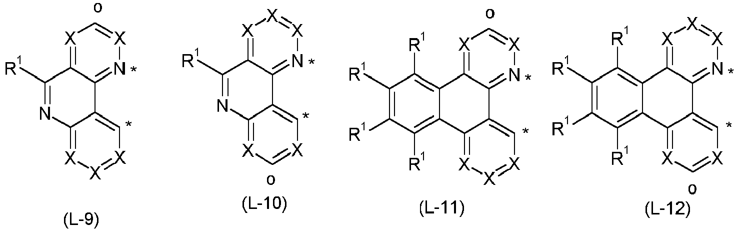 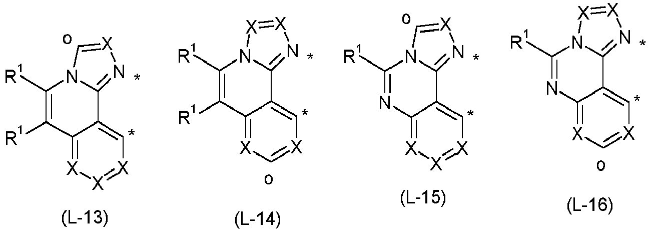 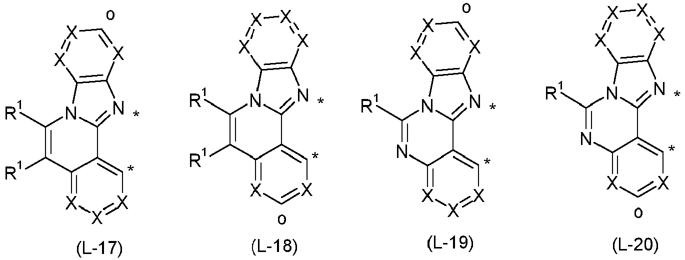 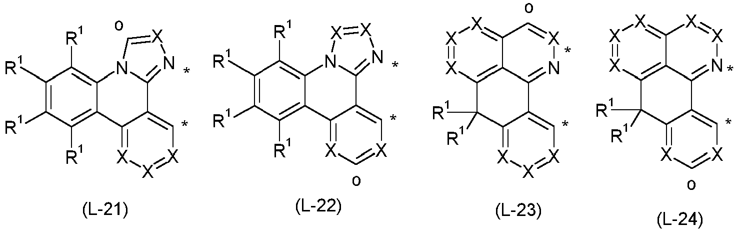 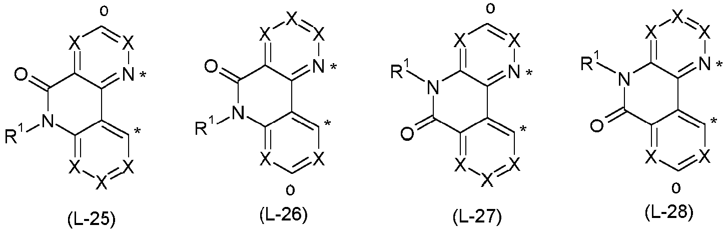 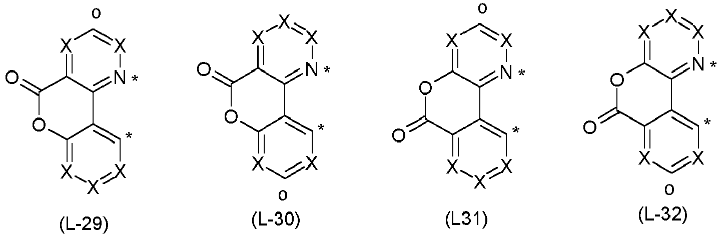 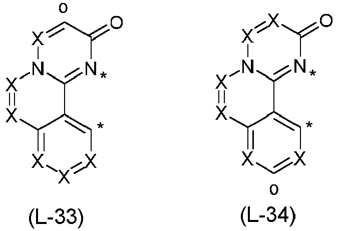 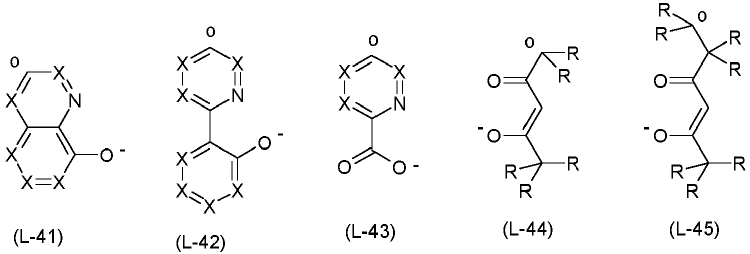 wobei die verwendeten Symbole die in Anspruch 1 genannten Bedeutungen aufweisen, * die Position der Koordination an das Metall kennzeichnet und "o" die Position der Bindung an die Brücke der Formel (1) darstellt.

10. Verbindung nach einem oder mehreren der Ansprüche 1 bis 9, **dadurch gekennzeichnet, dass** die Verbindung zwei Substituenten R enthält, die an benachbarte Kohlenstoffatome gebunden sind und die miteinander einen Ring gemäß einer der Formeln (43) bis (49) bilden, 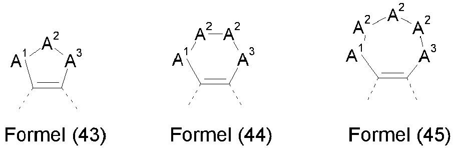 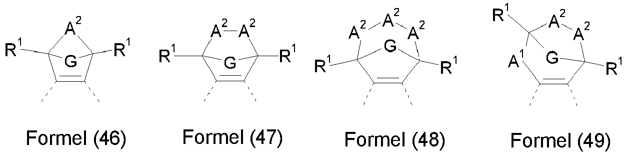 wobei R¹ und R² die in Anspruch 1 genannten Bedeutungen aufweisen, die gestrichelten Bindungen die Verknüpfung der beiden Kohlenstoffatome im Liganden andeuten und weiterhin gilt:
A¹, A³ ist gleich oder verschieden bei jedem Auftreten C(R³)₂, O, S, NR³ oder C(=O);
A² ist ist gleich oder verschieden bei jedem Auftreten C(R¹)₂, O, S, NR³ oder C(=O);
G ist eine Alkylengruppe mit 1, 2 oder 3 C-Atomen, welche mit einem oder mehreren Resten R² substituiert sein kann, -CR²=CR²- oder eine ortho-verknüpfte Arylen- oder Heteroarylengruppe mit 5 bis 14 aromatischen Ringatomen, welche durch einen oder mehrere Reste R² substituiert sein kann;
R³ ist gleich oder verschieden bei jedem Auftreten H, D, F, eine geradkettige Alkyl- oder Alkoxygruppe mit 1 bis 10 C-Atomen, eine verzweigte oder cyclische Alkyl- oder Alkoxygruppe mit 3 bis 10 C-Atomen, wobei die Alkyl- oder Alkoxygruppe jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können, oder ein aromatisches oder heteroaromatisches Ringsystem mit 5 bis 24 aromatischen Ringatomen, das jeweils durch einen oder mehrere Reste R² substituiert sein kann, oder eine Aryloxy- oder Heteroaryloxygruppe mit 5 bis 24 aromatischen Ringatomen, die durch einen oder mehrere Reste R² substituiert sein kann; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden;
mit der Maßgabe, dass in diesen Gruppen nicht zwei Heteroatome direkt aneinander gebunden sind und nicht zwei Gruppen C=O direkt aneinander gebunden sind.

11. Verfahren zur Herstellung einer Verbindung nach einem oder mehreren der Ansprüche 1 bis 10 durch Umsetzung des freien Liganden mit Metallalkoholaten der Formel (50), mit Metallketoketonaten der Formel (51), mit Metallhalogeniden der Formel (52) oder mit Metallcarboxylaten der Formel (53) oder mit Metallverbindungen, die sowohl Alkoholat- und/oder Halogenid- und/oder Hydroxy- wie auch Ketoketonatreste tragen, 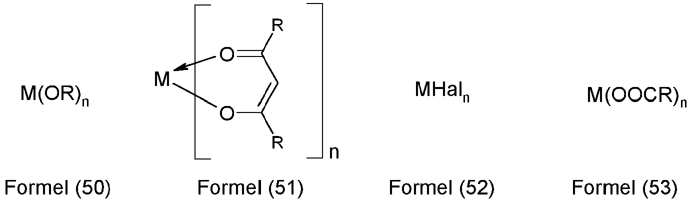 wobei M für das Metall des Metallkomplexes, der synthetisiert wird, steht, n für die Wertigkeit des Metalls M steht, R die in Anspruch 1 angegebenen Bedeutungen hat, Hal = F, Cl, Br oder I ist und die Metalledukte auch als die entsprechenden Hydrate vorliegen können.

12. Oligomer, Polymer oder Dendrimer enthaltend eine oder mehrere Verbindungen nach einem oder mehreren der Ansprüche 1 bis 10, wobei statt eines oder mehrerer Wasserstoffatome und/oder Substituenten ein oder mehrere Bindungen der Verbindung zum Polymer, Oligomer oder Dendrimer vorhanden sind.

13. Formulierung, enthaltend mindestens eine Verbindung nach einem oder mehreren der Ansprüche 1 bis 10, bzw. mindestens ein Oligomer, Polymer oder Dendrimer nach Anspruch 12, und mindestens ein Lösemittel.

14. Verwendung einer Verbindung nach einem oder mehreren der Ansprüche 1 bis 10 oder eines Oligomers, Polymers oder Dendrimers nach Anspruch 12 in einer elektronischen Vorrichtung oder als Sauerstoff-Sensibilisator.

15. Elektronische Vorrichtung enthaltend mindestens eine Verbindung nach einem oder mehreren der Ansprüche 1 bis 10 oder mindestens ein Oligomer, Polymer oder Dendrimer nach Anspruch 12.

16. Elektronische Vorrichtung nach Anspruch 15, **dadurch gekennzeichnet, dass** es sich um eine organische Elektrolumineszenzvorrichtung handelt und die Verbindung nach einem oder mehreren der Ansprüche 1 bis 10 als emittierende Verbindung in einer oder mehreren emittierenden Schichten oder als Lochblockiermaterial in einer Lochblockierschicht oder als Elektronentransportmaterial in einer Elektronentransportschicht eingesetzt wird.

## Claims

1. Monometallic compound containing a hexadentate tripodal ligand in which three bidentate part-ligands, which may be identical or different, are coordinated to a metal and the three bidentate part-ligands are linked to one another via a bridge of the formula (1): 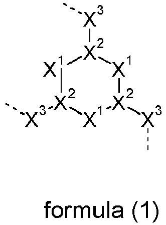 where the dashed bond represents the bond from the bidentate part-ligands to this structure, and the following applies to the symbols used:
X¹ is on each occurrence, identically or differently, CR₂ or O;
X² is on each occurrence, identically or differently, CR, P=O, B or Si, which is optionally substituted, with the proviso that, for X² equals P=O, B or Si, which is optionally substituted, X¹ stands for O; the substituents optionally present on X¹ and X² may in each case, and also with one another, form an aliphatic or heteroaliphatic ring system;
X³ is on each occurrence, identically or differently, -CR=CR-, -CR=N-, -C(=O)-O-, -C(=O)-NR"-,-C(=O)-S-, -C(=S)-O-, -C(=S)-NR"-, -C(=S)-S-;
R is on each occurrence, identically or differently, H, D, F, Cl, Br, I, N(R¹)₂, CN, NO₂, OR¹, SR¹, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R¹, where one or more non-adjacent CH₂ groups may be replaced by R¹C=CR¹, C≡C, Si(R¹)₂, C=O, NR¹, O, S or CONR¹, or an aromatic or heteroaromatic ring system having 5 to 40 aromatic ring atoms, which may in each case be substituted by one or more radicals R¹; two or more radicals R which are bonded to X¹ and/or X² may also form an aliphatic or heteroaliphatic ring system with one another; furthermore, two radicals R for X³ = -CR=CR- may also form an aliphatic, heteroaliphatic, aromatic or heteroaromatic ring system with one another;
R" is on each occurrence, identically or differently, H, D, a straight-chain alkyl group having 1 to 20 C atoms or a branched or cyclic alkyl group having 3 to 20 C atoms or an alkenyl group having 2 to 20 C atoms, where the alkyl group or alkenyl group may in each case be substituted by one or more radicals R¹ and where one or more non-adjacent CH₂ groups may be replaced by Si(R¹)₂, or an aromatic or heteroaromatic ring system having 5 to 40 aromatic ring atoms, which may in each case be substituted by one or more radicals R¹;
R¹ is on each occurrence, identically or differently, H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R², where one or more non-adjacent CH₂ groups may be replaced by R²C=CR², C≡C, Si(R²)₂, C=O, NR², O, S or CONR², or an aromatic or heteroaromatic ring system having 5 to 40 aromatic ring atoms, which may in each case be substituted by one or more radicals R²; a plurality of substituents R¹ may also form an aliphatic, heteroaliphatic, aromatic or heteroaromatic ring system with one another;
R² is on each occurrence, identically or differently, H, D, F or an aliphatic, aromatic and/or heteroaromatic organic radical, in particular a hydrocarbon radical, having 1 to 20 C atoms, in which, in addition, one or more H atoms may be replaced by F;
the three bidentate ligands may also be cyclised by a further bridge, in addition to the bridge of the formula (1), to form a cryptate.

2. Compound according to Claim 1, **characterised in that** the structure of the formula (1) is selected from the structures of the formulae (2) to (6), 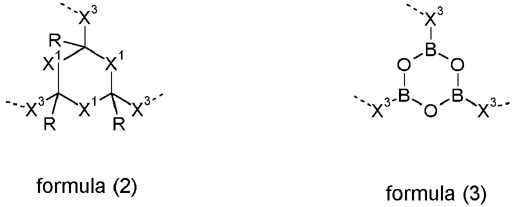 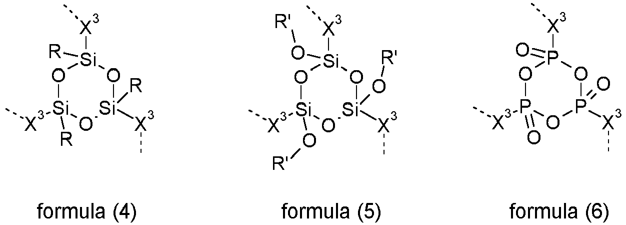 where the symbols used have the meanings given in Claim 1, where the following additionally applies:
R' is on each occurrence, identically or differently, H, D, F, Cl, Br, I, N(R¹)₂, CN, NO₂, OR¹, SR¹, COOH, C(=O)N(R¹)₂, C(=O)R¹, P(=O)(R¹)₂, S(=O)R¹, S(=O)₂R¹, OSO₂R¹, a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R¹, where one or more non-adjacent CH₂ groups may be replaced by R¹C=CR¹, C≡C, C=O, NR¹, O, S or CONR¹, or an aromatic or heteroaromatic ring system having 5 to 40 aromatic ring atoms, which may in each case be substituted by one or more radicals R¹.

3. Compound according to Claim 1 or 2, **characterised in that** the group of the formula (1) is selected from the structures of the formulae (2a) and (2b), 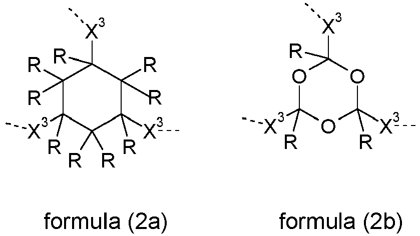 where the symbols have the meanings given in Claims 1 and 2.

4. Compound according to one or more of Claims 1 to 3, **characterised in that** the metal is selected from the group consisting of aluminium, gallium, indium, tin, chromium, molybdenum, tungsten, rhenium, ruthenium, osmium, rhodium, iridium, iron, cobalt, nickel, palladium, platinum, copper, silver and gold.

5. Compound according to one or more of Claims 1 to 4, **characterised in that** the bidentate part-ligands are in each case monoanionic and **in that** the three bidentate part-ligands are either selected identically or two of the bidentate part-ligands are selected identically and the third bidentate part-ligand is selected differently from the first two bidentate part-ligands and **in that** the coordinating atoms of the bidentate part-ligands are selected, identically or differently on each occurrence, from C, N and/or O.

6. Compound according to one or more of Claims 1 to 5, **characterised in that** the metal is Ir(III) and two of the bidentate part-ligands are coordinated to the iridium via in each case one carbon atom and one nitrogen atom or via two carbon atoms and the third of the bidentate part-ligands is coordinated to the iridium via one carbon atom and one nitrogen atom or via two carbon atoms or via two nitrogen atoms or via one nitrogen atom and one oxygen atom or via two oxygen atoms.

7. Compound according to one or more of Claims 1 to 6, **characterised in that** the bidentate part-ligands are selected, identically or differently on each occurrence, from the structures of the formulae (L-1), (L-2), (L-3) or (L-4), 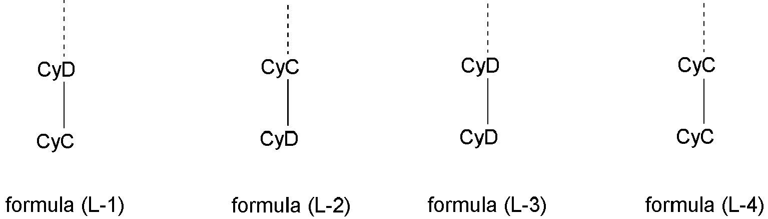 where the dashed bond represents the bond from the part-ligand to the bridge of the formula (1) and the following applies to the other symbols used:
CyC is, identically or differently on each occurrence, an optionally substituted aryl or heteroaryl group having 5 to 14 aromatic ring atoms, which is coordinated to the metal via a carbon atom and which is connected to CyD via a covalent bond;
CyD is, identically or differently on each occurrence, an optionally substituted heteroaryl group having 5 to 14 aromatic ring atoms, which is coordinated to the metal via a nitrogen atom or via a carbene carbon atom and which is connected to CyC via a covalent bond;
a plurality of the optional substituents may form a ring system with one another.

8. Compound according to Claim 7, **characterised in that** CyC is selected from the structures of the formulae (CyC-1) to (CyC-20), 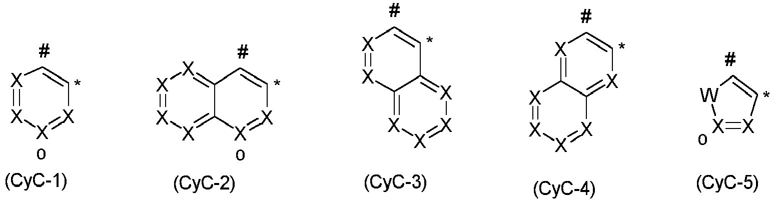 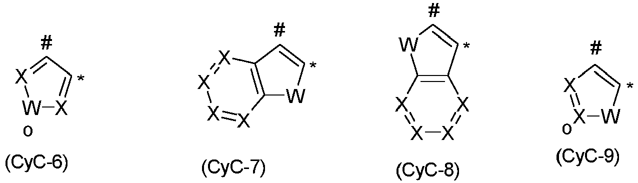 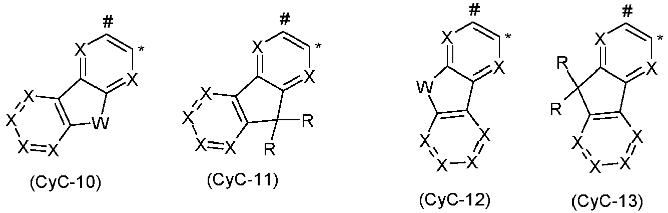 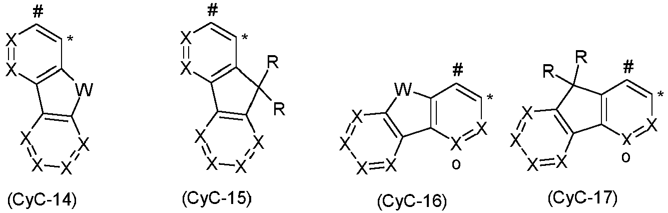 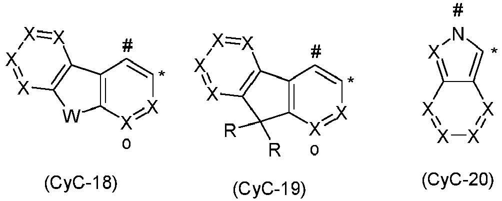 where the group is in each case bonded to CyD in (L-1) or (L-2) or to CyC in (L-4) at the position denoted by # and is coordinated to the metal at the position denoted by *;
and **in that** CyD is selected from the structures of the formulae (CyD-1) to (CyD-14), 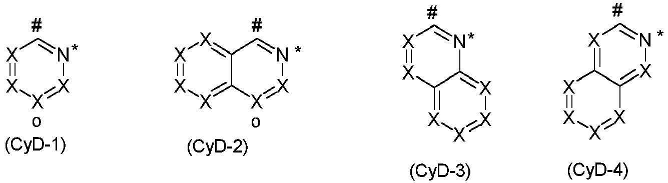 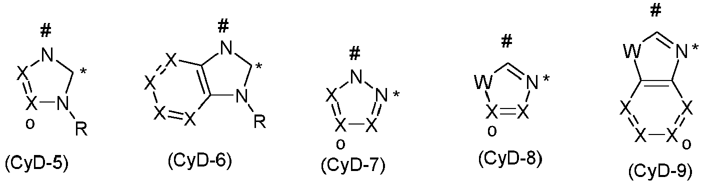 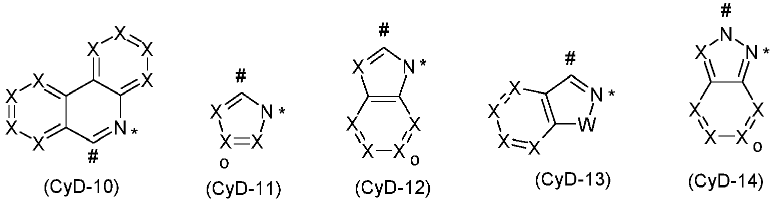 where the group is in each case bonded to CyC in (L-1) or (L-2) or to CyD in (L-3) at the position denoted by # and is coordinated to the metal at the position denoted by *;
furthermore, the following applies to the symbols used in CyC and CyD:
R has the meanings given in Claim 1, where two radicals R may also form an aliphatic, heteroaliphatic, aromatic or heteroaromatic ring system with one another:
X is on each occurence, identically or differently, CR or N, with the proviso that a maximum of two symbols X per ring stand for N;
W is on each occurence, identically or differently, NR, O or S;
the bonding of these groups to the bridge of the formula (1) takes place via a position marked by "o" in the formulae and the corresponding symbol X stands for C.

9. Compound according to one or more of Claims 1 to 8, **characterised in that** the bidentate part-ligands are selected from the following structures, 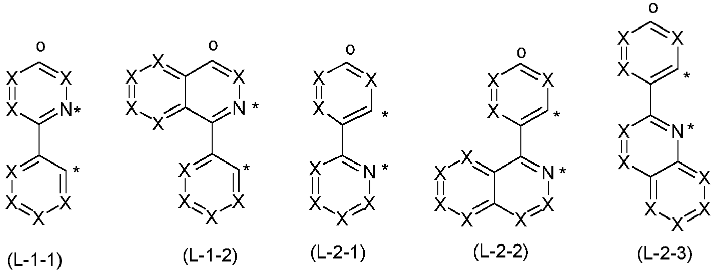 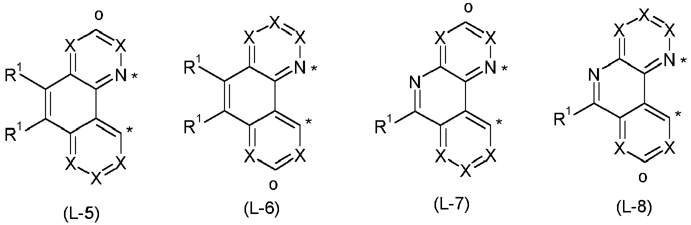 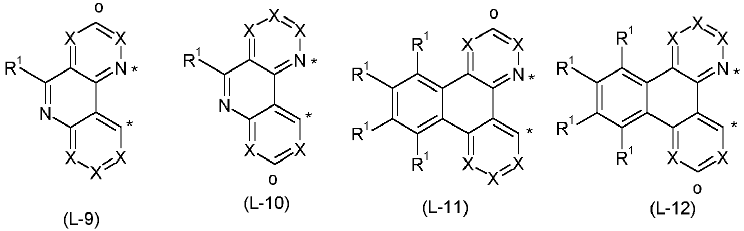 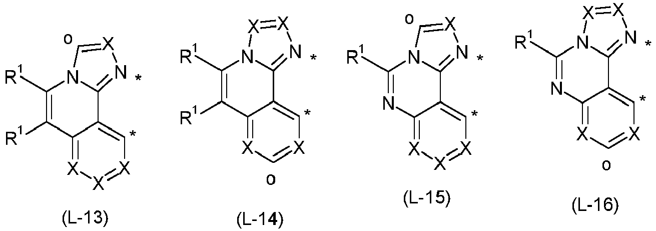 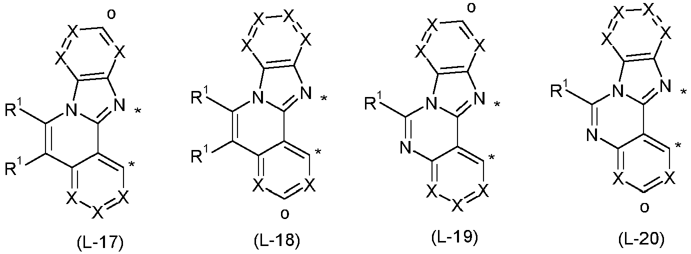 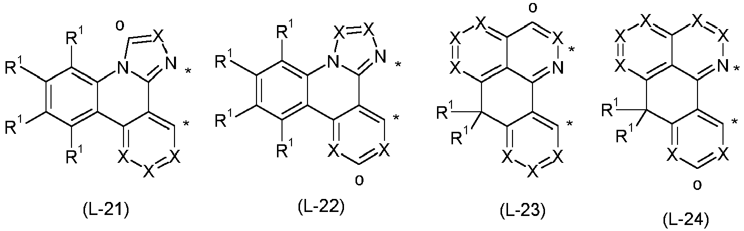 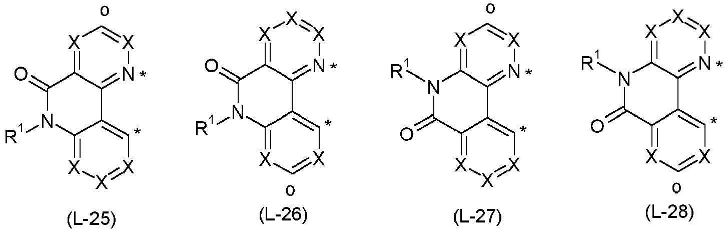 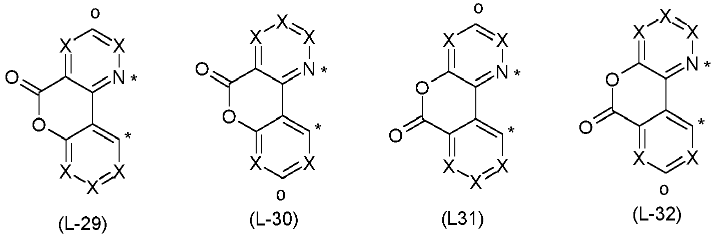 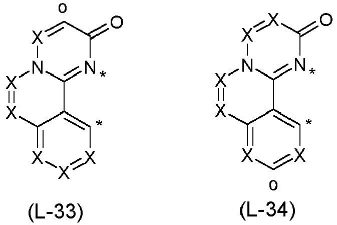 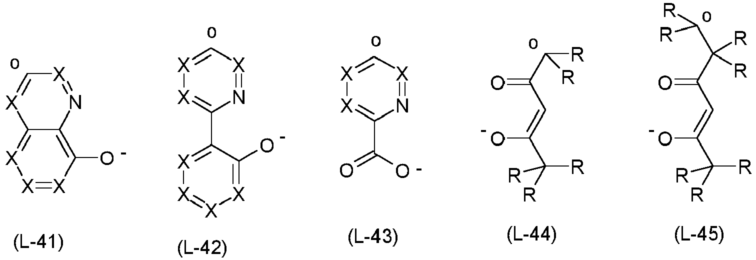 where the symbols used have the meanings given in Claim 1, * denotes the position of the coordination to the metal and "o" represents the position of the bond to the bridge of the formula (1).

10. Compound according to one or more of Claims 1 to 9, **characterised in that** the compound contains two substituents R, which are bonded to adjacent carbon atoms and which form a ring of one of the formulae (43) to (49) with one another, 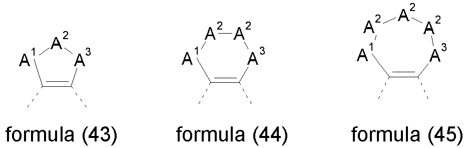 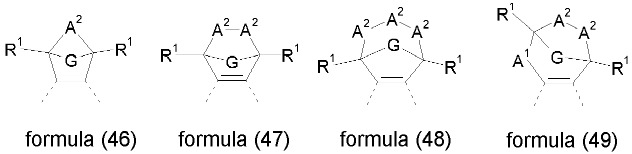 where R¹ and R² have the meanings given in Claim 1, the dashed bonds indicate the linking of the two carbon atoms in the ligand, and furthermore:
A¹, A³ are, identically or differently on each occurrence, C(R³)₂, O, S, NR³ or C(=O);
A² is, identically or differently on each occurrence, C(R¹)₂, O, S, NR³ or C(=O);
G is an alkylene group having 1, 2 or 3 C atoms, which may be substituted by one or more radicals R², or is -CR²=CR²- or an ortho-linked arylene or heteroarylene group having 5 to 14 aromatic ring atoms, which may be substituted by one or more radicals R²;
R³ is, identically or differently on each occurrence, H, D, F, a straight-chain alkyl or alkoxy group having 1 to 10 C atoms, a branched or cyclic alkyl or alkoxy group having 3 to 10 C atoms, where the alkyl or alkoxy group may in each case be substituted by one or more radicals R², where one or more non-adjacent CH₂ groups may be replaced by R²C=CR², C≡C, Si(R²)₂, C=O, NR², O, S or CONR², or an aromatic or heteroaromatic ring system having 5 to 24 aromatic ring atoms, which may in each case be substituted by one or more radicals R², or an aryloxy or heteroaryloxy group having 5 to 24 aromatic ring atoms, which may be substituted by one or more radicals R²; two radicals R³ which are bonded to the same carbon atom may form an aliphatic or aromatic ring system with one another and thus form a spiro system; furthermore, R³ may form an aliphatic ring system with an adjacent radical R or R¹;
with the proviso that in these groups, no two heteroatoms are bonded directly to one another and no two groups C=O are bonded directly to one another.

11. Process for the preparation of a compound according to the one or more of Claims 1 to 10 by reaction of the free ligand with metal alcoholates of the formula (50), with metal ketoketonates of the formula (51), with metal halides of the formula (52) or with metal carboxylates of the formula (53) or with metal compounds which carry both alcoholate and/or halide and/or hydroxyl and also ketoketonate radicals, 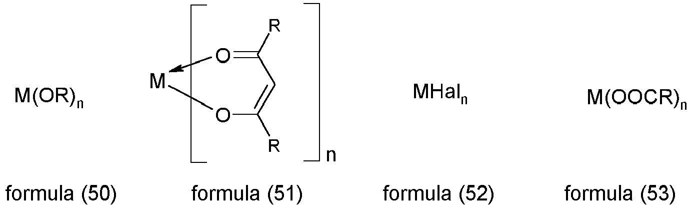 where M stands for the metal of the metal complex that is being synthesised, n stands for the valency of the metal M, R has the meanings indicated in Claim 1, Hal = F, Cl, Br or I, and the metal starting materials may also be in the form of the corresponding hydrates.

12. Oligomer, polymer or dendrimer containing one or more compounds according to one or more of Claims 1 to 10, where one or more bonds are present from the compound to the polymer, oligomer or dendrimer instead of one or more hydrogen atoms and/or substituents.

13. Formulation comprising at least one compound according to one or more of Claims 1 to 10, or at least one oligomer, polymer or dendrimer according to Claim 12, and at least one solvent.

14. Use of a compound according to one or more of Claims 1 to 10 or an oligomer, polymer or dendrimer according to Claim 12 in an electronic device or as oxygen sensitiser.

15. Electronic device containing at least one compound according to one or more of Claims 1 to 10 or at least one oligomer, polymer or dendrimer according to Claim 12.

16. Electronic device according to Claim 15, **characterised in that** it is an organic electroluminescent device and the compound according to one or more of Claims 1 to 10 is employed as emitting compound in one or more emitting layers or as hole-blocking material in a hole blocking layer or as electron-transport material in an electron-transport layer.

## Revendications

1. Composé monométallique contenant un ligand tripodal hexadenté dans lequel trois ligands partiels bidentés, qui peuvent être identiques ou différents, sont coordinés sur un métal et les trois ligands partiels bidentés sont liés les uns aux autres via un pont de la formule (1) : 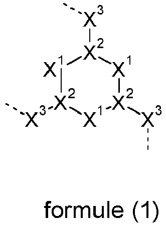 dans laquelle le lien en pointillés représente le lien depuis les trois ligands partiels bidentés sur cette structure, et ce qui suit s'applique aux symboles qui sont utilisés :
X¹ est pour chaque occurrence, de manière identique ou différente, CR₂ ou O ;
X² est pour chaque occurrence, de manière identique ou différente, CR, P=O, B ou Si, lequel est en option substitué, étant entendu que, pour X² égal à P=O, B ou Si, lequel est en option substitué, X¹ représente O ; les substituants qui sont en option présents sur X¹ et sur X² peuvent dans chaque cas, et également l'un avec l'autre ou les uns avec les autres, former un système de cycle aliphatique ou hétéroaliphatique ;
X³ est pour chaque occurrence, de manière identique ou différente, -CR=CR-, -CR=N-, -C(=O)-O-, -C(=O)-NR"-,-C(=O)-S-, -C(=S)-O-, -C(=S)-NR"-, -C(=S)-S- ;
R est pour chaque occurrence, de manière identique ou différente, H, D, F, Cl, Br, I, N(R¹)₂, CN, NO₂, OR¹, SR¹, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle en chaîne droite qui comporte de 1 à 20 atome(s) de C ou un groupe alkényle ou alkynyle qui comporte de 2 à 20 atomes de C ou un groupe alkyle ramifié ou cyclique qui comporte de 3 à 20 atomes de C, où le groupe alkyle, alkényle ou alkynyle peut dans chaque cas être substitué par un radical ou par plusieurs radicaux R¹, où un ou plusieurs groupe(s) CH₂ non adjacents peut/peuvent être remplacé(s) par R¹C=CR¹, C≡C, Si(R¹)₂, C=O, NR¹, O, S ou CONR¹, ou un système de cycle aromatique ou hétéroaromatique qui comporte de 5 à 40 atomes de cycle aromatique, lequel peut dans chaque cas être substitué par un radical ou par plusieurs radicaux R¹ ; deux radicaux R ou plus qui sont liés à X¹ et/ou à X² peuvent également former un système de cycle aliphatique ou hétéroaliphatique l'un avec l'autre ou les uns avec les autres ; qui plus est, deux radicaux R pour X³ = -CR=CR- peuvent également former un système de cycle aliphatique, hétéroaliphatique, aromatique ou hétéroaromatique l'un avec l'autre ;
R" est pour chaque occurrence, de manière identique ou différente, H, D, un groupe alkyle en chaîne droite qui comporte de 1 à 20 atome(s) de C ou un groupe alkyle ramifié ou cyclique qui comporte de 3 à 20 atomes de C ou un groupe alkényle qui comporte de 2 à 20 atomes de C, où le groupe alkyle ou le groupe alkényle peut dans chaque cas être substitué par un radical ou par plusieurs radicaux R¹ et où un ou plusieurs groupe(s) CH₂ non adjacents peut/peuvent être remplacé(s) par Si(R¹)₂, ou un système de cycle aromatique ou hétéroaromatique qui comporte de 5 à 40 atomes de cycle aromatique, lequel peut dans chaque cas être substitué par un radical ou par plusieurs radicaux R¹ ;
R¹ est pour chaque occurrence, de manière identique ou différente, H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², un groupe alkyle en chaîne droite qui comporte de 1 à 20 atome(s) de C ou un groupe alkényle ou alkynyle qui comporte de 2 à 20 atomes de C ou un groupe alkyle ramifié ou cyclique qui comporte de 3 à 20 atomes de C, où le groupe alkyle, alkényle ou alkynyle peut dans chaque cas être substitué par un radical ou par plusieurs radicaux R², où un ou plusieurs groupe(s) CH₂ non adjacents peut/peuvent être remplacé(s) par R²C=CR², C≡C, Si(R²)₂, C=O, NR², O, S ou CONR², ou un système de cycle aromatique ou hétéroaromatique qui comporte de 5 à 40 atomes de cycle aromatique, lequel peut dans chaque cas être substitué par un radical ou par plusieurs radicaux R² ; les substituants d'une pluralité de substituants R¹ peuvent également former un système de cycle aliphatique, hétéroaliphatique, aromatique ou hétéroaromatique les uns avec les autres ;
R² est pour chaque occurrence, de manière identique ou différente, H, D, F ou un radical organique aliphatique, aromatique et/ou hétéroaromatique, en particulier un radical hydrocarbone, qui comporte de 1 à 20 atome(s) de C, où, en outre, un ou plusieurs atome(s) de H peut/peuvent être remplacé(s) par F ;
les trois ligands bidentés peuvent également être cyclisés par un autre pont, en plus du pont de la formule (1), pour former un cryptate.

2. Composé selon la revendication 1, **caractérisé en ce que** la structure de la formule (1) est sélectionnée parmi les structures des formules (2) à (6), 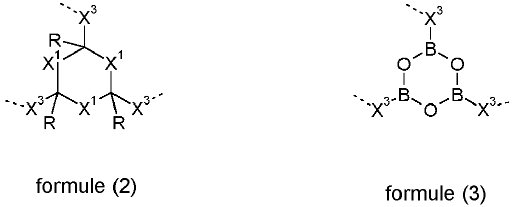 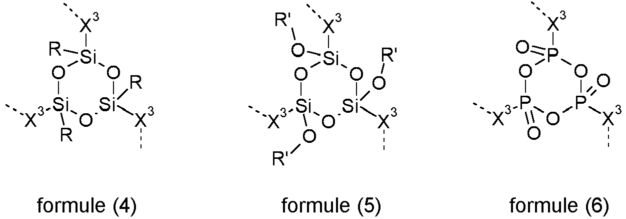 dans lesquelles les symboles qui sont utilisés présentent les significations qui ont été données selon la revendication 1, où ce qui suit s'applique de façon additionnelle :
R' est pour chaque occurrence, de manière identique ou différente, H, D, F, Cl, Br, I, N(R¹)₂, CN, NO₂, OR¹, SR¹, COOH, C(=O)N(R¹)₂, C(=O)R¹, P(=O)(R¹)₂, S(=O)R¹, S(=O)₂R¹, OSO₂R¹, un groupe alkyle en chaîne droite qui comporte de 1 à 20 atome(s) de C ou un groupe alkényle ou alkynyle qui comporte de 2 à 20 atomes de C ou un groupe alkyle ramifié ou cyclique qui comporte de 3 à 20 atomes de C, où le groupe alkyle, alkényle ou alkynyle peut dans chaque cas être substitué par un radical ou par plusieurs radicaux R¹, où un ou plusieurs groupe(s) CH₂ non adjacents peut/peuvent être remplacé(s) par R¹C=CR¹, C≡C, C=O, NR¹, O, S ou CONR¹, ou un système de cycle aromatique ou hétéroaromatique qui comporte de 5 à 40 atomes de cycle aromatique, lequel peut dans chaque cas être substitué par un radical ou par plusieurs radicaux R¹.

3. Composé selon la revendication 1 ou 2, **caractérisé en ce que** le groupe de la formule (1) est sélectionné parmi les structures des formules (2a) et (2b), 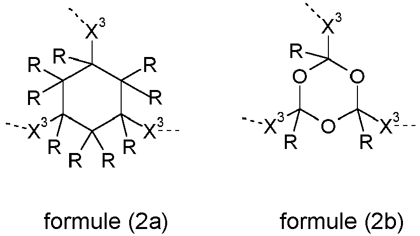 dans lesquelles les symboles présentent les significations qui ont été données selon les revendications 1 et 2.

4. Composé selon une ou plusieurs des revendications 1 à 3, **caractérisé en ce que** le métal est sélectionné parmi le groupe qui est constitué par l'aluminium, le gallium, l'indium, l'étain, le chrome, le molybdène, le tungstène, le rhénium, le ruthénium, l'osmium, le rhodium, l'iridium, le fer, le cobalt, le nickel, le palladium, le platine, le cuivre, l'argent et l'or.

5. Composé selon une ou plusieurs des revendications 1 à 4, **caractérisé en ce que** les trois ligands partiels bidentés sont dans chaque cas monoanioniques et **en ce que** soit les trois ligands partiels bidentés sont sélectionnés de façon identique, soit deux des ligands partiels bidentés sont sélectionnés de façon identique et le troisième ligand partiel bidenté est sélectionné de façon différente des deux premiers ligands partiel bidentés et **en ce que** les atomes de coordination des trois ligands partiels bidentés sont sélectionnés, de manière identique ou différente pour chaque occurrence, parmi C, N et/ou O.

6. Composé selon une ou plusieurs des revendications 1 à 5, **caractérisé en ce que** le métal est Ir(III) et deux des ligands partiels bidentés sont coordinés sur l'iridium via dans chaque cas un atome de carbone et un atome d'azote ou via deux atomes de carbone et le troisième ligand partiel bidenté est coordiné sur l'iridium via un atome de carbone et un atome d'azote ou via deux atomes de carbone ou via deux atomes d'azote ou via un atome d'azote et un atome d'oxygène ou via deux atomes d'oxygène.

7. Composé selon une ou plusieurs des revendications 1 à 6, **caractérisé en ce que** les ligands partiels bidentés sont sélectionnés, de manière identique ou différente pour chaque occurrence, parmi les structures des formules (L-1), (L-2), (L-3) ou (L-4), 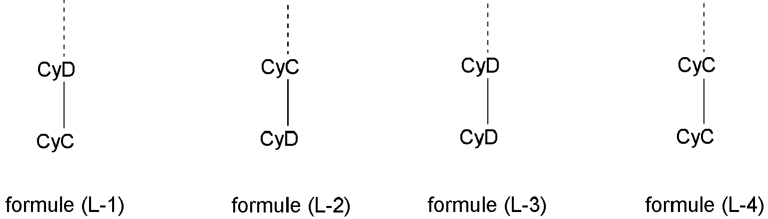 dans lesquelles le lien en pointillés représente le lien depuis le ligand partiel sur le pont de la formule (1) et ce qui suit s'applique aux autres symboles qui sont utilisés :
CyC est, de manière identique ou différente pour chaque occurrence, un groupe aryle ou hétéroaryle en option substitué qui comporte de 5 à 14 atomes de cycle aromatique, lequel est coordiné sur le métal via un atome de carbone et lequel est connecté à CyD via une liaison covalente ;
CyD est, de manière identique ou différente pour chaque occurrence, un groupe hétéroaryle en option substitué qui comporte de 5 à 14 atomes de cycle aromatique, lequel est coordiné sur le métal via un atome d'azote ou via un atome de carbone carbène et lequel est connecté à CyC via une liaison covalente ;
les substituants d'une pluralité des substituants optionnels peuvent former un système de cycle les uns avec les autres.

8. Composé selon la revendication 7, **caractérisé en ce que** CyC est sélectionné parmi les structures des formules (CyC-1) à (CyC-20), 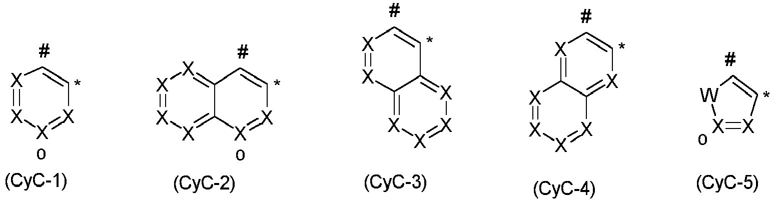 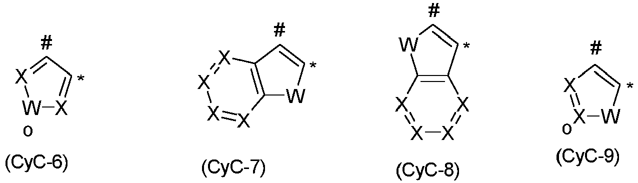 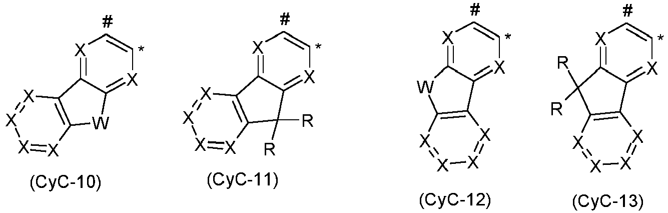 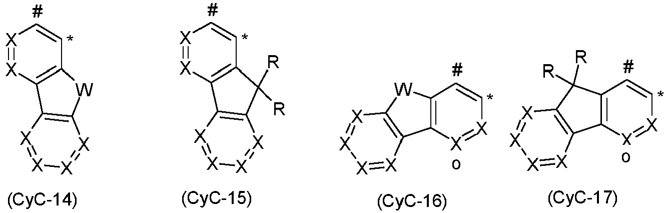 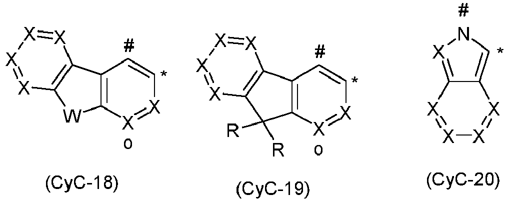 dans lesquelles le groupe est dans chaque cas lié à CyD dans la formule (L-1) ou dans la formule (L-2) ou à CyC dans la formule (L-4) à la position qui est indiquée par # et est coordiné sur le métal à la position qui est indiquée par * ;
et **en ce que** CyD est sélectionné parmi les structures des formules (CyD-1) à (CyD-14), 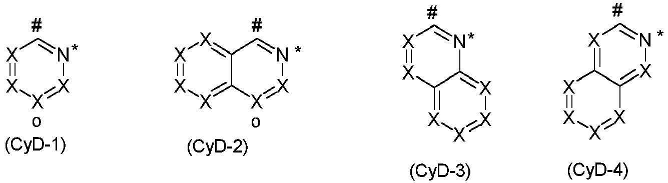 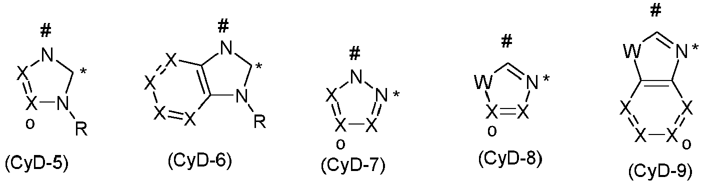 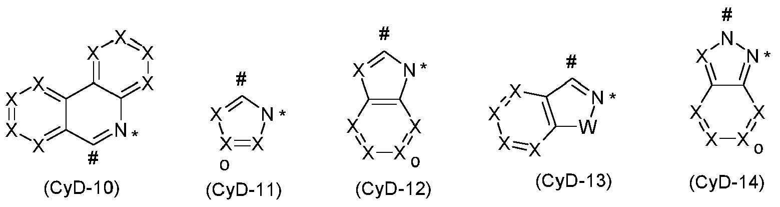 dans lesquelles le groupe est dans chaque cas lié à CyC dans la formule (L-1) ou dans la formule (L-2) ou à CyD dans la formule (L-3) à la position qui est indiquée par # et est coordiné sur le métal à la position qui est indiquée par * ;
en outre, ce qui suit s'applique aux symboles qui sont utilisés dans CyC et dans CyD :
R présente les significations qui ont été données selon la revendication 1, où deux radicaux R peuvent également former un système de cycle aliphatique, hétéroaliphatique, aromatique ou hétéroaromatique l'un avec l'autre ;
X est pour chaque occurrence, de manière identique ou différente, CR ou N, étant entendu qu'un maximum de deux symboles X par cycle représentent N ;
W est pour chaque occurrence, de manière identique ou différente, NR, O ou S ;
la liaison de ces groupes sur le pont de la formule (1) est réalisée via une position qui est indiquée par "o" dans les formules et le symbole correspondant X représente C.

9. Composé selon une ou plusieurs des revendications 1 à 8, **caractérisé en ce que** les ligands partiels bidentés sont sélectionnés parmi les structures qui suivent, 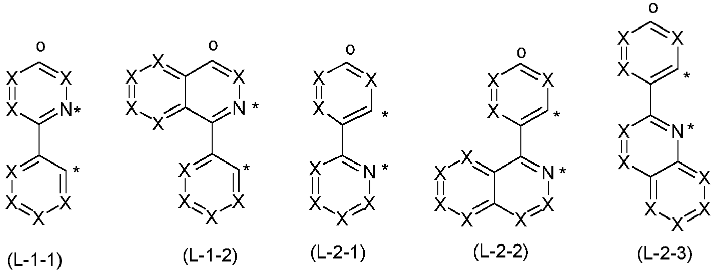 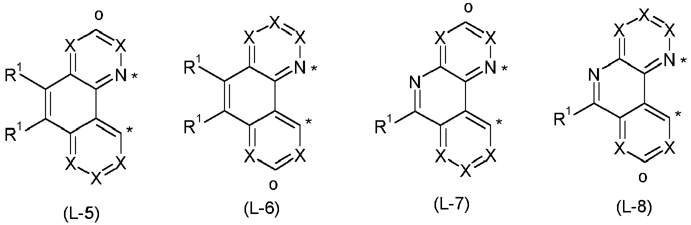 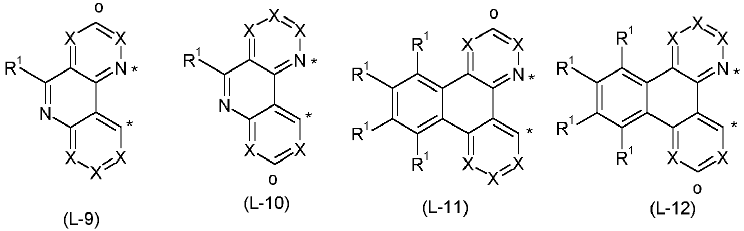 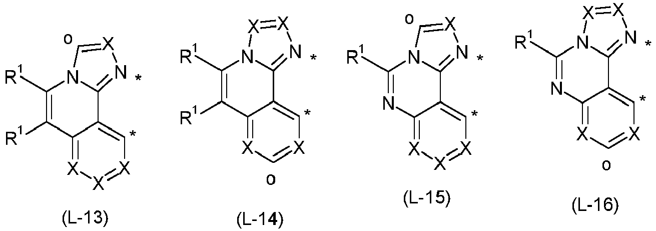 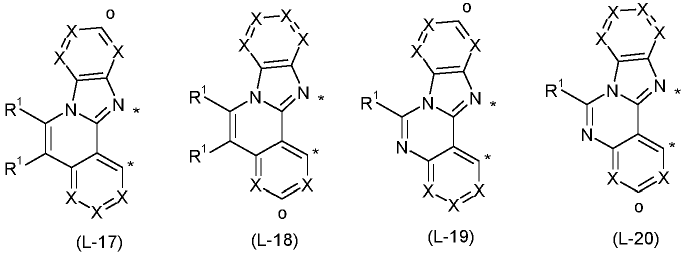 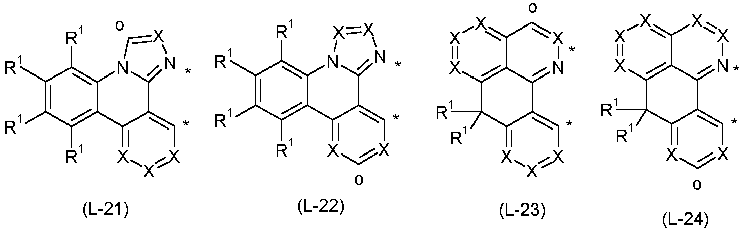 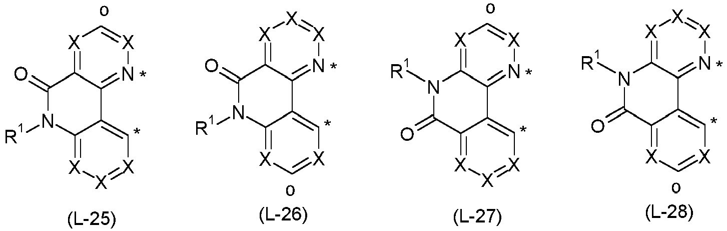 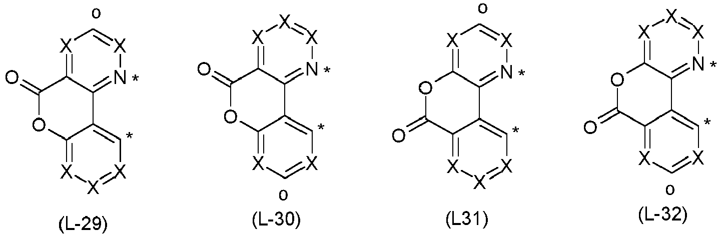 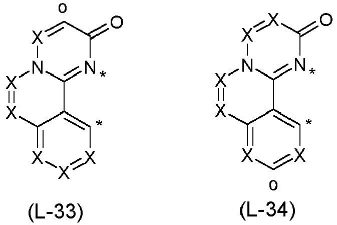 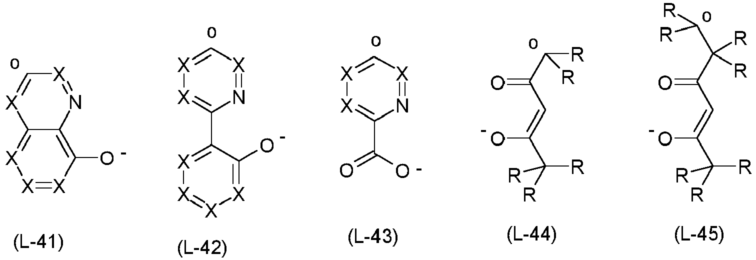 dans lesquelles les symboles qui sont utilisés présentent les significations qui ont été données selon la revendication 1, * représente la position de la coordination sur le métal et "o" représente la position de la liaison sur le pont de la formule (1).

10. Composé selon une ou plusieurs des revendications 1 à 9, **caractérisé en ce que** le composé contient deux substituants R, lesquels sont liés à des atomes de carbone adjacents et lesquels forment un cycle de l'une des formules (43) à (49) l'un avec l'autre, 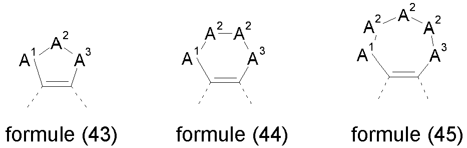 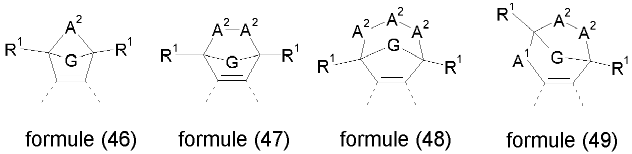 dans lesquelles R¹ et R² présentent les significations qui ont été données selon la revendication 1, les liens en pointillés indiquent la liaison des deux atomes de carbone dans le ligand, et en outre :
A¹, A³ sont, de manière identique ou différente pour chaque occurrence, C(R³)₂, O, S, NR³ ou C(=O) ;
A² est, de manière identique ou différente pour chaque occurrence, C(R¹)₂, O, S, NR³ ou C(=O) ;
G est un groupe alkylène qui comporte 1, 2 ou 3 atome(s) de C, lequel peut être substitué par un radical R² ou plus, ou est -CR²=CR²- ou un groupe arylène ou hétéroarylène ortho-lié qui comporte de 5 à 14 atomes de cycle aromatique, lequel peut être substitué par un radical ou par plusieurs radicaux R² ;
R³ est, de manière identique ou différente pour chaque occurrence, H, D, F, un groupe alkyle ou alcoxy en chaîne droite qui comporte de 1 à 10 atome(s) de C, un groupe alkyle ou alcoxy ramifié ou cyclique qui comporte de 3 à 10 atomes de C, où le groupe alkyle ou alcoxy peut dans chaque cas être substitué par un radical ou par plusieurs radicaux R², où un ou plusieurs groupe(s) CH₂ non adjacents peut/peuvent être remplacé(s) par R²C=CR², C≡C, Si(R²)₂, C=O, NR², O, S ou CONR², ou un système de cycle aromatique ou hétéroaromatique qui comporte de 5 à 24 atomes de cycle aromatique, lequel peut dans chaque cas être substitué par un radical ou par plusieurs radicaux R², ou un groupe aryloxy ou hétéroaryloxy qui comporte de 5 à 24 atomes de cycle aromatique, lequel peut être substitué par un radical ou par plusieurs radicaux R² ; deux radicaux R³ qui sont liés au même atome de carbone peuvent former un système de cycle aliphatique ou aromatique l'un avec l'autre et peuvent ainsi former un système spiro ; en outre, R³ peut former un système de cycle aliphatique avec un radical adjacent R ou R¹ ;
étant entendu que dans ces groupes, deux hétéroatomes ne sont pas liés directement l'un à l'autre et deux groupes C=O ne sont pas liés directement l'un à l'autre.

11. Procédé pour la préparation d'un composé selon une ou plusieurs des revendications 1 à 10 par réaction du ligand libre avec des alcoolates de métal de la formule (50), avec des cétocétonates de métal de la formule (51), avec des halogénures de métal de la formule (52) ou avec des carboxylates de métal de la formule (53) ou avec des composés de métal qui sont porteurs à la fois de radicaux alcoolate et/ou halogénure et/ou hydroxyle et également cétocétonate, 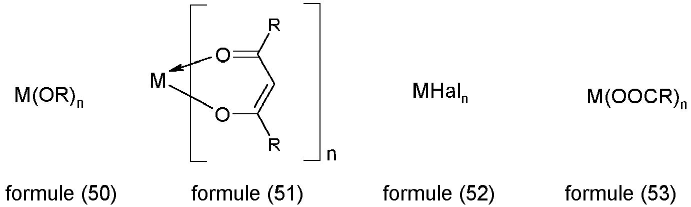 dans lesquelles M représente le métal du complexe de métal qui est synthétisé, n représente la valence du métal M, R présente les significations qui ont été indiquées selon la revendication 1, Hal = F, Cl, Br ou I, et les matériaux de départ du métal peuvent également être sous la forme des hydrates correspondants.

12. Oligomère, polymère ou dendrimère contenant un ou plusieurs composé(s) selon une ou plusieurs des revendications 1 à 10, où un ou plusieurs lien(s) est/sont présent(s) depuis le composé jusqu'au polymère, à l'oligomère ou au dendrimère en lieu et place d'un ou de plusieurs atome(s) d'hydrogène et/ou substituant(s).

13. Formulation comprenant au moins un composé selon une ou plusieurs des revendications 1 à 10, ou au moins un oligomère, un polymère ou un dendrimère selon la revendication 12, et au moins un solvant.

14. Utilisation d'un composé selon une ou plusieurs des revendications 1 à 10 ou d'un oligomère, d'un polymère ou d'un dendrimère selon la revendication 12 dans un dispositif électronique ou en tant qu'agent sensibilisant par rapport à l'oxygène.

15. Dispositif électronique contenant au moins un composé selon une ou plusieurs des revendications 1 à 10 ou au moins un oligomère, un polymère ou un dendrimère selon la revendication 12.

16. Dispositif électronique selon la revendication 15, **caractérisé en ce qu'**il s'agit d'un dispositif électroluminescent organique et le composé selon une ou plusieurs des revendications 1 à 10 est utilisé en tant que composé d'émission dans une ou plusieurs couche(s) d'émission ou en tant que matériau de blocage de trous dans une couche de blocage de trous ou en tant que matériau de transport d'électrons dans une couche de transport d'électrons.
